# EUROPEAN PATENT APPLICATION

(11) **EP 2 065 369 A1**
(43) Date of publication of application: **03.06.2009**
(21) Application number: 07792835.6
(22) Date of filing: 22.08.2007
(51) Int. Cl.: C07D 209/08, A61K 31/445, A61K 31/454, A61K 31/4545, A61K 31/495, A61K 31/496, A61K 31/501, A61K 31/53, A61K 31/5377, A61P 13/02, A61P 13/10, C07D 211/44, C07D 213/74, C07D 235/30, C07D 237/22, C07D 249/14, C07D 251/42, C07D 285/135, C07D 295/16, C07D 401/12, C07D 405/12

(54) **UREA COMPOUND OR SALT THEREOF**

(30) Priority: 23.08.2006 JP 2006226072
(71) Applicant: Astellas Pharma Inc., Tokyo 103-8411 (JP)
(72) Inventor: ISHII, Takahiro, Chuo-ku Tokyo, 103-8411 (JP); SUGANE, Takashi, Chuo-ku Tokyo, 103-8411 (JP); KAKEFUDA, Akio, Chuo-ku Tokyo, 103-8411 (JP); TAKAHASHI, Tatsuhisa, Chuo-ku Tokyo, 103-8411 (JP); KANAYAMA, Takatoshi, Chuo-ku Tokyo, 103-8411 (JP); SATO, Kentaro, Chuo-ku Tokyo, 103-8411 (JP); KURIWAKI, Ikumi, Chuo-ku Tokyo, 103-8411 (JP); KITADA, Chika, Chuo-ku Tokyo, 103-8411 (JP); SUZUKI, Jotaro, Chuo-ku Tokyo, 103-8411 (JP)
(74) Representative: HOFFMANN EITLE
(86) International application number: PCT/JP2007/066236
(87) International publication number: WO 2008/023720

(57) **Abstract**

[Object] To provide a compound which can be used for the treatment of a disease associated with fatty acid amide hydrolase (FAAH), particularly urinary frequency, urinary incontinence and/or overactive bladder.

[Means for solution] It is confirmed that a urea compound chemical-structurally **characterized by** having a piperidine or piperazine ring or a salt thereof has an excellent FAAH-inhibitory activity, and thus the present invention is completed. The urea compound or its pharmaceutically acceptable salt of the present invention can increase the effective bladder capacity and ameliorate the state of urinary frequency, and is therefore useful as an agent for treating urinary frequency, urinary incontinence and/or overactive bladder.

## Description

### Technical Field

The present invention relates to a urea compound or a pharmaceutically acceptable salt thereof which is useful as a medicine, particularly as an agent for treating urinary frequency, urinary incontinence and/or overactive bladder.

### Background Art

Overactive bladder refers to a clinical condition showing an urgency of urination regardless of incontinence, which is usually associated with a urinary frequency and/or nocturia (Non-Patent Document 1). For a treatment thereof, currently an anticholinergic agent is mainly used, and certain treatment results are given. However, it has been reported that the anticholinergic agent is difficult to be used for patients with prostatic hypertrophy or elderly patients because it is known to cause side-effects such as dry mouth, constipation and blurred vision, as well as a risk of urinary retention. In addition, there are patients showing no effectiveness in the treatment with the anticholinergic agent. From the above facts, there is a great expectation for a drug with a new mechanism of action for overactive bladder.

Fatty acid amide hydrolase (FAAH) is known to hydrolyze and inactivate endocannabinoid (Non-Patent Documents 2 to 5). Endocannabinoid is a generic term for a biological substance that acts on a cannabinoid receptor thereby exhibiting the physiological activity *in vivo*. Typical endocannabinoids are anandamide, palmitoyl ethanolamide, oleamide, and 2-arachidonoyl glycerol; and they are known to be hydrolyzed and lose their activity by FAAH. Furthermore, Δ9-tetrahydrocannabinol that is considered as an active ingredient of Cannabis (marijuana) is known to activate a cannabinoid receptor (Non-Patent Document 6).
In mammals, two types of cannabinoid receptor CB1 and CB2 have heretofore been known. CB1 is expressed in central and peripheral nervous systems, and when activated, it exhibits mental action and analgesic action. CB2 is expressed in immune systems, and when activated, it exhibits antiinflammatory action and analgesic (and inflammatory) action.
Meanwhile, in a cystitis rat model, a cannabinoid receptor agonist increases the bladder capacity and the urination threshold (Non-Patent Document 7 and Non-Patent Document 8); and the side effects, such as hallucination, delusion, tachycardia and orthostatic hypotension, which are observed when a cannabinoid receptor agonist is administered to animals, are not observed if an FAAH inhibitor is administered (Non-Patent Document 9). From these, the FAAH inhibitor is expected as a novel therapeutic agent for urinary frequency, urinary incontinence, and/or overactive bladder, which has less risks of causing the side effects of cannabinoid and compatibility problems.

The following compounds have been reported as piperazinyl urea derivatives or piperidyl urea derivatives which have an FAAH inhibitory activity.
For example, Patent Document 1 discloses the following compound: 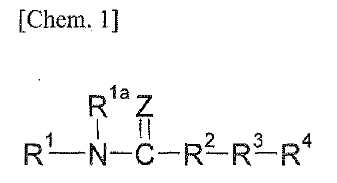 (wherein R¹ represents aryl which may be substituted, or a heterocyclic group which may be substituted, R^{1a} represents H, a hydrocarbon group, or the like, Z represents O or S, R² represents piperidine-1,4-diyl which may be substituted or piperazine-1,4-diyl which may be substituted, R³ represents -CO-, -CO-O-, -CONH-, or a heterocyclic group, and R⁴ represents a hydrocarbon group which may be substituted or a heterocyclic group which may be substituted, provided that if R³ is -CO-, -CO-O-or -CONH-, R⁴ represents benzoisoxazolyl. For details, refer to the publication.), and
Patent Document 2 discloses the following compound: 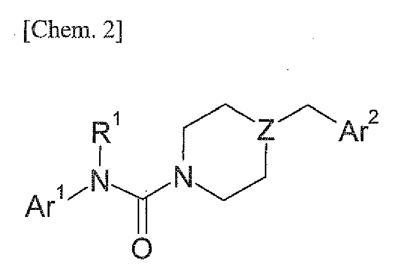 (wherein Ar¹ represents 2-thiazolyl, 2-pyridyl, 4-pyridyl, 2-pyrimidinyl, 4-pyrimidinyl, 5-pyrimidinyl, or phenyl, each of which may be substituted with one or two R^{a}, R¹ represents H or C₁₋₄ alkyl, Z represents N or CH, and Ar² represents a phenyl or heterocyclic group, each of which may be substituted with a specific group or condensed. For details, refer to the publication.). However, none of the publications has a specific disclosure of the compound according to the present invention.
Furthermore, a carbamate derivative having an FAAH inhibitory activity has been reported, for example, in Patent Documents 3 and 4, as well as in Patent Document 5 that has been published after the priority date of the present invention.

[Patent Document 1] Pamphlet of International Publication No. WO2006/054652
[Patent Document 2] Pamphlet of International Publication No. WO2006/074025
[Patent Document 3] Pamphlet of International Publication No. WO2003/065989
[Patent Document 4] Pamphlet of International Publication No. WO2004/033422
[Patent Document 5] Pamphlet of International Publication No. WO2006/088075
Non-Patent Document 1: "Neurourology and Urodynamics", (UK), 2002, Vol. 21, p. 167-78
Non-Patent Document 2: "Prostaglandins Leukotrienes and Essential Fatty Acids", (UK), 2002, Vol. 66, p. 143-160
Non-Patent Document 3: "British Journal of Pharmacology", (UK), 2004, Vol. 141, p. 253-262
Non-Patent Document 4: "Nature", (UK), 1996, Vol. 384, p. 83-87
Non-Patent Document 5: "Biochemical Pharmacology", (US), 2001, Vol. 62, p. 517-526
Non-Patent Document 6: "Current Medicinal Chemistry", (US), 1999, Vol. 6, p. 635-664
Non-Patent Document 7: "The Journal of Neuroscience", 2002, Vol. 22, p. 7147-7153
Non-Patent Document 8: "Pain", 1998, Vol. 76, p. 189-199
Non-Patent Document 9: "Nature Medicine", (UK), 2003, Vol. 9, p. 76-81

### Disclosure of the Invention

### Problem that the Invention is to Solve

It is an object of the present invention to provide a compound that is useful as a medicine having an FAAH inhibitory action, particularly as an agent for treating urinary frequency, urinary incontinence and/or overactive bladder.

### Means for Solving the Problem

As described above, an FAAH inhibitor can be expected as a very safe therapeutic agent with few side effects such as dry mouth and urinary retention found in an anticholinergic agent, which is for urinary frequency, urinary incontinence and/or overactive bladder. The present inventors have devotedly investigated a compound having an FAAH inhibitory activity in order to provide a compound useful for treating urinary frequency, urinary incontinence, and/or overactive bladder. As a result, they have found that a urea compound of the present invention has an excellent FAAH inhibitory action, and a compound having an FAAH inhibitory activity increases an affective bladder capacity in a rat with urinary frequency caused by cyclophosphamide (CPA), and thus they have completed the present invention.

Specifically, the present invention relates to the following.
[1] A urea compound represented by the formula (I) or a pharmaceutically acceptable salt thereof. 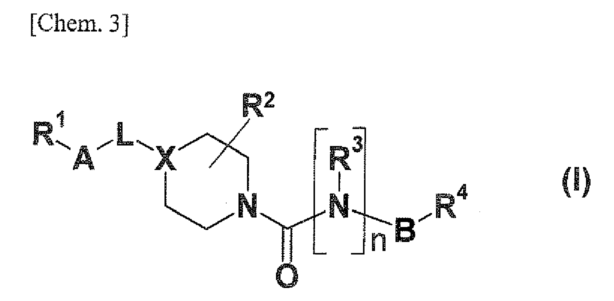 [in the formula, the symbols have the following meanings:
   R¹: H, aryl, aryl-O-, aryl-lower alkylene-, aryl-lower alkenylene-, aryl-lower alkylene-O-, aryl-lower alkylene-NR⁰-, aryl-NR⁰-lower alkylene-, aryl-C(O)-NR⁰-, aryl-SO₂-NR⁰-, heteroaryl, beteroaryl-lower alkylene-O-, cycloalkyl-lower alkylene-, cycloalkyl-lower alkylene-O-, cycloalkyl-lower alkylene-NR⁰-, a nitrogen-containing heterocyclic group having a bonding arm on N as a ring-constituting element, or oxygen-containing saturated heterocyclic group-lower alkylene-O-,
   wherein each aryl and each heteroaryl in R¹ may be substituted with group(s) selected from the following Group G¹,
   Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
   R⁰: the same or different, H or lower alkyl,
   A: a benzene ring or a hetero ring, each of which may be substituted with group(s) selected from the following Group G²,
   Group G²: halogen, lower alkyl, -O-C₁₋₈ alkyl, -OH, -NO₂, -C(O)-OR⁰, and -C(O)-N(R⁰)₂,
   X: N or CH,
   L: lower alkylene, lower alkenylene, -O-, -O-lower alkylene-, -S(O)ₘ-, -lower alkylene-S(O)ₘ-, -C(O)-, -lower alkylene-C(O)-, -lower alkenylene-C(O)-, -NR⁰-, -C(O)-NR⁰-, -NR⁰-C(O)-, -O-lower alkylene-C(O)-, -lower alkylene-O-C(O)-, or -lower alkylene-NR⁰-C(O)-,
   (provided that when X is N, L is not -O-, -S-, -NR⁰-, and -C(O)-NR⁰-),
   m: the same or different, 0, 1, or 2,
   R² and R³: the same or different, H or lower alkyl,
   n: 0 or 1,
   B: (i) in a case of n=1, a single bond, or a benzene ring or aromatic hetero ring, each of which may be substituted with group(s) selected from the following Group G³, and (ii) in a case of n=0, a single bond,
   Group G³: halogen, lower alkyl, -O-lower alkyl, halogeno-lower alkyl, -O-halogeno-lower alkyl, -OH, -O-benzyl, -O-C(O)-lower alkyl, -lower alkylene-OR⁰, -lower alkylene-O-C(O)-lower alkyl, -CN, -NO₂ -C(O)-OR⁰, -C(O)-N(R⁰)₂, -N(R⁰)₂, -NR⁰-C(O)-lower alkyl, -NR⁰-SO₂-lower alkyl, and -S(O)ₘ-lower alkyl,
   R⁴: (i) in a case of n=1 and B=a single bond,
   -C(O)-Z or -S(O)ₘ-Z,
   [wherein
   Z: lower alkyl, cycloalkyl, aryl, heteroaryl, -lower alkylene-O-lower alkyl, or -lower alkylene-O-benzyl],
   (ii) in a case of n=1 and B= other than a single bond,
   H, or phenyl, a nitrogen-containing heterocyclic group, a -C(O)-nitrogen-containing heterocyclic group, each of which may be substituted with group(s) selected from the following Group G⁴, or -W-lower alkylene-Y,
   [wherein
   W: -lower alkylene-C(O)-NR⁰-, -C(O)-NR⁰-, -O-, or a single bond, and
   Y: -OH, -N(R⁰)₂, -C(O)-OR⁰, or -C(O)-N(R⁰)₂], and
   (iii) in a case of n=0,
   a nitrogen-containing heterocyclic group having a bonding arm on N as a ring-constituting element, which may be substituted with group(s) selected from the following Group G⁴, and
   Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂,
   provided that when L is -C(O)- or-lower alkylene-C(O)-, X is N, n=1, and R¹, R², R³, and R⁴ are all H, for B, unsubstituted benzoisoxazole is excluded. The same shall apply hereinafter.]
[2] The urea compound as described in [1], which is represented by the formula (I-A) or a pharmaceutically acceptable salt thereof. 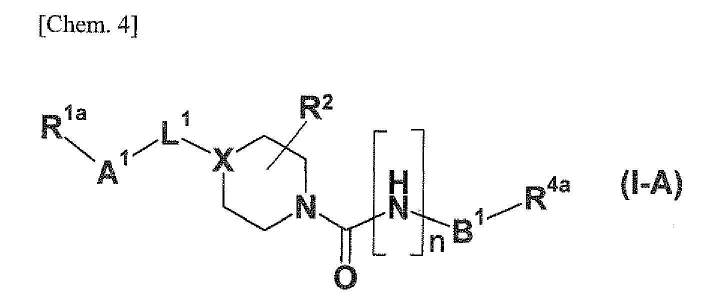 [in the formula, the symbols have the following meanings:
   R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
   wherein each aryl and each heteroaryl in R^{1a} may be substituted with group(s) selected from the following Group G¹,
   Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
   R⁰: the same or different, H or lower alkyl,
   A¹: a benzene ring or aromatic hetero ring, each of which may be substituted with group(s) selected from the following Group G²,
   Group G²: halogen, lower alkyl, -O-C₁₋₈ alkyl, -OH, -NO₂, -C(O)-OR⁰, and -C(O)-N(R⁰)₂,
   x: N or CH,
   L¹: methylene, -O-, -S(O)ₘ-, -C(O)-, or -NR⁰-,
   (provided that when X is N, L¹ is methylene, -S(O)₂-, or -C(O)-), m: the same or different, 0, 1, or 2,
   R²: H or lower alkyl,
   n: 0 or 1,
   B¹: (i) in a case of n=1, a single bond, or a 5- or 6-membered aromatic nitrogen-containing hetero ring which may be substituted with group(s) selected from the following Group G³, (ii) in a case of n=0, a single bond,
   Group G³: halogen, lower alkyl, -O-lower alkyl, halogeno-lower alkyl, -O-halogeno-lower alkyl, -OH, -O-benzyl, -O-C(O)-lower alkyl, -lower alkylene-OR⁰, -lower alkylene-O-C(O)-lower alkyl, -CN, -NO₂, -C(O)-OR⁰, -C(O)-N(R⁰)₂, -N(R⁰)₂, -NR⁰-C(O)-lower alkyl, -NR⁰-SO₂-lower alkyl, and -S(O)ₘ-lower alkyl,
   R^{4a}: (i) in a case of n=1 and B¹=a single bond,
   -C(O)-Z or -S(O)ₘ-Z,
   [wherein
   Z: lower alkyl, cycloalkyl, aryl, heteroaryl, -lower alkylene-O-lower alkyl, or -lower alkylene-O-benzyl],
   (ii) in a case of n=1 and B¹= a 5- or 6-membered aromatic nitrogen-containing hetero ring which may be substituted with group(s) selected from the above-mentioned Group G³,
   H, or phenyl, a nitrogen-containing heterocyclic group, -C(O)-nitrogen-containing heterocyclic group, each of which may be substituted with group(s) selected from the following Group G⁴, or -W-lower alkylene-Y,
   [wherein
   W: -lower alkylene-C(O)-NR⁰-, -C(O)-NR⁰-, -O-, or a single bond, and
   Y: -OH, -N(R⁰)₂, -C(O)-OR⁰, or -C(O)-N(R⁰)₂], and
   (iii) in a case of n=0,
   a 5-membered aromatic nitrogen-containing heterocyclic group having a bonding arm on N as a ring-constituting element, which may be substituted with group(s) selected from the following Group G⁴, and
   Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂. The same shall apply hereinafter.]
[3] The urea compound as described in [2], which is represented by the formula (I-B) or a pharmaceutically acceptable salt thereof. 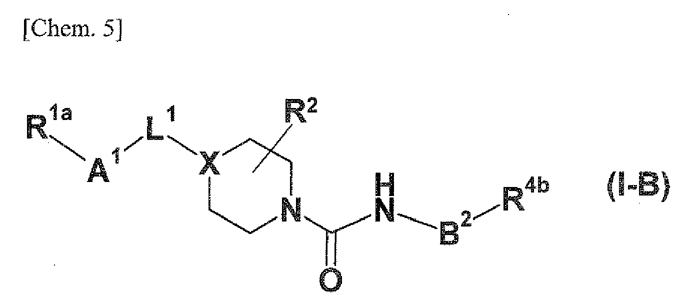 [in the formula, the symbols have the following meanings:
   R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
   wherein each aryl and each heteroaryl in R^{1a} may be substituted with group(s) selected from the following Group G¹,
   Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
   R⁰: the same or different, H or lower alkyl,
   A¹: a benzene ring or aromatic hetero ring, each of which may be substituted with group(s) selected from the following Group G²,
   Group G²: halogen, lower alkyl, -O-C₁₋₈ alkyl, -OH, -NO₂, -C(O)-OR⁰, and -C(O)-N(R⁰)₂,
   X: N or CH,
   L¹: methylene, -O-, -S(O)ₘ-, -C(O)-, or -NR⁰-,
   (provided that when X is N, L¹ is methylene, -S(O)₂- or -C(O)-),
   m: the same or different, 0, 1, or 2,
   R²: H or lower alkyl,
   B²: a 5- or 6-membered aromatic nitrogen-containing hetero ring, which may be substituted with group(s) selected from the following Group G³,
   Group G³: halogen, lower alkyl, -O-lower alkyl, halogeno-lower alkyl, -O-halogeno-lower alkyl, -OH, -O-benzyl, -O-C(O)-lower alkyl, -lower alkylene-OR⁰, -lower alkylene-O-C(O)-lower alkyl, -CN, -NO₂, -C(O)-OR⁰, -C(O)-N(R⁰)₂, -N(R⁰)₂, -NR⁰-C(O)-lower alkyl, -NR⁰-SO₂-lower alkyl, and -S(O)ₘ-lower alkyl,
   R^{4b}: H, or phenyl, a nitrogen-containing heterocyclic group, -C(O)-nitrogen-containing heterocyclic group, each of which may be substituted with group(s) selected from the following Group G⁴, or -W-lower alkylene-Y,
   [wherein
   W: -lower alkylene-C(O)-NR⁰-, -C(O)-NR⁰-, -O-, or a single bond, and
   Y: -OH, -N(R⁰)₂, -C(O)-OR⁰, or -C(O)-N(R⁰)₂], and
   Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂. The same shall apply hereinafter.]
[4] The urea compound as described in [3], wherein A¹ is a benzene ring or a 5-or 6-membered aromatic hetero ring or a pharmaceutically acceptable salt thereof.
[5] The urea compound as described in [4], wherein R^{1a} is aryl-lower alkylene-O-, aryl-lower alkylene-NR⁰-, heteroaryl-lower alkylene-O-, cycloalkyl-lower alkylene-O-, cycloalkyl-lower alkylene-NR⁰-, or oxygen-containing saturated heterocyclic group-lower alkylene-O- or a pharmaceutically acceptable salt thereof.
[6] The urea compound as described in [5], wherein X=N, and L¹ is -C(O)- or methylene or a pharmaceutically acceptable salt thereof.
[7] The urea compound as described in [5], wherein X=CH, and L¹ is -O- or a pharmaceutically acceptable salt thereof.
[8] The urea compound as described in [2], which is represented by the formula (I-C) or a pharmaceutically acceptable salt thereof. 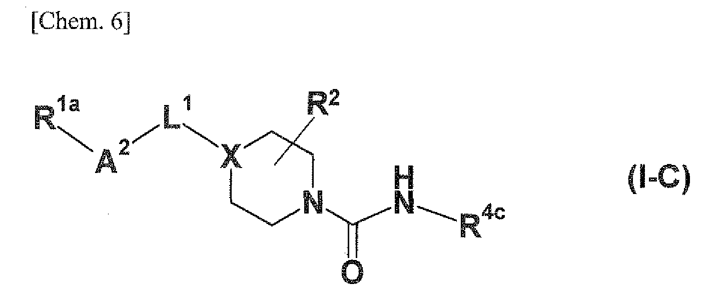 [in the formula, the symbols have the following meanings:
   R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
   wherein each aryl and each heteroaryl in R^{1a} may be substituted with group(s) selected from the following Group G¹,
   Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
   R⁰: the same or different, H or lower alkyl,
   A²: a benzene ring or 5- or 6-membered aromatic hetero ring, each of which may be substituted with group(s) selected from the following Group G²,
   Group G²: halogen, lower alkyl, -O-C₁₋₈ alkyl, -OH, -NO₂, -C(O)-OR⁰, and -C(O)-N(R⁰)₂,
   X: N or CH,
   L¹: methylene, -O-, -S(O)ₘ-, -C(O)-, or -NR⁰-,
   (provided that when X is N, L¹ is methylene, -S(O)₂-, or -C(O)-),
   m: the same or different, 0, 1, or 2,
   R²: H or lower alkyl, and
   R^{4c}: -C(O)-Z or -S(O)ₘ-Z,
   [wherein
   Z: lower alkyl, cycloalkyl, aryl, heteroaryl, -lower alkylene-O-lower alkyl, or -lower alkylene-O-benzyl. The same shall apply hereinafter.]
[9] The urea compound as described in [2], which is represented by the formula (I-D) or a pharmaceutically acceptable salt thereof. 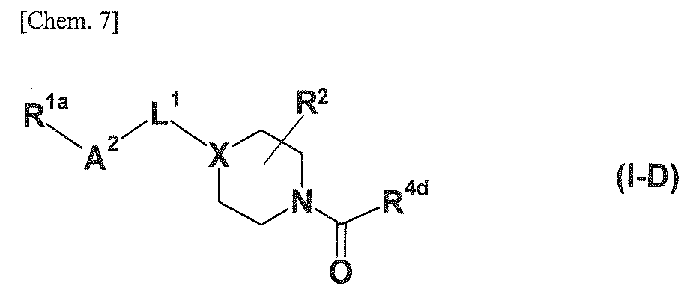 [in the formula, the symbols have the following meanings:
   R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
   wherein each aryl and each heteroaryl in R^{1a} may be substituted with group(s) selected from the following Group G¹,
   Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
   R⁰: the same or different, H or lower alkyl,
   A²: a benzene ring or 5- or 6-membered aromatic hetero ring, each of which may be substituted with group(s) selected from the following Group G²,
   Group G²: halogen, lower alkyl, -O-C₁₋₈ alkyl, -OH, -NO₂, -C(O)-OR⁰, and -C(O)-N(R⁰)₂,
   X: N or CH,
   L¹: methylene, -O-, -S(O)ₘ-, -C(O)-, or -NR⁰-,
   (provided that when X is N, L¹ is methylene, -S(O)₂-, or -C(O)-),
   m: 0, 1, or 2,
   R²: H or lower alkyl,
   R^{4d}: a 5-membered aromatic nitrogen-containing heterocyclic group having a bonding arm on N as a ring-constituting element, which may be substituted with group(s) selected from the following Group G⁴, and
   Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂. The same shall apply hereinafter.]
[10] The compound as described in [1], which is selected from the group consisting of
   4-{4-[(3-fluorobenzyl)oxy]phenoxy}-N-pyridin-3-ylpiperidine-1-carboxamide,
   4-{4-[(3-fluorobenzyl)oxy]benzyl}-N-pyridin-3-ylpiperazine-1-carboxamide,
   4-{4-[(2-cyclohexylethyl)(methyl)amino]benzoyl}-N-pyridin-3-ylpiperazine-1-carboxamide,
   4-[3-(2-cyclohexylethoxy)benzoyl]-N-pyrimidin-2-ylpiperazine-1-carboxamide,
   4-[3-(2-cyclohexylethoxy)benzoyl]-N-pyridazin-3-ylpiperazine-1-carboxamide,
   4-[3-(2-cyclohexylethoxy)benzoyl]-N-pyrazin-2-ylpiperazine-1-carboxamide,
   4-[2-(2-cyclohexylethoxy)isonicotinoyl]-N-pyrazin-2-ylpiperazine-1-carboxamide,
   4-[3-(2-cyclohexylethoxy)benzyl]-N-pyridin-3-ylpiperazine-1-carboxamide,
   4-[3-(2-cyclohexylethoxy)benzoyl]-N-pyrimidin-5-ylpiperazine-1-carboxamide,
   N-(6-aminopyridin-3-yl)-4-[3-(2-cyclohexylethoxy)benzoyl]piperazine-1-carboxamide,
   4-[3-(2-cyclohexylethoxy)benzyl]-N-pyridazin-3-ylpiperazine-1-carboxamide,
   4-{3-[(2,3-difluorobenzyl)oxy]benzyl}-N-pyridin-3-ylpiperazine-1-carboxamide,
   4-{[2-(2-cyclohexylethoxy)pyridin-4-yl]methyl}-N-pyridin-3-ylpiperazine-1-carboxamide,
   4-{3-[2-(3-fluorophenyl)ethoxy]benzoyl}-N-pyrazin-2-ylpiperazine-1-carboxamide,
   4-{[5-(2-cyclohexylethoxy)pyridin-2-yl]carbonyl}-N-pyrazin-2-ylpiperazine-1-carboxamide,
   4-[3-(2-cyclohexylethoxy)-5-fluorobenzoyl]-N-pyrazin-2-ylpiperazine-1-carboxamide,
   4-[3-(2-cyclohexylethoxy)-4-fluorobenzoyl]-N-pyrazin-2-ylpiperazine-1-carboxamide,
   4-{2-[2-(2-fluorophenyl)ethoxyl]isonicotinoyl}-N-pyrazin-2-ylpiperazine-1-carboxamide,
   N-(3-chloropyrazin-2-yl)-4-[3-(2-cyclohexylethoxy)benzoyl]piperazine-1-carboxamide,
   N-(3-chloropyrazin-2-yl)-4-[3-(2-cyclohexylethoxy)phenoxy]piperidine-1-carboxamide,
   4-[5-(2-cyclohexylethoxy)-2-fluorobenzoyl]-N-pyrazin-2-ylpiperazine-1-carboxamide,
   N-(3-chloropyrazin-2-yl)-4-[2-(2-cyclohexylethoxy)isonicotinoyl]piperazine-1-carboxamide,
   N-(3-chloropyrazin-2-yl)-4-{[2-(2-cyclohexylethoxy)pyridin-4-yl]methyl}piperazine-1-carboxamide,
   4-{[2-(2-cyclohexylethoxy)pyridin-4-yl]methyl}-N-pyrazin-2-ylpiperazine-1-carboxamide,
   N-(3-chloropyrazin-2-yl)-4-{2-[2-(3-fluorophenyl)ethoxy]isonicotinoyl}piperazine-1-carboxamide,
   4-{2-[2-(2-chlorophenyl)ethoxy]isonicotinoyl}-N-(3-chloropyrazin-2-yl)piperazine-1-carboxamide,
   N-(3-chloropyrazin-2-yl)-4-(2-{2-[2-(trifluoromethyl)phenyl]ethoxy}isonicotinoyl)piperazine-1-carboxamide,
   N-(3-chloropyrazin-2-yl)-4-{2-[2-(2-fluorophenyl)ethoxy]isonicotinoyl}piperazine-1-carboxamide,
   4-{4-[(3-fluorobenzyl)oxy]phenoxy}-1-(1H-imidazol-1-ylcarbonyl)piperidine,
   1-{4-[(3-fluorobenzyl)oxy]benzoyl}-4-(1H-imidazol-1-ylcarbonyl)piperazine,
   4-({4-[3-(2-cyclohexylethoxy)benzoyl]piperazin-l-yl}carbonyl)-4H-1,2,4-triazol-3-amine,
   1-({4-[3-(2-cyclohexylethoxy)benzoyl]piperazin-1-yl}carbonyl)-1H-pyrazol-5-amine, and
   4-[3-(2-cyclohexylethoxy)benyl]-N-(methoxyacetyl)piperazine-1-carboxamide, or a pharmaceutically acceptable salt thereof.
[11] A pharmaceutical composition comprising the compound as described in [1] or a pharmaceutically acceptable salt thereof as an active ingredient.
[12] The pharmaceutical composition as described in [11], which is an FAH inhibitor.
[13] The pharmaceutical composition as described in [11], which is an agent for treating urinary frequency, urinary incontinence, and/or overactive bladder.
[14] A use of the compound as described in [1] or a pharmaceutically acceptable salt thereof for the preparation of an agent for treating urinary frequency, urinary incontinence, and/or overactive bladder.
[15] A method for treating urinary frequency, urinary incontinence, and/or overactive bladder, comprising administering an effective amount of the compound as described in [1] or a pharmaceutically acceptable salt thereof to a patient.

### Effects of the Invention

Since it is confirmed that the compound of the present invention has an excellent FAAH inhibitory action as seen from the following Test Examples 1 to 3, and increases an effective bladder capacity thereby relieving the condition of urinary frequency as seen from the following Test Example 4, the compound is useful as an agent for treating urinary frequency, urinary incontinence, and/or overactive bladder.

### BEST MODE FOR CARRYING OUT THE INVENTION

Hereinafter, the present invention will be described in more detail.
As used in the present specification, the term "lower" means a linear or branched hydrocarbon chain having 1 to 6 carbon atoms (hereinafter simply referred to as C₁₋₆), unless otherwise specifically mentioned.

The "lower alkyl" means C₁₋₆ alkyl. Specifically, examples thereof include methyl, ethyl, n-propyl, isopropyl, n-butyl, isobutyl, sec-butyl, tert-butyl, n-pentyl, n-hexyl and the like. Preferred is alkyl having 1 to 3 carbon atoms, and more preferred is methyl, ethyl or isopropyl.
The "lower alkylene" means a divalent group in which one hydrogen at any position of the "lower alkyl" is removed. Specifically, examples thereof include methylene, ethylene, methylmethylene, dimethylmethylene, trimethylene and the like. Preferred is methylene, ethylene or trimethylene, and more preferred is methylene or ethylene.
The "lower alkenylene" means a divalent group having at least one double bond at any position of the "lower alkylene". Specifically, examples thereof include vinylene, propenylene, 1-butenylene 2-butenylene and the like. Preferred is vinylene.
The "cycloalkyl" means a C₃₋₁₀ saturated hydrocarbon ring, or it may form a bridged ring. Specifically, examples thereof include cyclopropyl, cyclobutyl, cyclopentyl, cyclohexyl, cycloheptyl, cyclooctyl, adamantyl, norbornyl and the like. Preferred is a monocyclic C₃₋₈ cycloalkyl having no bridge, and more preferred is cyclohexyl.

The "halogen" means F, Cl, Br, and I. Preferred is F, Cl, or Br.
The "halogeno-lower alkyl" means the "lower alkyl" as defined above in which any one or more hydrogen atoms are substituted with the same or different "halogen" as defined above. Specifically, examples thereof include fluoromethyl, difluoromethyl, trifluoromethyl, 2,2,2-trifluoroethyl, pentafluoroethyl and the like. Preferred is trifluoromethyl.
The "aryl" is a C₆₋₁₄ mono-, bi-, or tricyclic aromatic hydrocarbon ring group, and examples thereof include a ring group that is condensed with a C₅₋₇ cyclolalkene ring. However, if the C₅₋₇ cycloalkene ring is condensed, a bonding arm is positioned on the aromatic ring. Specifically, examples thereof include phenyl, naphthyl, indanyl, tetrahydronaphthyl, fluorenyl and the like. Preferred is phenyl.

The "hetero ring" is a 4- to 12-membered, mono- or bicyclic saturated or unsaturated ring containing 1 to 4 hetero atoms selected from O, S and N. Examples of the unsaturated ring include aromatic hetero rings. Furthermore, the ring atom, S or N, may be oxidized to form an oxide or a dioxide. Specifically, examples of the monocyclic ring include azetidine, pyrrolidine, piperidine, piperazine, morpholine, thiomorpholine, azepane, diazepane, oxetane, tetrahydrofuran, tetrahydropyran, 1,3-dioxole, 2,3-dihydro-1,4-dioxine, pyrazolidine, furan, thiophene, pyrrole, imidazole, pyrazole, thiazole, oxazole, isothiazole, isoxazole, triazole, tetrazole, thiadiazole, oxadiazole, pyridine, pyrazine, pyrimidine, pyridazine, triazine, 2,3-dihydro-1,3-oxazole and the like, and examples of the bicyclic ring include 1,3-benzodioxole, 2,3-dihydro-1,4-benzodioxine, indole, benzofuran, benzothiophene, benzoxazole, benzisoxazole, benzothiazole, benzisothiazole, benzimidazole, indazole, benzotriazole, quinoline, isoquinoline, 1,2,3,4-tetrahydroquinoline, 1,2,3,4-tetrahydroisoquinoline, quinoxaline, quinazoline, phthalazine and the like. Preferred is a monocyclic hetero ring. The "heterocyclic group" means a ring group consisting of the above-mentioned hetero ring.

The "aromatic hetero ring" means, among the above-mentioned "hetero rings", a ring selected from i) a monocyclic, 5- or 6-membered aromatic hetero ring containing 1 to 4 hetero atoms selected from O, S and N, ii) a bicyclic hetero ring in which the aromatic hetero ring in the above-described i) is condensed (provided that the two aromatic hetero rings to be condensed may be the same as or different from each other), and iii) a bicyclic hetero ring in which a benzene ring and the aromatic hetero ring in the above-described i) or 5- to 7-membered cycloalkane is condensed. Specifically, examples thereof include i) pyridine, pyrazine, pyrimidine, pyridazine, triazine, pyrrole, furan, thiophene, imidazole, pyrazole, triazole, tetrazole, oxazole, isoxazole, oxadiazole, thiazole, isothiazole, and thiadiazole, ii) naphthylidine, imidazopyridine, pyrrolopyrimidine, thienopyridine, and thienopyrroline, and iii) benzimidazole, benzofuran, benzothiophene, benzothiadiazole, benzothiazole, benzisothiazole, benzoxazole, benzisoxazole, quinoline, isoquinoline, 5,6,7,8-tetrahydroquinoline, 5,6,7,8-tetrahydroisoquinoline, quinazoline, quinoxaline, phthalazine, indole, isoindole, tetrahydrobenzimidazole, chromane, indazole and the like. Preferred is the above-described the i) or iii), and more preferred is the above-described i) monocyclic, 5- or 6-membered aromatic hetero ring. The "heteroaryl" means a ring group consisting of the above-mentioned aromatic hetero ring.

The "nitrogen-containing hetero rings" means a hetero ring containing at least one or more N(s) as a ring-constituting element in the above-mentioned "hetero ring". Specifically, examples thereof include pyrrolidine, piperidine, piperazine, morpholine, thiomorpholine, azepane, diazepane, 1,2,3,4-tetrahydroquinoline, 1,2,3,4-tetrahydroisoquinoline, pyridine, pyrazine, pyrimidine, pyridazine, triazine, pyrrole, imidazole, pyrazole, triazole, tetrazole, oxazole, isoxazole, oxadiazole, thiazole, isothiazole, thiadiazole, benzimidazole, benzothiadiazole, benzothiazole, benzisothiazole, benzoxazole, benzisoxazole, quinoline, isoquinoline, 5,6,7,8-tetrahydroquinoline, 5,6,7,8-tetrahydroisoquinoline, quinazoline, quinoxaline, phthalazine, indole, isoindole, tetrahydrobenzimidazole, indazole and the like. The "nitrogen-containing heterocyclic group" means a ring group consisting of the above-mentioned nitrogen-containing hetero ring.

The "5- or 6-membered aromatic nitrogen-containing hetero ring" means an aromatic, monocyclic 5- or 6-membered ring among the above-mentioned "nitrogen-containing hetero rings". Specifically, examples thereof include pyridine, pyrazine, pyrimidine, pyridazine, triazine, pyrrole, imidazole, pyrazole, triazole, tetrazole, oxazole, isoxazole, oxadiazole, thiazole, isothiazole, thiadiazole and the like.
The "nitrogen-containing heterocyclic group having a bonding arm on N as a ring-constituting element" means a ring group necessarily having a bonding arm on N as a ring-constituting element among the above-mentioned "nitrogen-containing heterocyclic groups", such as 1H-imidazol-1-yl, 4H-1,2,4-triazol-4-yl, 1H-pyrazol-1-yl, 1- pyrrolidinyl, 1-piperidyl, 1-piperazinyl, 4-morpholinyl and the like.
The "5-membered aromatic nitrogen-containing heterocyclic group having a bonding arm on N as a ring-constituting element" means a ring group consisting of aromatic, monocyclic 5-membered rings among the above-mentioned "nitrogen-containing heterocyclic group having a bonding arm on N as a ring-constituting element". Specifically, examples thereof include 1H-imidazol-1-yl, 4H-1,2,4-triazol-4-yl, 1H-pyrazol-1-yl and the like.

The "oxygen-containing saturated hetero ring" means a saturated hetero ring necessarily having at least one or more O(s) as a ring-constituting element, and, in addition, may have a hetero atom selected from O, S, and N among the above-mentioned "hetero rings". Preferred is a saturated hetero ring having C and O as a ring-constituting element, such as oxetane, tetrahydrofuran, tetrahydropyran, oxepane, 1,4-dioxane and the like. The "oxygen-containing saturated heterocyclic group" means a ring group consisting of the above-mentioned oxygen-containing hetero ring.

The expression "may be substituted" means that "is not substituted" or "is substituted with the same or different 1 to 5 substituents, preferably with 1 to 2 substituents".
Furthermore, in the case where a plurality of the groups exists as in R⁰ in -N(R⁰)₂, each group (R⁰ in this case) may be the same or different from each other.

Preferred embodiments of the compound (I) of the present invention are as follows.
(1) R¹ is preferably R^{1a}, more preferably aryl-lower alkylene-O-, aryl-lower alkylene-NR⁰-, heteroaryl-lower alkylene-O-, cycloalkyl-lower alkylene-O-, cycloalkyl-lower alkylene-NR⁰-, or oxygen-containing saturated heterocyclic group-lower alkylene-O-, further more preferably aryl-lower alkylene-O-, aryl-lower alkylene-NR⁰-, cycloalkyl-lower alkylene-O-, or cycloalkyl-lower alkylene-NR⁰-, and even further more preferably aryl-lower alkylene-O-, aryl-lower alkylene-NR⁰-, or cycloalkyl-lower alkylene-O-. Each aryl and each heteroaryl in R¹ may be substituted with group(s) selected from the above-mentioned Group G¹, but preferably it is not substituted, or it is substituted with the same or different I or 2 substituents selected from the group consisting of halogen, lower alkyl, -O-lower alkyl, and halogeno-lower alkyl, and more preferably it is not substituted, or it is substituted with the same or different 1 or 2 substituents selected from the group consisting of halogen and halogeno-lower alkyl. In R¹, the aryl is preferably phenyl, and the cycloalkyl is preferably 5 to 7-membered cycloalkyl, and more preferably cyclohexyl. Furthermore, in R¹, the lower alkylene is preferably methylene, ethylene, or trimethylene, and more preferably ethylene.
(2) R⁰ is preferably H or methyl.
(3) A is preferably A¹, more preferably a benzene ring or 5- or 6-membered aromatic hetero ring, each of which may be substituted, further more preferably a benzene ring or a 6-membered an aromatic hetero ring, and even further more preferably a benzene ring or a pyridine ring. In A, the benzene ring and the hetero ring may be substituted with group(s) selected from the above-mentioned Group G², but preferably, it is not substituted, or it is substituted with the same or different 1 or 2 substituents selected from the group consisting of halogen, lower alkyl, and -O-C₁₋₈ alkyl, and more preferably it is not substituted, or it is substituted with one halogen.
(4) L is preferably L¹, more preferably methylene, -O- or -C(O)-, and further more preferably (i) when X is CH, it is -O-, (ii) when X is N, it is methylene or -C(O)-.
(5) R² is preferably H or methyl, and more preferably H.
(6) B is preferably B¹, more preferably a single bond or a 6-membered aromatic nitrogen-containing hetero ring, and further more preferably a single bond, a pyridine ring, a pyrazine ring, a pyrimidine ring, a pyridazine ring or a triazine ring. In B, the benzene ring and the aromatic hetero ring may be substituted with group(s) selected from the above-mentioned Group G³, but preferably, it is not substituted, or it is substituted with one substituent selected from the group consisting of halogen, lower alkyl, -O-lower alkyl, -OH, -lower alkylene-OR⁰, -CN, -C(O)-N(R⁰)₂, -N(R⁰)₂ and - NR⁰-C(O)-lower alkyl, and more preferably it is not substituted, or it is substituted with one halogen.
(7) R⁴ is preferably R^{4a}, and
   (i) when n=1 and B= a single bond,
      it is more preferably -C(O)-Z. Here, Z is preferably lower alkyl, cycloalkyl, heteroaryl, or -lower alkylene-O-lower alkyl, more preferably lower alkyl, heteroaryl or -lower alkylene-O-lower alkyl, and further more preferably methyl, pyridyl or methoxymethyl;
   (ii) when n=1 and B= a 5- or 6-membered aromatic nitrogen-containing hetero ring which may be substituted with group(s) selected from the above-mentioned Group G³,
      it is more preferably H or a nitrogen-containing heterocyclic group, and further more preferably H; and
   (iii) when n=0,
      it is more preferably 5-membered aromatic nitrogen-containing heterocyclic group having a bonding arm on N as a ring-constituting element, unsubstituted or substituted with -NH₂, and further more preferably 1H-imidazol-1-y1,3-amino-4H-1,2,4-triazol-4-yl or 5-amino-1H-pyrazol-1-yl.
A particularly preferred embodiment of the compound (I) of the present invention is a compound obtained by the combination of each preferable group as described in (1) to (7) as above. Furthermore, another preferred embodiments of the compound (I) of the present invention are a compound represented by the formula (I-A), the formula (I-B), the formula (I-C) or the formula (I-D).

The compound of the present invention may in some cases exist in the form of other tautomers or geometrical isomers, depending on the kind of the substituents. As used in the present specification, the compound can be described in only one form of isomers, but the present invention includes the isomers, an isolated form of the isomers, or a mixture of these isomers.
Furthermore, the compound (I) may have asymmetric carbons or axial asymmetry, and correspondingly, it may exist in the form of optical isomers such as an (R)-form and an (S)-form. The compound of the present invention includes both of a mixture and an isolated form of these optical isomers.
Further, the "pharmaceutically acceptable prodrugs" of the compound (I) are also included in the present invention. The "pharmaceutically acceptable prodrug" is a compound having a group which can be converted into an amino group, OH, CO₂H, or the like, by solvolysis or under a physiological condition. Examples of the group capable of forming a prodrug include those described in "Prog. Med., 5, 2157-2161 (1985)", or "Iyakuhin no Kaihatsu (Development of Drugs) (Hirokawa Shoten, 1990), vol. 7, Bunshi Sekkei (Molecular Design)", pp. 163-198.

Furthermore, the compounds of the present invention may form an acid-addition salt or a salt with a base, depending on the kind of the substituents, and these salts are included in the present invention, as long as they are pharmaceutically acceptable salts. Specifically, examples thereof include acid-addition salts with inorganic acids such as hydrochloric acid, hydrobromic acid, hydroiodic acid, sulfuric acid, nitric acid and phosphoric acid, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid and glutamic acid. The compounds may form a salt with a base, and examples thereof include salts with inorganic bases such as sodium, potassium, magnesium, calcium and aluminum, and organic bases such as methylamine, ethylamine, ethanolamine, lysine and ornithine, and ammonium salts.
In addition, the present invention also includes various hydrates and solvates, and polymorphic crystal substances of the compound of the present invention and a pharmaceutically acceptable salt thereof. Furthermore, the present invention also includes the compounds that are labeled with various radioactive isotopes or nonradioactive isotopes.

In the present specification, the "urinary frequency" refers to a condition in which urination frequency increases over a normal range. Further, the "urinary incontinence" refers to a condition of involuntary leakage of urine that gives rise to problems in the social or sanitary aspects.
In the present specification, the "overactive bladder" refers to a syndrome diagnosed by self-consciousness of urinary frequency and/or urinary urgency (Non-Patent Document 1). Examples of the causes of developing the conditions include neurologic disorders (for example, disorders due to neurogenic bladder, or cerebral infarction), lower urinary tract obstruction (for example, prostate hypertrophy), and aging, which are each considered to have hyperactivity of a capsaicin-sensitive afferent nerve as a common disease-developing mechanism.
The overactive bladder can be treated by relieving the symptoms of urinary frequency, urinary incontinence, urinary urgency or the like. It is clear, for example, from the fact that by administering Oxybutynin Hydrochloride (Japan Standard Industrial Classification No. 87259; Aventis Pharmaceuticals, Inc.) as an anticholinergic agent to a patient with overactive bladder three times a day, at a dose of 2 to 3 mg once, the symptoms of urinary frequency, urinary incontinence, urinary urgency or the like is relieved, thereby it being possible to treat overactive bladder.

Demonstration of the therapeutic effect for urinary frequency, urinary incontinence and/or overactive bladder can be carried out by using a method known to a person skilled in the art or a modified method thereof. For example, a pathologic model caused by administration of 50 to 200 mg of cyclophosphamide (CPA) to a rat, a guinea pig, a dog or the like is very frequently used in the art (Ozawa et. al., The Journal of Urology, Vol. 162, pp. 2211-2216, 1999; Boucher et. al., The Journal of Urology, Vol. 164, pp. 203-208, 2000). This model is a pathologic model accompanied by hemorrhagic cystitis, but since a capsaicin-sensitive afferent nerve is involved role in the symptom-developing mechanism for urinary frequency, the present model is considered to be suitable for various types of overactive bladder including neurogenic bladder (Carlo Alberto Maggi et. al., Journal of the Autonomic Nervous System, Vol. 38, pp. 201-208, 1992). The condition of urinary frequency can be confirmed from the reduction in the effective bladder capacity. For this pathologic model animal, by repeatedly administering once or several times an effective dose of a pharmaceutical composition in oral, intraperitoneal or intravenous administration mode, the therapeutic effect for urinary frequency, urinary incontinence and/or overactive bladder can be confirmed from the increase in the effective bladder capacity.

### (Production Processes)

The compound of the present invention and a pharmaceutically acceptable salt thereof can be prepared by applying various known synthetic methods, by the use of the characteristics based on their basic backbones or the kind of the substituents. Here, depending on the kind of the functional groups, it is in some cases effective from the viewpoint of the preparation techniques to substitute the functional group with an appropriate protecting group (a group which may be easily converted into the functional group), during the steps from starting materials to intermediates. Examples of such a functional group include an amino group, a hydroxyl group, a carboxyl group and the like, and examples of a protecting group thereof include those as described in "Protective Groups in Organic Synthesis", edited by Greene and Wuts, 3^{rd} edition, 1999, which may be optionally selected and used in response to the reaction conditions. By such a method, a desired compound can be obtained by introducing a protecting group to carry out the reaction, and then, if desired, removing the protecting group.
In addition, a prodrug of the compound (I) can be prepared by introducing a specific group during the steps from starting materials to intermediate, in the same manner as for the aforementioned protecting groups, or by carrying out the reaction with the obtained compound (I). The reaction can be carried out by employing a method known to a person skilled in the art, such as common esterification, amidation, dehydration and the like.
Hereinbelow, the representative production processes of the compounds of the present invention are described. Further, the production processes of the present invention are not limited to the examples as shown below.
(The symbols in the sentences have the following meanings:
DMF: N,N-dimethylformamide; DMSO: dimethylsulfoxide; THF: tetrahydrofuran; Tol: toluene; EtOAc: ethyl acetate; and TEA: triethylamine. The same shall apply hereinafter.)
In addition, if the same substituents in the compound of the present invention are present on the position other than those as shown in Reaction Schemes in the Production Processes, a reaction for modifying a substituent can be used to easily prepare the compound included in the present invention.

### (Production Process 1)

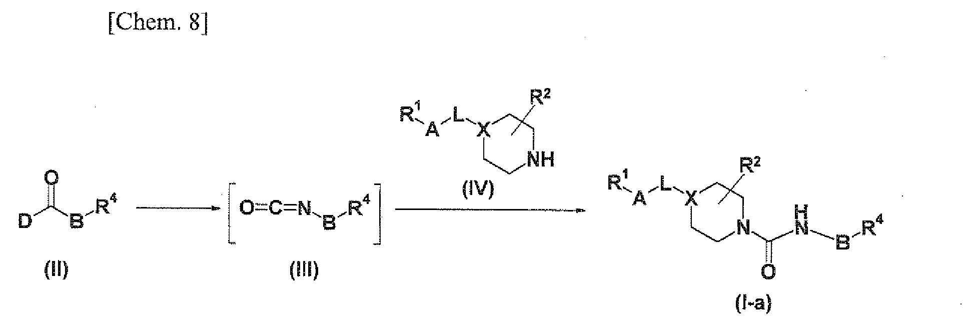

(wherein D represents a leaving group or -OH, which is advantageous for the present reaction. The same shall apply hereinafter.)
The present production process is a process in which an isocyanate (III) is produced from a carboxylic acid derivative (II), and then allowed to undergo a reaction with a cyclic amine derivative (IV) to produce a urea compound (I-a), which is the compound (I) of the present invention, wherein n=1, and R³ is H, or a benzene ring or an aromatic hetero ring, each of which may be substituted. Examples of the leaving group of D include halogen. In the case where D is halogen, or the like, the production of isocyanate (III) is carried out in the following manner: sodium azide is allowed to undergo a reaction in a solvent inert to the reaction such as acetonitrile from under cooling to room temperature to form a corresponding acid azide, and then excessive sodium azide is removed by a liquid separation process, the solvent is substituted by a solvent inert to the reaction such as Tol, and heated (preferably to around 110°C). The production of the isocyanate (III) may be carried out in the following manner: using a free carboxylic acid wherein D is -OH as a starting material, diphenylphosphorylazide (DPPA) is allowed to undergo a reaction in a solvent inert to the reaction such as Tol, in the presence of an organic base such as TEA, from under cooling to room temperature, and the same process as above is then performed. The reaction of the isocyanate (III) and the cyclic amine derivative (IV) can be carried out by using a solution of the isocyanate (III) produced as above in Tol, in a solvent inert to the reaction such as THF, from under cooling to heating under reflux. Furthermore, if the isocyanate (III) is commercially available, the reaction can also be carried out by using the commercially available one.

### (Production Process 2)

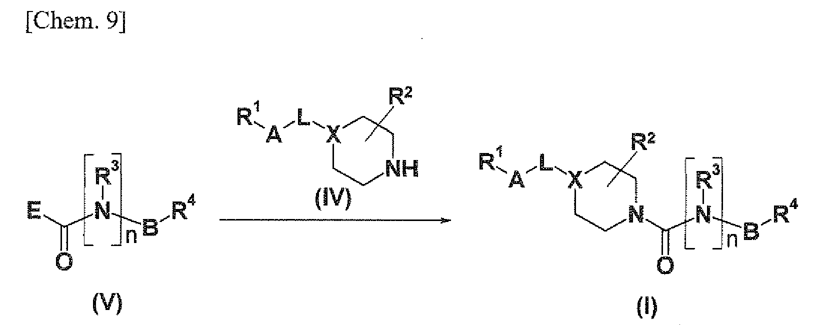

(wherein E represents a leaving group that is advantageous in the present reaction).
The present production process is a process in which a compound (V) having a leaving group obtained by allowing a nitrogen-containing compound to undergo a reaction with triphosgene, 1,1'-carbonyldimidazole (CDI), phenyl chloroformate, or ethyl chlorate, is reacted with a cyclic amine derivative (IV), in the presence or absence of an organic base such as TEA and pyridine, in a solvent inert to the reaction such as dichloromethane and THF, from under cooling to room temperature to produce the compound (I) of the present invention. The compound (V) may be used in the reaction after isolation, or used as it is without isolation after being produced in the reaction system, and examples of the leaving group of E include chloro, imidazolyl, phenoxy, or ethoxy. The reaction is carried out in a solvent inert to the reaction such as dichloromethane, DMF, DMSO, THF, and dioxane, from cooling to heating under reflux.

### (Production Process 3)

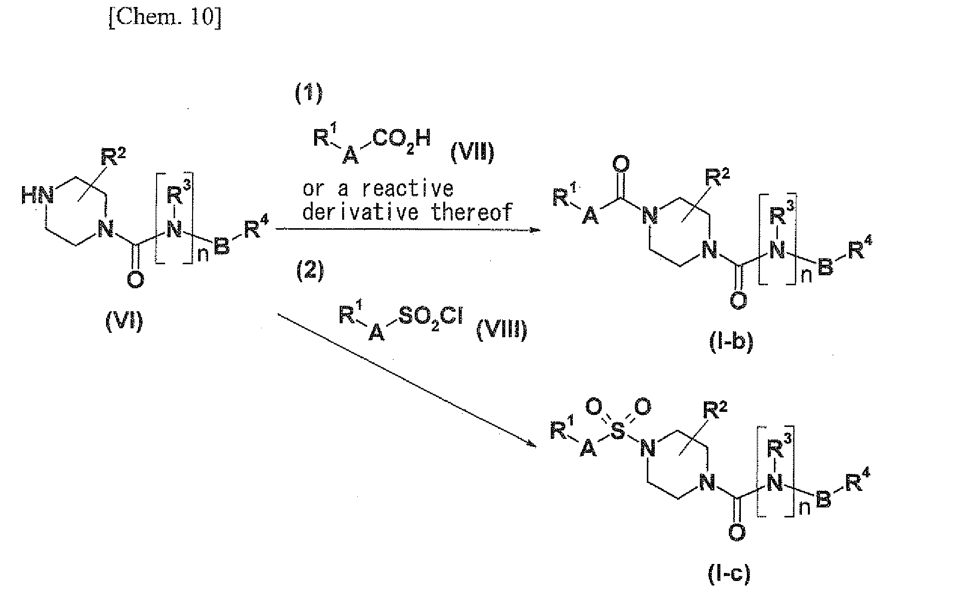

(1) The present production process is a process in which a cyclic amine derivative (VI) is reacted with a carboxylic acid (VII) or a reactive derivative thereof to produce a compound (I-b) that is the compound (I) of the present invention in which X is N, and L is -C(O)-. The reaction can be carried out in the presence of a condensing agent (for example, dicyclohexylcarbodimide (DCC), diisopropylcarbodimide (DIPC),1-ethyl-3-(3-dimethylaminopropyl)carbodiimide (WSC), and 1,1'-carbonyldimidazole (CDI)), if desired, an additive (for example, N-hydroxysuccinimide (HONSu), 1-hydroxybenzotriazole (HOBt), organic bases such as 4-(N,N-dimethylamino)pyridine (DMAP) and TEA). As the reactive derivative of the carboxylic acid, an acid halide, an acid anhydride, an active ester, and the like can be used. The reaction can be carried out, for example, with reference to a method as described in "Jikken Kagaku Koza (Courses in Experimental Chemistry) (4th edition)", edited by The Chemical Society of Japan, vol. 22 (1992) (Maruzen), and the like.
(2) The present production process is a process in which a cyclic amine derivative (VI) is reacted with sulfonyl chloride (VIII) to produce a compound (I-c) that is the compound (I) of the present invention in which X is N, and L is -S(O)₂-. The reaction can be carried out, for example, with the use of the sulfonylation condition as described in "Protective Groups in Organic Synthesis" as described above. Specifically, it can be carried out in a solvent such as THF, dichloromethane, and acetonitrile, and if desired, in the presence of a base such as TEA and pyridine, from under cooling to heating under reflux.

### (Production Process 4)

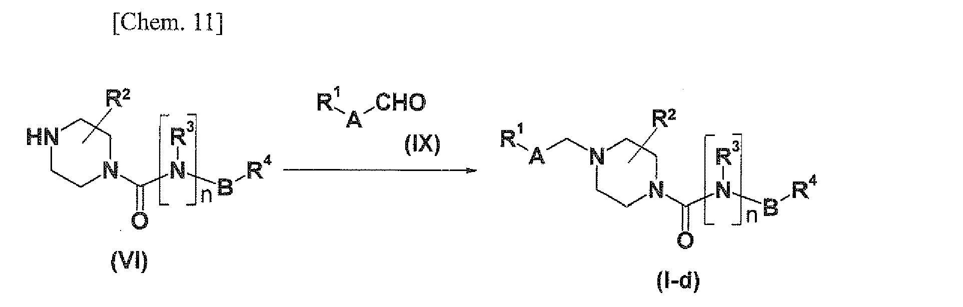

The present production process is a process in which reductive alkylation is carried out using a cyclic amine derivative (VI) and an aldehyde (IX) to produce a compound (I-d) that is the compound (I) of the present invention in which X is N, and L is methylene. The reaction can be carried out, for example, with reference to a method as described in "Jikken Kagaku Koza (Courses in Experimental Chemistry) (4th edition)", edited by The Chemical Society of Japan, vol. 20 (1992) (Maruzen), p. 300, or the like. Specifically, it is preferably carried out without a solvent, or in a solvent inert to the reaction, for example, halogenated hydrocarbons such as dichloromethane, 1,2-dichloroethane, and chloroform, aromatic hydrocarbons such as benzene, Tol, and xylene, esters such as EtOAc, ethers such as diethylether, THF, and dioxane, alcohols such as methanol and ethanol, and acetic acid, using a reducing agent such as sodium borohydride (NaBH₄), sodium cyanoborohydride (NaBH₃CN), and sodium triacetoxyborohydride (NaBH(OAc)₃), under cooling, or from under cooling to heating under reflux. According to the compounds, it is desirable in some cases to carry out the reaction in the presence of mineral acids such as sulfuric acid, hydrochloric acid, and hydrobromic acid, organic acids such as formic acid and acetic acid, or Lewis acid such as titanium tetrachloride. Furthermore, the reaction can be carried out, for example, using palladium-carbon, Raney nickel, platinum, or the like as a catalyst, at a normal pressure to elevated pressure under a hydrogen atmosphere, in a solvent inert to the reaction, such as aromatic hydrocarbons, esters, ethers, halogenated hydrocarbons, DMF, N,N-dimethylacetamide (DMA), N-methylpyrrolidone (NMP), acetonitrile, and acetic acid, as described above, from room temperature to heating under reflux.

### (Production Process 5)

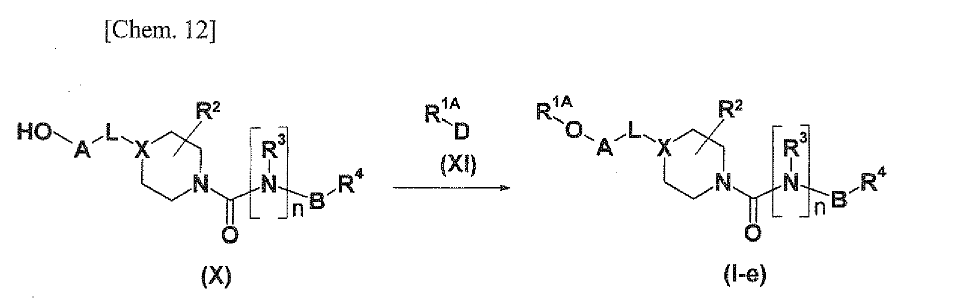

(wherein R^{1A} represents aryl-lower alkylene- which may be substituted with the above-mentioned Group G¹, and cycloalkyl-lower alkylene-.)
The present production process is a process in which a phenol derivative or an aromatic hetero ring derivative (X) having a hydroxyl group is alkylated using a compound (XI) having a leaving group to produce a compound (I-e) that is the compound (I) of the present invention in which R¹ is aryl-lower alkylene-O- which may be substituted with the above-mentioned Group G¹, or cycloalkyl-lower alkylene-O-. The leaving group of D may be any of the leaving groups that are commonly used in nucleophilic substitution reactions, and as the leaving group, halogen such as chloro and bromo, sulfonyloxy such as methanesulfonyloxy, p-toluenesulfonyloxy, and trifluoromethanesulfonyloxy, sulfonyl such as lower alkylsulfonyl and arylsulfonyl, or the like are suitably used. For the alkylation reaction in the present process, alkylation that can be usually used by a person skilled in the art can be employed. The reaction can be carried out, for example, in a solvent inert to the reaction, for example, aromatic hydrocarbons such as benzene, Tol, and xylene, esters such as EtOAc, ethers such as diethylether, THF, and dioxane, halogenated hydrocarbons such as dichloromethane, 1,2-dichloroethane, and chloroform, DMF, DMF, NMP, DMSO, and acetonitrile, or in a solvent such as alcohols, from room temperature to heating under reflux. According to the compounds, it is in some cases advantageous in advancing the reaction smoothly to carry out the reaction in the presence of organic bases (TEA, diisopropylethylamine, N-methylmorpholine, pyridine, DMAP, and the like are suitably used), or metal salt bases (potassium carbonate, cesium carbonate, sodium hydroxide, potassium hydroxide, sodium hydride, potassium tert-butoxide are suitably used).

In addition, the reaction can be carried out using a compound in the formula (XI) in which D is -OH, in a solvent such as ethers such as THF, dioxane, and diethylether, halogenated hydrocarbons such as methylene chloride and chloroform, aromatic hydrocarbons such as Tol and benzene, and DMF, in the presence of phosphines such as triphenylphosphine (Ph₃P) and tri-n-butylphosphine, and azodicarboxylates such as diethylazodicarboxylate (DEAD) and diisopropylazodicarboxylate, from under cooling to at room temperature.

### (Starting Material Synthesis 1)

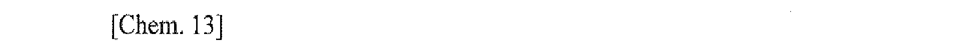

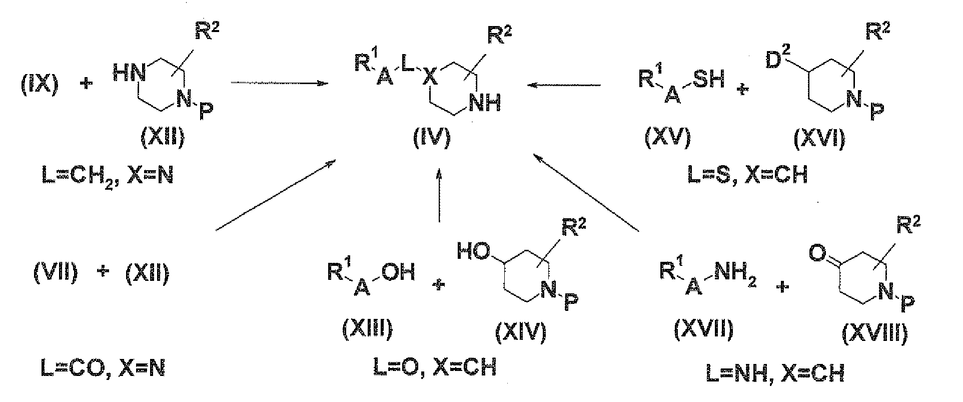

(wherein P represents a protecting group of an amino group, and preferably a Boc group. Further, D² represents a leaving group, and preferably a methanesulfonyloxy group. The same shall apply hereinafter.)
The cyclic amine derivative (IV) can be produced using various methods according the embodiments of L and X. For example, it can be produced by reductive amination of an aldehyde derivative (IX) and an amine (XII), amidation of a carboxylic acid (VII) or a reactive derivative thereof and an amine (XII), Mitsunobu reaction of a compound (XIII) and an alcohol (XIV), S-alkylation of a compound (XV) and a compound (XVI), and reductive amination of a compound (XVII) and a ketone (XVIII).
("Comprehensive Organic Functional Group Transformations II", written by A. R. Katritzky and R. J. K. Taylor, Vol. 2, Elsevier Pergamon, 2005,
"Jikken Kagaku Koza (Courses in Experimental Chemistry) (5th edition)", edited by The Chemical Society of Japan, vol. 14 (2005) (Maruzen),
"Organic Functional Group Preparations", written by S. R. Sandler and W. Karo, 2nd edition, Vol. 1, Academic Press Inc., 1991)

### (Starting Material Synthesis 2)

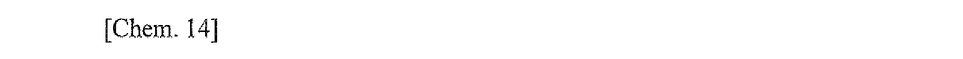

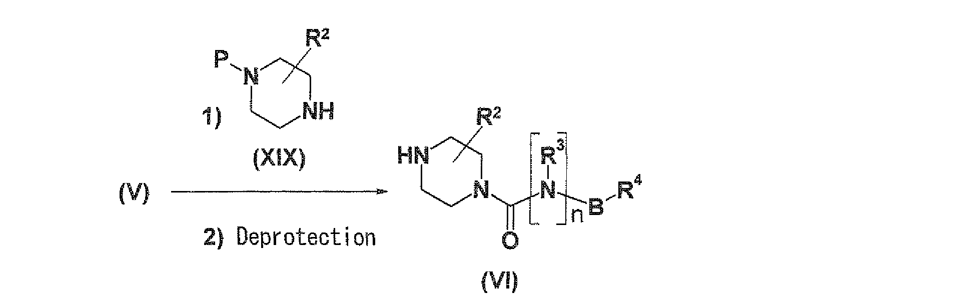

The cyclic amine derivative (VI) can be produced by reacting the compound (V) with an amine (XIX), and then deprotecting the amino group.

Moreover, several compounds represented by the formula (I) can be prepared from the compound of the present invention thus produced, by the method as described in the following Examples, and alternative methods thereof, or by any combination of well-known processes that can be usually employed by a person skilled in the art, such as alkylation, acylation, substitution reaction, oxidation, reduction, hydrolysis, and deprotection.

The compound of the present invention is isolated and purified as its free compound, a pharmaceutically acceptable salt, a hydrate, and a solvate thereof, or a polymorphic crystal substances. The pharmaceutically acceptable salt of the compound (I) of the present invention can be produced after carrying out a conventional salt formation treatment.
The isolation and purification can be carried out by employing common chemical operations such as extraction, fractional crystallization, and various types of chromatography.
Various isomers can be isolated by selecting a suitable starting compound, or by making use of the difference in a physicochemical property between isomers. For example, the optical isomers can be derived into a stereochemically pure isomers by means of general optical resolution methods (for example, fractional crystallization for inducing diastereomers with optically active bases or acids, and a technique such as a chiral filler-aided column chromatography). In addition, the isomers can also be produced using an appropriate optically active starting material.

The pharmacological effects of the compounds of the present invention were confirmed by the following tests.

### Test Example 1 Screening for an FAAH activity inhibiting substance using a rat brain homogenate

### (1) Preparation of a rat brain homogenate

The head of a 10-week-old SD-line male rat (Japan SLC., Inc.) was cut off, and its cerebrum was taken out and weighed. Five times by volume its weight of an ice-cooled buffer (50 mM Tris-HCl (pH 7.4), 0.32 M sucrose) was added, and this was homogenized with a homogenizer in ice to give a uniform suspension. This was centrifuged (1500×g, 4°C, 15 minutes), and the supernatant was again centrifuged (15000×g, 4°C, 20 minutes) to obtain a precipitate. Further, using an ultrasonic wave generator (UR-20P, TOMY SEIKO CO., LTD.), this was ultrasonicated (power dial 4) for 5 seconds. The protein concentration of the resulting homogenate was measured according to a dye-coupling method (protein assay CBB solution, NACALAI TESQUE, INC.). Using a buffer (50 mM Tris-HCl (pH 8.0), 1 mM EDTA, 0.1 mg/ml BSA, 100 mM NaCl), the rat brain homogenate was diluted so that its protein concentration could be 60 µg/ml, thereby preparing an enzyme solution.

### (2) Screening for an FAAH activity inhibiting substance

A substrate solution was prepared, comprising 2 µCi/ml radiolabeled anandamide (Anandamide [ethanolamine 1-³H] (American Radiolabeled Chemical)), 8 µM anandamide (Funakoshi), 50 mM Tris-HCl (pH 8.0), 1 mM EDTA, 0.1 mg/ml BSA and 100 mM NaCl. Test substance solutions were prepared, dissolved in DMSO to have a concentration from 1 nM to 100 µM. 50 µl of the substrate solution and 1 µl of the test substance solution were added to 50 µl of the enzyme solution, and left for 1 hour. As a control, DMSO was added in place of the test substance solution. To this, added was 200 µl of a 1:1 (ratio by volume) solution of chloroform/methanol, followed by vortexing. This was centrifuged (15000 rpm, 2 minutes), whereby the decomposed product ethanolamine (ethanolamine 1-³H) was separated in the upper layer (water/methanol layer) and the unreacted radiolabeled anandamide (Anandamide [ethanolamine 1-³H]) was in the lower layer (chloroform layer). 30 µl of the upper layer was transferred into a 96-well organic solvent-resistant white microplate (PicoPlate-96; Perkin Elmer, Inc.), 150 µl of Microscint-20 (Perkin Elmer, Inc.) was added thereto, and this was measured with a microplate scintillation counter (TopCount^{™}; Beckman Coulter, Inc.). As compared with the control, the substance that gave a decreased value was selected as an FAAH activity-inhibiting substance.

### (3) Measurement of the IC₅₀ value of the FAAH activity inhibiting substance

The compound was dissolved in DMSO to have a varying concentration from 1 nM to 100 µM to prepare test substance solutions. According to the method mentioned above, the compound was analyzed for its influence on FAAH activity. As a control, DMSO was used. A measured value of a case where a buffer (50 mM Tris-HCl (pH 8.0), 1 mM EDTA, 0.1 mg/ml BSA, 100 mM NaCl) was reacted in place of the enzyme solution was subtracted from every measured value. Based on the measured value of the control, 100%, the IC₅₀ value of the test substance was determined. These measurement results are shown in Table 1. Furthermore, Ex represents the Example No. as denoted below.

**[Table 1]**

| Ex | IC50 (nM) |
|---|---|
| 30 | 30 |
| 38 | 5.0 |
| 155 | 25 |
| 210 | 3.3 |
| 334 | 42 |
| 439 | 25 |

### Test Example 2 Screening for an FAAH activity-inhibiting substance with a human bladder epithelial cancer-derived cell

### (1) Screening for an FAAH activity inhibiting substance

Human bladder epithelial cancer-derived cell line 5637 cells (HTB-9; ATCC) were seeded on a 48-well cell culture plate in an amount of 1×10⁵ cell/well, using 10% fetal bovine serum (HyClone)-containing RPMI1640 medium (Invitrogen Corporation). After incubated at 37°C for 12 hours or longer, the cells were washed with 400 µl/well of a buffer (Hank's Balanced Salt Solution, 20 mM Hepes-NaOH (pH 7.4)). A test substance dissolved in DMSO was added to a substrate solution (the above buffer containing 3 µCi/ml radiolabeled anandamide (Anandamide [ethanolamine 1-³H]) and 10 µM anandamide) so as to have a concentration from 0.003 nM to 30 nM. As a control, DMSO alone was added. 100 µl/well of the substrate solution was added to the above cells, and incubated in a CO₂ incubator at 37°C for 30 minutes. Next, the cell culture plate was transferred onto ice, and the substrate solution was removed by suction; and 75 µl/well of a cytolytic solution (the above buffer containing 0.5% Triton X-100, and 10 µM of FAAH-inhibitory activity-having compound, 3'-carbamoylbiphenyl-3-yl ester (URB597; Cayman chemical; Kathuria et al., Nature Med., Vol. 9, pp. 76-81, 2003)) was added thereto, followed by stirring. The resulting cell lysate in every well was individually transferred into a 1.5 ml sample tube, to which was added 150 µl of 1:1 (ratio by volume) chloroform/methanol solution, followed by vortexing. This was centrifuged (15000 rpm, 2 minutes), whereby the decomposed product, ethanolamine (ethanolamine 1-³H) was separated in the upper layer (water/methanol layer) and the unreacted radiolabeled anandamide was in the lower layer (chloroform layer). 25 µl of the upper layer was transferred into a 96-well organic solvent-resistant white microplate (PicoPlate-96; Perkin Elmer, Inc.), 150 µl of Microscint-20 (Perkin Elmer, Inc.) was added thereto, and this was measured with a microplate scintillation counter (TopCount^{™}; Beckman Coulter, Inc.). As compared with the control, the substance that gave a decreased value was selected as an FAAH activity-inhibiting substance.

### (2) Measurement of the IC₅₀ value of the FAAH activity inhibiting substance

The compound dissolved in DMSO to have a concentration of 10 mM was dissolved in the substrate solution so as to have a varying concentration from 0.003 nM to 30 nM. According to the method mentioned above, the compound was analyzed for its influence on FAAH activity. As a negative control, DMSO was used. As a positive control, URB597 was added to the substrate solution to have a concentration of 10 µM. Based on the measured value of the positive control, 0%, and on the measured value of the negative control, 140%, the IC₅₀ value of the test substance was obtained. The measurement results are shown in Table 2. Furthermore, Ex represents Example No. as denoted below.

**[Table 2]**

| Ex | IC₅₀ (nM) | Ex | IC₅₀ (nM) |
|---|---|---|---|
| 1 | 5.6 | 179 | 0.21 |
| 12 | 0.060 | 210 | 0.16 |
| 30 | 0.28 | 300 | 0.16 |
| 38 | 0.70 | 317 | 0.57 |
| 59 | 0.82 | 320 | 0.24 |
| 75 | 0.81 | 322 | 0.54 |
| 133 | 2.8 | 334 | 0.18 |
| 136 | 0.22 | 388 | 0.080 |
| 155 | 0.54 | 426 | 0.12 |
| 156 | 0.33 | 439 | 0.43 |
| 159 | 0.29 | 488 | 0.85 |

### Test Example 3 Screening for an FAAH activity inhibiting substance using a rat tissue homogenate administered with a test substance

### (1) Administration to a rat, and preparation of a tissue homogenate

A test substance suspended in 0.5% methyl cellulose (MC) solution was orally administered to two 9-week-old Wistar male rats (Japan SLC, Inc.) at a dose from 1 to 3 mg/kg. As a control, 0.5% MC solution was orally administered to two rats. After 30 minutes, the blood was collected from the rat under ether anesthesia through its aorta. With that, the head of each rat was cut off, and its cerebrum was taken out.
3 ml of the collected blood was diluted with the same amount of physiological saline water, and gently put on 3 ml of a hemocyte-separating agent (Nycoplep; AXIS-SHIELD) in a centrifugal tube. This was centrifuged (400xg, 20 minutes) to collect the monocytic layer. The resulting monocytes were washed twice with physiological saline, and frozen and stored at -20°C until their use for measurement.
To the collected rat brain, added was five times by volume its weight of an ice-cooled buffer (50 mM Tris-HCl (pH 8.0), 1 mM EDTA), and this was homogenized with a homogenizer in ice to give a uniform suspension. Further, using an ultrasonic wave generator (UR-20P (power dial 4), TOMY SEIKO CO., LTD.), this was ultrasonicated for 5 seconds. To the above frozen monocytes, added was 100 µl of an ice-cooled buffer (50 mM Tris-HCl (pH 8.0), 1 mM EDTA), and using an ultrasonic wave generator (UR-20P (power dial 4), TOMY SEIKO CO., LTD.), this was ultrasonicated for 5 seconds. The protein concentration of each of the homogenates of brain and monocytes was measured according to a dye-coupling method (protein assay CBB solution, NACALAI TESQUE, INC.). Using a buffer (50 mM Tris-HCl (pH 8.0), 1 mM EDTA, 0.1 mg/ml BSA, 100 mM NaCl), the homogenates of brain and monocytes were diluted so that their protein concentration could be 80 µg/ml and 400 µg/ml, thereby preparing enzyme solutions.

### (2) Measurement of FAAH activity

50 µl of the enzyme solution was reacted with 50 µl of a substrate solution (2 µCi/ml radiolabeled anandamide (Anandamide [ethanolamine 1-³H] (American Radiolabeled Chemical)), 8 µM anandamide (Funakoshi), 50 mM Tris-HCl (pH 8.0), 1 mM EDTA) added thereto, at room temperature for 1 hour. 200 µl of a 1:1 (ratio by volume) solution of chloroform and methanol was added to it, followed by vortexing. This was centrifuged (12000×g, 2 minutes), whereby the decomposed product ethanolamine (ethanolamine 1-³H) was separated in the upper layer (water/methanol layer) and the unreacted radiolabeled anandamide (Anandamide [ethanolamine 1-³H]) was in the lower layer (chloroform layer). 25 µl of the upper layer was transferred into a 96-well organic solvent-resistant white microplate (PicoPlate-96; PerkinElmer, Inc.), 150 µl of Microscinti-20 (Perkin Elmer, Inc.) was added thereto, and this was measured with a microplate scintillation counter (TopCount^{™}; Beckman Coulter, Inc.).
Based on the FAAH activity of the control, test substance-free, rat brain or monocyte homogenate, 100%, and on the FAAH activity of the tissue homogenate-free buffer (50 mM Tris-HCl (pH 8.0), 1 mM EDTA, 0.1 mg/ml BSA, 100 mM NaCl), 0%, the relative value (%) of the FAAH activity of the tissue homogenate of the rat administered with the test substance was obtained. The substance that decreased the relative value of FAAH activity was selected as an FAAH activity-inhibiting substance.
The above results confirm that by administering a test substance to a test animal, and then measuring the test substance-dependent FAAH activity change in the taken tissue homogenate, an FAAH activity-inhibiting substance can be screened.

### Test Example 4 Effect of compound on cyclophosphamide (CPA)-induced urinary frequency in a rat

Compounds were tested for their bladder irritation-relieving effect, using pathologic models. It is known that systemic administration of cyclophosphamide (CPA) converts the compound into its metabolite, acrolein, and, as existing in urine, this injures the bladder mucosa. In rats, CPA administration induces bladder pain or urinary frequency accompanied by hemorrhagic cystitis, and therefore using such rats, it is possible to evaluate the potency of drug for these symptoms. In this experiment, used were 9-week-old Wistar female rats (Charles River). CPA (100 mg/kg) was intraperitoneally administered to the rats, and after 2 days, the rats were tested. A test compound was orally administered (p.o.) to the rats; and after 15 minutes, distilled water (30 ml/kg) was forcedly orally administered thereto. The rats were put in a metabolic cage, and the weight of their urine was continuously measured for 1 hour. The overall urine amount was divided by the overall urination frequency, and the effective bladder capacity was thus calculated. As a result, in the group administered with the solvent, 0.5% methyl cellulose (MC), the effective bladder capacity reduced, and the rats showed urinary frequency. In oral administration, the effective doses of compounds of Examples 155 and 210 were 10 mg/kg and 3 mg/kg, respectively. These compounds increased the reduced effective bladder capacity, and relieved the condition of urinary frequency.

The above-described results confirmed that the compound of the present invention has an excellent FAAH inhibitory action, increases the reduced effective bladder capacity, and relieves the condition of urinary frequency. In this regard, it is apparent that the compound of the present invention is useful as an agent for treating FAAH-related diseases, in particular, urinary frequency, urinary incontinence, and/or overactive bladder.
Furthermore, the compound of the present invention has an excellent FAAH inhibitory action, and thus is useful as an agent for treating (1) neuropsychiatric disorders (e.g., anxiety, depression, and epilepsy), (2) brain disorders, and neurodegenerative disorders (e.g., head injury, cerebral ischemia, and cognitive symptoms (dementia)), (3) pains, (4) immunological and inflammatory diseases, (5) vomiting, (6) eating disorders, (7) irritable bowel syndrome and ulcerative colitis, (8) hypertension, (9) glaucoma, or (10) sleep disorders.

The pharmaceutical composition containing one or two or more kinds of the compound (I) of the present invention or a salt thereof as an active ingredient can be prepared in accordance with a method that is generally employed, using a pharmaceutically acceptable carrier, an excipient, and the like, generally used in the art.
The administration of the composition can be accompanied by any mode of oral administration via tablets, pills, capsules, granules, powders or liquid preparations; and parenteral administration via injections such as intraarticular, intravenous, or intramuscular injections, suppositories, eye drops, eye ointments, transdermal liquid preparations, ointments, transdermal patches, transmucosal liquid preparations, transmucosal patches, and inhalations.
Regarding the solid composition of the present invention for oral administration, tablets, powders, granules, or the like are used. In such a solid composition, one or two or more kinds of active ingredients are mixed with at least one inactive excipient such as lactose, mannitol, glucose, hydroxypropylcellulose, microcrystalline cellulose, starch, polyvinyl pyrrolidone, and/or magnesium aluminometasilicate. In a conventional method, the composition may contain inactive additives such as lubricants such as magnesium stearate, disintegrators such as carboxymethylstarch sodium, stabilizing agents, and solubilizing agents. Tablets or pills may be coated with a film of a sugar coating, or a gastric or enteric coating agent if necessary.
The liquid composition for oral administration includes pharmaceutically acceptable emulsions, solutions, suspensions, syrups, elixirs, or the like, and contains a generally used inert diluent such as purified water or ethanol. In addition to the inert diluent, this liquid composition may also contain auxiliary agents such as solubilizing agents, moistening agents, and suspending agents, sweeteners, flavors, aromatics, and antiseptics.
Injection for parenteral administration includes aseptic aqueous or non-aqueous liquid preparations, suspensions and emulsions. As the aqueous solvent, for example, distilled water for injection and physiological saline are included. Examples of the non-aqueous solvent include propylene glycol, polyethylene glycol, plant oils such as olive oil, alcohols such as ethanol, and Polysorbate 80 (Pharmacopeia). Such a composition may further contain tonicity agents, antiseptics, moistening agents, emulsifying agents, dispersing agents, stabilizing agents, or solubilizing agents. These are sterilized, for example, by filtration through a bacteria retaining filter, blending of a germicide, or irradiation. In addition, these can also be used by producing sterile solid compositions, and dissolving or suspending it in sterile water or a sterile solvent for injection prior to their use.

Medicaments for external use include ointments, plasters, creams, jellies, patches, sprays, lotions, eye drops, and eye ointments. The medicament contains generally used ointment bases, lotion bases, aqueous or non-aqueous solutions, suspensions, emulsions, and the like. Examples of the ointment bases or lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, and sorbitan sesquioleate.
Regarding transmucosal agents such as inhalations and transnasal agents, those in a solid, liquid, or semi-solid state are used, and may be produced in accordance with a conventionally known method. For example, known excipients, and also pH adjusting agents, antiseptics, surfactants, lubricants, stabilizing agents, thickening agents, and the like may be optionally added thereto. For their administration, appropriate devices for inhalation or insufflation may be used. For example, a compound may be administered alone or as a powder of formulated mixture, or as a solution or suspension by combining it with a pharmaceutically acceptable carrier, using a conventionally known device or sprayer, such as a measured administration inhalation device. The dry powder inhalers or the like may be for single or multiple administration use, and dry powders or powder-containing capsules may be used. Alternatively, this may be in a form such as a high pressure aerosol spray which uses an appropriate propellant, for example, a suitable gas such as chlorofluoroalkane, hydrofluoroalkane, and carbon dioxide.

In the case of oral administration, the daily dose may be generally from about 0.001 to 100 mg/kg, preferably from 0.1 to 30 mg/kg, and further more preferably 0.1 to 10 mg/kg, per body weight, and this is administered in one portion or dividing it into 2 to 4 portions. Also, in the case of intravenous administration, the daily dose is from about 0.0001 to 10 mg/kg per day per body weight, once a day or two or more times a day. In addition, a transmucosal agent is administered at a dose from about 0.001 to 100 mg/kg per body weight, once a day or two or more times a day. The dose is appropriately determined in response to an individual case by taking the symptoms, the age, and the gender of the subject, and the like into consideration.

The compound of the present invention can be used in combination of various therapeutic or prophylactic agents for the diseases, in which the compound of the present invention is considered effective. The combined preparation may be administered simultaneously or separately and continuously, or at a desired time interval. The preparations to be co-administerd may be a blend, or prepared individually.

### Examples

Hereinbelow, the processes for producing the compound (I) of the present invention will be described in more detail with reference to the following Examples, but the compounds of the present invention are not limited to the compounds described in the following Examples. Furthermore, the processes for producing the starting compounds will be described in Reference Examples.
In addition, the following abbreviations are used in Examples, Reference Examples, and Tables as below.
Rf: Reference Example No., Ex: Example No., Str: structural formula, Syn: production process (the numeral shows that it was produced using a corresponding starting material, similar to the case of an Example compound having its number as the Example No. In the case where R is provided before the number, the numeral shows that it was produced using a corresponding starting material, similar to the case of a Reference Example compound having its number as the Reference Example No.). Dat: Physicochemical data (EI: EI-MS ([M]⁺); EP: ESI-MS (Pos) (in a case of no description, [M+H]⁺); EN: ESI-MS(Neg) ([M-H]⁻); FP: FAB-MS (Pos) (in a case of no description, [M+H]⁺); FN: FAB-MS (Neg) (in a case of no description, [M-H]⁻); NMR1 : δ (ppm) of the peaks in ¹H-NMR using DMSO-d₆; NMR2: δ (ppm) of the peaks in ¹H-NMR using CDCl₃ DIBAL: Diisobutylaluminum hydride; Pd (PPh₃)₄: Tetrakis(triphenylphosphine) palladium; pTsOH: p-toluene sulfonic acid; Me: methyl; Et: ethyl.

### Reference Example 1

To a solution of tert-butyl 4-hydroxypiperidine-1-carboxylate (7.85 g) in THF (60 ml) was added PPh₃ (10.2 g), and further a solution of 4-benzyloxyphenol (6.01 g) and DEAD (6.14 ml, 40% Tol solution) in THF (50 ml) was added dropwise thereto under ice-cooling, followed by stirring at room temperature for 20 hours. The reaction mixture was concentrated under reduced pressure, and to the obtained residue was added an aqueous sodium hydroxide solution, followed by extraction with EtOAc. The organic layer was dried over anhydrous sodium sulfate, and then concentrated under reduced pressure. The residue was purified by silica gel column chromatography (eluent; hexane:EtOAc=4:1 (V/V)) to obtain tert-butyl 4-[4-(benzyloxy)phenoxy]piperidine-1-carboxylate (9.13 g).
To a solution of the obtained tert-butyl 4-[(4-benzyloxy)phenoxy]piperidine-1-carboxylate (9.13 g) in methanol was added 10% palladium carbon (0.913 g), followed by stirring at room temperature for 3 hours under a hydrogen atmosphere. The insoluble material was removed by filtration, and the filtrate was then concentrated under reduced pressure. The obtained residue was recrystallized from hexane/EtOAc to obtain tert-butyl 4-(4-hydroxyphenoxy)piperidine-1-carboxylate (5.04 g).
To a solution of the obtained tert-butyl 4-(4-hydroxyphenoxy)piperidine-1-carboxylate (3.82 g) in acetonitrile (50 ml) were added potassium carbonate (2.70 g) and 3-fluorobenzylbromide (2.95 g), followed by stirring at 80°C for 3 hours. The reaction mixture was cooled to room temperature, and water was added thereto, followed by extraction with chloroform. The organic layer was dried over anhydrous sodium sulfate, and then concentrated under reduced pressure. The residue was purified by silica gel column chromatography (eluent; hexane:EtOAc=4:1 (V/V)) to obtain tert-butyl 4-{4-[(3-fluorobenzyl)oxy]phenoxy}piperidine-1-carboxylate (5.77 g).
To a solution of the obtained tert-butyl 4-[4-(3-fluorobenzyloxy)phenoxy]pipendine-1-carboxylate (5.77 g) in EtOAc (20 ml) was added a 4 M hydrochloride/EtOAc solution (19.5 ml), followed by stirring at room temperature for 5 hours. The precipitated solid was collected by filtration, and washed with EtOAc. The obtained solid was dissolved in water, alkalified by addition of an aqueous sodium hydroxide solution, and extracted with chloroform. The organic layer was dried over anhydrous sodium sulfate, and then concentrated under reduced pressure to obtain 4-{4-[(3-fluorobenzyl)oxy]phenoxy}piperidine (3.56 g).

### Reference Example 2

To a solution of ethyl 3-bromobenzoate (2.29 g) and (3-hydroxyphenyl) boronic acid (1.66 g) in acetonitrile (40 ml) were sequentially added a 0.5 M aqueous sodium carbonate solution (40 ml) and Pd (PPh₃)₄ (578 mg), followed by stirring at 90°C for 1 hour. The reaction mixture was cooled to room temperature, and the insoluble material was then removed by filtration through Celite. To the filtrate was added water, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=2:1 (V/V)) to obtain a pale yellow oily substance (2.62 g).
To a solution of tert-butyl-4-hydroxypiperidine-1-carboxylate (3.00 g) and PPh₃ (3.91 g) in THF (20 ml) was added dropwise a solution of the obtained compound (2.61 g) and DEAD (2.59 g, 40% Tol solution) in THF (10 ml) under ice-cooling, followed by stirring at room temperature for 20 hours. To the reaction mixture was added an aqueous sodium hydroxide solution, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=4:1 (V/V)) to obtain a colorless and oily substance (4.77 g).
To a solution of the obtained compound (4.75 g) in EtOAc (20 ml) was added a 4 M hydrochloride/EtOAc solution (19.8 ml), followed by stirring at room temperature for 6 hours. The reaction mixture was concentrated under reduced pressure, and the obtained residue was crystallized from a mixed solvent of EtOAc/acetonitrile/ethanol to obtain a colorless powder of ethyl 3'-(piperidin-4-yloxy)biphenyl-3-carboxylate hydrochloride (2.81 g).

### Reference Example 3

To a solution of 4-hydroxythiophenol (1.26 g) in DMA (15 ml) were added cesium carbonate (3.91 g) and tert-butyl-4-[(methanesulfonyl)oxy]piperidine-1-carboxylate (3.35 g), followed by heating at 50°C for 2 hours. The reaction mixture was cooled to room temperature, and water was added thereto, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=2:1 (V/V)) to obtain a colorless and oily substance (2.28 g).
To a solution of the obtained compound (1.24 g) in acetonitrile (20 ml) were added potassium carbonate (829 mg) and 1-(bromomethyl)-3-fluorobenzene (832 mg), followed by stirring at 80°C for 3 hours. The reaction mixture was cooled to room temperature, and an aqueous sodium hydroxide solution was added thereto, followed by extraction with chloroform. The organic layer was washed with saturated brine, dried over anhydrous sodium sulfate, and then concentrated under reduced pressure. The residue was purified by silica gel column chromatography (eluent; hexane:EtOAc=4:1 (V/V)) to obtain a colorless and oily substance (1.86 g).
To a solution of the obtained oily substance (1.85 g) in EtOAc (10 ml) was added a 4 M hydrochloride/EtOAc solution (4.94 ml), followed by stirring at room temperature for 4 hours. The precipitated solid was collected by filtration, and dried under heating to obtain a colorless powder of 4-({4-[(3-fluorobenzyl)oxy]phenyl}sulfanyl)piperidine hydrochloride (1.20 g).

### Reference Example 4

To a solution of 4-aminophenol (1.09 g) in DMF (40 ml) were sequentially added potassium tert-butoxide (1.35 g), and 1-(bromomethyl)-3-fluorobenzene (1.89 g) under ice-cooling, followed by stirring at room temperature for 1 hour. To the reaction mixture was added water under ice-cooling, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=1:1 (V/V)) to obtain a pale yellow oily substance (1.87 g).
To a solution of the obtained oily substance (1.05 g) in acetic acid (15 ml) were sequentially added 1-(tert-butoxycarbonyl)-4-piperidone (996 mg) and NaBH(OAc)₃ (1.27 g), followed by stirring at room temperature for 3 hours. The reaction mixture was concentrated under reduced pressure, acetic acid was removed by distillation, and then to the obtained residue was added a 5 M aqueous sodium hydroxide solution, followed by extraction with chloroform. The organic layer was dried over anhydrous sodium sulfate, and then concentrated under reduced pressure. The residue was purified by silica gel column chromatography (eluent; hexane:EtOAc=2:1 (V/V)) to obtain a yellow oily substance (1.96 g).
To a solution of the obtained oily substance (1.96 g) in acetic acid (10 ml) were sequentially added a 35% aqueous formalin solution (2.10 g) and NaBH(OAc)₃ (2.07 g), followed by stirring at room temperature for 16 hours. The solution was concentrated under reduced pressure, acetic acid was removed by distillation, and then to the obtained residue was added a 5 M aqueous sodium hydroxide solution, followed by extraction with chloroform. The organic layer was dried over anhydrous sodium sulfate, and then concentrated under reduced pressure. The residue was purified by silica gel column chromatography (eluent; hexane:EtOAc=2:1 (V/V)) to obtain a yellow oily substance (1.88 g).
To a solution of the obtained oily substance (1.88 g) in EtOAc (10 ml) was added a 4 M hydrochloride/EtOAc solution (9.05 ml), followed by stirring at room temperature for 5 hours. The reaction mixture was concentrated under reduced pressure, and the obtained residue was crystallized from acetonitrile/methanol to obtain a pale yellow powder of N-{4-[(3-fluorobenzyl)oxy]phenyl}-N-methylpiperidin-4-amine dihydrochloride (1.29 g).

### Reference Example 5

To a 48% aqueous hydrobromic acid solution (20 ml) was added 4-(4-methoxybenzoyl)piperidine hydrochloride (1.05 g), followed by heating under reflux for 19 hours. The reaction mixture was cooled to room temperature, and then concentrated under reduced pressure to obtain a pale yellow solid.
To a mixed solution of the obtained solid in dioxane (10 ml)/water (5 ml) were sequentially added TEA (1.24 g), and a solution of di(tert-butyl) dicarbonate (984 mg) in dioxane (5 ml), followed by stirring at room temperature for 2 hours. To the reaction mixture was added a 5% aqueous citric acid solution, followed by extraction with EtOAc. The organic layer was washed with saturated brine, dried over anhydrous sodium sulfate, and then concentrated under reduced pressure. The residue was purified by silica gel column chromatography (eluent; chloroform:methanol=24:1 (V/V)) to obtain a pale yellow oily substance (1.36 g).
To a solution of the obtained oily substance (1.34 g) in acetonitrile (15 ml) were sequentially added potassium carbonate (840 mg) and 1-(bromomethyl)-3-fluorobenzene (843 mg), followed by stirring at 80°C for 3 hours. The reaction mixture was cooled to room temperature, and an aqueous sodium hydroxide solution was added thereto, followed by extraction with EtOAc. The organic layer was washed with saturated brine, dried over anhydrous sodium sulfate, and then concentrated under reduced pressure. The residue was crystallized from hexane/EtOAc to obtain a colorless powder (1.25 g).
To a solution of the obtained powder (1.22 g) in EtOAc (10 ml) was added a 4 M hydrochloride/EtOAc solution (7.38 ml), followed by stirring at room temperature for 10 hours. The precipitated solid was collected by filtration to obtain a colorless powder of {4-[(3-fluorobenzyl)oxy]phenyl}(piperidin-4-yl) methanone hydrochloride (1.01 g).

### Reference Example 6

To a solution of methyl 4-hydroxybenzoate (4.56 g) in acetonitrile (50 ml) were sequentially added potassium carbonate (4.98 g) and 1-(bromomethyl)-3-fluorobenzene (6.24 g), followed by stirring at 80°C for 2 hours. The reaction mixture was cooled to room temperature, and water was added thereto, followed by extraction with chloroform. The organic layer was washed with saturated brine, dried over anhydrous sodium sulfate, and then concentrated under reduced pressure. The residue was washed with hexane to obtain a colorless powder of methyl 4-[(3-fluorobenzyl)oxy]benzoate (6.92 g).
To a mixed solution of methyl 4-[(3-fluorobenzyl)oxy]benzoate (6.92 g) in THF (30 ml)/methanol (30 ml) was added a 1 M aqueous sodium hydroxide solution (39.9 ml), followed by stirring at 50°C for 2 hours. The reaction mixture was cooled to room temperature, and then concentrated under reduced pressure. The residue was acidified by addition of a 1 M aqueous hydrochloric acid solution, and the precipitated solid was collected by filtration to obtain a colorless powder of 4-[(3-fluorobenzyl)oxy]benzoic acid (6.66 g).
To a solution of 1-(tert-butoxycarbonyl)-4-piperidone (1.99 g) in chloroform (30 ml) were sequentially added acetic acid (1.80 g), a 40% methyl amine-containing methanol solution (2.33 g), and NaBH(OAc)₃ (3.18 g), followed by stirring at room temperature for 3 hours. The reaction mixture was alkalified by addition of a 1 M aqueous sodium hydroxide solution, followed by extraction with chloroform. The organic layer was dried over anhydrous sodium sulfate, and then concentrated under reduced pressure, and the residue was purified by silica gel column chromatography (eluent; chloroform:methanol=21:4(V/V)) to obtain a yellow oily substance of tert-butyl 4-(methylamino)piperidine-1-carboxylate (2.01 g).
To a solution of tert-butyl 4-(methylamino)piperidine-1-carboxylate (53 6 mg) in THF (10 ml) were sequentially added 4-[(3-fluorobenzyl)oxy]benzoic acid (616 mg), HOBt (169 mg), and WSC (527 mg), followed by stirring at room temperature for 18 hours. To the reaction mixture was added an aqueous hydrochloric acid solution, followed by extraction with EtOAc. The organic layer was washed with an aqueous sodium hydroxide solution and water, dried over anhydrous sodium sulfate, and then concentrated under reduced pressure. The residue was crystallized from hexane/EtOAc to obtain a colorless powder of a compound (940 mg).
To a solution of the obtained compound (930 mg) in EtOAc (10 ml) was added a 4 M hydrochloride/EtOAc solution (5.25 ml), followed by stirring at room temperature for 14 hours. The precipitated solid was collected by filtration to obtain a colorless powder of 4-[(3-fluorobenzyl)oxy]-N-methyl-N-piperidin-4-ylbenzamide hydrochloride (674 mg).

### Reference Example 7

To a solution of 4-(4-methoxybenzoyl)piperidine hydrochloride (1.05 g) in dioxane (10 ml) were sequentially added TEA (1.06 g), and a solution of di(tert-butyl) dicarbonate (1.09 g) in dioxane (10 ml), followed by stirring at room temperature for 1 hour. To the reaction mixture was added a 5% aqueous citric acid solution, followed by extraction with EtOAc. The organic layer was washed with saturated brine, dried over anhydrous sodium sulfate, and then concentrated under reduced pressure to obtain a pale yellow oily substance (1.89 g).
To a solution of the obtained oily substance (1.89 g) in methanol (20 ml) was added NaBH₄ (284 mg), followed by stirring at room temperature for 30 minutes. To the reaction mixture was added water, followed by extraction with chloroform. The organic layer was dried over anhydrous sodium sulfate, and then concentrated under reduced pressure, and the residue was purified by silica gel column chromatography (eluent; hexane:EtOAc=1:1 (V/V)) to obtain a yellow oily substance (1.65 g).
To a solution of the obtained oily substance (1.63 g) in ethanol (30 ml) was added 10% palladium carbon (796 mg), followed by stirring at room temperature for 17 hours under a hydrogen gas atmosphere of 3 kgf/cm². The catalyst was removed by filtration, and the filtrate was concentrated under reduced pressure. The residue was purified by silica gel column chromatography (eluent; hexane:EtOAc=4:1 (V/V)) to obtain a colorless and oily substance (1.31 g).
The obtained oily substance (1.29 g) was dissolved in a 48% aqueous hydrobromic acid solution (20 ml), followed by heating under reflux for 24 hours. The reaction mixture was cooled to room temperature, and then concentrated under reduced pressure to obtain a pale yellow solid.
To a mixed solution of the obtained solid in dioxane (10 ml)/water (5 ml) were sequentially added TEA (1.29 g), and a solution of di(tert-butyl) dicarbonate (1.01 g) in dioxane (5 ml), followed by stirring at room temperature for 30 minutes. To the reaction mixture was added a 5% aqueous citric acid solution, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=2:1 (V/V)) to obtain a colorless and oily substance (1.05 g).
To a solution of the obtained oily substance (1.03 g) in acetonitrile (15 ml) were sequentially added potassium carbonate (733 mg) and 1-(bromomethyl)-3-fluorobenzene (733 mg), followed by stirring at 80°C for 14 hours. The reaction mixture was cooled to room temperature, and water was added thereto, followed by extraction with chloroform. The organic layer was washed with water, dried over anhydrous sodium sulfate, and then concentrated under reduced pressure. The residue was purified by silica gel column chromatography (eluent; hexane:EtOAc=4.1 (V/V)) to obtain a colorless and oily substance (1.49 g).
To a solution of the obtained oily substance (1.47 g) in EtOAc (10 ml) was added a 4 M hydrochloride/EtOAc solution (8.70 ml), followed by stirring at room temperature for 4 hours. The resulting solid was collected by filtration to obtain a colorless powder of 4-{4-[(3-fluorobenzyl)oxy]benzyl}piperidine hydrochloride (802 mg).

### Reference Example 8

To a solution of 4-[(3-fluorobenzyl)oxy]aniline hydrochloride (761 mg) in THF (10 ml) were sequentially added TEA (304 mg), I -(tert-butoxycarbonyl)piperidine-4-carboxylic acid (688 mg), HOBt (203 mg), and WSC (633 mg), followed by stirring at room temperature for 16 hours. To the reaction mixture was added an aqueous hydrochloric acid solution, followed by extraction with EtOAc. The organic layer was washed with an aqueous sodium hydroxide solution and saturated brine, dried over anhydrous sodium sulfate, and then concentrated under reduced pressure. The residue was crystallized from hexane/EtOAc to obtain a colorless powder of a compound (880 mg).
To a solution of the obtained powder (855 mg) in DMF (10 ml) was added 60% sodium hydride (96 mg) under ice-cooling, followed by stirring at room temperature for 20 minutes. To the reaction mixture was added methyl iodide (566 mg), followed by stirring at 40°C for 15 minutes. The reaction mixture was cooled to room temperature, and water was added thereto, followed by extraction with EtOAc. The organic layer was washed with saturated brine, dried over anhydrous sodium sulfate, and then concentrated under reduced pressure. The residue was purified by silica gel column chromatography (eluent; chloroform:methanol=24:1 (V/V)) to obtain a pale yellow oily substance (1.03 g).
To a solution of the obtained oily substance (1.01 g) in EtOAc (10 ml) was added a 4 M hydrochloride/EtOAc solution (4.85 ml), followed by stirring at room temperature for 4 hours. The reaction mixture was concentrated under reduced pressure, and the residue was crystallized from acetonitrile/diethylether to obtain a colorless powder of N-{4-[(3-fluorobenzyl)oxy]phenyl}-N-methylpiperidine-4-carboxamide hydrochloride (590 mg).

### Reference Example 9

To a solution of cyclohexylmethanol (514 mg) and PPh₃ (1.18 g) in THF (5 ml) was added dropwise a solution of methyl 4-hydroxybenzoate (456 mg) and DEAD (784 mg, 40% Tol solution) in THF (5 ml) under ice-cooling, followed by stirring at room temperature for 21 hours. To the reaction mixture was added an aqueous sodium hydroxide solution, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=4:1 (V/V)) to obtain a colorless solid (934 mg).
To a mixed solution of the obtained compound (920 mg) in methanol (5 ml)/THF (3 ml) was added a 1 M aqueous sodium hydroxide solution (4.43 ml), followed by stirring at 50°C for 6 hours. The reaction mixture was cooled to room temperature, an aqueous sodium hydroxide solution was added thereto, and the water layer was washed with EtOAc. The water layer was acidified with concentrated hydrochloric acid, followed by extraction with chloroform. The organic layer was dried over anhydrous sodium sulfate, and then concentrated under reduced pressure, and the residue was crystallized from hexane/EtOAc to obtain a colorless powder of 4-(cyclohexylmethoxy) benzoic acid (603 mg).

### Reference Example 10

To a solution of 2,2-dimethylpropanol (397 mg) in DMF (5 ml) was added potassium tert-butoxide (505 mg) under ice-cooling, followed by stirring at room temperature for 30 minutes. To the reaction mixture was added a solution of 4-fluorobenzonitrile (363 mg) in DMF (5 ml) under ice-cooling, followed by stirring at room temperature for 3 hours. To the reaction mixture was added water, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=4:1 (V/V)) to obtain a colorless and oily substance (587 mg).
To a solution of the obtained compound (575 mg) in ethanol (5 ml) was added a 5 M aqueous sodium hydroxide solution (5.90 ml), followed by stirring at 100°C for 23 hours. The reaction mixture was cooled to room temperature, and then concentrated under reduced pressure. To the residue were added water and a 1 M aqueous hydrochloric acid solution, and the precipitated solid was collected by filtration, and dried under heating to obtain a colorless powder of 4-(2,2-dimethylpropoxy) benzoic acid (560 mg).

### Reference Example 11

To a solution of methyl 3-hydroxybenzoate (610 mg) in acetonitrile (10 ml) were sequentially added potassium carbonate (1.11 g) and bromomethylcyclohexane (1.06 g), followed by stirring at 80°C for 28 hours. The reaction mixture was cooled, and an aqueous sodium hydroxide solution was added thereto, followed by extraction with chloroform. The organic layer was dried over anhydrous sodium sulfate, and then concentrated under reduced pressure, and the residue was purified by silica gel column chromatography (eluent; hexane:EtOAc=4:1 (V/V)) to obtain a colorless and oily substance (475 mg).
To a mixed solution of the obtained compound (465 mg) in methanol (4 ml)/THF (4 ml) was added a 1 M aqueous sodium hydroxide solution (3.75 ml), followed by stirring at 50 °C for 3 hours. The reaction mixture was cooled to room temperature, and then concentrated under reduced pressure, and the obtained residue was dissolved in water. The solution was acidified by addition of a 1 M aqueous hydrochloric acid solution, and the precipitated solid was collected by filtration, and dried under heating to obtain a colorless powder of 3-(cyclohexylmethoxy) benzoic acid (371 mg).

### Reference Example 12

To a solution of ethyl 4-amino benzoate (1.65 g) in chloroform (30 ml) were sequentially added acetic acid (3.00 g), 3-fluorobenzaldehyde (1.30 g), and NaBH(OAc)₃ (2.54 g), followed by stirring at room temperature for 17 hours. To the reaction mixture was added a 1 M aqueous sodium hydroxide solution, followed by extraction with chloroform. The organic layer was dried over anhydrous sodium sulfate, and then concentrated under reduced pressure, and the residue was purified by silica gel column chromatography (eluent; hexane:EtOAc=2:1 (V/V)) to obtain a pale yellow solid (1.68 g).
To a mixed solution of the obtained compound (683 mg) in ethanol (5 ml)/THF (5 ml) was added a 1 M aqueous sodium hydroxide solution (12.5 ml), followed by stirring at 50°C for 24 hours. The reaction mixture was cooled to room temperature, and then concentrated under reduced pressure, and the obtained residue was dissolved in water. The solution was acidified by addition of a 1 M aqueous hydrochloric acid solution, and the precipitated solid was collected by filtration, and dried under heating to obtain a colorless powder of 4-[(3-fluorobenzyl)amino]benzoic acid (574 mg).

### Reference Example 13

To a solution of cyclohexylmethanol (685 mg) in DMF (10 ml) was added potassium tert-butoxide (673 mg) under ice-cooling, followed by stirring at room temperature for 30 minutes. To the reaction mixture was added a solution of 2-chloro-4-cyanopyridine (693 mg) in DMF (2 ml) under ice-cooling, followed by stirring at room temperature for 2 hours. To the reaction mixture was added water, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=4:1 (V/V)) to obtain a colorless and oily substance (807 mg).
To a solution of the obtained compound (792 mg) in ethanol (5 ml) was added a 5 M aqueous sodium hydroxide solution (7.32 ml), followed by stirring at 100°C for 2 hours. The reaction mixture was cooled to room temperature, and then concentrated under reduced pressure, and the obtained residue was dissolved in water. The solution was acidified by addition of a 1 M aqueous hydrochloric acid solution, and the precipitated solid was collected by filtration, and dried under heating to obtain a pale yellow powder of 2-(cyclohexylmethoxy) isonicotinic acid (864 mg).

### Reference Example 14

To a solution of 6-chloronicotinonitrile (508 mg) and TEA (1.02 ml) in acetonitrile (10 ml) was added (cyclohexylmethyl)methylamine hydrochloride (600 mg), followed by stirring at 90°C overnight. The reaction mixture was concentrated under reduced pressure, and then diluted with EtOAc, and the organic layer was washed with a saturated aqueous sodium hydrogen carbonate solution, dried over anhydrous magnesium sulfate, and then concentrated under reduced pressure. The residue was purified by basic silica gel column chromatography (eluent; hexane:EtOAc=10: 1 (V/V)), the obtained oily substance was dissolved in ethanol (5 ml), and then a 5 M aqueous sodium hydroxide solution (5 ml) was added thereto, followed by stirring at 100°C for 6 hours. The reaction mixture was cooled to room temperature, and neutralized by addition of 1 M hydrochloric acid, and the precipitated crystal was collected by filtration, and dried under heating to obtain 6-[(cyclohexylmethyl)(methyl)amino]nicotinic acid (530 mg).

### Reference Example 15

To a solution of cyclohexylmethanol (594 mg) and PPh₃ (1.36 g) in THF (10 ml) was added dropwise a solution of methyl 5-hydroxynicotinate (613 mg) and DEAD (2.26 g, 40% Tol solution) in THF (30 ml) under ice-cooling, followed by stirring at room temperature for 18 hours. To the reaction mixture was added an aqueous sodium hydroxide solution, followed by extraction with EtOAc. The organic layer was washed with saturated brine, dried over anhydrous sodium sulfate, and then concentrated under reduced pressure. The residue was purified by silica gel column chromatography (eluent; hexane:EtOAc=2:1 (V/V)) to obtain a pale yellow oily substance of methyl 5-(cyclohexylmethoxy) nicotinate (1.17 g).

### Reference Example 16

To a solution of 2-cyclohexylethanol (1.00 g) in DMF (20 ml) was added potassium tert-butoxide (0.972 g) under ice-cooling, followed by stirring at room temperature for 30 minutes. To the reaction mixture was added 2-chloroisonicotinonitrile (1.11 g), followed by stirring at room temperature for 3 days. To the reaction mixture was added water, followed by extraction with EtOAc. The organic layer was washed with saturated brine, dried over anhydrous magnesium sulfate, and then concentrated under reduced pressure. The residue was purified by silica gel column chromatography (eluent; hexane:EtOAc=4:1 (V/V)) to obtain 2-(2-cyclohexylethoxy) isonicotinonitrile (1.27 g).
To a solution of 2-(2-cyclohexylethoxy)isonicotinonitrile (1.25 g) in Tol (40 ml) was added DIBAL (0.543 ml, 1 M Tol solution) at -78°C, followed by stirring for 2 hours. To the reaction mixture was added DIBAL (0.109 ml, 1 M Tol solution), followed by stirring for additional 2 hours. To the reaction mixture was added a saturated aqueous ammonium chloride solution, followed by extraction with EtOAc, and the organic layer was dried over anhydrous magnesium sulfate, and concentrated under reduced pressure. The residue was purified by silica gel column chromatography (eluent; hexane:EtOAc=4:1 (V/V)) to obtain 2-(2-cyclohexylethoxy)isonicotinaldehyde (338 mg) as a colorless and oily substance.

### Reference Example 17

To a solution of tert-butylpiperazine-1-carboxylate (331 mg) in THF (15 ml) were sequentially added 4-[(3-fluorobenzyl)oxy]benzoic acid (1.23 g), HOBt (338 mg), and WSC (1.05 g), followed by stirring at room temperature for 7 hours. To the reaction mixture was added an aqueous hydrochloric acid solution, followed by extraction with EtOAc. The organic layer was washed with an aqueous sodium hydroxide solution and water in this order, dried over anhydrous sodium sulfate, and then concentrated under reduced pressure. The residue was crystallized from hexane/EtOAc to obtain a colorless powder of a compound (1.79 g).
To a solution of the obtained compound (1.75 g) in EtOAc (10 ml) was added a 4 M hydrochloride/EtOAc solution (10.6 ml), followed by stirring at room temperature for 7 hours. The solution was concentrated under reduced pressure, and the residue was then crystallized from EtOAc to obtain a colorless powder of 1-{4-[(3-fluorobenzyl)oxy]benzoyl}piperazine hydrochloride (1.47 g).

### Reference Example 18

To a solution of tert-butylpiperazine-1-carboxylate hydrochloride (12.4 g) in DMF (200 ml) were sequentially added 3-acetyl benzoic acid (10 g), HOBt (9.0 g), and WSC (10.3 g), followed by stirring at room temperature overnight. To the reaction mixture was added an aqueous hydrochloric acid solution, followed by extraction with EtOAc. The organic layer was washed with a saturated aqueous sodium hydrogen carbonate solution and saturated brine in this order, dried over anhydrous sodium sulfate, and then concentrated under reduced pressure. The residue was crystallized from hexane/EtOAc to obtain a white powder of tert-butyl 4-(3-acetoxybenzoyl)piperazine-1-carboxylate (16.5 g).
To a solution of tert-butyl 4-(3-acctoxybenzoyl)piperazine-1-carboxylate (8.0 g) in THF (100 ml) was added a 1 M aqueous sodium hydroxide solution (45.9 ml), followed by stirring at room temperature for 1 hour. The reaction mixture was concentrated under reduced pressure, a 1 M aqueous hydrochloric acid solution was then added thereto and neutralized, and the precipitated solid was collected by filtration, and dried under heating to obtain tert-butyl 4-(3-hydroxybenzoyl)piperazine-1-carboxylate (7.0 g) as a white powder.
To a solution of PPh₃ (3.85 g) in THF (60 ml) was added dropwise DEAD (2.26 g, 40% Tol solution) under ice-cooling, followed by stirring at room temperature for 1 hour. Under ice-cooling, to the reaction mixture were sequentially added 3-phenyl-1-propanol (2.00 ml) and tert-butyl 4-(3-hydroxybenzoyl)piperazine-1-carboxylate (3.0 g), followed by stirring at room temperature overnight. To the reaction mixture was added a saturated aqueous sodium hydrogen carbonate solution, followed by extraction with EtOAc. The organic layer was washed with water, dried over anhydrous sodium sulfate, and then concentrated under reduced pressure. The residue was purified by silica gel column chromatography (eluent; hexane:EtOAc=3:1 (V/V)) to obtain a pale yellow oily substance of tert-butyl 4-[3-(3-phenylpropoxy)benzoyl]piperazine-1-carboxylate (3.60 g).
To a solution of tert-butyl 4-[3-(3-phenylpropoxy)benzoyl]piperazine-1-carboxylate (3.60 g) in EtOAc (15 ml) was added a 4 M hydrochloride/EtOAc solution (10.6 ml), followed by stirring at room temperature for 2 hours. The reaction mixture was concentrated under reduced pressure, and the residue was then crystallized from diisopropylether to obtain a white powder of 1-[3-(3-phenylpropoxy)benzoyl]piperazine hydrochloride (2.40 g).

### Reference Example 19

To a solution of 4-fluoro-3-hydroxybenzoic acid (1.0 g), tert-butylpiperazine-1-carboxylate hydrochloride (1.79 g), and HOBt (1.04 g) in DMF (20 ml) was added WSC (1.47 g), followed by stirring at room temperature overnight. The reaction mixture was diluted with EtOAc, washed with a saturated aqueous sodium hydrogen carbonate solution, dried over anhydrous magnesium sulfate, and concentrated under reduced pressure. The obtained solid was washed with hexane/EtOAc, and then dried under heating to obtain tert-butyl 4-(4-fluoro-3-hydroxybenzoyl)piperazine-1-carboxylate (1.70 g) as a white solid.
To a solution of tert-butyl 4-(4-fluoro-3-hydroxybenzoyl)piperazine-1-carboxylate (400 mg), 2-cyclohexylethanol (240 mg), and tributylphosphine (374 mg) in THF (13 ml) was added 1,1'-(azodicarbonyl)dipiperidine (466 mg) under ice-cooling, followed by stirring at room temperature overnight. The reaction mixture was diluted with EtOAc, the insoluble material was filtered, and the filtrate was then concentrated under reduced pressure. The residue was purified by silica gel column chromatography (eluent; hexane:EtOAc=2:1 (V/V)), and the obtained solid was washed with hexane/EtOAc, and then dried under heating to obtain tert-butyl 4-[3-(2-cyclohexyethoxy)-4-fluorobenzoyl]piperazine-1-carboxylate (255 mg) as a white solid.
To a solution of tert-butyl 4-[3-(2-cyclohexylethoxy)-4-fluorobenzoyl]piperazine-1-carboxylate (240 mg) in EtOAc (3 ml) was added a 4 M hydrochloride/EtOAc solution (2 ml), followed by stirring at room temperature overnight. The precipitated solid was collected by filtration, washed with EtOAc, and then dried under heating to obtain 1-[3-(2-cyclohexylethoxy)-4-fluorobenzoyl]piperazine hydrochloride (193 mg) as a white solid.

### Reference Example 20

To a solution of 2-methylpiperazine (1.38 g) in THF (40 ml) was added di(tert-butyl)dicarbonate (2.00 g) under ice-cooling, followed by stirring at room temperature overnight. The reaction mixture was concentrated under reduced pressure, and the obtained residue was purified by silica gel column chromatography (eluent; chloroform:methanol=85:15(V/V)) to obtain tert-butyl-3-methylpiperazine-1-carboxylate (966 mg) as a colorless and oily substance.
A suspension of 3-(2-cyclohexylethoxy) benzoic acid (300 mg), tert-butyl-3-methylpiperazine-1-carboxylate (371 mg), HOBt (202 mg), and WSC (287 mg) in dichloromethane (4.5 ml) was stirred at room temperature for 2 days. The reaction mixture was sequentially washed with water, a saturated aqueous sodium hydrogen carbonate solution, and saturated brine, dried over anhydrous magnesium sulfate, and then concentrated under reduced pressure. The residue was purified by silica gel column chromatography (eluent; chloroform:methanol=97:3 (V/V)) to obtain tert-butyl 4-[3-(2-cyclohexylethoxy)benzoyl]-2-methylpiperazine-1-carboxylate (601 mg) as a colorless and oily substance.
To tert-butyl 4-[3-(2-cyclohexylethoxy)benzoyl]-3-methylpiperazine-1-carboxylate (522 mg) was added a 4 M hydrochloride/EtOAc solution, followed by stirring at room temperature for 1 hour. The reaction mixture was concentrated under reduced pressure to obtain 4-[3-(2-cyclohexylethoxy)benzoyl]-2-methylpiperazine hydrochloride (444 mg) as a colorless and oily substance.

### Reference Example 21

A suspension of 3-(2-cyclohexylethoxy) benzoic acid (350 mg), HOBt (190 mg) and WSC (270 mg) in dichloromethane was stirred at room temperature for 2 hours, and 2-methylpiperazine was then added thereto, followed by stirring overnight. The reaction mixture was washed with a saturated aqueous sodium hydrogen carbonate solution and saturated brine in this order, dried over anhydrous magnesium sulfate, and concentrated under reduced pressure. The residue was purified by silica gel column chromatography (eluent; chloroform:methanol=93: 7(V/V)) to obtain 1-[3-(2-cyclohexylethoxy)benzoyl]-3-methylpiperazine (466 mg) as a colorless and oily substance.

### Reference Example 22

To a solution of 3-(benzyloxy) benzaldehyde (8.00 g) in THF (150 ml) were added tert-butylpiperazine-1-carboxylate hydrochloride (7.72 g), acetic acid (2.16 ml), and NaBH(OAc)₃ (9.59 g) under ice-cooling, followed by stirring at room temperature overnight. The reaction mixture was alkalified by addition of a 1 M aqueous sodium hydroxide solution, followed by extraction with chloroform. The organic layer was dried over anhydrous magnesium sulfate, and then concentrated under reduced pressure. The residue was purified by silica gel column chromatography (eluent; hexane:EtOAc=4.1 (V/V)) to obtain tert-butyl 4-[3-(beznzyloxy)benzyl]piperazine-1-carboxylate (13.4 g) as a pale yellow oily substance.
To a solution of tert-butyl 4-[3-(benzyloxy)benzyl]piperazine-1-carboxylate (13.4 g) in EtOAc (200 ml) was added 10% palladium carbon (1.12 g), followed by stirring at room temperature for 10 hours under a hydrogen atmosphere. The reaction mixture was filtered through Celite, and the filtrate was concentrated under reduced pressure to obtain tert-butyl 4-(3-hydroxybenzyl)piperazine-1-carboxylate (9.36 g).
To a solution of tert-butyl 4-(3-hydroxybenzyl)piperazine-1-carboxylate (10.6 g), 2-cyclohexylethanol (5.54 ml), and PPh₃ (14.2 g) in THF (140 ml) was added DEAD (24.6 ml, 40% Tol solution) under ice-cooling, followed by stirring at room temperature for 2 days. The reaction mixture was concentrated under reduced pressure, and the obtained residue was then purified by silica gel column chromatography (eluent; hexane:EtOAc=4:1 (V/V)), and again by silica gel column chromatography (eluent; chloroform) to obtain tert-butyl 4-[3-(2-cyclohexylethoxy)benzyl]piperazine-1-carboxylate (5.06 g) as a pale yellow oily substance.
To a solution oftert-butyl 4-[3-(2-cyclohexylethoxy)benzyl]piperazine-1-carboxylate (5.06 g) in EtOAc (31.4 ml) was added a 4 M hydrochloride/EtOAC solution (31.4 ml), followed by stirring at room temperature overnight. The precipitated solid was collected by filtration, and dried under heating to obtain 1-[3-(2-cyclohexylethoxy)benzyl]piperazine dihydrochloride (3.45 g) as a colorless powder.

### Reference Example 23

To a solution of 6-methyl-3-pyridinol (2.00 g), 2-cyclohexylethanol (3.1 ml), and PPh₃ (5.77 g) in THF (50 ml) were added dropwise DEAD (10 ml, 40% Tol solution), followed by stirring at room temperature overnight. The reaction mixture was concentrated under reduced pressure, and the obtained residue was then purified by silica gel column chromatography (eluent; chloroform:methanol=20:1 (V/V)) to obtain a colorless and oily 5-(2-cyclohexylethanol)-2-methylpyridine (3.93 g).
To a solution of 5-(2-cyclohexylethanol)-2-methylpyridine (3.90 g) in chloroform (50 ml) was added m-chloroperbenzoic acid (3.38 g), followed by stirring at room temperature for 3 hours. To the reaction mixture was added a 10% aqueous sodium thiosulfate solution (50 ml), followed by stirring for 5 minutes, and then extraction with EtOAc. The organic layer was washed with a saturated aqueous sodium hydrogen carbonate solution, and saturated brine in this order, dried over anhydrous magnesium sulfate, concentrated under reduced pressure. The residue was purified by silica gel column chromatography (eluent; hexane:EtOAc=2:1 (V/V)) to obtain a colorless crystalline 5-(2-cyclohexylethoxy)-2-methylpyridine 1-oxide (4.11 g).
A mixture of 5-(2-cyclohexylethoxy)-2-methylpyridine-1-oxide (3.9 g) and anhydrous acetic acid (1.69 g) was stirred at 100°C for 2 hours. To the reaction mixture was added Tol, followed by concentration under reduced pressure. The solution was diluted with EtOAc, and then washed with a saturated aqueous sodium hydrogen carbonate solution. The solution was dried over anhydrous magnesium sulfate, and then concentrated under reduced pressure, and the obtained residue was purified by silica gel column chromatography (eluent; hexane:EtOAc=4:1 (V/V)) to obtain [5-(2-cyclohexylethoxy)pyridin-2-yl]methylacetate (3.9 g) as an oily substance.
To a solution of [5-(2-cyclohexylethoxy)pyridin-2-yl]methylacetate (3.9 g) in methanol (70 ml) was added a 1 M aqueous sodium hydroxide solution (30 ml), followed by stirring at room temperature overnight. To the reaction mixture was added a 1 M aqueous hydrochloric acid solution (30 ml), followed by extraction with EtOAc. The organic layer was washed with water, dried over anhydrous magnesium sulfate, and then concentrated under reduced pressure to obtain [5-(2-cyclohexylethoxy)pyridin-2-yl]methanol (3.26 g) as an oily substance.
To a solution of [5-(2-cyclohexylethoxy)pyridin-2--yl]methanol (1.0 g) in chloroform (30 ml) was added manganese dioxide (3.70 g), followed by stirring at 65°C for 1 hour. The reaction mixture was filtered through Celite, and the filtrate was then concentrated to obtain 5-(2-cyclohexylethoxy)pyridine-2-carboaldehyde (840 mg) as an oily substance.
To a solution of 5-(2-cyclohexylethoxy)pyridine-2-carbaldehyde (830 mg), acetic acid (430 mg), and tert-butyl 1 -piperazinecarboxylate (660 mg) in dichloromethane (16 ml) was added NaBH(OAc)₃ (1.13 g), followed by stirring at room temperature for 2 hours. The reaction mixture was diluted with dichloromethane, washed with a saturated aqueous sodium hydrogen carbonate solution, dried over anhydrous magnesium sulfate, and then concentrated under reduced pressure. The obtained solid was washed with hexane/EtOAc, and dried under heating to obtain tert-butyl 4-{[5-(2-cyclohexylethoxy)pyridin-2-yl]methyl}piperazine-1-carboxylate (830 mg) as a white solid.
To a solution of tert-butyl 4-{[5-(2-cyclohexylethoxy)pyridin-2-yl]methyl}piperazine-1-carboxylate (800 mg) in methanol (10 ml) was added a 4 M hydrochloride/dioxane solution (4 ml), followed by stirring at room temperature overnight. The reaction mixture was concentrated under reduced pressure, and the obtained solid was then washed with EtOAc, and dried under heating to obtain 1-{[5-(2-cyclohexylethoxy)pyridin-2-yl]methyl}piperazine trihydrochloride (730 mg) as a white solid.

### Reference Example 24

To a solution of 5-(benzyloxy)-1H-indole-2-carboxylic acid (3.0 g), tert-butylpiperazine-1-carboxylate hydrochloride (2.51 g), and HOBt (1.82 g) in DMF (30 ml) was added WSC (2.58 g), followed by stirring at room temperature for 2 hours. To the reaction mixture was added a saturated aqueous sodium hydrogen carbonate solution, and the precipitated solid was collected by filtration. The obtained solid was washed with water, and then dried under heating to obtain tert-butyl 4-{[5-(benzyloxy)-1H-indol-2-yl]carbonyl}piperazine-1-carboxylate (4.48 g) as a white solid.
A mixture of tert-butyl 4-{[5-(benzyloxy)-1H-indol-2-yl]carbonyl}piperazine-1-carboxylate (1.0 g), 1,4-diazabicyclo[2.2.2]octane (25 mg), dimethyl carbonate (10 ml), and DMF (1 ml) was stirred at 100°C overnight. The reaction mixture was diluted with EtOAc, washed with water, and then dried over anhydrous magnesium sulfate. The solution was concentrated under reduced pressure, and the obtained solid was then washed with hexane/EtOAc, and dried under heating to obtain tert-butyl 4-{[5-(benzyloxy)-1-methyl-1H-indol-2-yl]carbonyl}piperazine-1-carboxylate (940 mg) as a white solid.
To a mixed solution of tert-butyl 4-{[5-(benzyloxy)-1-methyl-1H-indol-2-yl]carbonyl}piperazine-1-carboxylate (630 mg) in THF (15 ml)/ethanol (9 ml) was added 10% palladium carbon (30 mg), followed by stirring at room temperature for 5 hours under a hydrogen atmosphere. The catalyst was removed by filtration through Celite, and the filtrate was concentrated under reduced pressure to obtain tert-butyl 4-[(5-hydroxy-1-methyl-1H-indol-2-yl)carbonyl]piperazine-1-carboxylate (500 mg) as a colorless amorphous substance.
To a solution of tert-butyl 4-[(5-hydroxy-1-methyl-1H-indol-2-yl)carbonyl]piperazine-1-carboxylate (490 mg), PPh₃ (540 mg), and cyclohexylmethanol (233 mg) in THF (10 ml) was added DEAD (0.93 ml, 40% Tol solution) under ice-cooling, followed by stirring at room temperature overnight. The reaction mixture was diluted with EtOAc, washed with water and a saturated aqueous sodium hydrogen carbonate solution in this order, and then dried over anhydrous magnesium sulfate. The solution was concentrated under reduced pressure, and the obtained residue was then purified by silica gel column chromatography (eluent; hexane:EtOAc=2:1 (V/V)) to obtain tert-butyl 4-{[5-(cyclohexylmethoxy)-1-methyl-1H-indol-2-yl]carbonyl}piperidine-1-carboxylate (310 mg).
To a solution of tert-butyl 4-{[5-(cyclohexylmethoxy)-1-methyl-1H-indol-2-yl]carbonyl}piperidine-1-carboxylate (300 mg) in EtOAc (10 ml) was added a 4 M hydrochloride/EtOAc solution (3 ml), followed by stirring at room temperature for 6 hours. The precipitated solid was collected by filtration, washed with EtOAc, and then dried under heating to obtain 5-(cyclohexylmethoxy)-1-methyl-2-(piperazin-1-ylcarbonyl)-1H-indole hydrochloride (236 mg) as a white solid.

### Reference Example 25

To a solution of methyl 3-(3-cyclohexylpropyl)benzoate (1.50 g) in THF (20 ml) was added dropwise DIBAL (13 ml, 1 M Tol solution) at -78°C, followed by stirring at 0°C for 1 hour. To the reaction mixture were added a saturated aqueous (±)-sodium tartrate potassium solution (30 ml) and EtOAc, followed by vigorously stirring for 30 minutes, and extraction with EtOAc. The organic layer was dried over anhydrous magnesium sulfate, and concentrated under reduced pressure. The residue was purified by silica gel column chromatography (eluent; hexane:EtOAc =3:1 (V/V)) to obtain a colorless and oily [3-(3-cyclohexylpropyl)phenyl]methanol (1.30 g).
To a solution of [3-(3-cyclohexylpropyl)phenyl]methanol (1.30 g) in chloroform (30 ml) was added manganese dioxide (25.0 g), followed by stirring at room temperature for 1 hour. The reaction mixture was filtered, manganese dioxide was removed by filtration through Celite, and the filtrate was concentrated under reduced pressure. The residue was purified by silica gel column chromatography (eluent; hexane:EtOAc=4:1 (V/V)) to obtain a colorless and oily 3-(3-cyclohexylpropyl)benzaldehyde (1.10 g).
To a solution of 3-(3-cyclohexylpropyl)benzaldehyde (600 mg) in THF (30 ml) were added tert-butylpiperazine-1-carboxylate hydrochloride (583 mg), acetic acid (0.30 ml), and NaBH(OAc)₃ (1.11 g) under ice-cooling, followed by stirring at room temperature for 2 hours. The reaction mixture was alkalified with a 1 M aqueous sodium hydroxide solution, and extracted with chloroform. The organic layer was dried over anhydrous magnesium sulfate, and concentrated under reduced pressure. The residue was purified by silica gel column chromatography (eluent; hexane:EtOAc=3:1 (V/V)) to obtain a colorless and oily tert-butyl 4-[3-(3-cyclohexylpropyl)benzyl]piperazine-1-carboxylate (800 mg).
To a solution of tert-butyl 4-[3-(3-cyclohexylpropyl)benzyl]piperazine-1-carboxylate (800 mg) in EtOAc (10 ml) was added a 4 M hydrochloride/EtOAc solution (2.0 ml) followed by stirring at room temperature overnight. The precipitated solid was washed with EtOAc, and dried under heating to obtain a white solid of 1-[3-(3-cyclohexyl)benzyl]piperazine (530 mg).

### Reference Example 26

A suspension of tert-butyl (5-bromo-3-pyridyl)carbamate (300 mg), palladium acetate (II) (12.3 mg), 2-(di-tert-butylphosphino)biphenyl(32.8 mg), morpholine (0.287 ml), and potassium phosphate (699 mg) in dioxane (10 ml) was stirred at 100°C overnight. The reaction mixture was cooled to room temperature, and palladium acetate (II) (12.3 mg), 2-(di-tert-butylphosphino)biphenyl(32.8 mg), and morpholine (0.287 ml) were further added thereto, followed by stirring at 100°C for 24 hours. The reaction mixture was cooled to room temperature, and then filtered through Celite, and the filtrate was concentrated under reduced pressure. The residue was purified by silica gel column chromatography (eluent; hexane:EtOAc=2:1 (V/V)) to obtain tert-butyl (5-morpholin-4-ylpyridin-3-yl)carbamate (147 mg) as a colorless and oily substance.
To a solution of tert-butyl (5-morpholin-4-ylpyridin-3-yl)carbamate (147 mg) in dioxane (4.0 ml) was added a 4 M hydrochloride/dioxane solution (4.0 ml), followed by stirring overnight. The reaction mixture was concentrated under reduced pressure to obtain 5-morpholin-4-ylpyridin-3 amine dihydrochloride (132 mg) as a colorless amorphous substance.

### Reference Example 27

To a suspension of nicotinic acid choride hydrochloride (3.03 g) in acetonitrile (40 ml) were sequentially added sodium azide (2.76 g) and TEA (5.16 g), followed by stirring for 1 hour under ice-cooling. To the reaction mixture was added an aqueous hydrochloric acid solution, followed by extraction with EtOAc. The organic layer was washed with an aqueous sodium hydroxide solution and water, dried over anhydrous sodium sulfate, and then concentrated under reduced pressure to obtain a brown solid (2.08 g).
A solution of the obtained solid (2.08 g) in Tol (20 ml) was stirred at 110°C for 40 minutes. The reaction mixture was cooled to obtain a brown solution.
To a solution of tert-butylpiperazine-1-carboxylate hydrochloride (1.86 g) in THF (20 ml) was added the obtained brown solution under ice-cooling, followed by stirring at room temperature for 15 hours. To the reaction mixture was added an aqueous sodium hydroxide solution, followed by extraction with EtOAc. The organic layer was washed with water, dried over anhydrous sodium sulfate, and then concentrated under reduced pressure. The residue was purified by silica gel column chromatography (eluent; chloroform:methanol=23:2 (V/V)) to obtain a pale yellow oily substance (3.64 g).
To a solution of the obtained oily substance (3.62 g) in EtOAc (20 ml) was added a 4 M hydrochloride/EtOAc solution (19.9 ml), followed by stirring at room temperature for 5 hours. The precipitated solid was collected by filtration, and dried under heating to obtain a brown powder of N-pyridin-3-ylpiperidine-1-carboxamide dihydrochloride (2.57 g).

### Reference Example 28

To a suspension of 60% sodium hydride (4.62 g) in THF (200 ml) was added 2-amino pyrazine (10.0 g) under ice-cooling, followed by stirring at room temperature for 1 hour. To the reaction mixture was added diphenylcarbonate (22.5 g) under ice-cooling, followed by heating at 45°C overnight. To the reaction smixture was added a 1 M aqueous hydrochloric acid solution under ice-cooling, followed by extraction with EtOAc. The organic layer was washed with water, dried over anhydrous sodium sulfate, and then concentrated under reduced pressure. The obtained solid was washed with hexane to obtain a beige powder of phenylpyrazin-2-ylcarbamate (20.5 g).
To a solution of tert-butylpiperazine-1-carboxylate hydrochloride (10.0 g) in acetonitrile (200 ml) were added phenylpyrazin-2-ylcarbamate (11.6 g) and TEA (6.3 ml), followed by stirring at room temperature overnight. To the reaction mixture was added a saturated aqueous sodium hydrogen carbonate solution, followed by extraction with EtOAc. The organic layer was washed with water, dried over anhydrous sodium sulfate, and then concentrated under reduced pressure. The residue was purified by silica gel column chromatography (eluent; hexane:EtOAc=2:1 (V/V)) to obtain tert-butyl 4-(pyrazin-2-ylcarbamoyl)piperazine-1-carboxylate (12.0 g) as a white powder.
To a mixed solution of tert-butyl 4-(pyrazin-2-ylcarbamoyl)piperazine-1-carboxylate (12.0 g) in dioxane (30 ml)/methanol (10 ml) was added a 4 M hydrochloride/dioxane solution (48.8 ml), followed by stirring at room temperature for 3 hours. The precipitated solid was suspended in EtOAc, collected by filtration, and dried under heating to obtain a pale yellow powder of N-pyrazin-2-ylpiperazine-1-carboxamide dihydrochloride (9.50 g).

### Reference Example 29

To a suspension of 1-[3-(2-cyclohexylethoxy)benzoyl]piperazine hydrochloride (200 mg) in dichloromethane were added diisopropylethylamine (126 mg) and triphosgene (84 mg), followed by stirring at room temperature for 3 hours. Further, TEA (0.4 ml) and ammonium choride (152 mg) were added thereto, followed by stirring for 15 hours. To the reaction mixture was added water, and extracted with chloroform, and the organic layer was dried over anhydrous magnesium sulfate. The solution was concentrated under reduced pressure, and the residue was then purified by silica gel column chromatography (eluent; chloroform:methanol=10:1 (V/V)), and crystallized from EtOAc to obtain 4-[3-(2-cyclohexylethoxy)benzoyl]piperazine-1-carboxamide (117 mg) as a white solid.

### Reference Example 96

To a solution of N-2-pyrazinyl-1-piperazinecarboxamide dihydrochloride (210 mg), 5-hydroxy-1-benzothiophene-2-carboxylic acid (145 mg), WSC (172 mg), and HOBt (121 mg) in DMF (5 ml) was added TEA (0.31 ml), followed by stirring at room temperature overnight. To the reaction mixture was added water, followed by extraction with EtOAc. The organic layer was washed with a saturated aqueous sodium hydrogen carbonate solution, dried over anhydrous magnesium sulfate, and then concentrated under reduced pressure. The obtained solid was washed with hexane/EtOAc, and then dried under heating to obtain 4-(5-hydroxy-1-benzothiophene-2-yl)carbonyl]-N-pyrazin-2-ylpiperazine-1-carboxamide (216 mg) as a white solid.

### Reference Example 97

To a solution of 5-phenylthiophene-2-aldehyde (640 mg) and acetic acid (0.39 ml) in dichloromethane (13 ml) was added tert-butylpiperazine-1-carboxylate (696 mg), followed by stirring at room temperature for 1 hour. Further, NaBH(OAc)₃ (1.08 g) was added thereto, followed by stirring overnight. To the reaction mixture was added water, and extracted with chloroform. The organic layer was washed with a saturated aqueous sodium hydrogen carbonate solution, dried over anhydrous magnesium sulfate, and then concentrated under reduced pressure. The residue was dissolved in EtOAc (15 ml), and a 4 M hydrochloride/EtOAc solution (5 ml) was added thereto, followed by stirring overnight. The precipitated solid was collected by filtration, washed with EtOAc, and dried under heating to obtain 1-[(5-phenyl-2-thienyl)methyl]piperazine dihydrochloride (1.02 g) as a white solid.

### Reference Example 98

To a suspension of 60% sodium hydride (4.62 g) in THF (200 ml) was added amino pyrazine (10 g) under ice-cooling, followed by stirring at room temperature for 1 hour. Further, diphenyl carbonate (22.5 g) was added portionwise thereto under ice-cooling, followed by stirring at 45°C for 4 hours. To the reaction solution was added a 1 M aqueous hydrochloric acid solution (120 ml), followed by extraction with EtOAc. The organic layer was washed with a saturated aqueous sodium hydrogen carbonate solution, dried over anhydrous magnesium sulfate, and then concentrated under reduced pressure. The obtained solid was washed with hexane, and then dried under heating to obtain phenylpyrazin-2-ylcarbamate (12.4 g).

### Reference Example 99

To a solution of 2-amino-3-chloropyrazine (500 mg) in pyridine (5 ml) was added phenyl chloroformate (1.1 ml) under ice-cooling, followed by stirring at room temperature for 2 hours. To the reaction mixture was added toluene, and concentrated under reduced pressure. The residue was washed with water, and then dried under heating to obtain diphenyl(3-chloropyrazin-2-yl)imidedicarbonate (1.41 g) as a brown solid.

### Reference Example 100

To a solution of diphenyl(3-chloropyrazin-2-yl)imidedicarbonate (3.5 g) and tert-butylpiperazine-1-carboxylate (3.52 g)in DMF (35 ml) was added TEA (2.64 ml), followed by stirring at 80°C for 1 hour. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; hexane:EtOAc=1:1 (V/V)) to obtain tert-butyl 4-[(3-chloropyrazin-2-yl)carbamoyl]piperazine-1-carboxylate (2.28 g) as a white solid.

### Reference Example 101

To a solution of tert-butyl 4-[(3-chloropyrazin-2-yl)carbamoyl]piperazine-1-carboxylate (2.28 g) in isopropanol (30 ml) was added a 4 M hydrochloride/EtOAc (15 ml), followed by stirring at room temperature overnight. The reaction mixture was concentrated under reduced pressure, and the obtained solid was washed with isopropanol, and then dried under heating to obtain N-(3-chloropyrazin-2-yl)piperazine-1-carboxamide dihydrochloride (1.13 g).

### Reference Example 102

To a solution of 2-chloroisonicotinonitrile (1.00 g) in dimethoxyethane (20 ml) were added [3-(benzyloxy)phenyl]boronic acid (1.81 g), Pd (PPh₃)₄(417 mg), sodium carbonate (1.53 g), and water (10 ml), followed by stirring at 80°C for 3 hours. The reaction mixture was cooled to room temperature, followed by extraction with EtOAc. The organic layer was washed with saturated brine, dried over anhydrous magnesium sulfate, and then concentrated under reduced pressure. The residue was purified by silica gel column chromatography (eluent; hexane:EtOAc=6:1 (V/V)) to obtain 2-[3-(benzyloxy)phenyl]isonicotinonitrile (1.50 g) as a white powder.
To a solution of 2-[3-(benzyloxy)phenyl]isonicotinonitrile (1.15 g) in ethanol (23 ml) was added a 4 M aqueous sodium hydroxide solution (10 ml), followed by stirring at 80°C for 4 hours. The reaction mixture was cooled to room temperature, ethanol was then removed by distillation, and the residue was neutralized with an aqueous hydrochloric acid solution. The precipitated solid was collected by filtration, washed with water, and then dried under heating to obtain 2-[3-(benzyloxy)phenyl]isonicotinic acid (1.20 g) as a white powder.

### Reference Example 103

To a solution of 2-chloroisonicotinonitrile (1.00 g) in toluene (30 ml) were added 2,5-difluorobenzylamine (1.10 g), palladium acetate (81 mg), 2,2'-bis(diphenylphosphino)-1,1'-binaphthyl (224 mg), and sodium tert-butoxide (762 mg), followed by stirring at 50°C for 2 hours. The reaction mixture was cooled to room temperature, and then water was added thereto, followed by extraction with EtOAc. The organic layer was washed with saturated brine, dried over anhydrous magnesium sulfate, and then concentrated under reduced pressure. The residue was purified by silica gel column chromatography (eluent; hexane:EtOAc=6:1 (V/V)) to obtain 2-[(2,5-fluorobenzyl)amino]isonicotinonitrile (800 mg) as a white powder.
To a solution of 2-[(2,5-fluorobenzyl)amino]isonicotinonitrile (800 mg) in ethanol (16 ml) was added a 4 M aqueous sodium hydroxide solution (8 ml), followed by stirring at 80°C for 4 hours. The reaction mixture was cooled to room temperature, ethanol was then removed by distillation, and the residue was neutralized with an aqueous hydrochloric acid solution. The precipitated solid was collected by filtration, washed with water, and then dried under heating to obtain 2-[(2,5-difluorobenzyl)amino]isonicotinic acid (240 mg) as a pale yellow powder.

### Reference Example 104

To a solution of 3-(benzyloxy)phenol (5.00 g), tert-butyl 4-hydroxypiperidine-1-carboxylate (7.53 g) and PPh₃ (9.82 g) in THF (250 ml) was added DEAD (17 ml, 40% Tol solution) under ice-cooling, followed by stirring at room temperature for 48 hours. The reaction mixture was concentrated under reduced pressure, added with EtOAc, washed with a 1 M aqueous sodium hydroxide solution and water in this order, and dried over anhydrous magnesium sulfate. The solution was concentrated under reduced pressure, and the residue was then purified by silica gel column chromatography (eluent; hexane:EtOAc=4:1 (V/V)) to obtain tert-butyl 4-[3-(benzyloxy)phenoxy]piperidine-1-carboxylate (7.67 g) as a colorless and oily substance.

### Reference Example 105

To a solution of tert-butyl 4-[3-(benzyloxy)phenoxy]piperidine-1-carboxylate (4.67 g) in ethanol was added 10% palladium carbon (500 mg), followed by stirring at room temperature overnight under a hydrogen atmosphere. The catalyst was removed by filtration through Celite, and the filtrate was concentrated under reduced pressure. The obtained solid was washed with hexane/EtOAc, and then dried under heating to obtain tert-butyl 4-(3-hydroxyphenoxy)piperidine-1-carboxylate (3.08 g) as a white solid.

### Reference Example 106

To a solution of tert-butyl 4-(3-hydroxyphenoxy)piperidine-1-carboxylate (400 mg), 2-cyclohexylethanol (262 mg), and tributylphosphine (413 mg) in THF (15 ml) was added 1,1'-(azodicarbonyl)dipiperidine (516 mg) under ice-cooling, followed by stirring at room temperature overnight. The reaction mixture was concentrated under reduced pressure, added with EtOAc, washed with a 1 M aqueous sodium hydroxide solution, dried over anhydrous magnesium sulfate, and then concentrated under reduced pressure. The residue was purified by silica gel column chromatography (eluent; hexane:EtOAc=7: 3 (V/V)) to obtain an oily substance. The obtained oily substance was dissolved in a mixed solvent of isopropanol/EtOAc, and a 4 M hydrochloride/EtOAc was added thereto, followed by stirring at room temperature overnight. The reaction mixture was concentrated under reduced pressure, and the obtained solid was washed with hexane/EtOAc, and then dried under heating to obtain 4-[3-(2-cyclohexylethoxy)phenoxy]piperidine hydrochloride (252 mg) as a white solid.

### Reference Example 107

A solution of 4-[3-(benzyloxy)phenoxy]piperidine hydrochloride (2.16 g), phenylpyrazin-2-ylcarbamate (1.45 g), and TEA (1.88 ml) in acetonitrile (43 ml) was stirred at 80°C for 1 hour. The reaction mixture was concentrated under reduced pressure, added with EtOAc, washed with a saturated aqueous sodium hydrogen carbonate solution, dried over anhydrous magnesium sulfate, and then concentrated under reduced pressure. The residue was purified by silica gel column chromatography (eluent; hexane:EtOAc=1:1 (V/V)) to obtain an oily substance. The obtained oily substance was crystallized from hexane/EtOAc, and dried under heating to obtain 4-[3-(benzyloxy)phenoxy]-N-pyrazin-2-ylpiperazine-1-carboxamide (2.3 g) as a white solid.

### Reference Example 108

To a solution of 4-[3-(benzyloxy)phenoxy]-N-pyrazin-2-ylpiperazine-1-carboxamide (2.2 g) in ethanol (50 ml) was added 10% palladium carbon (200 mg), followed by stirring at room temperature overnight under a hydrogen atmosphere. The catalyst was removed by filtration through Celite, and the filtrate was then concentrated under reduced pressure. The residue was crystallized from hexane/EtOAc, and dried under heating to obtain 4-(3-hydroxyphenoxy)-N-pyrazin-2-ylpiperidine-1-carboxamide (1.67 g) as a white solid.

### Reference Example 109

To a solution of 2-chloro-4-nitropyridine (1 g) and tert-butyl 4-hydroxypiperidine-1-carboxylate (1.40 g) in DMF (30 ml) was added 60% sodium hydride (277 mg) under ice-cooling, followed by stirring at room temperature for 2 hours. To the reaction mixture was added water, and the precipitated solid was washed with water and hexane in this order, and dried under heating to obtain tert-butyl-4-[(2-chloropyridin-4-yl)oxy]piperidine-1-carboxylate (1.56 g) as a white solid.

### Reference Example 136

To a solution of 4-[(3-fluorobenzyl)oxy]benzaldehyde (1.40 g) in THF (20 ml) were sequentially added t-butylpiperazine-1-carboxylate (1.23 g), acetic acid (0.343 ml), and NaBH(OAc)₃ (1.53 g) under ice-cooling, followed by stirring at room temperature for 19 hours. The reaction mixture was alkalified by addition of a 1 M aqueous sodium hydroxide solution under ice-cooling, followed by extraction with chloroform. The organic layer was dried over anhydrous sodium sulfate, and then concentrated under reduced pressure. The obtained residue was purified by silica gel column chromatography (eluent; chloroform:methanol=96: 4(V/V)) to obtain t-butyl 4-{4-[(3-fluorobenzyl)oxy]benzyl}piperazine-1-carboxylate (2.41 g) as a pale yellow oily substance.
To a solution of t-butyl 4-{4-[(3-5uorobenzyl)oxy]benzyl}piperazine-1-carboxylate (2.39 g) in methanol (10 ml) was added a 4 M hydrochloride/EtOAc solution (11.9 ml) under ice-cooling, followed by stirring at room temperature for 5 hours. The reaction mixture was concentrated under reduced pressure, and the obtained residue was suspended in acetonitrile, and collected by filtration, to obtain 1-{4-[(3-fluorobenzyl)oxy]benzyl}piperazine dihydrochloride (1.71 g) as a colorless solid.

The compounds of Reference Examples 1 to 137 were prepared in the same manner as the methods of Reference Examples 1 to 29, 96 to 109, and 136, using each corresponding starting materials as shown in the following Tables 3 to 17. The structures, the production processes, and the physicochemical data of the compounds of Reference Examples are shown in Tables 3 to 17.

### Example 1

Nicotinic acid chloride hydrochloride (356 mg) was suspended in acetonitrile (10 ml), and sodium azide (325 mg) and TEA (607 mg) were added thereto under ice-cooling, followed by stirring for 1 hour under ice-cooling. To the reaction mixture was added with water, followed by extraction with EtOAc. The organic layer was dried over anhydrous sodium sulfate, and then concentrated under reduced pressure, and the obtained residue was dissolved in Tol (10 ml), followed by stirring at 110°C for 30 minutes. The reaction mixture was cooled, and then used for the next reaction.
To a solution of 4-{4-[(3-fluorobenzyl)oxy]phenoxy}piperidine (301 mg) in THF (5 ml) was added the above-described Tol solution under ice-cooling, followed by stirring at room temperature for 3 hours. To the reaction mixture was added an aqueous sodium hydroxide solution, followed by extraction with EtOAc, and the organic layer was dried over anhydrous sodium sulfate, and then concentrated under reduced pressure. The residue was purified by silica gel column chromatography (eluent; chloroform:methanol=24:1 (V/V)). The obtained oily substance was dissolved in EtOAc (5 ml), added with a 4 M hydrochloride/EtOAc solution (0.30 ml) under ice-cooling, and then concentrated under reduced pressure. The obtained residue was recrystallized from a mixed solvent of acetonitrile/methanol to obtain 4-{4-[(3-fluorobenzyl)oxy]phenoxy}-N-(pyridin-3-yl)piperidine-1-carboxamide hydrochloride (325 mg).

### Example 2

To a solution of 5-aminopyridine-2-carbonitrile (1.0 g) in pyridine (10 ml) was added phenyl chloroformate (1.05 ml), followed by stirring at room temperature for 2 hours. The reaction mixture was concentrated under reduced pressure, and the obtained residue was then washed with a saturated aqueous sodium hydrogen carbonate solution and acetonitrile in this order, and then dried under heating to obtain phenyl(6-cyanopyridin-3-yl)carbamate (1.01 g) as a white solid.
To a solution of 4-{4-[(3-fluorobenzyl)oxy]phenoxy}piperidine (594 mg) and TEA (0.30 ml) in DMF (10 ml) was added phenyl(6-cyanopyridin-3-yl)carbamate (519 mg), followed by stirring at 100°C for 2 hours. The reaction mixture was diluted with EtOAc, washed with a saturated aqueous sodium hydrogen carbonate solution. The solution was dried over anhydrous magnesium sulfate, and then concentrated under reduced pressure, and the residue was purified by silica gel column chromatography (eluent; chloroform:methanol=95: 5(V/V)). The obtained solid was washed with hexane/EtOAc to obtain N-(6-cyanopyridin-3-yl)-4-{4-[(3-fluorobenzyl)oxy]phenoxy}piperidine-1-carboxamide (530 mg) as a colorless powder.

### Example 3

To a solution of 5-bromonicotinic acid (1.21 g) and TEA (1 ml) in Tol (20 ml) was added diphenylphosphorylazide (1.82 g) under ice-cooling, followed by stirring at room temperature for 2 days. The reaction mixture was diluted with EtOAc, washed with a saturated aqueous sodium hydrogen carbonate solution and saturated brine in this order, dried over anhydrous magnesium sulfate, and then concentrated under reduced pressure. The obtained residue was dissolved in Tol (30 ml), followed by stirring at 110°C for 2 hours. To the reaction mixture was added a solution of 4-[4-(3-fluorobenzyloxy)phenoxy]piperidine hydrochloride (1.5 g) in THF (30 ml) under ice-cooling, followed by stirring at room temperature for 5 hours. The reaction mixture was diluted with EtOAc, washed with a 1 M aqueous sodium hydroxide solution and saturated brine, dried over anhydrous magnesium sulfate, and then concentrated under reduced pressure. The obtained residue was recrystallized from EtOAc/acetonitrile to obtain N-(5-bromopyridin-3-yl)-4-{4-[(3-fluorobenzyl)oxy]phenoxy}piperidine-1-carboxamide (1.73 g) as a white solid.

### Example 4

A suspension of N-(5-bromopyridin-3-yl)-4-{4-[(3-fluorobenzyl)oxy]phenoxy}piperidine-1-carboxamide (720 mg), dicyanozinc (101 mg), tris(dibenzylidene acetone)dipalladium (7.0 mg) and 1,1'-bis(diphenylphosphino)ferrocene (10 mg) in DMF was stirred at 120°C for 15 hours. The reaction mixture was diluted with EtOAc, and washed with a saturated aqueous sodium hydrogen carbonate solution and saturated brine. The solution was dried over anhydrous sodium sulfate, and then concentrated under reduced pressure, and the residue was purified by silica gel column chromatography (eluent; hexane:EtOAc=1:1 to 1:9(V/V)) to obtain N-(5-cyanopyridin-3-yl)-4-{4-[(3-fluorobenzyl)oxy]phenoxy}piperidine-1-carboxamide (500 mg).
To a solution of N-(5-cyanopyridin-3-yl)-4-{4-[(3-fluorobenzyl)oxy]phenoxy}piperidine-1-carboxamide (500 mg) in ethanol was added a 10 M aqueous sodium hydroxide solution (0.67 ml), followed by stirring at 80°C for 15 hours. The reaction mixture was concentrated under reduced pressure, to the residue were then added water (20 ml) and methanol (5 ml), and the insoluble material was removed by filtration. To the filtrate was added a 1 M aqueous hydrochloric acid solution under ice-cooling, and the precipitated crystal was taken by filtration, and then dried under heating to obtain 5-{[(4-{4-[(3-fluorobenzyl)oxy]phenoxy}piperidin-1-yl)carbonyl]amino}nicotinic acid (129 mg) as a white solid.

### Example 5

To a solution of methyl 4-aminobenzoate (2.0 g) in pyridine was added phenyl chlorofomate under ice-cooling, followed by stirring at room temperature for 3 hours. The reaction mixture was concentrated under reduced pressure, EtOAc was added thereto, and the organic layer was washed with water, a saturated aqueous sodium hydrogen carbonate solution and a saturated sodium chloride solution in this order. The solution was dried over anhydrous magnesium sulfate, and then concentrated under reduced pressure to obtain methyl 4-[(phenoxycarbonyl)amino]benzoate (4.23 g) as a white solid.
A solution of methyl 4-[(phenoxycarbonyl)amino]benzoate (1.0 g), 4-[4-(benzyloxy)phenoxy]piperidine hydrochloride (1.2 g) and TEA (0.96 g) in acetonitrile was stirred at 60°C for 3 hours. The reaction mixture was concentrated under reduced pressure, and the residue was purified by silica gel column chromatography (eluent; hexane:EtOAc=2:1 to 1:1 (V/V)) to obtain methyl 4-[({4-[4-(benzyloxy)phenoxy]piperidin-1-yl}carbonyl)amino]benzoate (1.25 g) as a white solid.
To a mixed solution of methyl 4-[({4-[4-(benzyloxy)phenoxy]piperidin-1-yl}carbonyl)amino]benzoate (800 mg) in THF (16 ml)/methanol (8 ml) was added a 1 M aqueous sodium hydroxide solution, followed by stirring at 70°C for 15 hours. The reaction mixture was concentrated under reduced pressure, water was then added to the residue for dissolution, and acidified by addition of a 1 M aqueous hydrochloric acid solution. The precipitated crystal was taken by filtration, and then dried under heating to obtain 4-[({4-[4-(benzyloxy)phenoxy]piperidin-1-yl}carbonyl)amino]benzoic acid (690 mg) as a white solid.

### Example 6

To a solution of 3-[({4-[4-(benzyloxy)phenoxy]piperidin-1-yl}carbonyl)amino]benzoic acid (103 mg) in DMF (2.0 ml) were sequentially added WSC (53 mg), HOBt (46 mg), and piperidine (29 mg), followed by stirring at room temperature for 12 hours. To the reaction mixture was added a saturated aqueous sodium hydrogen carbonate solution, followed by extraction with EtOAc, and the organic layer was washed with saturated brine, and then dried over anhydrous sodium sulfate. The solution was concentrated under reduced pressure, and the residue was then purified by silica gel column chromatography (eluent; hexane:EtOAc=1:1 (V/V)). The residue was crystallized from diisopropylether, and dried under heating to obtain 4-[4-(benzyloxy)phenoxy]-N-[3-(piperidin-1-ylcarbonyl)phenyl]piperidine-1-carboxamide (63 mg).

### Example 7

To a solution of 4-{4-[(3-fluorobenzyl)oxy]phenoxy}piperazine (400 mg) in THF (8 ml) was added ethyl 3-isocyanatebenzoate (305 mg), followed by stirring at room temperature for 24 hours. The reaction mixture was concentrated under reduced pressure, diluted with chloroform, and then washed with a saturated aqueous sodium hydrogen carbonate solution. The solution was dried over anhydrous magnesium sulfate, and then concentrated under reduced pressure, and the residue was purified by silica gel column chromatography (eluent; hexane:EtOAc=2:1 (V/V)). The obtained solid was recrystallized from hexane/EtOAc to obtain ethyl 3-{[(4-{4-[(3-fluorobenzyl)oxy]phenoxy}pipendin-1-yl)carbonyl]amino}benzoate (562 mg) as a colorless powder.

### Example 8

To a mixed solution of ethyl 3-{[(4-{4-[(3-fluorobenzyl)oxy]phenoxy}piperidin-1-yl)carbonyl]amino}benzoate (400 mg) in THF (5 ml)/methanol (3 ml) was added a 1 M aqueous sodium hydroxide solution (3 ml), followed by stirring at room temperature for 10 hours. To the reaction mixture was added a I M aqueous hydrochloric acid solution (3 ml), and the precipitated solid was collected by filtration, washed with water, and then dried under heating to obtain 3-{[(4-{4-[(3-fluorobenzyl)oxy]phenoxy}piperidin-1-yl)carbonyl]amino}benzoic acid (361 mg) as a colorless powder.

### Example 9

To a solution of 3-{[(4-{4-[(3-fluorobenzyl)oxy]phenoxy}piperidin-1-yl)carbonyl]amino}benzoic acid (253 mg), and HOBt (88 mg) in DMF (5 ml) was added WSC (124 mg), followed by stirring at room temperature for 2 hours. To the reaction mixture was added concentrated aqueous ammonia (0.5 ml), followed by stirring at room temperature for 1 hour. To the reaction mixture was added water, and the precipitated solid was collected by filtration, and recrystallized from ethanol to obtain N-(3-carbamoylphenyl)-4-{4-[(3-fluorobenzyl)oxy]phenoxy}piperidine-1-carboxamide (163 mg) as a colorless powder.

### Example 10

To a suspension of N-(6-cyanopyridin-3-yl)-4-{4-[(3-fluorobenzyl)oxy]phenoxy}piperidine-1-carboxamide (285 mg) and potassium carbonate (88 mg) in DMSO(3 ml) was added a 31% aqueous hydrogen peroxide (0.3 ml) under ice-cooling, followed by stirring at room temperature for 1 hour. To the reaction mixture was added water, and the precipitated solid was collected by filtration. The solid was washed with water, and then dried under heating to obtain 5-{[(4-{4-[(3-fluorobenzyl)oxy]phenoxy}piperidin-1-yl)carbonyl]amino}pyridine-2-carboxamide (272 mg) as a colorless powder.

### Example 11

To a solution of 3-{[(4-{4-[(3-fluorobenzyl)oxy]phenoxy}piperidin-1-yl)carbonyl]amino}phenyl acetate (1.0 g) in methanol (10 ml) was added a 1 M aqueous sodium hydroxide solution (5 ml), followed by stirring at room temperature for 4 hours. AIM aqueous hydrochloric acid solution (5 ml) was added thereto, and the precipitated solid was collected by filtration, washed with water, and then dried under heating to obtain 4-{4-[(3-fluorobenzyl)oxy]phenoxy}-N-(3-hydroxyphenyl)piperidine-1-carboxamide (0.85 g) as a colorless powder.

### Example 12

To a solution of 4-{4-[(3-fluorobenzyl)oxy]phenoxy}piperazine (500 mg) in dichloromethane (5 ml) was added CDI (269 mg) under ice-cooling, followed by stirring at room temperature overnight. The reaction mixture was concentrated, diluted with chloroform, and then washed with water. The solution was concentrated under reduced pressure, and the obtained residue was then crystallized from hexane/EtOAc and recrystallized from hexane/EtOAc to obtain 4-{4-[(3-fluorobenzyl)oxy]phenoxy}-1-(1H-imidazol-1-ylcarbonyl)piperidine (370 mg) as a colorless powder.

### Example 13

To a solution of 4-{4-[(3-fluorobenzyl)oxy]phenoxy}-N-pyridin-3-ylpiperidine-1-carboxamide in chloroform (8 ml) was added m-chloroperbenzoic acid (281 mg), followed by stirring at room temperature for 2 hours. The reaction mixture was diluted with chloroform, washed with a saturated aqueous sodium hydrogen carbonate solution, and then dried over anhydrous magnesium sulfate. The solution was concentrated under reduced pressure, and the obtained solid was then washed with hexane/EtOAc, and recrystallized from ethanol/EtOAc to obtain 4-{4-[(3-fluorobenzyl)oxy]phenoxy}-N-(1-oxidepyridin-3-yl)piperidine-1-carboxamide (185 mg) as a colorless powder.

### Example 14

To a solution of 1H-pyrazol-3-amine (2.0 g) and TEA (3.7 ml) in THF (40 ml) was added phenyl chloroformate (3.3 ml), followed by stirring at room temperature overnight. The reaction mixture was concentrated under reduced pressure, and the obtained residue was then diluted with EtOAc, and washed with a saturated aqueous sodium hydrogen carbonate solution. The solution was dried over anhydrous magnesium sulfate, and then concentrated under reduced pressure, and the residue was purified by silica gel column chromatography (eluent; hexane:EtOAc=5:1 to 0:1 (V/V)) to obtain a white solid (2.1 g).
To a solution of triphosgene (118 mg) in THF (5 ml) was added dropwise a solution of the solid obtained as above (203 ml) and TEA (0.20 ml) in THF (3 ml) under ice-cooling, followed by stirring at room temperature for 30 minutes. To the reaction mixture was added dropwise a solution of 4-{4-[(3-fluorobenzyl)oxy]phenoxy}piperazine (250 mg) and TEA (0.14 ml) in THF (2 ml) under ice-cooling, followed by stirring at room temperature for 2 hours. The reaction mixture was concentrated under reduced pressure, diluted with EtOAc, and then washed with a saturated aqueous sodium hydrogen carbonate solution. The solution was dried over anhydrous magnesium sulfate, and then concentrated under reduced pressure, and the residue was purified by silica gel column chromatography (eluent; hexane:EtOAc=5:1 (V/V)) to obtain a solid. The obtained solid was washed with hexane/EtOAc, and then dried under heating to obtain a white solid (318 mg).
To a solution of the solid obtained as above (310 mg) in acetonitrile (5 ml) was added lithium hydroxide monohydrate (245 mg), followed by stirring at 50°C for 3 hours. To the reaction mixture was added water, and the precipitated solid was washed with water, and then recrystallized from EtOAc to obtain 4-{4-[(3-fluorobenzyl)oxy]phenoxy}-N-1H-pyrazol-3-ylpiperidine-1-carboxamide (168 mg) as a colorless powder.

### Example 15

To a solution of 3-[({4-[4-(benzyloxy)phenoxy]piperidin-1-yl}carbonyl)amino]benzoic acid (300 mg) in DMF (6.7 ml) were sequentially added WSC (193 mg), HOBt (136 mg), N-methyl-1,2-benzene diamine (410 mg), and TEA (340 mg), followed by stirring at room temperature for 12 hours. To the reaction mixtue was added water and a saturated aqueous sodium hydrogen carbonate solution, followed by extraction with EtOAc, and the organic layer was washed with saturated brine, and then dried over anhydrous sodium sulfate. The solution was concentrated under reduced pressure, and the residue was then purified by silica gel column chromatography (eluent; hexane:EtOAc=1:2 (V/V)) to obtain 4-[4-(benzyloxy)phenoxy]-N-(3-{[2-(methylamino)phenyl]carbamoyl}phenyl)piperidine-1-carboxamide (234 mg).
A solution of 4-[4-(benzyloxy)phenoxy]-N-(3-{[2-(methylamino)phenyl]carbamoyl}phenyl)piperidine-1-carboxamide (230 mg) in acetic acid (6.0 ml) solution was stirred at 80°C for 3 hours, and then concentrated under reduced pressure. The residue was purified by basic silica gel column chromatography (eluent; hexane:EtOAc=1:2 (V/V)), crystallized from diisopropylether, and then dried under heating to obtain 4-[4-(benzyloxy)phenoxy]-N-[3-(1-methyl-1H-benzimidazole-2-yl)phenyl]piperidine-1-carboxamide (100 mg).

### Example 16

To a solution of 3-(benzyloxy)benzoic acid (3.50 g), N-pyridin-3-ylpiperazine-1-carboxamide dihydrochloride (4.29 g), WSC (4.41 g), and HOBt (3.11 g) in DMF (100 ml) was added dropwise TEA (6.5 ml), followed by stirring at room temperature overnight. To the reaction mixture was added water (100 ml), followed by extraction with EtOAc. The organic layer was washed with a saturated aqueous sodium hydrogen carbonate solution and saturated brine in this order, dried over anhydrous magnesium sulfate, and concentrated under reduced pressure. The residue was purified by silica gel column chromatography (eluent; chloroform:methanol=20:1 (V/V)) to obtain 4-[3-(benzyloxy)benzoyl]-N-pyridin-3-ylpiperazine-1-carboxamide (5.80 g) as a colorless and oily substance. The obtained oily substance (230 mg) was dissolved in ethanol (10 ml), and oxalic acid (99 mg) was added thereto to obtain 4-[3-(benzyloxy)benzoyl]-N-pyridin-3-ylpiperazine-1-carboxamide oxalate (202 mg).

### Example 17

To a solution of methyl 5-(cyclohexylmethoxy)nicotinate (303 mg) in methanol (5 ml) was added a 1 M aqueous sodium hydroxide solution (1.56 ml), followed by stirring at room temperature for 2 hours. The reaction mixture was acidified by addition with a 1 M aqueous hydrochloric acid solution, and then concentrated under reduced pressure, and the residue was used for the next reaction.
To a solution of N-pyridin-3-ylpiperazine-1-carboxamide dihydrochloride (223 mg) in DMF (5 ml) were sequentially added TEA (162 mg), a solution of thus obtained residue in DMF (3 ml), HOBt (108 mg), and WSC (230 mg), followed by stirring at room temperature for 4 hours. To the reaction mixture was added an aqueous sodium hydroxide solution, followed by extraction with chloroform. The organic layer was dried over anhydrous sodium sulfate, and then concentrated under reduced pressure, and the residue was purified by silica gel column chromatography (eluent; chloroform:methanol=22:3 (V/V)) to obtain a pale yellow oily substance (332 mg). To a solution of the obtained oily substance of methanol (5 ml) was added oxalic acid (141 mg), and then crystallized by addition of acetonitrile to obtain a pale yellow powder of 4- {[5-(cyclohexylmethoxy)pyridin-3-yl]carbonyl}-N-pyridin-3-ylpiperazine-1-carboxamide oxalate (351 mg).

### Example 18

To a solution of N-pyridin-3-ylpiperidine-1-carboxamide dihydrochloride (280 mg) in DMF (15 ml) were added TEA (0.42 ml) and 4-pentylbenzene-1-sulfonyl chloride (370 mg), followed by stirring at room temperature overnight. To the reaction mixture was added with water, followed by extraction with EtOAc. The organic layer was washed with water, dried over anhydrous sodium sulfate, and then concentrated under reduced pressure. The residue was crystallized from hexane/EtOAc to obtain a pale orange powder of 4-[(4-pentylphenyl)sulfonyl]-N-pyridin-3-ylpiperidine-1-carboxamide (244 mg).

### Example 19

To a solution of 3,5-bis(acetyloxy)benzoic acid (202 mg) and TEA (103 mg) in Tol (5 ml) was added diphenylphosphorylazide (257 mg), followed by stirring for 3 hours. To the reaction mixture was added water, followed by extraction with EtOAc, and the organic layer was washed with saturated brine, and then dried over anhydrous magnesium sulfate. The solution was concentrated under reduced pressure, and the obtained residue was dissolved in Tol, followed by stirring at 100°C for 4 hours. The reaction mixture was cooled to room temperature, and added to a solution of 1-[3-(2-cyclohexylethoxy)benzoyl]piperazine hydrochloride (200 mg) and TEA (58 mg) in acetonitrile (10 ml), followed by stirring for 2 hours. The reaction mixture was diluted with EtOAc, and washed with water and saturated brine, and dried over anhydrous magnesium sulfate. The solution was concentrated under reduced pressure, and the residue was then purified by silica gel column chromatography (eluent; hexane: EtOAc=1:1 to 1:2 (V/V)) to obtain 5-[({4-[3-(2-cyclohexylethoxy)benzoyl]piperazin-1-yl}carbonyl)amino]-1,3-phenylene diacetate (260 mg) as an amorphous substance.
To a solution of 5-[({4-[3-(2-cyclohexylethoxy)benzoyl]piperazin-1-yl}carbonyl)amino]-1,3-phenylene diacetate (260 mg) in isopropanol (5 ml) was added a 1 M aqueous sodium hydroxide solution (2.4 ml), followed by stirring for 3 hours. The reaction mixtue was acidified by addition of a 1 M aqueous hydrochloric acid solution, followed by extraction with EtOAc. The organic layer was washed with saturated brine, dried over anhydrous magnesium sulfate, and then concentrated under reduced pressure. The residue was purified by silica gel column chromatography (eluent; chloroform:methanol=10:0 to 10:1 (V/V)), and then purified by silica gel column chromatography (eluent; hexane:EtOAc=2:1 to 1:2 (V/V)) to obtain 4-[3-(2-cyclohexylethoxy)benzoyl]-N-(3,5-dihydroxyphenyl)piperazine-1-carboxamide (54 mg) as a white solid.

### Example 20

A solution (20 ml) of methyl 5-(azidecarbonyl)nicotinate (1.2 g) in Tol was stirred at 120°C for 1 hour. This reaction mixture was added to a solution of 1-[3-(2-cyclohexylethoxy)benzoyl]piperazine hydrochloride (700 mg) and TEA (241 mg) in THF (20 ml) under ice-cooling, followed by stirring at room temperature for 10 hours. The reaction mixture was diluted with EtOAc, and then washed with water, a saturated aqueous sodium hydrogen carbonate solution and saturated brine in this order, dried over anhydrous magnesium sulfate, and concentrated under reduced pressure. The residue was purified by silica gel column chromatography (eluent; chloroform:methanol=10:0 to 5:1 (V/V)) to obtain methyl 5-[({4-[3-(2-cyclohexylethoxy)benzoyl]piperazin-1-yl}carbonyl)amino]nicotinate (1.0 g) as an amorphous substance.
To a mixed solution of methyl 5-[({4-[3-(2-cyclohexylethoxy)benzoyl]piperazin-1-yl}carbonyl)amino]nicotinate (100 mg) in THF (5 ml)/ethanol (1 ml) was added lithium borohydride (44 mg), followed by stirring for 3 hours. To the reaction mixture was added a saturated aqueous sodium hydrogen carbonate solution, followed by stirring for 10 minutes. The reaction mixture was extracted with EtOAc, and the organic layer was then washed with saturated brine, dried over anhydrous magnesium sulfate, and concentrated under reduced pressure. The residue was purified by silica gel column chromatography (eluent; chloroform:methanol=5.1 (V/V)) to obtain 4-[3-(2-cyclohexylethoxy)benzoyl]-N-[5-(hydroxymethyl)pyridin-3-yl]piperazine-1-carboxamide (38 mg) as an amorphous substance.
To a solution of 4-[3-(2-cyclohexylethoxy)benzoyl]-N-[5-(hydroxymethyl)pyridin-3-yl]piperazine-1-carboxamide (38 mg) in EtOAc (2.0 ml) was added a 4 M hydrochloride/EtOAc solution (0.1 ml), followed by stirring at room temperature for 3 hours. The reaction mixture was concentrated under reduced pressure to obtain 4-[3-(2-cyclohexylethoxy)benzoyl]-N-[5-(hydroxymethyl)pyridin-3-yl]piperazine-1-carboxamide hydrochloride (30 mg) as an amorphous substance.

### Example 21

To a solution of 5-bromonicotinic acid (20.0 g) in Tol (300 ml) was added TEA (12.0 g), and further diphenylphosphorylazide (30.0 g) was added thereto under ice-cooling. The reaction mixture was stirred at room temperature for 3 hours, and the reaction mixture was then diluted with EtOAc, and washed with a saturated aqueous sodium hydrogen carbonate solution and saturated brine in this order. The organic layer was dried over anhydrous magnesium sulfate, and then concentrated under reduced pressure to obtain 5-bromonicotinoylazide (26.5 g) as a white solid.
A solution of 5-bromonicotinoylazide (2.95 g) in Tol (30 ml) was stirred at 110°C for 1 hour. To the reaction mixture was added a solution of 1-[3-(2-cyclohexylethoxy)benzoyl]piperazine hydrochloride (3.0 g) and TEA (1.72 g) in THF (50 ml) under ice-cooling, followed by stirring at room temperature for 3 hours. The reaction mixture was concentrated under reduced pressure, and the residue was then purified by silica gel column chromatography (eluent; chloroform:methanol=10:0 to 10:1 (V/V)) to obtain N-(5-bromopyridin-3-yl)-4-[3-(2-cyclohexylethoxy)benzoyl]piperazine-1-carboxamide (3.57 g) as an amorphous substance.
To a suspension of N-(5-bromopyridin-3-yl)-4-[3-(2-cyclohexylethoxy)benzoyl]piperazine-1-carboxamide (150 mg), (3-aminocarbonylphenyl)boronic acid (96 mg), and Pd(PPh₃)₄ (67 mg) in Tol (7.5 ml) was added a 2 M aqueous sodium carbonate solution (0.44 ml), followed by stirring at 100°C for 3 hours. To the reaction mixture was added a saturated aqueous sodium hydrogen carbonate solution, followed by extraction with EtOAc. The organic layer was washed with saturated brine, dried over anhydrous magnesium sulfate, and then concentrated under reduced pressure. The residue was purified by silica gel column chromatography (eluent; chloroform:methanol=20:1 (V/V)) to obtain N-[5-(3-carbamoylphenyl)pyridin-3-yl]-4-[3-(2-cyclohexylethoxy)benzoyl]piperazine-1-carboxamide (66 mg) as a white solid.

### Example 22

To a solution of methyl 5-hydroxynicotinate (5.0 g) in acetone (150 ml) were added potassium carbonate (13.5 g) and benzyl bromide (9.71 ml), followed by heating under reflux for 5 hours. The reaction mixture was cooled to room temperature, the insoluble material was then removed by filtration, and the filtrate was concentrated under reduced pressure. The residue was purified by silica gel column chromatography (eluent; EtOAc: methanol=10:1 (V/V)) to obtain methyl 5-benzyloxynicotinate (1.70 g) as a pale yellow crystal.
To a mixed solution of methyl 5-benzyloxynicotinate (1.70 g) in methanol (40 ml)/THF (10 ml) was added a 1 M aqueous sodium hydroxide solution (7.34 ml) under ice-cooling, followed by stirring at room temperature overnight. The reaction mixture was concentrated under reduced pressure, and the precipitated crystal was then collected by filtration, and washed with EtOAc to obtain sodium 5-benzyloxynicotinate (1.66 g).
To a solution of sodium 5-benzyloxynicotinate (1.66 g) in DMF (26 ml) was added TEA (1.10 ml) at room temperature, and further diphenylphosphorylazide (1.56 ml) was added thereto under ice-cooling, followed by stirring at room temperature for 5 hours. To the reaction mixture was added water, followed by extraction with EtOAc, and the organic layer was washed with a saturated aqueous sodium hydrogen carbonate solution and saturated brine in this order, dried over anhydrous sodium sulfate, and then concentrated under reduced pressure to obtain 5-(benzyloxy)nicotinoylazide.
A solution of 5-(benzyloxy)nicotinoylazide (1.68 g) in Tol (20 ml) was stirred at 120°C for 2 hours. This solution was added to a solution of 1-[3-(2-cyclohexylethoxy)benzoyl]piperazine hydrochloride (1.55 g) and TEA (1.23 ml) in THF (20 ml) under ice-cooling, followed by stirring for 2 hours, and further stirring at room temperature for 10 hours. To the reaction mixture was added water, followed by extraction with EtOAc, and the organic layer was washed with a saturated aqueous sodium hydrogen carbonate solution, and saturated brine in this order, and then dried over anhydrous sodium sulfate. The solution was concentrated under reduced pressure, and the residue was then purified by silica gel column chromatography (eluent; hexane:EtOAc=2:1 (V/V)) to obtain N-[5-(benzyloxy)pyridin-3-yl]-4-[3-(2-cyclohexylethoxy)benzoyl]piperazine-1-carboxamide (1.78 g) as a pale yellow amorphous substance.
To a solution of N-[5-(benzyloxy)pyridin-3-yl]-4-[3-(2-cyclohexylethoxy)benzoyl]piperazine-1-carboxamide (1.78 g) in methanol (20 ml) was added 5% palladium carbon (698 mg), followed by stirring at room temperature overnight under a hydrogen atmosphere. The catalyst was removed by filtration through Celite, and the filtrate was concentrated under reduced pressure. The residue was purified by silica gel column chromatography (eluent; chloroform:methanol=10:1 (V/V)) to obtain 4-[3-(2-cyclohexylethoxy)benzoyl]-N-(5-hydroxypyridin-3-yl)piperazine-1-carboxamide (1.15 g) as a pale yellow amorphous substance.

### Example 23

To a solution of 4-[3-(2-cyclohexylethoxy)benzoyl]-N-(5-hydroxypyridin-3-yl)piperazine-1-carboxamide (200 mg), 2-(benzyloxy)ethanol (0.126 ml) and PPh₃ (232 mg) in THF (4.0 ml) was added DEAD (203 mg, 40% Tol solution) under ice-cooling, followed by stirring at room temperature overnight. To the reaction mixture was added water, followed by extraction with EtOAc, and the organic layer was washed with saturated brine, and then dried over anhydrous sodium sulfate. The solution was concentrated under reduced pressure, and the residue was then purified by silica gel column chromatography (eluent; chloroform:methanol=10:1 (V/V)) to obtain N-{5-[2-(benzyloxy)ethoxy]pyridin-3-yl}-4-[3-(2-cyclohexylethoxy)benzoyl]piperazine-1-carboxamide (94 mg) as a brown oily substance.
To a solution of N-{5-[2-(benzyloxy)ethoxy]pyridin-3-yl}-4-[3-(2-cyclohexylethoxy)benzoyl]piperazine-1-carboxamide (94 mg) in EtOAc (5.0 ml) was added 5% palladium carbon (34 mg), followed by stirring overnight under a hydrogen atmosphere. Further, 5% palladium carbon (100 mg) was added thereto, followed by stirring for 24 hours under a hydrogen atmosphere. The catalyst was removed by filtration through Celite, and the filtrate was concentrated under reduced pressure. The residue was purified by silica gel column chromatography (eluent; chloroform:methanol=10:1 (V/V)) to obtain 4-[3-(2-cyclohexylethoxy)benzoyl]-N-[5-(2-hydroxyethoxypyridin-3-yl]piperazine-1-carboxamide (33 mg) as a pale yellow amorphous substance.

### Example 24

To a solution of indole (116 mg) in acetonitrile (10 ml) were added CDI (169 mg) and a catalytic amount of DMAP, followed by stirring at 80°C for 6 hours. The reaction mixture was cooled to room temperature, and a solution of 1-[3-(2-cyclohexylethoxy)benzoyl]piperazine hydrochloride (350 mg) in DMF (5 ml) was added thereto, followed by heating at 80°C for 10 hours. The reaction mixture was cooled to room temperature, and water was added thereto, followed by extraction with EtOAc. The organic layer was washed with water, dried over anhydrous sodium sulfate, and then concentrated under reduced pressure. The residue was purified by silica gel column chromatography (eluent; hexane:EtOAc=2:1 (V/V)) to obtain 1-({4-[3-(2-cyclohexylethoxy)benzoyl]piperazin-1-yl}carbonyl)-1H-indole (288 mg) as a white powder.

### Example 25

To a solution of 4-[3-(benzyloxy)benzoyl]-N-pyridin-3-ylpiperazine-1-carboxamide (5.4 g) in ethanol (100 ml) was added 10% palladium carbon (500 mg), followed by stirring for 5 hours under a hydrogen atmosphere. The catalyst was removed by filtration through Celite, and the filtrate was concentrated under reduced pressure. The residue was crystallized from EtOAc to obtain 4-(3-hydroxybenzoyl)-N-pyridin-3-ylpiperazine-1-carboxamide (3.9 g) as a white solid.

### Example 26

To a solution of 4-(3-hydroxybenzoyl)-N-pyridin-3-ylpiperazine-1-carboxamide (300 mg), 2-fluorobenzyl alcohol (0.10 ml) and PPh₃ (362 mg) in THF (10 ml) was added dropwise DEAD (0.63 ml, 40% Tol solution), followed by stirring at room temperature overnight. The reaction mixture was concentrated under reduced pressure, and the residue was then purified by silica gel column chromatography (eluent; chloroform:methanol=20:1) to obtain a colorless and oily 4-{3-[(2-fluorobenzyl)oxy]benzoyl}-N-pyridin-3-ylpiperazine-1-carboxamide (298 mg). The obtained compound was dissolved in ethanol (10 ml), and oxalic acid (186 mg) was added thereto, followed by stirring at room temperature for 30 minutes. The precipitated solid was collected by filtration, washed with ethanol, and dried under heating to obtain 4-{3-[(2-fluorobenzyl)oxy]benzoyl}-N-pyridin-3-ylpiperazine-1-carboxamide oxalate (140 mg) as a white powder.

### Example 27

A suspension of 4-[3-(2-cyclohexylethoxy)benzoyl]-N-(5-hydroxypyridin-3-yl)piperazine-1-carboxamide (150 mg), ethyl bromoacetate (0.077 ml) and potassium carbonate (48 mg) in DMF (2.0 ml) was stirred at room temperature overnight. To the reaction mixture was added water, followed by extraction with EtOAc, and the organic layer was washed with saturated brine, and then dried over anhydrous sodium sulfate. The solution was concentrated under reduced pressure, and the residue was then purified by silica gel column chromatography (eluent; chloroform:methanol=10:1 (V/V)) to obtain ethyl({5-[({4-[3-(2-cyclohexylethoxy)benzoyl]piperazin-1-yl}carbonyl)amino]pyridin-3-yl}oxy)acetate (66 mg) as a brown oily substance.
To a mixed solution of ethyl({5-[({4-[3-(2-cyclohexylethoxy)benzoyl]piperazin-1-yl}carbonyl)amino]pyridin-3-yl}oxy)acetate (66 mg) in THF (5.0 ml)/methanol (1.0 ml) was added a 1 M aqueous sodium hydroxide solution (0.130 ml), followed by stirring at room temperature overnight. The reaction mixture was concentrated under reduced pressure, and the obtained residue was purified by silica gel column chromatography (eluent; chloroform:methanol=10:1 (V/V)) to obtain ({5-[({4-[3-(2-cyclohexylethoxy)benzoyl]piperazin-1-yl}carbonyl)amino]pyridin-3-yl}oxy)acetic acid (38 mg) as a brown amorphous substance.

### Example 28

To a solution of 3-(benzyloxy)benzaldehyde (15.0 g) in THF (250 ml) were added tert-butylpiperazine-1-carboxylate hydrochloride (19.8 g), acetic acid (6.1 ml), and NaBH(OAc)₃ (24.0 g) under ice-cooling, followed by stirring at room temperature for 2 hours. The reaction mixture was alkalified by addition of a 1 M aqueous sodium hydroxide solution, and extracted with chloroform. The organic layer was dried over anhydrous magnesium sulfate, and concentrated under reduced pressure. The residue was purified by silica gel column chromatography (eluent; hexane:EtOAc=3:1 (V/V)) to obtain a colorless and oily tert-butyl 4-[3-(benzyloxy)benzyl]piperazine-1-carboxylate (26.4 g).
To a solution of tert-butyl 4-[3-(benzyloxy)benzyl]piperazine-1-carboxylate (24.0 g) in EtOAc was added a 4 M hydrochloride/EtOAc solution (70 ml), followed by stirring at room temperature for 5 hours. The precipitated solid was collected by filtration, and washed with EtOAc to obtain a white solid of 1-[3-(benzyloxy)benzyl]piperazine hydrochloride (18.8 g).
To a solution of 1-[3-(benzyloxy)benzyl]piperazine hydrochloride (3.00 g) and phenyl 3-pyridinecarbamate (2.02 g) in DMF (45 ml) was added TEA (3.3 ml), followed by stirring at 80°C for 1 hour. The reaction mixture was cooled to room temperature, and a saturated aqueous sodium hydrogen carbonate solution was added thereto, followed by extraction with EtOAc. The organic layer was washed with saturated brine, dried over anhydrous magnesium sulfate, and concentrated under reduced pressure. The residue was purified by silica gel column chromatography (eluent; chloroform:methanol=20:1 (V/V)) to obtain 4-[3-(benzyloxy)benzyl]-N-pyridin-3-ylpiperazine-1-carboxamide (1.76 g) as a pale brown amorphous substance.
4-[3-(Benzyloxy)benzyl]-N-pyridin-3-ylpiperazine-1-carboxamide (200 mg) was dissolved in ethanol (10 ml), and oxalic acid (90.0 mg) was added thereto, followed by stirring at room temperature for 30 minutes. The precipitated solid was collected by filtration, washed with ethanol, and then dried under heating to obtain 4-[3-(benzyloxy)benzyl]-N-pyridin-3-ylpiperazine-1-carboxamide oxalate (135 mg) as a white powder.

### Example 29

OXONE (registered trademark, 376 mg) was suspended in a mixed solvent of water (5 ml)/THF (5 ml), and a solution of 4-[3-(2-cylohexylethoxy)benzoyl]-N-[5-(methylthio)-1,3,4-thiadiazol-2-yl]piperazine-1-carboxamide (200 mg) in THF (5 ml) was added dropwise thereto at room temperature, followed by stirring for 4 hours. To the reaction mixture was added water, followed by extraction with EtOAc, and the organic layer was washed with water and saturated brine, dried over anhydrous magnesium sulfate, and then concentrated under reduced pressure. The residue was purified by silica gel column chromatography (eluent; chloroform:methanol=20:1 (V/V)), and crystallized from hexane to obtain 4-[3-(2-cyclohexylethoxy)benzoyl]-N-[5-(methylsulfonyl)-1,3,4-thiadiazol-2-yl]piperazine-1-carboxamide (209 mg) as a colorless crystal.

### Example 30

To a solution of N-3-pyridinyl-1-piperazinecarboxamide dihydrochloride (227 mg) in THF (3.8 ml) was added TEA (0.114 ml) at room temperature, followed by stirring for 1 hour. To the reaction mixture was added 2-(2-cyclohexylethoxy)isonicotinaldehyde (190 mg), NaBH(OAc)₃ (207 mg) and acetic acid (0.0470 ml), followed by stirring overnight. The reaction mixture was alkalified by addition of a 1 M aqueous sodium hydroxide solution, followed by extraction with chloroform, and the organic layer was dried over anhydrous magnesium sulfate. The solution was concentrated under reduced pressure, and the residue was then purified by silica gel column chromatography (eluent; EtOAc:methanol=10:1 (V/V)) to obtain a colorless and oily substance. This oily substance was dissolved in EtOAc (5.0 ml), and a 4 M hydrochloride/EtOAc solution (0.36 ml) was added thereto, followed by stirring at room temperature for 2 hours. The precipitated crystal was collected by filtration to obtain 4-{[2-(2-cyclohexylethoxy)pyridin-4-yl]methyl}-N-pyridin-3-ylpiperazine-1-carboxamide trihydrochloride (338 mg) as a colorless powder.

### Example 31

To a solution of 5-[(tert-butoxycarbonyl)amino]nicotinic acid (900 mg) in Tol (10 ml) were added TEA (459 mg) and diphenylphosphorylazide (1.25 g), followed by stirring for 1 hour. The reaction mixture was diluted with EtOAc, washed with a saturated aqueous sodium hydrogen carbonate solution and saturated brine in this order, and dried over anhydrous magnesium sulfate. The solution was concentrated under reduced pressure, and the obtained residue was then dissolved in Tol (10 ml), followed by stirring at 100°C for 3 hours. The reaction mixture was cooled to room temperature, and then a solution of TEA (382 mg) and 1-[3-(2-cyclohexylethoxy)benzoyl]piperazine hydrochloride (1.33 g) in THF (10 ml) was added thereto, followed by stirring for 15 hours. The reaction mixtue was concentrated under reduced pressure, and the obtained residue was then purified by silica gel column chromatography (eluent; hexane:EtOAc=2:1 (V/V)) to obtain tert-butyl{5-[({4-[3-(2-cyclohexylethoxy)benzoyl]piperazin-1-yl}carbonyl)amino]pyridin-3-yl}carbamate (1.31 g) as a white solid.
To a solution of tert-butyl{5-[({4-[3-(2-cyclohexylethoxy)benzoyl]piperazin-1-yl}carbonyl)amino]pyridin-3-yl}carbamate (1.31 g) in EtOAc (20 ml) was added a 4 M hydrochloride/EtOAc solution (20 ml), followed by stirring at room temperature for 3 hours. The precipitated solid was collected by filtration, and dried under heating to obtain N-(5-aminopyridin-3-yl)-4-[3-(2-cyclohexylethoxy)benzoyl]piperazine-1-carboxamide hydrochloride (956 mg) as a pale yellow solid.

### Example 32

To a solution of N-(5-aminopyridin-3-yl)-4-[3-(2-cyclohexylethoxy)benzoyl]piperazine-1-carboxamide hydrochloride (150 mg) in dichloromethane (5 ml) were added TEA (78 mg) and methanesulfonyl chloride(39 mg) under ice-cooling, followed by stirring for 3 hours. To the reaction mixture was added a saturated aqueous sodium hydrogen carbonate solution, followed by extraction with chloroform. The organic layer was dried over anhydrous magnesium sulfate, and then concentrated under reduced pressure, and the residue was purified by silica gel column chromatography (eluent; chloroform:methanol=10:0 to 20:1 (V/V)) to obtain 4-[3-(2-cyclohexylethoxy)benzoyl]-N-{5-[(methylsulfonyl)amino]pyridin-3-yl}piperazine-1-carboxamide (63 mg) as a white solid.

### Example 33

To a suspension of 6-chloronicotinonitrile (272 mg) and potassium carbonate (544 mg) in DMF (10 ml) was added (2-cyclohexylethyl)methylamine hydrochloride (350 mg), followed by stirring at 120°C for 2 hours. The reaction mixture was cooled to room temperature, diluted with EtOAc, washed with a saturated aqueous sodium hydrogen carbonate solution, and then dried over anhydrous magnesium sulfate. The solution was concentrated under reduced pressure, and the obtained residue was then dissolved in ethanol (5 ml), a 5 M aqueous sodium hydroxide solution (5 ml) was added thereto, followed by stirring at 100°C for 6 hours. The reaction mixture was cooled to room temperature, and then neutralized by addition of a 1 M aqueous hydrochloric acid solution, and the precipitated crystal was collected by filtration, and then dried under heating to obtain a brown solid. To a solution of the obtained brown solid, N-pyridin-3-ylpiperazine-1-carboxamide dihydrochloride (159 mg), HOBt (92 mg), and TEA (0.16 ml) in DMF (3 ml) was added WSC (131 mg), followed by stirring at room temperature overnight. The reaction mixture was diluted with EtOAc, washed with a saturated aqueous sodium hydrogen carbonate solution, and then dried over anhydrous magnesium sulfate. The solution was concentrated under reduced pressure, and the obtained residue was then purified by preparative HPLC(ODS column, eluent; water:acetonitrile=60:40 to 5:95(V/V)) to obtain a solid. The obtained solid was washed with hexane/EtOAc, and then dried under heating to obtain 4-({6-[(2-cyclohexylethyl)(methyl)amino]pyridin-3-yl}carbonyl)-N-pyridin-3-ylpiperazine-1-carboxamide (157 mg) as a colorless powder.

### Example 34

To a solution of methyl 3-(bromomethyl)benzoate (821 mg) and N-pyridin-3-ylpiperazine-1-carboxamide dihydrochloride (1.00 g) in DMF (20 ml) was added potassium carbonate (1.49 g), followed by stirring at room temperature for 2 hours. To the reaction mixture was added a saturated aqueous sodium hydrogen carbonate solution, followed by extraction with EtOAc. The organic layer was washed with saturated brine, dried over anhydrous magnesium sulfate, and then concentrated under reduced pressure. The residue was purified by silica gel column chromatography (eluent; chloroform: methanol=2 0: (V/V)) to obtain a colorless and oily methyl 3-({4-[(pyridin-3-ylamino)carbonyl]piperazin-1-yl}methyl)benzoate (995 mg).
To a solution of methyl 3-({4-[(pyridin-3-ylamino)carbonyl]piperazin-1-yl}methyl)benzoate (995 mg) in THF (20 ml) was added a 1 M aqueous sodium hydroxide solution (2.9 ml), followed by stirring at room temperature overnight. The reaction mixture was concentrated under reduced pressure, and the residue was azeotroped with Tol (50 ml), and dehydrated. The EtOAc was added thereto, followed by heating under reflux, and the precipitated solid was then collected by filtration to obtain sodium 3-({4-[(pyridin-3-ylamino)carbonyl]piperazin-1-yl}methyl)benzoate (995 mg) as a pink powder.

### Example 35

To a solution of 3-hydroxybenzaldehyde (5.0 g), 2-cyclohexylethanol (6.82 g) and PPh₃ (14.0 g) in THF (100 ml) was added DEAD (21 ml, 40% Tol solution) under ice-cooling, followed by stirring at room temperature for 5 hours. The reaction mixture was concentrated under reduced pressure, and the residue was then purified by silica gel column chromatography (eluent; hexane:EtOAc=10:1 (V/V)) to obtain 3-(2-cyclohexylethoxy)benzaldehyde (8.81 g) as a brown oily substance.
To a solution of 3-(2-cyclohexylethoxy)benzaldehyde (8.81 g) in THF (100 ml) were added tert-butylpiperazine-1-carboxylate hydrochloride (7.1 g), acetic acid (4.3 ml) and NaBH(OAc)₃ (16.1 g) under ice-cooling, followed by stirring at room temperature for 2 hours. The reaction mixture was alkalified by addition of a 1 M aqueous sodium hydroxide solution, followed by extraction with EtOAc. The organic layer was washed with saturated brine, and dried over anhydrous magnesium sulfate. The solution was concentrated under reduced pressure, and the residue was then purified by silica gel column chromatography (eluent; hexane:EtOAc=3:1 (V/V)) to obtain tert-butyl 4-[3-(2-cyclohexylethoxy)benzyl]piperazine-1-carboxylate (11.6 g) as a colorless and oily substance.
To a solution of tert-butyl 4-[3-(2-cyclohexylethoxy)benzyl]piperazine-1-carboxylate (11.6 g) in EtOAc (100 ml) was added a 4 M hydrochloride/EtOAc solution (100 ml), followed by stirring for 3 hours. To the reaction mixture was added diethylether (100 ml), followed by stirring for 10 minutes. The precipitated solid was taken by filtration, and dried under heating to obtain 1-[3-(2-cyclohexylethoxy)benzyl]piperazine dihydrochloride (9.67 g) as a white solid.
To a solution of 1-[3-(2-cyclohexylethoxy)benzyl]piperazine dihydrochloride (2.04 g) and potassium cyanate (880 mg) in methanol (50 ml) was added a 1 M aqueous hydrochloric acid solution (11 ml), followed by stirring for 1 hour. To the reaction mixture was alkalified by addition of a 1 M aqueous sodium hydroxide solution, followed by extraction with EtOAc, and the organic layer was washed with water and saturated brine in this order, and then dried over anhydrous magnesium sulfate. The solution was concentrated under reduced pressure, and then obtained solid was recrystallized from hexane/EtOAc to obtain 4-[3-(2-cyclohexylethoxy)benzyl]piperazine-1-carboxamide (1.41 g) as a white solid.
To a solution of 4-[3-(2-cyclohexylethoxy)benzyl]piperazine-1-carboxamide (200 mg) in dichloromethane were added 1,8-diazabicyclo[5.4.0]-7-undecene (530 mg) and acetylchloride (272 mg), followed by stirring at room temperature for 3 hours. To the reaction mixture was added water under ice-cooling, followed by extraction with EtOAc. The organic layer was washed with saturated brine, dried over anhydrous magnesium sulfate, and then concentrated under reduced pressure. The obtained residue was dissolved in ethanol, and a 1 M aqueous sodium hydroxide solution (0.6 ml) was added thereto under ice-cooling, followed by stirring for 30 minutes. The reaction mixture was concentrated under reduced pressure, and then water was added thereto, followed by extraction with EtOAc. The organic layer was washed with saturated brine, dried over anhydrous magnesium sulfate, and then concentrated under reduced pressure. The residue was purified by silica gel column chromatography (eluent; hexane:EtOAc=10:1 to 1:1 (V/V)). The obtained oily substance (142 mg) was dissolved in acetone, and oxalic acid (10 mg) was added thereto, followed by stirring for 10 minutes. The precipitated solid was taken by filtration, and dried under heating to obtain N-acetyl-4-[3-(2-cyclohexylethoxy)benzyl]piperazine-1-carboxamide oxalate (122 mg) as a white solid.

### Example 36

To a mixed solution of N-(6-acetamidepyridin-3-yl)-4-[3-(2-cyclohexylethoxy)benzoyl]piperazine-1-carboxamide (276 mg) in methanol (5 ml)/THF (3 ml) was added a 8 M aqueous sodium hydroxide solution (0.124 ml), followed by stirring at 60°C overnight. The reaction mixture was diluted with EtOAc, washed with saturated brine, dried over anhydrous magnesium sulfate, and then concentrated under reduced pressure. The residue was purified by silica gel column chromatography (eluent; chloroform:methanol=10:1 (V/V)), and recrystallized from EtOAc/isopropanol to obtain N-(6-aminopyridin-3-yl)-4-[3-(2-cyclohexylethoxy)benzoyl]piperazine-1-carboxamide (22 mg) as a pale yellow solid.

### Example 37

1-[3-(2-Cyclohexylethoxy)benzoyl]piperazine hydrochloride (300 mg) and TEA (0.12 ml) were suspended in acetonitrile (4.5 ml), and CDI (137 mg) was added thereto at room temperature, followed by stirring until it becomes a transparent solution. To the reaction mixture were added methanesulfonamide (243 mg) and 1,8-diazabicyclo[5.4.0]-7-undecene (0.38 ml), followed by stirring at 60°C overnight. The reaction mixture was cooled to room temperature, and then concentrated under reduced pressure, and EtOAc was added thereto, followed by washing with water and saturated brine in this order. The solution was dried over anhydrous magnesium sulfate, and then concentrated under reduced pressure. The residue was purified by silica gel column chromatography (eluent; chloroform:methanol=8:2 (V/V)), and the obtained oily substance was crystallized from ethanol/water, and dried under heating to obtain 4-[3-(2-cyclohexylethoxy)benzoyl]-N-(mothylsulfonyl)piperazine-1-carboxamide (343 mg) as a white crystal.

### Example 38

To a solution of 2 -cyclohexyl ethanol (6.03 ml) in DMF (75 ml) was added potassium tert-butoxide (4.86 g) under ice-cooling, followed by stirring at room temperature for 30 minutes. To the reaction mixture was added 2-chloroisonicotinonitrile (5.00 g), followed by stirring at room temperature for 3 days. To the reaction mixture was added water, followed by extraction with EtOAc, and the organic layer was washed with saturated brine, and then dried over anhydrous magnesium sulfate. The solution was concentrated under reduced pressure, and the residue was then purified by silica gel column chromatography (eluent; hexane:EtOAc=4:1 (V/V)) to obtain 2-(2-cyclohexylethoxy)isonicotinonitrile (6.97 g) as a colorless powder.
To a solution of 2-(2-cyclohexylethoxy)isonicotinonitrile (6.97 g) in ethanol (80 ml) was added a 5 M aqueous sodium hydroxide solution (60 ml), followed by stirring at 100°C overnight. The reaction mixture was cooled to room temperature, and the reaction mixture was acidified by addition of a 1 M aqueous hydrochloric acid solution was added. The precipitated solid was collected by filtration, and dried under heating to obtain 2-(2-cyclohexylethoxy)isonicotinic acid (6.90 g) as a colorless powder.
A solution of 2-(2-cyclohexylethoxy)isonicotinic acid (3.00 g), WSC (2.77 g), and HOBt (1.95 g) in DMF (60 ml) was stirred for 30 minutes, and tert-butylpiperazine-1-carboxylate hydrochloride (2.24 g) was added thereto, followed by stirring for 4 hours. To the reaction mixture was added water, followed by extraction with EtOAc, and the organic layer was washed with saturated brine, and then dried over anhydrous magnesium sulfate. The solution was concentrated under reduced pressure, and the residue was then purified by silica gel column chromatography (eluent; hexane:ETOAC=4:1 (V/V)) to obtain tert-butyl 4-[2-(2-cyclohexylethoxy)isonicotinoyl]piperazine-1-carboxylate (5.14 g) as a colorless amorphous substance.
To a solution of tert-butyl 4-[2-(2-cyclohexylethoxy)isonicotinoyl]piperazine-1-carboxylate (5.14 g) in EtOAc (50 ml) was added a 4 M hydrochloride/EtOAc solution (50 ml), followed by stirring overnight. The resulting solid was collected by filtration, and dried under heating to obtain 1-[2-(2-cyclohexylethoxy)isonicotinoyl]piperazine dihydrochloride (4.78 g) as a colorless powder.
To a solution of 2-aminopyrazine (5.00 g) in pyridine (50 ml) was added phenyl chloroformate (6.97 ml) was added under ice-cooling, followed by stirring at room temperature for 4 hours. The reaction mixture was concentrated under reduced pressure, and then to the residue was added a saturated aqueous sodium hydrogen carbonate solution, followed by extraction with EtOAc, and the organic layer was washed with saturated brine, and then dried over anhydrous sodium sulfate. The solution was concentrated under reduced pressure, and the residue was then purified by silica gel column chromatography (eluent; hexane:EtOAc=2:1 (V/V)) to obtain phenylpyrazin-2-ylcarbamate (2.11 g) as a colorless powder.
To a solution of 1-[2-(2-cyelohexylethoxy)isonicotinnyl]piperazine dihydrochloride (362 mg) and phenylpyrazin-2-ylcarbamate (200 mg) in acetonitrile (10 ml) was added TEA (0.259 ml), followed by stirring overnight. To the reaction mixture was added a saturated aqueous sodium hydrogen carbonate solution, followed by extraction with EtOAc, and the organic layer was washed with saturated brine, and then dried over anhydrous magnesium sulfate. The solution was concentrated under reduced pressure, and the residue was then purified by silica gel column chromatography (eluent; EtOAc) to obtain 4-[2-(2-cyclohexylethoxy)isonicotinoyl]-N-pyrazin-2-ylpiperazine-1-earboxamide (136 mg) as a colorless amorphous substance.

### Example 39

To a solution of 4-(3-hydroxybenzyl)-N-pyridin-3-ylpiperazine-l-carboxamide (600 mg), methyl 3-(hydroxymethyl)benzoate (479 mg), and PPh₃ (756 mg) in THF (20 ml) was added dropwise DEAD (1.4 ml, 40% Tol solution), followed by stirring at room temperature overnight. The reaction mixture was concentrated under reduced pressure, and the obtained residue was purified by silica gel column chromatography (eluent; chloroforn:methanol=20:1 (V/V)) to obtain a colorless and oily methyl 3-{[3-({4-[(pyridin-3-ylamino)carbonyl]piperazm-1-yl}methyl)phenoxy]methyl}benzoate (618 mg).
To a mixed solution of methyl 3-{[3-({4-[(pyridin-3-ylamino)carbonyl]piperazin-1-yl}methyl)phenoxy]methyl}benzoate (615 mg) in THF (15 ml)/methanol (1.5 ml) was added a 1 M aqueous sodium hydroxide solution (1.4 ml), followed by stirring at 50° C for 1 hour. The reaction mixture was concentrated under reduced pressure, and the residue was azeotroped by addition of Tol (50 ml) for dehydration. To the residue was added EtOAc, followed by heating under reflux for 30 minutes, and the resulting solid was collected by filtration to obtain sodium 3-{[3-({4-[(pyridin-3-ylamino)carbonyl]piperazin-1-yl}methyl)phenoxy]methyl}benzoate (608 mg) as a white solid.

### Example 40

A solution of sodium 3-{[3-({4-[(pyridin-3-ylamino)carbonyl]piperazin-1-yl}methyl)phenoxy]methyl}benzoate (320 mg), ammonium choride (110 mg), WSC (197 mg), and HOBt (139 mg) in DMF (15 ml) was stirred at room temperature overnight. To the reaction mixture was added a saturated aqueous sodium hydrogen carbonate solution, followed by extraction with EtOAc. The organic layer was washed with saturated brine, dried over anhydrous magnesium sulfate, and then concentrated under reduced pressure. The residue was purified by silica gel column chromatography (eluent; chloroform:methanol=10:1 (V/V)) to obtain a colorless and oily 4-(3-{[3-(aminocarbonyl)benzyl]oxy}benzyl)-N-pyridin-3-ylpiperazine-1-carboxamide (267 mg).
To a solution of 4-(3-{[3-(aminocarbonyl)benzyl]oxy}benzyl)-N-pyridin-3-ylpiperazine-1-carboxamide (265 mg) in ethanol (10 ml) was added oxalic acid (161 mg), followed by stirring at room temperature for 30 minutes. The precipitated solid was collected by filtration, and then washed with ethanol to obtain 4-(3-{[3-(aminocarbonyl)benzyl]oxy}benzyl)-N-pyridin-3-ylpiperazine-1-carboxamide dioxalate (201 mg) as a white powder.

### Example 41

To a solution of [3-(methoxycarbonyl)benzyl](triphezayl)phosphonium bromide (1.42 g) and cyclohexyl acetoaldehyde (5.50 g) in DMF (80 ml) was added 60% sodium hydride (513 mg) under ice-cooling, followed by stirring at room temperature overnight. To the reaction mixture was added water (100 ml), followed by extraction with EtOAc. The organic layer was washed with saturated brine, dried over anhydrous magnesium sulfate, and concentrated under reduced pressure. To the residue was added diethylether (100 ml) and hexane (200 ml), and the insoluble material was removed by filtration. The filtrate was concentrated under reduced pressure, and the residue was purified by silica gel column chromatography (eluent; hexane:EtOAc=9:1 (V/V)) to obtain a colorless and oily methyl 3-[3-cyclohexyl-1-propen-1-yl]benzoate (2.29 g).
To a solution of methyl 3-[3-cyclohexyl-1-propen-1-yl]benzoate (2.28 g) in ethanol was added 10% palladium carbon (500 mg) followed by stirring for 5 hours under a hydrogen atmosphere. The catalyst was removed by filtration through Celite, and the filtrate was concentrated under reduced pressure to obtain a colorless and oily methyl 3-(3-cyclohexylpropyl)benzoate (2.29 g).
To a solution of methyl 3-(3-cyclohexylpropyl)benzoate (720 mg) in methanol (10 ml) was added a 1 M aqueous sodium hydroxide solution (5.0 ml), followed by stirring at 50°C for 2 hours. The reaction mixture was cooled to room temperature, acidified by addition of a 1 M aqueous hydrochloric acid solution, and extracted with EtOAc. The organic layer was dried over anhydrous magnesium sulfate, and concentrated under reduced pressure to obtain a white crystal of 3-(3-cyclohexylpropyl)benzoic acid (660 mg).
To a solution of 3-(3-cyclohexylpropyl)benzoic acid (300 mg), N-pyridin-3-ylpiperazine-1-carboxamide dihydrochloride (374 mg), WSC (257 mg), and HOBt (182 mg) in DMF (10 ml) was added TEA (0.40 ml), followed by stirring at room temperature overnight. To the reaction mixture was added water (20 ml), followed by extraction with EtOAc. The organic layer was washed with a saturated aqueous sodium hydrogen carbonate solution and saturated brine in this order, dried over anhydrous magnesium sulfate, and concentrated under reduced pressure. The residue was purified by silica gel column chromatography (eluent; chloroform:methanol=20:1 (V/V)) to obtain a colorless and oily 4-[3-(3-cyclohexylpropyl)benzoyl]-N-pyridin-3-ylpiperazine-1-carboxamide (185 mg).
To a solution of 4-[3-(3-cyclohexylpropyl)benzoyl]-N-pyridin-3-ylpiperazine-1-carboxamide (180 mg) in ethanol (15 ml) was added oxalic acid (112 mg), followed by stirring at room temperature for 30 minutes. The precipitated solid was collected by filtration, washed with ethanol, and then dried under heating to obtain 4-[3-(3-cyclohexylpropyl)benzoyl]-N-pyridin-3-ylpiperazine-1-carboxamide oxalate (158 mg) as a white powder.

### Example 42

To a solution of 6-chloronicotinonitrile (2.00 g) and 2-cyclohexylethanol (2.23 g) in DMF (30 ml) was added potassium tert-butoxide (1.95 g) under ice-cooling, followed by stirring at room temperature overnight. To the reaction mixture was added with water, followed by extraction with EtOAc. The organic layer was washed with saturated brine, dried over anhydrous magnesium sulfate, and concentrated under reduced pressure. The residue was purified by silica gel column chromatography (eluent; hexane:EtOAc=4:1 (V/V)) to obtain a colorless and oily 6-(2-cyclohexylethoxy)nicotinonitrile (2.86 g).
To a solution of 6-(2-cyclohexylethoxy)nicotinonitrile (2.00 g) in Tol (150 ml) was added dropwise DIBAL (8.7 ml, 1 M Tol solution) at -78°C, followed by stirring at -78°C for 1 hour. To the reaction mixture were added a saturated aqueous ammonium chloride solution (30 ml) and EtOAc, followed by stirring for 30 minutes and extraction with EtOAc. The organic layer was dried over anhydrous magnesium sulfate, and concentrated under reduced pressure. The residue was purified by silica gel column chromatography (eluent; hexane. EtOAc=3:1 (V/V)) to obtain a colorless and oily 6-(2-cyclohexylethoxy)nicotinaldehyde (1.62 g).
To a suspension of 6-(2-cyclohexylethoxy)nicotinaldehyde (200 mg) in THE (20 ml) was added TEA (0.12 ml), followed by stirring at room temperature for 1 hour. N-pyridin-3-ylpiperazine-1-carboxamide (240 mg), acetic acid (50 ul), and NaBH(OAc)₃ (219 mg) were added thereto in this order, followed by stirring at room temperature overnight. The reaction mixture was alkalified with a I M aqueous sodium hydroxide solution, and extracted with chloroform. The organic layer was dried over anhydrous magnesium sulfate, and concentrated under reduced pressure. The residue was purified by silica gel column chromatography (eluent; hexane:EtOAc=3:1 (V/V)) to obtain a colorless and oily 4-{[6-(2-cyclohexylethoxy)pyridin-3-yl]methyl}-N-pyridin-3-ylpiperazine-1-carboxamide (297 mg).
To a solution of 4-{[6-(2-cyclohexylethoxy)pyridin-3-yl]methyl}-N-pyridin-3 ylpiperazine-1-carboxamide (297 mg) in ethanol (15 ml) was added oxalic acid (190 mg), followed by stirring at room temperature for 30 minutes. The precipitated solid was collected by filtration, washed with ethanol, and then dried under heating to obtain 4-{[6-(2-cyclohexylethoxy)pyridin-3-y1]methyl}-N-pyridin-3-ylpiperazine-1-carboxamide dioxalate (390 mg) as a white powder.

### Example 43

To benzyl alcohol (72 ml) was added 60% sodium hydride (2.0 g) under ice-cooling, followed by stirring for 15 minutes. To the reaction mixture was added 6-chloro-3-pyridazineamine (3.0 g), followed by stirring at room temperature for 2 hours, at 50°C for 2 hours, and at 80°C overnight. The reaction mixture was cooled to room temperature, and water was added thereto, followed by extraction with EtOAc. The organic layer was dried over anhydrous sodium sulfate. The solution was concentrated under reduced pressure, and the residue was then purified by silica gel column chromatography (eluent; EtOAc:methanol=10:1 (V/V)) to obtain 6-(benzyloxy)pyridazin-3-amine (1.78 g) as a brown powder.
To a solution of 6-(benzyloxy)pyridazin-3-amine (867 mg) in pyridine (10 ml) was added phenyl chloroformate (0.571 ml) under ice-cooling, followed by stirring for 1 hour, and then stirring at room temperature for 2 hours. The reaction mixture was concentrated under reduced pressure, and then to the residue was added EtOAc, and the precipitated solid was washed with diisopropylether and water in this order, and dried under heating to obtain phenyl [6-(benzyloxy)pyridazin-3-yl]carbamate (1.05 g) as a brown powder.
To a solution of phenyl [6-(benzyloxy)pyridazin-3-yl]carbamate (500 mg) and 1-[3-(2-cyclohexylethoxy)benzoyl]piperazine hydrochloride (500 mg) in acetonitrile (10 ml) was added TEA (0.197 ml), followed by stirring overnight. To the reaction mixture was added EtOAc, the resulting insoluble material was removed by filtration, and the filtrate was concentrated under reduced pressure. The residue was purified by silica gel column chromatography (eluent; EtOAc), and crystallized from diisopropylether to obtain N-[6-(benzyloxy)pyridazin-3-yl]-4-[3-(2-cyclohexylethoxy)benzoyl]piperazine-1-carboxamide (716 mg) as a colorless powder.

### Example 44

To a mixed solution of N-[6-(benzyloxy)pyridazin-3-yl]-4-[3-(2-cyclohexylethoxy)benzoyl]piperazine-1-carboxamide (448 mg) in ethanol (40 ml)/methanol (20 ml) was added 10% palladium carbon (44 mg), followed by stirring overnight under a hydrogen atmosphere. The catalyst was removed by filtration through Celite, and the filtrate was concentrated under reduced pressure. The obtained solid was washed with hexane to obtain 4-[3-(2-cyclohexylethoxy)benzoyl]-N-(6-hydroxypyridazin-3-yl)piperazine-1-carboxaznide (299 mg) as an off-white powder.

### Example 45

To a suspension of 4-[3-(2-cyclohexylethoxy)benzoyl]-N-(methylsulfonyl)piperazine-1-carboxamide (110 mg) and potassium carbonate (105 mg) in DMF (1.6 ml) was added methyl iodide (0.08 ml), followed by stirring at room temperature for 3 days. To the reaction mixture was added EtOAc, followed by washing with water, a saturated aqueous sodium hydrogen carbonate solution, and saturated brine in this order. The solution was dried over anhydrous magnesium sulfate, and then concentrated under reduced pressure. The residue was purified by silica gel column chromatography (eluent; chloroform:methanol=97:3 (V/V)) to obtain 4-[3-(2-cyclohexylethoxy)benzoyl]-N-methyl-N-(methylsulfonyl)piperazine-1-carboxamide (57 mg) as a white crystal.

### Example 46

To a solution of N-(5-amino-2-pyridyl)acetamide (810 mg) in pyridine (20 ml) was added phenyl chloroformate (0.744 ml) under ice-cooling, followed by stirring at room temperature for 1 hour. The reaction mixture was concentrated under reduced pressure, and then to the residue was added water, followed by extraction with EtOAc. The organic layer was washed with saturated brine, dried over anhydrous magnesium sulfate, and then concentrated under reduced pressure to obtain phenyl(6-acetamidepyridin-3-yl)carbamate (1800 mg) as a pale yellow powder.
To a solution of phenyl(6-acetamidepyridin-3-yl)carbamate (291 mg) and 1-[3-(2-cyclohexylethoxy)benzyl]piperazine dihydrochloride (383 mg) in acetonitrile (20 ml) was added TEA (0.284 ml), followed by stirring for 1 day. The precipitated solid was collected by filtration, and purified by silica gel column chromatography (eluent; chloroform:methanol=10:1 (V/V)) to obtain N-(6-acetamidepyridin-3-yl)-4-[3-(2-cyelohexylethoxy)benzyl]piperazine-1-carboxamide (353 mg) as a colorless amorphous substance.
To a mixed solution of N-(6-acetamidepyridin-3-yl)-4-[3-(2-cyclohexylethoxy)benzyl]piperazine-1-carboxamide (353 mg) in THF (5.0 ml)/methanol (3.0 ml) was added a 8 M aqueous sodium hydroxide solution (0.28 ml) at room temperature, followed by stirring at 70°C for 2 days. To the reaction mixture was added with water, followed by extraction with EtOAc. The organic layer was washed with saturated brine, dried over anhydrous magnesium sulfate, and then concentrated under reduced pressure. The residue was purified by basic silica gel chromatography (eluent; EtOAc:methanol=10:1 (V/V)), and the obtained pale yellow oily substance was crystallized from diisopropylether to obtain N-(6-aminopyridin-3-yl)-4-[3-(2-cyclohexylethoxy)benzyl]piperazine-1-carboxamide (96 mg) as a pale yellow powder.

### Example 441

To a solution of -4-(5-hydroxy-1-benzothiophene-2-yl)carbonyl]-N-pyrazin-2 ylpiperazine-1-carbaxamide (188 mg), 3-fluorobenzylalcohol (93 mg), and PPh₃ (192 mg) in THF (5 ml) was added DEAD (0.33 ml, 40% Tol solution) under ice-cooling, followed by stirring at room temperature overnight. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; chloroform:methanol=20:1 (V/V)) to obtain a solid. The obtained solid was washed with hexane/EtOAc, and then dried under heating to obtain 4-({5-[(3-fluorobenzyl)oxy]-1-benzothiophene-2-yl}carbonyl)-N-pyrazin-2-ylpiperazine-1-carboxamide (71 mg) as a white powder.

### Example 442

To a solution of 1-[(5-phenyl-2-thienyl)methyl]piperazine dihydrochloride (0.30 g) and phenylpyrazin-2-ylcarbamate (390 mg) in DMF (6 ml) was added TEA (0.38 ml, followed by stirring at 80°C for 2 hours. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; chloroform:methanol=20:1 (V/V)), and purified by basic silica gel column chromatography (eluent; hexane:EtOAc=1:1 (V/V)) to obtain 4-[(5-phenyl-2-thienyl)methyl]-N-pyrazin-2-ylpiperazine-1-carboxamide (143 mg) as a white powder.

### Example 443

To a solution of 4-phenylthiophene-2-aldehyde (134 mg) and N-2-pyrazinyl-1-piperazinecarboxamide dihydrochloride (200 mg) in THF (5 ml) were added acetic acid (43 mg) and DMF (1.25 ml), followed by stirring at room temperature for 1 hour. To the reaction mixture was added NaBH(OAc)₃ (302 mg), followed by stirring at room temperature overnight. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; chloroform:methanol=20:1 (V/V)) to obtain a solid. The obtained solid was washed with hexane/EtOAc, and then dried under heating to obtain 4-[(4-phenyl-2-thienyl)methyl]-N-pyrazin-2-ylpiperazine-1-carboxamide (65 mg) as a white solid.

### Example 444

To a solution of diphenyl(3-chloropyrazin-2-yl)imidedicarbonate (284 mg) and 1-[2-(2-cyclohexylethoxy)isonicotinoyl]piperazine dihydrochloride (300 mg) in acetonitrile (10 ml) was added TEA (0.43 ml), followed by stirring at 80°C for 1 hour. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; chloroform:methanol=20:1 (V/V)) to obtain a solid. The obtained solid was washed with hexane/EtOAc, and then dried under heating to obtain N-(3-chloropyrazin-2-yl)-4-[2-(2-cyclohexylethoxy)isonicotinoyl]piperazine-1-carboxamide (133 mg) as a white solid.

### Example 445

To a solution of 4-(methylamino)-N-pyrazin-2-ylpiperidine-1-carboxamide dihydrochloride (314 mg) and 5-chloro-3-phenyl-1,2,4-thiadiazole (200 mg) in DMF (5 ml) was added TEA (0.57 ml), followed by stirring at room temperature overnight. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; hexane:EtOAe=1:1 (V/V)). The obtained solid was washed with hexane/EtOAc, and then dried under heating to obtain 4-[methyl(3-phenyl-1,2,4-thiadiazol-5-yl)amino]-N-pyrazin-2-ylpiperidine-1-carboxamide (254 mg) as a white solid.

### Example 446

To a solution of N-(3-chloropyxazin-2-yl)piperazine-1-carboxamide dihydrochloride (100 mg), 2-[2-(2-chlorophenyl)ethoxy]isonicotinic acid (97 mg), WSC (76 mg), and HOBt (54 mg) in DMF (3 ml) was added TEA (0.088 ml), followed by stirring at room temperature overnight. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; chloroform:methanol=20:1 (V/V)). The obtained solid was washed with hexane/EtOAc, and then dried under heating to obtain 4-{2-[2-(2-chlorophenyl)ethoxy]isonicotinoyl}-N-(3-chloropyrazin-2-yl)piperazine-1-carboxaanide (89 mg) as a white solid.

### Example 447

To a solution of N-(3-chloropyrazin-2-yl)piperazine-1-carboxamide dihydrochloride (150 mg), 2-{[2-(3-fluorophenyl)ethyl]amino}isonicotinic acid (124 mg), and O-(7-azabenzotriazole-1-yl)-N,N,N',N'-tetramethyluroniumhexafluorophosphate (271 mg) in DMF (5 ml) was added diisopropylethylamine (0.25 ml), followed by stirring at room temperature overnight. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; chloroform:methanol=10:1 (V/V)), and the obtained solid was washed with hexane/EtOAc, and then dried under heating to obtain N-(3-chloropyrazin-2-yl)-4-(2-{[2-(3-fluorophenyl)ethyl]amino}isonicotinoyl)piperazine-1-carboxamide (22 mg) as a white solid.

### Example 448

To a solution of 4-(3-hydroxyphenoxy)-N-pyrazin-2-ylpiperidine-1-carboxamide (150 mg) and 2-cyclohexylethanol (91 mg) and PPh₃ (187 mg) in THF (5 ml) was added DEAD (0.33 ml, 40% Tol solution), followed by stirring at room temperature overnight. The reaction mixture was diluted with EtOAc, washed with a 1 M aqueous sodium hydroxide solution and water in this order, dried over anhydrous magnesium sulfate, and then concentrated under reduced pressure. The residue was purified by silica gel column chromatography (eluent; hexane:EtOAc= 1:2 (V/V)) to obtain 4-[3-(2-cyclohexyl)phenoxy]-N-pyrazin-2-ylpiperidine-1-carboxamide (72 mg) as an amorphous substance.

### Examples 449 and 450

To a solution of tert-butyl 4-[(2-chloropyridin-4-yl)oxy]piperidine-1-carboxylate (500 mg) and 2-cyclohexylethanol (307 mg) in DMF (10 ml) was added 60% sodium hydride (95 mg), followed by stirring at 80°C for 2 hours. The reaction mixture was diluted with EtOAc, washed with a saturated aqueous sodium hydrogen carbonate solution, dried over anhydrous magnesium sulfate, and then concentrated under reduced pressure. The residue was purified by silica gel column chromatography (eluent; hexane:EtOAc==3:1 (V/V)) to obtain an oily substance. The obtained oily substance was dissolved in EtOAc (5 mL), and a 4 M hydrochloride/EtOAc (5 mL) was added thereto, followed by stirring at room temperature overnight. The reaction mixture was concentrated under reduced pressure, and then phenylpyrazin-2-ylcarbamate (129 mg), TEA (0.14 ml), and DMF (3 ml) were added thereto, followed by stirring at 80°C for 1 hour. The reaction mixture was diluted with EtOAc, washed with water and a saturated aqueous sodium hydrogen carbonate solution in this order, dried over anhydrous magnesium sulfate, and then concentrated under reduced pressure. The residue was purified by preparative HPLC (ODS column, eluent; water: acetonitrile=1:1) to obtain 4-{[2-(2-cyclohexylethoxy)pyridin-4-yl]oxy}-N-pyrazin-2-ylpiperazine-1-carboxamide (43 mg: Example 449), 4-{[4-(2-cyclohexylethoxy)pyridin-2-yl]oxy}-N-pyrazin-2-ylpiperazine-1-carboxamide (55 mg: Example 450) as white solids, respectively.

### Example 451

To a suspension of 60% sodium hydride (95 mg) in DMF (12 ml) was added 4-(1H-indole-3-ylmethyl)-N-pyrazin-2-ylpiperazine-1-carboxamide (400 mg), followed by stirring at room temperature for 30 minutes. To the reaction mixture was added 1-(bromomethyl)-3-fluorobenzene (247 mg), followed by stirring at room temperature overnight. To the reaction mixture was added with water, followed by extraction with EtOAc. The organic layer was washed with saturated brine, dried over anhydrous magnesium sulfate, and then concentrated under reduced pressure. The residue was purified by silica gel column chromatography (eluent; chloroform:methanol=10:1 (V/V)) to obtain a pale yellow oily substance. The obtained oily substance was dissolved in EtOAc (5 ml), a 4 M hydrochloride/EtOAc solution (1 ml) was added thereto, and the precipitated solid was collected by filtration, and dried under heating to obtain a pale yellow powder of 4-{[1-(3-fluorobenzyl)-1H-indole-3-yl]methyl}-N-pyrazin-2-ylpiperazine-1-carboxamide dihydrochloride (315 mg).

### Example 452

To a solution of 1-(2-phenylethyl)piperazine dihydrochloride (400 mg) in DMF (8 ml) were added benzoylisocyanate (245 mg) and TEA (0.42 ml), followed by stirring at room temperature overnight. To the reaction mixture was added a saturated aqueous sodium hydrogen carbonate solution, followed by extraction with EtOAc. The organic layer was washed with saturated brine, dried over anhydrous magnesium sulfate, and then concentrated under reduced pressure. The residue was purified by silica gel column chromatography (eluent; chloroform:methanol=95:5(V/V)) to obtain N-benzoyl-4-(2-phenylethyl)piperazine-1-carboxamide (512 mg) as a white powder.

The compounds of Examples 1 to 492 were prepared in the same manner as the methods of Examples 1 to 46, and 441 to 452, using each corresponding starting materials as shown in the following Tables 18 to 76. The production processes and the physicochemical data of the compounds of Examples are shown in Tables 18 to 76. Further, NMR data of the compounds of a few Examples are shown in Tables 77 to 81.

**[Table 3]**

| Rf | Syn | Str | Dat |
|---|---|---|---|
| 1 | R1 | 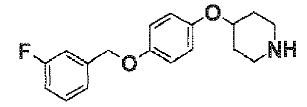 | FP: 302 |
| 2 | R2 | 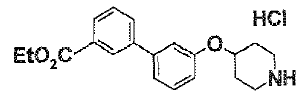 | FP: 326 |
| 3 | R3 | 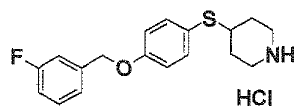 | FP: 318 |
| 4 | R4 | 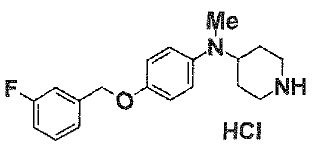 | FP: 315 |
| 5 | R5 | 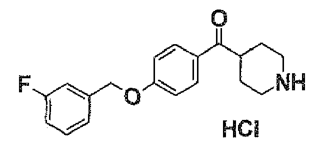 | FP: 314 |
| 6 | R6 | 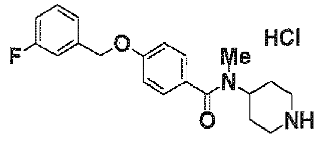 | FP: 343 |
| 7 | R7 | 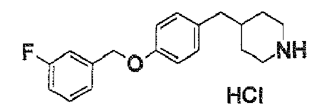 | FP: 300 |
| 8 | R8 | 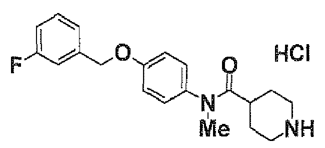 | FP: 343 |
| 9 | R9 | 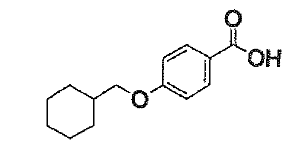 | FP: 235 |
| 10 | R10 | 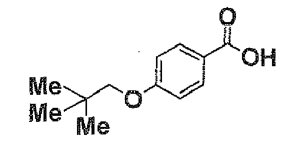 | FP: 209 |

**[Table 4]**

| Rf | Syn | Str | Dat |
|---|---|---|---|
| 11 | R11 | 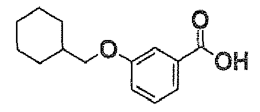 | EI: 235 [M+H]⁺ |
| 12 | R12 | 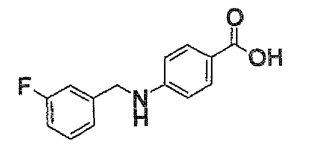 | FN: 244 |
| 13 | R13 | 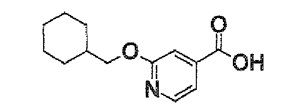 | FP: 236 |
| 14 | R14 | 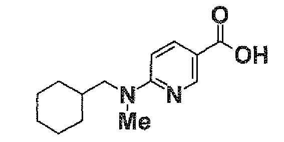 | EP: 249 |
| 15 | R15 | 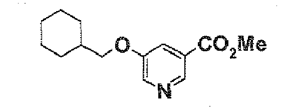 | FP: 250 |
| 16 | R16 | 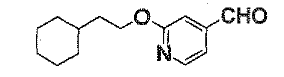 | EP: 234 |
| 17 | R17 | 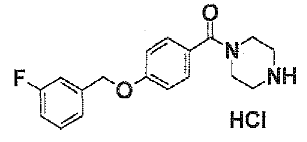 | FP: 315 |
| 18 | R18 | 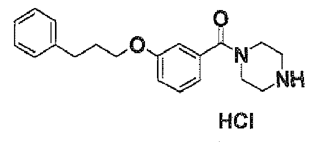 | EP: 325 |
| 19 | R19 | 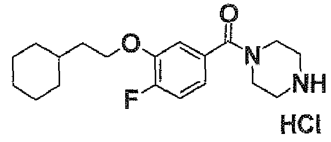 | FP: 335 |
| 20 | R20 | 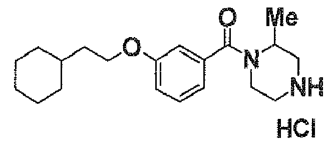 | FP: 331 |

**[Table 5]**

| Rf | Syn | Str | Dat |
|---|---|---|---|
| 21 | R21 | 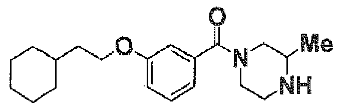 | FP: 331 |
| 22 | R22 | 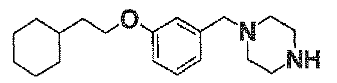 | FP: 303 |
| 23 | R23 | 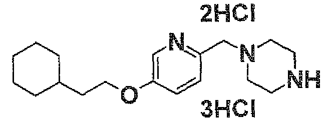 | EP: 304 |
| 24 | R24 | 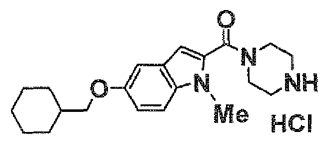 | FP: 356 |
| 25 | R25 | 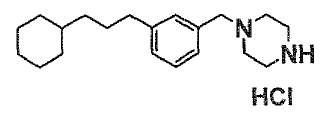 | EP: 301 |
| 26 | R26 | 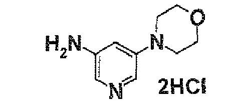 | EP: 180 |
| 27 | R27 | 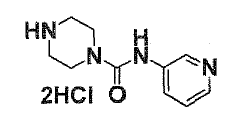 | FP: 207 |
| 28 | R28 | 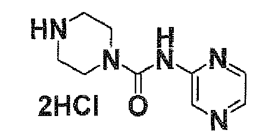 | FP: 208 |
| 29 | R29 | 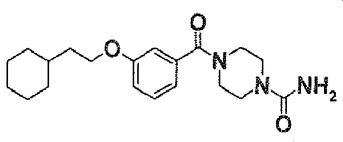 | FP: 360 |
| 30 | R1 | 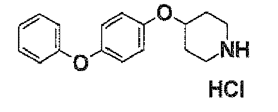 | FP: 270 |

**[Table 6]**

| Rf | Syn | Str | Dat |
|---|---|---|---|
| 31 | R1 | 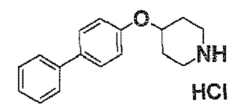 | FP: 254 |
| 32 | R1 | 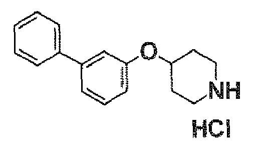 | FP: 254 |
| 33 | R1 | 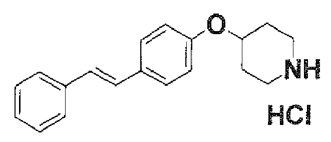 | FP: 280 |
| 34 | R1 | 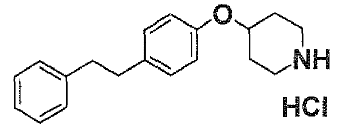 | FP: 282 |
| 35 | R1 | 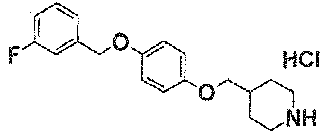 | FP: 316 |
| 36 | R1 | 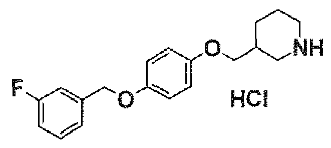 | FP: 316 |
| 37 | R1 | 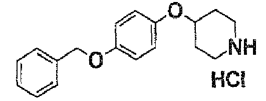 | FP: 284 |
| 38 | R5 | 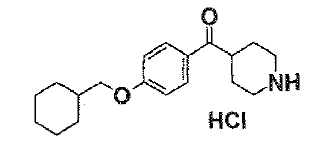 | FP: 302 |
| 39 | R5 | 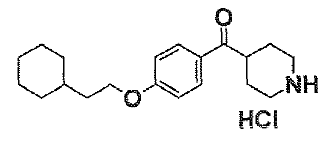 | FP: 316 |

**[Table 7]**

| Rf | Syn | Str | Dat |
|---|---|---|---|
| 40 | R5 | 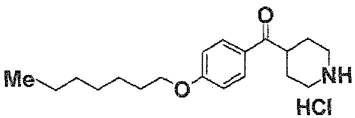 | FP: 304 |
| 41 | R8 | 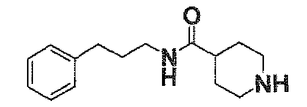 | FP: 247 |
| 42 | R9 | 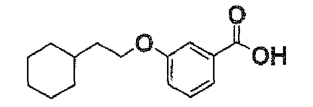 | FP: 249 |
| 43 | R9 | 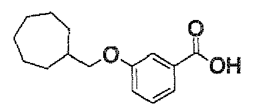 | FP: 249 |
| 44 | R9 | 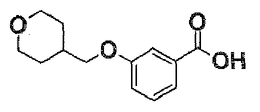 | FP: 237 |
| 45 | R9 | 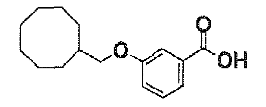 | FP: 263 |
| 46 | R9 | 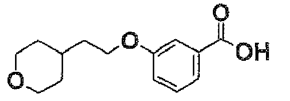 | FP: 251 |
| 47 | R9 | 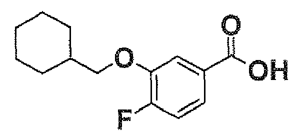 | FP: 253 |
| 48 | R9 | 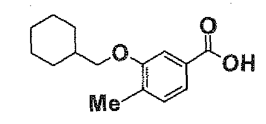 | FP: 249 |
| 49 | R9 | 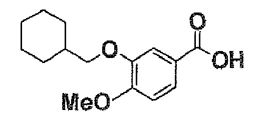 | FP: 265 |

**[Table 8]**

| Rf | Syn | Str | Dat |
|---|---|---|---|
| 50 | R9 | 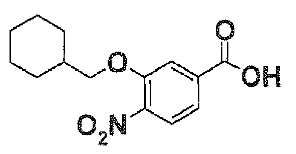 | FN: 278 |
| 51 | R9 | 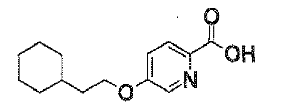 | EP: 250 |
| 52 | R9 | 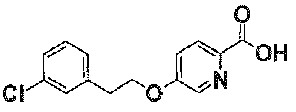 | EP: 278 |
| 53 | R9 | 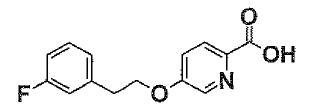 | FN: 260 |
| 54 | R10 | 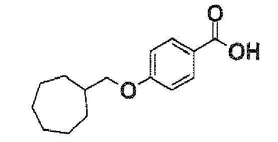 | FP: 249 |
| 55 | R10 | 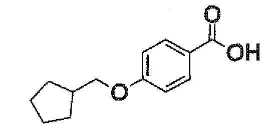 | EP: 221 |
| 56 | R10 | 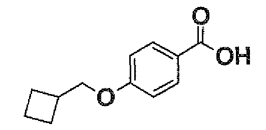 | EP: 207 |
| 57 | R10 | 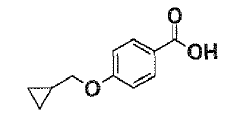 | EP: 193 |
| 58 | R10 | 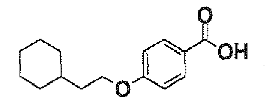 | EP: 249 |
| 59 | R10 | 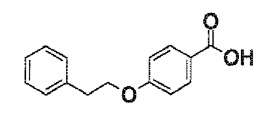 | FP: 243 |

**[Table 9]**

| Rf | Syn | Str | Dat |
|---|---|---|---|
| 60 | R10 | 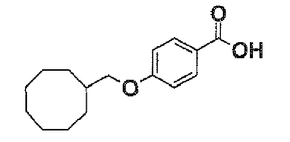 | FP: 263 |
| 61 | R10 | 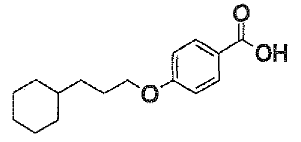 | FP: 263 |
| 62 | R10 | 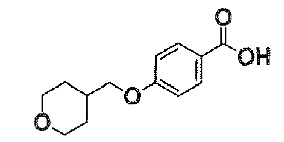 | FP: 237 |
| 63 | R10 | 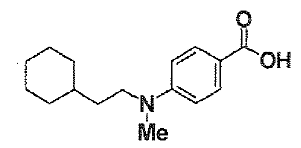 | FP: 262 |
| 64 | R10 | 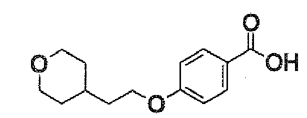 | FP: 251 |
| 65 | R10 | 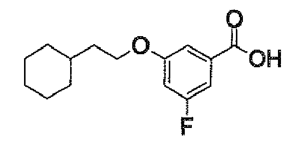 | EN: 265 |
| 66 | R12 | 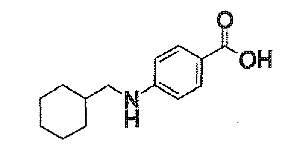 | FP: 234 |
| 67 | R12 | 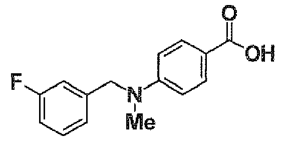 | FP: 260 |
| 68 | R12 | 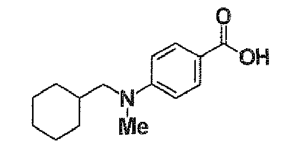 | FP: 248 |

**[Table 10]**

| Rf | Syn | Str | Dat |
|---|---|---|---|
| 69 | R12 | 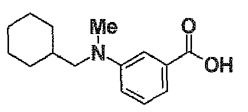 | FP: 248 |
| 70 | R12 | 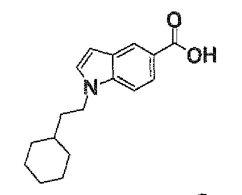 | Fop: 272 |
| 71 | R13 | 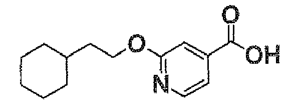 | FP: 250 |
| 72 | R13 | 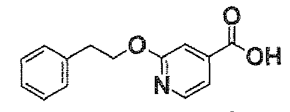 | FP: 244 |
| 73 | R13 | 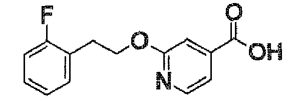 | FP: 262 |
| 74 | R13 | 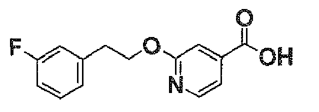 | EP: 262 |
| 75 | R13 | 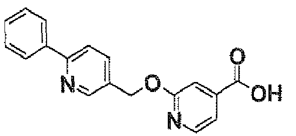 | EP: 307 |
| 76 | R13 | 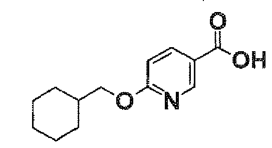 | FP: 236 |
| 77 | R13 | 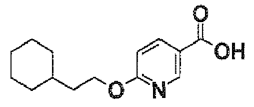 | FP: 250 |
| 78 | R13 | 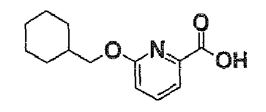 | FP: 236 |

**[Table 11]**

| Rf | Syn | Str | Dat |
|---|---|---|---|
| 79 | R13 | 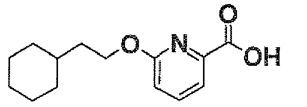 | FP: 250 |
| 80 | R15 | 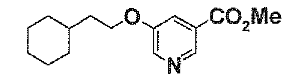 | FP: 264 |
| 81 | R17 | 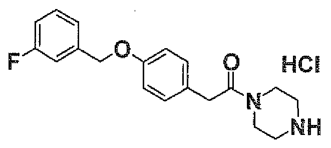 | FP: 329 |
| 82 | R17 | 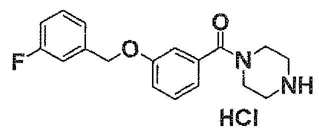 | FP: 315 |
| 83 | R17 | 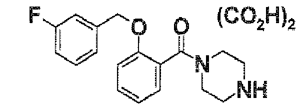 | FP: 315 |
| 84 | R17 | 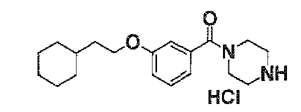 | FP: 317 |
| 85 | R17 | 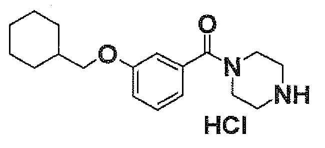 | FP: 303 |
| 86 | R17 | 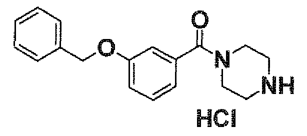 | EP: 297 |
| 87 | R17 | 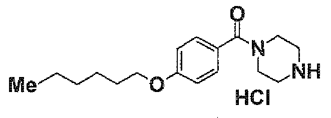 | EP: 291 |
| 88 | R17 | 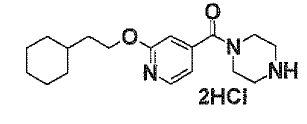 | EP: 318 |

**[Table 12]**

| Rf | Syn | Str | Dat |
|---|---|---|---|
| 89 | R17 | 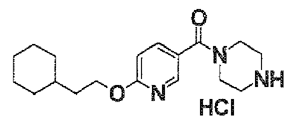 | EP: 318 |
| 90 | R17 | 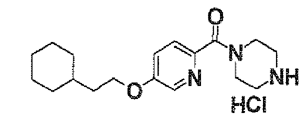 | EP: 318 |
| 91 | R18 | 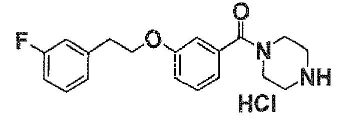 | FP: 329 |
| 92 | R18 | 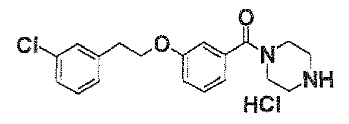 | EP: 345 |
| 93 | R18 | 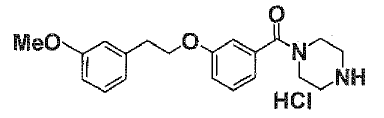 | FP: 341 |
| 94 | R18 | 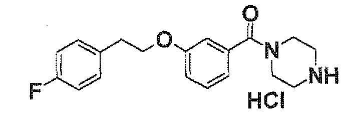 | EP: 329 |
| 95 | R24 | 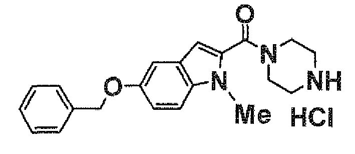 | EP: 350 |

**[Table 13]**

| Rf | Syn | Str | Dat |
|---|---|---|---|
| 96 | R96 | 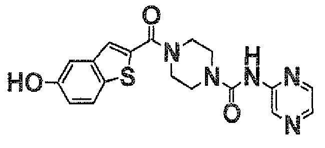 | FP:384 |
| 97 | R97 | 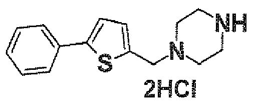 | FP:259 |
| 98 | R98 | 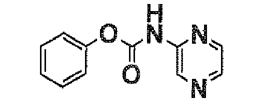 | EP: 216 |
| 99 | R99 | 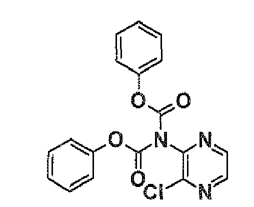 | FP:370 |
| 100 | R100 | 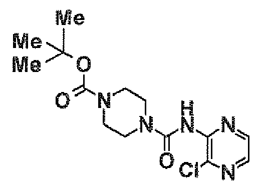 | EN:340 |
| 101 | R101 | 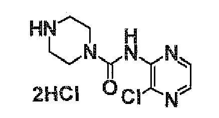 | EP:242 |
| 102 | R102 | 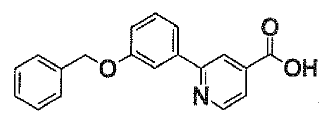 | EP:306 |
| 103 | R103 | 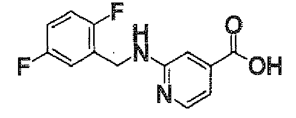 | EP:265 |

**[Table 14]**

| Rf | Syn | Str | Dat |
|---|---|---|---|
| 104 | R104 | 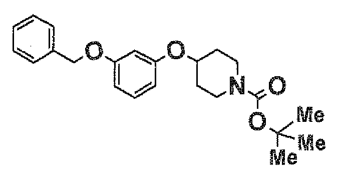 | EP:406 [M+Na]⁺ |
| 105 | R105 | 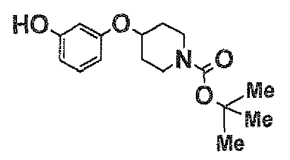 | FP:294 |
| 106 | R106 | 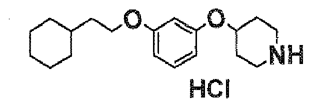 | FP:304 |
| 107 | R107 | 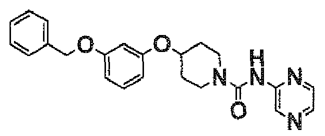 | FP:405 |
| 108 | R108 | 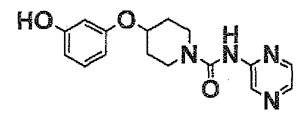 | EP:315 |
| 109 | R109 | 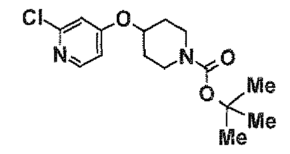 | EP:313 |
| 110 | R9 | 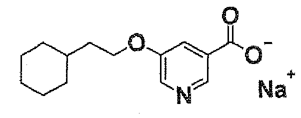 | FP:250 |

**[Table 15]**

| Rf | Syn | Str | Dat |
|---|---|---|---|
| 111 | R16 | 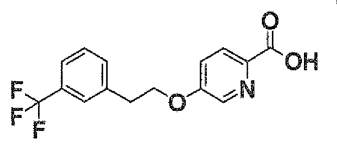 | EP:312 |
| 112 | R16 | 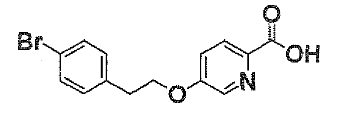 | FP:324 |
| 113 | R96 | 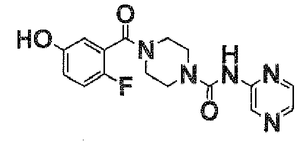 | FP:346 |
| 114 | R97 | 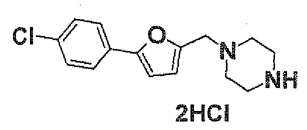 | EP:277 |
| 115 | R97 | 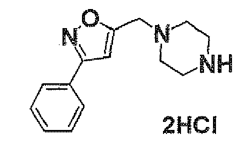 | EP:244 |
| 116 | R97 | 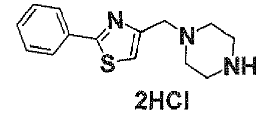 | EP:260 |
| 117 | R97 | 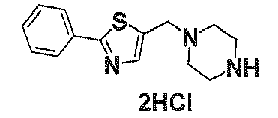 | FP:260 |
| 118 | R19 | 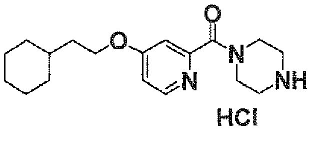 | EP:318 |

**[Table 16]**

| Rf | Syn | Str | Dat |
|---|---|---|---|
| 119 | R19 | 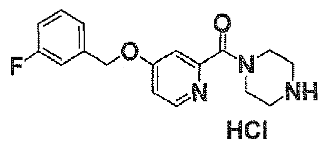 | FP:316 |
| 120 | R99 | 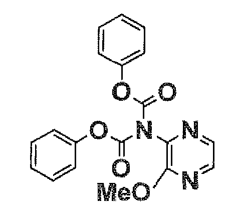 | EP:366 |
| 121 | R28 | 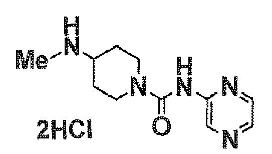 | FP:236 |
| 122 | R28 | 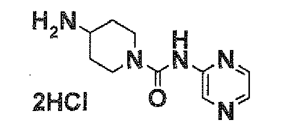 | FP:222 |
| 123 | R13 | 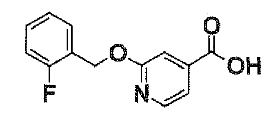 | EI:247 |
| 124 | R13 | 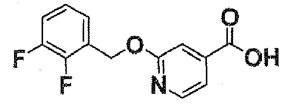 | FP:266 |
| 125 | R13 | 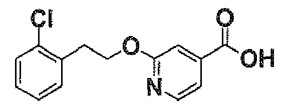 | FP:278 |
| 126 | R13 | 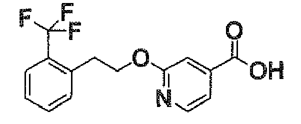 | FP:312 |
| 127 | R103 | 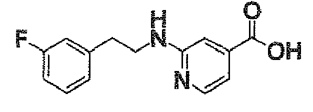 | EP:261 |

**[Table 17]**

| Rf | Syn | Str | Dat |
|---|---|---|---|
| 128 | R13 | 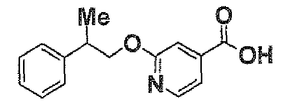 | FP:258 |
| 129 | R104 | 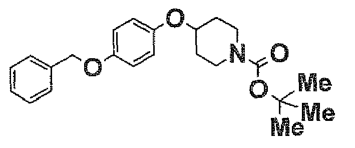 | EP:406 [M+Na]⁺ |
| 130 | R105 | 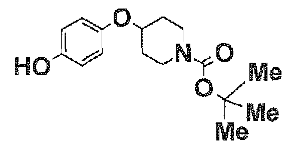 | EN:292 |
| 131 | R106 | 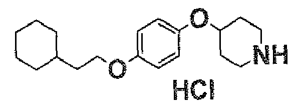 | FP:304 |
| 132 | R106 | 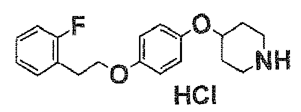 | FP:316 |
| 133 | R106 | 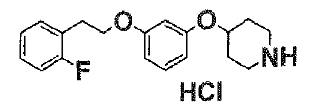 | EP:316 |
| 134 | R13 | 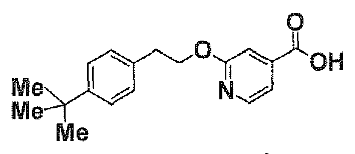 | FP:300 |
| 135 | R17 | 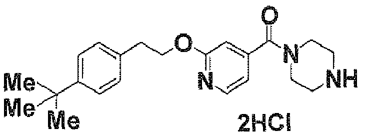 | EP:368 |
| 136 | R136 | 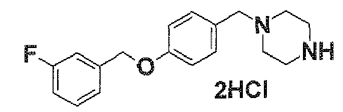 | FP:301 |
| 137 | R136 | 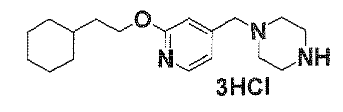 | FP:304 |

**[Table 18]**

| Ex | Syn | Str | Dat |
|---|---|---|---|
| 1 | 1 | 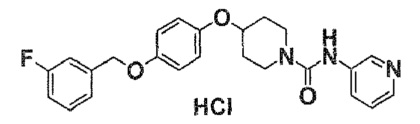 | FP: 422 |
| 2 | 2 | 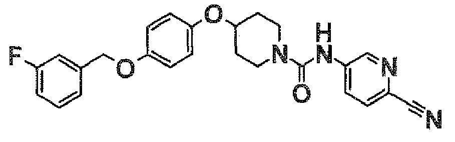 | FP: 447 |
| 3 | 3 | 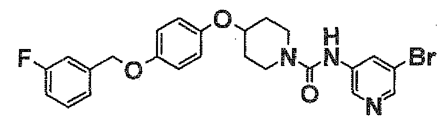 | FP: 500 502 |
| 4 | 4 | 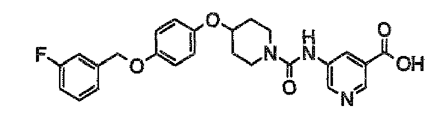 | EP: 466 |
| 5 | 5 | 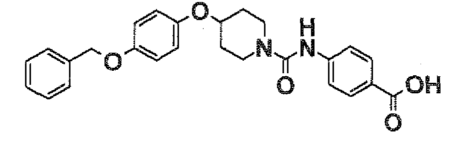 | FP: 447 |
| 6 | 6 | 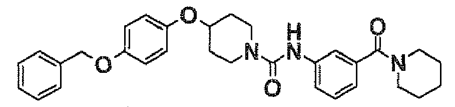 | FP: 514 |
| 7 | 7 | 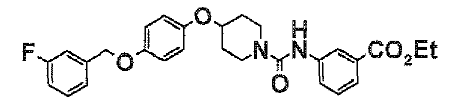 | FP: 492 |
| 8 | 8 | 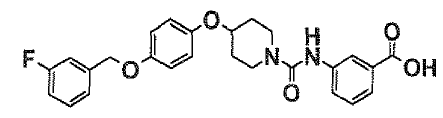 | FP: 465 |
| 9 | 9 | 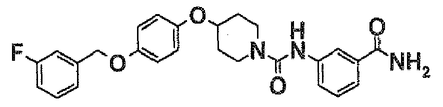 | FP: 464 |
| 10 | 10 | 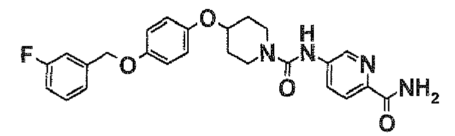 | FP: 465 |

**[Table 19]**

| Ex | Syn | Str | Dat |
|---|---|---|---|
| 11 | 11 | 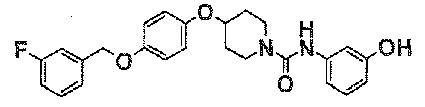 | FP: 437 |
| 12 | 12 | 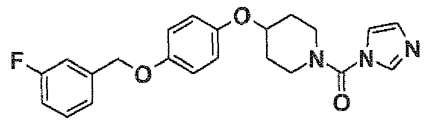 | FP: 396 |
| 13 | 13 | 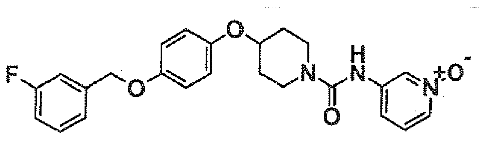 | FP: 438 |
| 14 | 14 | 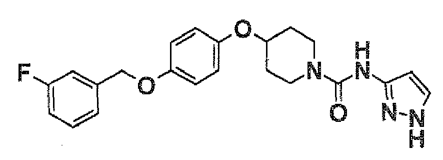 | FP: 411 |
| 15 | 15 | 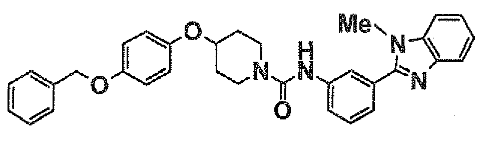 | FP: 533 |
| 16 | 16 | 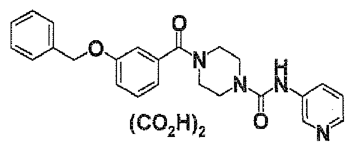 | FP: 417 |
| 17 | 17 | 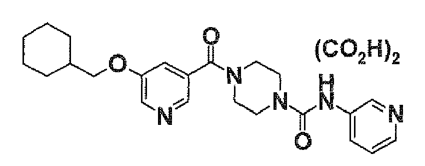 | FP: 424 |
| 18 | 18 | 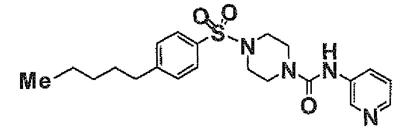 | EP: 417 |
| 19 | 19 | 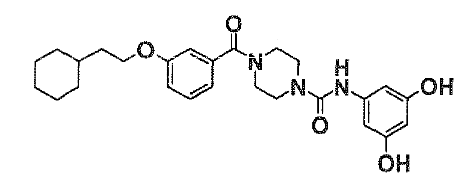 | EP: 468 |

**[Table 20]**

| Ex | Syn | Str | Dat |
|---|---|---|---|
| 20 | 20 | 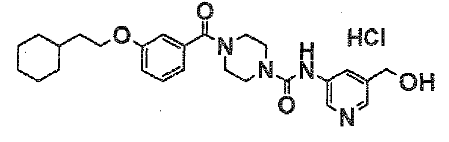 | FP: 467 |
| 21 | 21 | 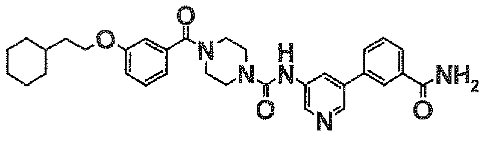 | FP: 556 |
| 22 | 22 | 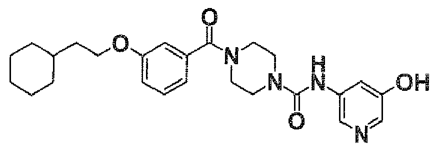 | FP: 453 |
| 23 | 23 | 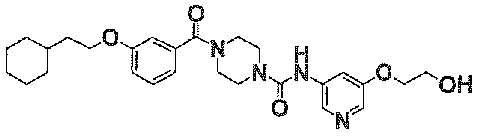 | FP: 497 |
| 24 | 24 | 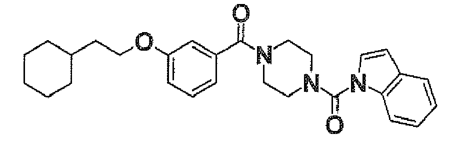 | FP: 460 |
| 25 | 25 | 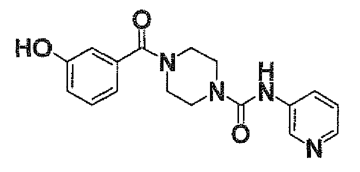 | FP: 327 |
| 26 | 26 | 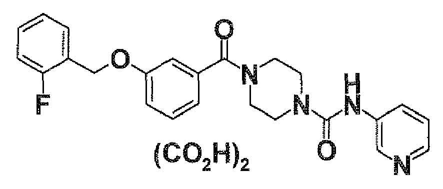 | EP: 435 |
| 27 | 27 | 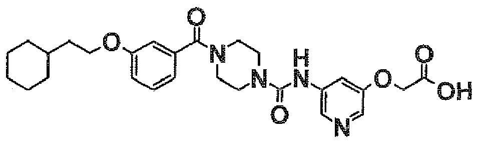 | EP: 511 |

**[Table 21]**

| Ex | Syn | Str | Dat |
|---|---|---|---|
| 28 | 28 | 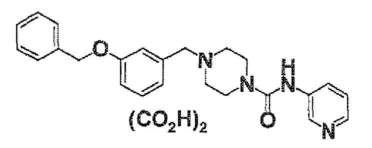 | FP: 403 |
| 29 | 29 | 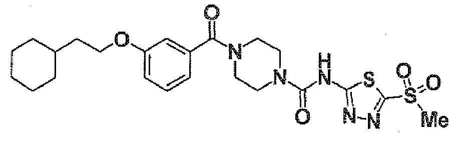 | EP: 522 |
| 30 | 30 | 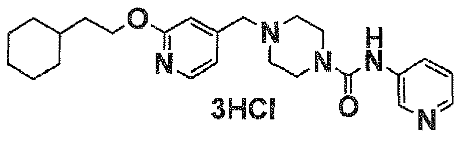 | FP: 424 |
| 31 | 31 | 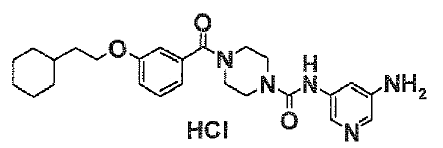 | FP: 452 |
| 32 | 32 | 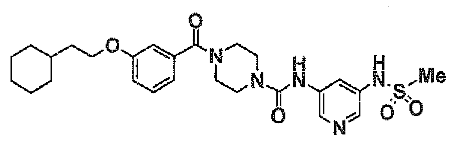 | FP: 530 |
| 33 | 33 | 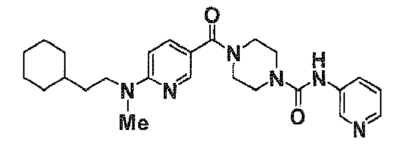 | FP: 451 |
| 34 | 34 | 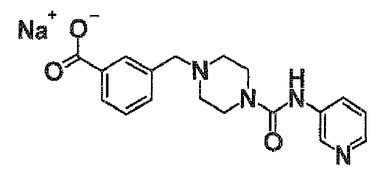 | EP: 361 |
| 35 | 35 | 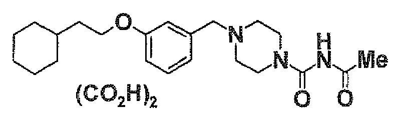 | FP: 388 |
| 36 | 36 | 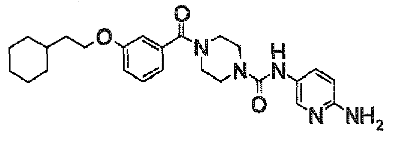 | FP: 452 |

**[Table 22]**

| Ex | Syn | Str | Dat |
|---|---|---|---|
| 37 | 37 | 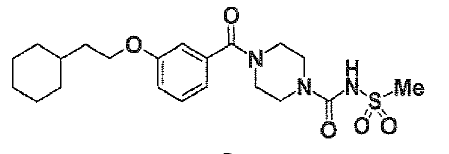 | FP: 438 |
| 38 | 38 | 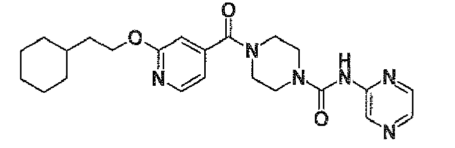 | FP: 439 |
| 39 | 39 | 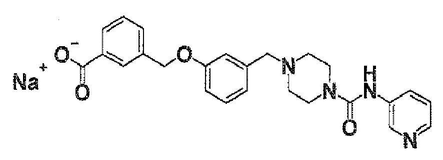 | EP: 459 |
| 40 | 40 | 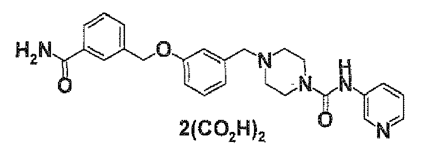 | EP: 446 |
| 41 | 41 | 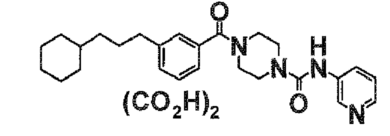 | FP: 435 |
| 42 | 42 | 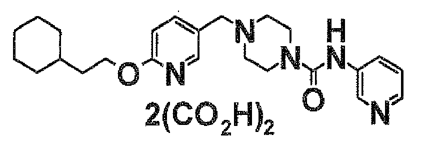 | FP: 424 |
| 43 | 43 | 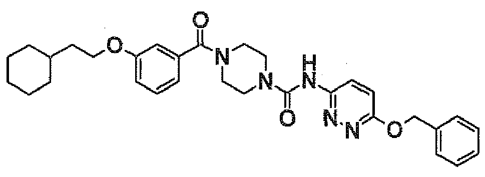 | FP: 544 |
| 44 | 44 | 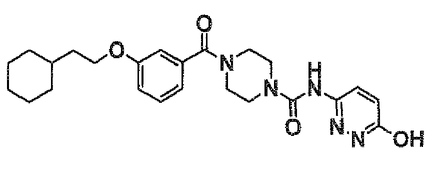 | EP: 454 |

**[Table 23]**

| Ex | Syn | Str | Dat |
|---|---|---|---|
| 45 | 45 | 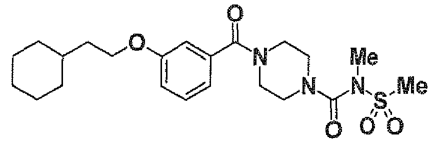 | FP: 452 |
| 46 | 46 | 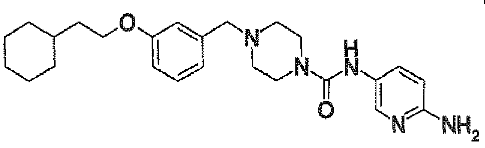 | FP: 438 |
| 47 | 1 | 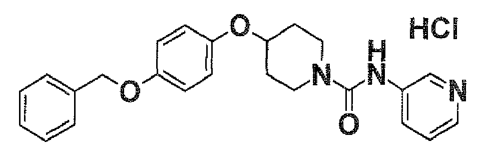 | FP: 404 |
| 48 | 1 | 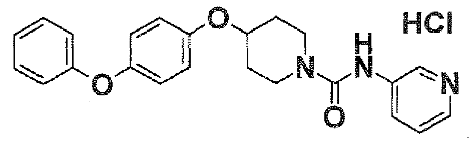 | FP: 390 |
| 49 | 1 | 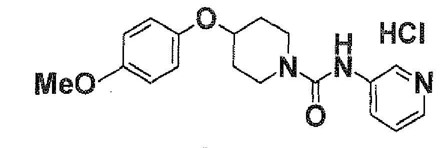 | FP: 328 |
| 50 | 1 | 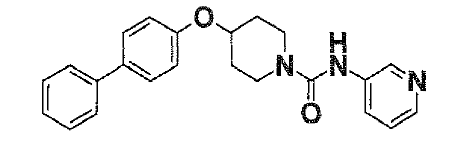 | FP: 374 |
| 51 | 1 | 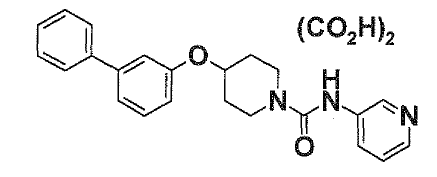 | FP: 374 |
| 52 | 1 | 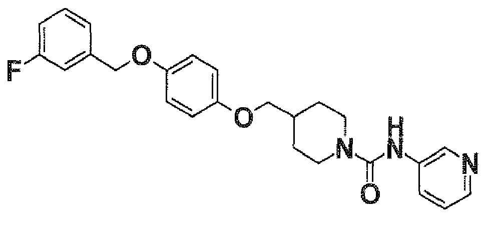 | FP: 436 |

**[Table 24]**

| Ex | Syn | Str | Dat |
|---|---|---|---|
| 53 | 1 | 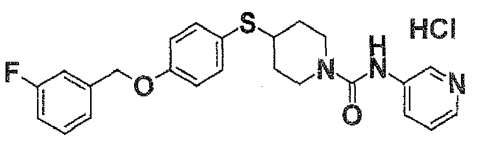 | FP: 438 |
| 54 | 1 | 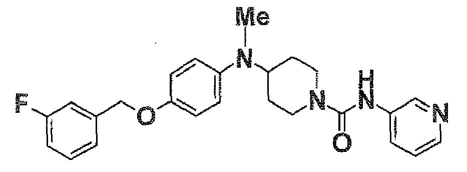 | FP: 435 |
| 55 | 1 | 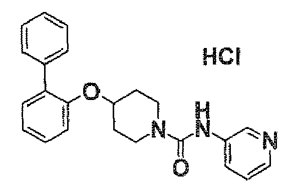 | FP: 374 |
| 56 | 1 | 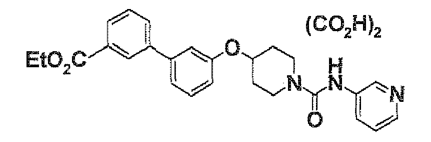 | FP: 446 |
| 57 | 1 | 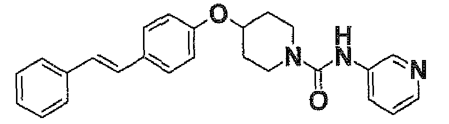 | FP: 400 |
| 58 | 1 | 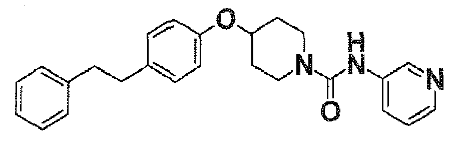 | FP: 402 |
| 59 | 1 | 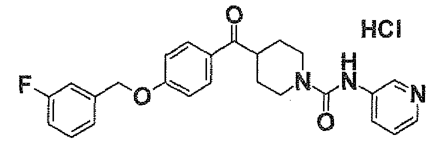 | FP: 434 |
| 60 | 1 | 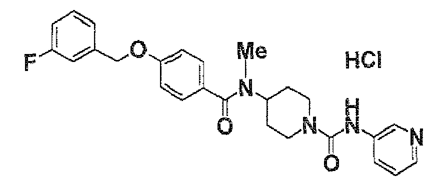 | FP: 463 |

**[Table 25]**

| Ex | Syn | Str | Dat |
|---|---|---|---|
| 61 | 1 | 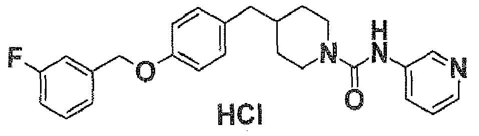 | FP: 420 |
| 62 | 1 | 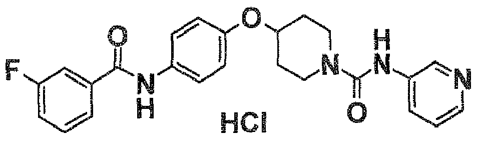 | FP: 435 |
| 63 | 1 | 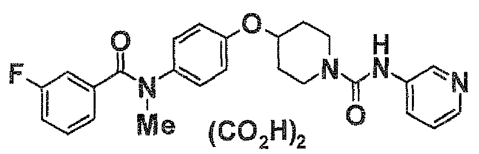 | FP: 449 |
| 64 | 1 | 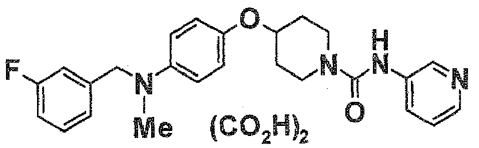 | FP: 435 |
| 65 | 1 | 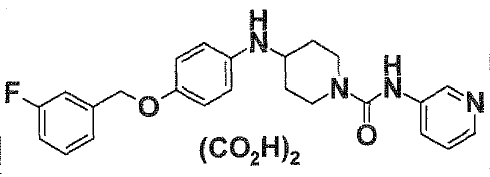 | FP: 421 |
| 66 | 1 | 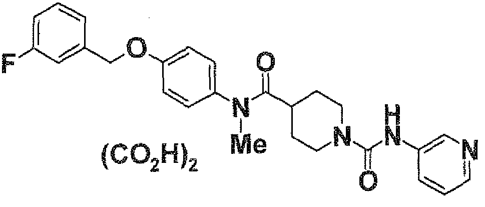 | FP: 463 |
| 67 | 1 | 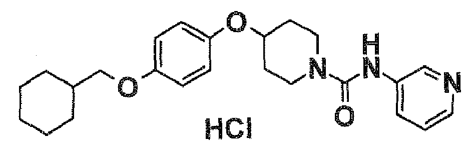 | FP: 410 |
| 68 | 1 | 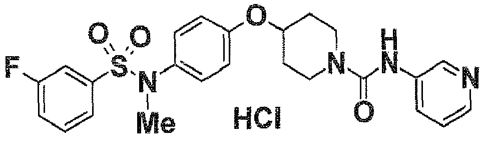 | FP: 485 |

**[Table 26]**

| Ex | Syn | Str | Dat |
|---|---|---|---|
| 69 | 1 | 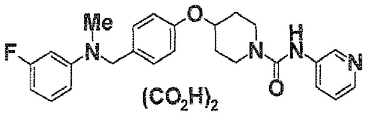 | FP: 435 |
| 70 | 1 | 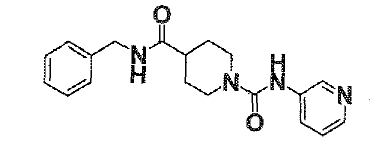 | EP: 339 |
| 71 | 1 | 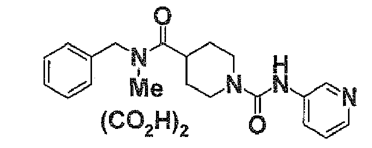 | EP: 353 |
| 72 | 1 | 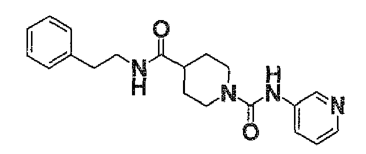 | EP: 353 |
| 73 | 1 | 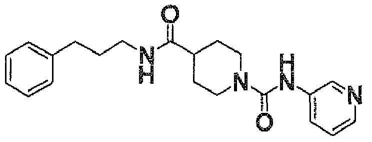 | FP: 367 |
| 74 | 1 | 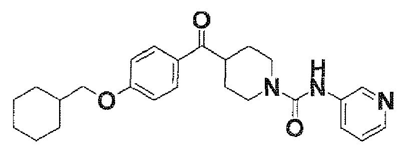 | FP: 422 |
| 75 | 1 | 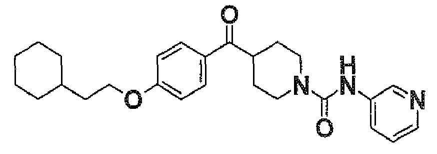 | FP: 436 |
| 76 | 1 | 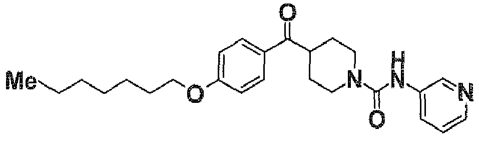 | FP: 424 |
| 77 | 1 | 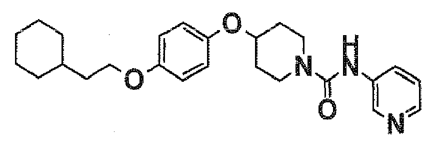 | EP: 424 |

**[Table 27]**

| Ex | Syn | Str | Dat |
|---|---|---|---|
| 78 | 1 | 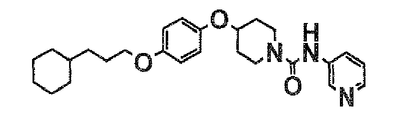 | FP: 438 |
| 79 | 1 | 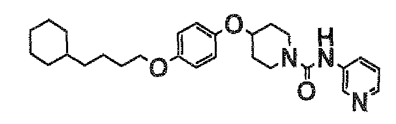 | FP: 452 |
| 80 | 2 | 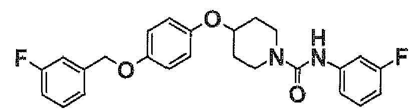 | FP: 439 |
| 81 | 2 | 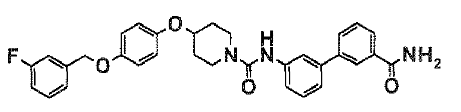 | FP: 540 |
| 82 | 2 | 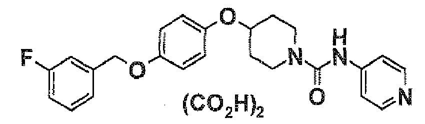 | FP: 422 |
| 83 | 2 | 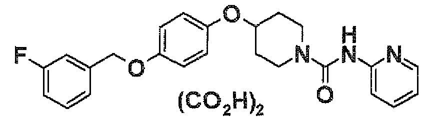 | EP: 422 |
| 84 | 2 | 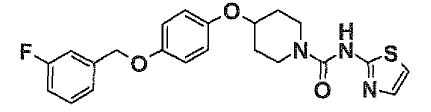 | EP: 427 |
| 85 | 2 | 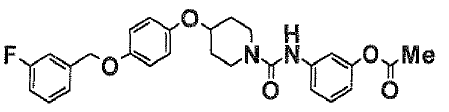 | FP: 479 |
| 86 | 2 | 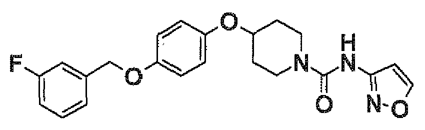 | FP: 412 |
| 87 | 2 | 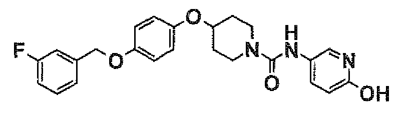 | FP: 438 |

**[Table 28]**

| Ex | Syn | Str | Dat |
|---|---|---|---|
| 88 | 2 | 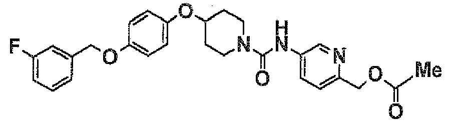 | FP: 494 |
| 89 | 2 | 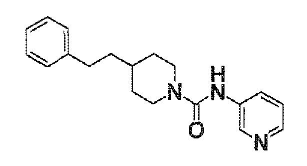 | FP: 310 |
| 90 | 2 | 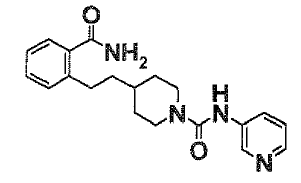 | FP: 353 |
| 91 | 2 | 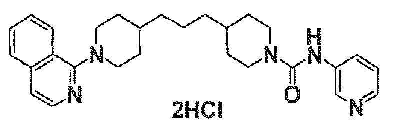 | EP: 458 |
| 92 | 2 | 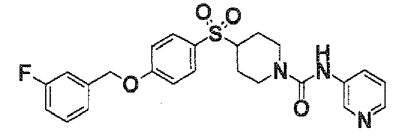 | FP: 470 |
| 93 | 2 | 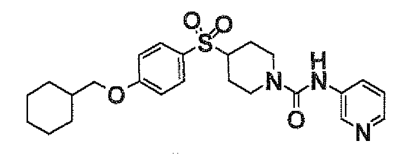 | FP: 458 |
| 94 | 2 | 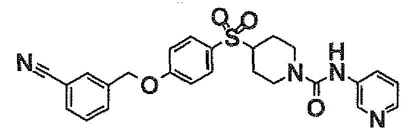 | FP: 477 |
| 95 | 2 | 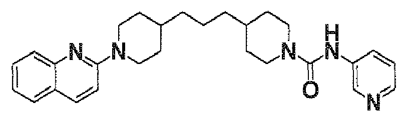 | EP: 458 |

**[Table 29]**

| Ex | Syn | Str | Dat |
|---|---|---|---|
| 96 | 2 | 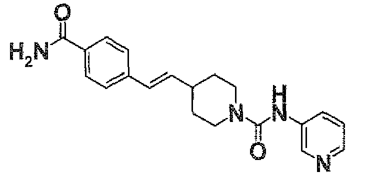 | FP: 351 |
| 97 | 2 | 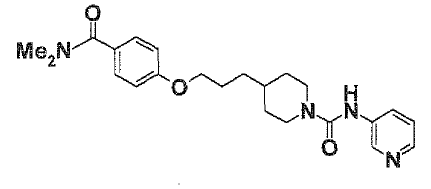 | FP: 411 |
| 98 | 2 | 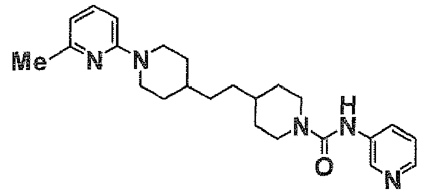 | FP: 408 |
| 99 | 2 | 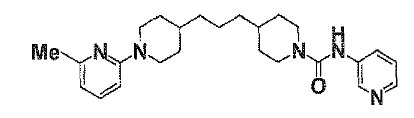 | FP: 422 |
| 100 | 2 | 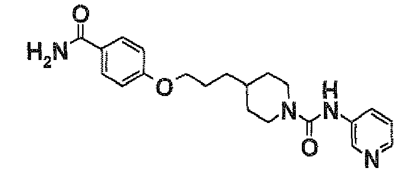 | FP: 383 |
| 101 | 2 | 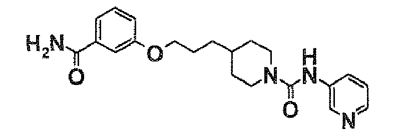 | FP: 383 |
| 102 | 2 | 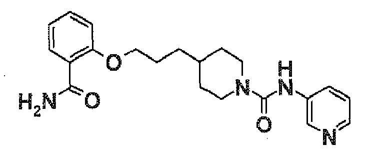 | FP: 383 |
| 103 | 2 | 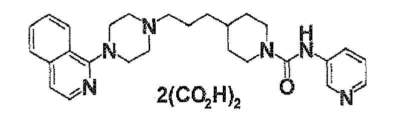 | EP: 459 |

**[Table 30]**

| Ex | Syn | Str | Dat |
|---|---|---|---|
| 104 | 2 | 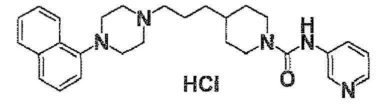 | EP: 458 |
| 105 | 2 | 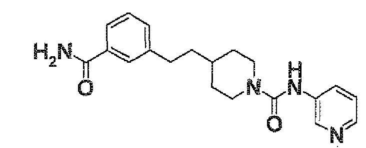 | FP: 353 |
| 106 | 2 | 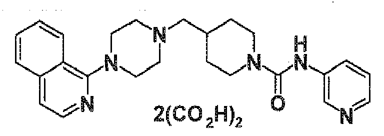 | FP: 431 |
| 107 | 2 | 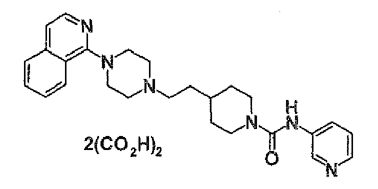 | EP: 458 |
| 108 | 2 | 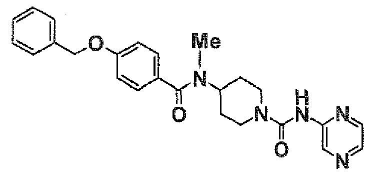 | FP: 445 |
| 109 | 2 | 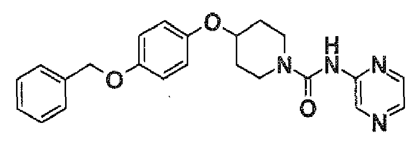 | EP: 405 |
| 110 | 3 | 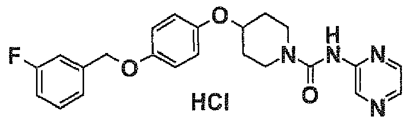 | FP: 423 |
| 111 | 5 | 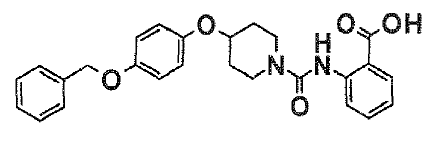 | FP: 447 |

**[Table 31]**

| Ex | Syn | Str | Dat |
|---|---|---|---|
| 112 | 5 | 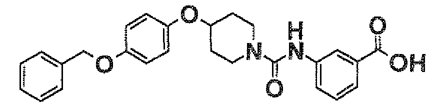 | FP: 447 |
| 113 | 5 | 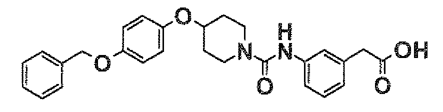 | FP: 461 |
| 114 | 5 | 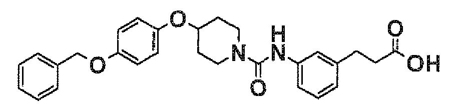 | FP: 475 |
| 115 | 6 | 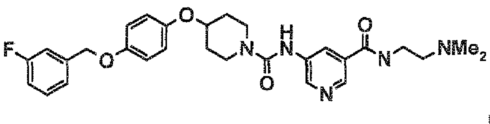 | FP: 536 |
| 116 | 6 | 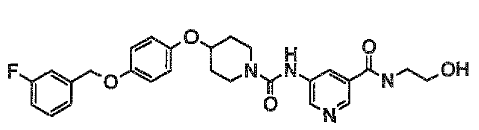 | FP: 509 |
| 117 | 6 | 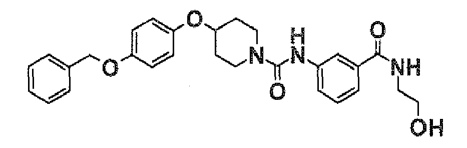 | FP: 490 |
| 118 | 6 | 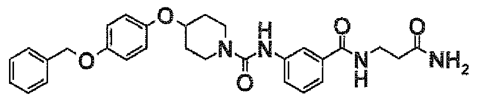 | FP: 517 |
| 119 | 6 | 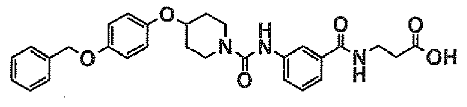 | FP: 518 |
| 120 | 7 | 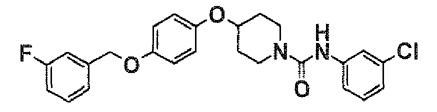 | FP: 455 |
| 121 | 7 | 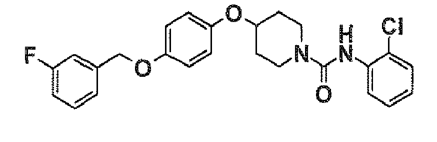 | EP: 455 |

**[Table 32]**

| Ex | Syn | Str | Dat |
|---|---|---|---|
| 122 | 7 | 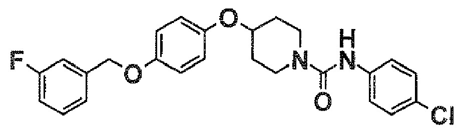 | EP: 455 |
| 123 | 7 | 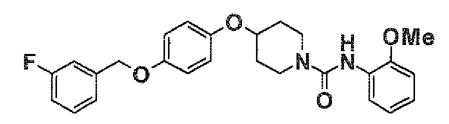 | FP: 451 |
| 124 | 7 | 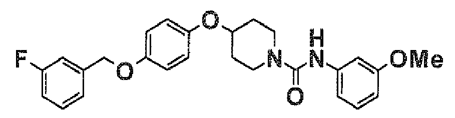 | FP: 451 |
| 125 | 7 | 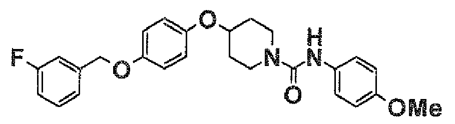 | FP: 451 |
| 126 | 7 | 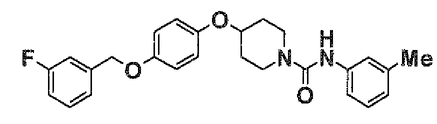 | FP: 435 |
| 127 | 7 | 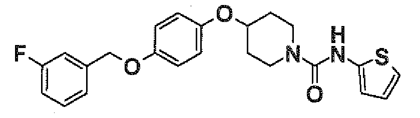 | FP: 427 |
| 128 | 7 | 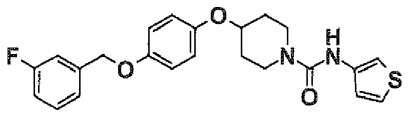 | FP: 427 |
| 129 | 7 | 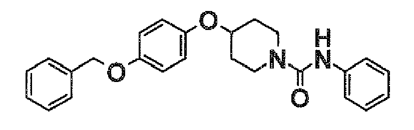 | FN: 401 |
| 130 | 8 | 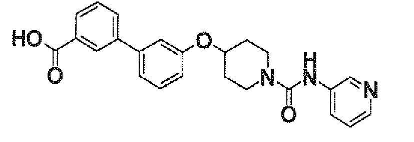 | FN: 415 |

**[Table 33]**

| Ex | Syn | Str | Dat |
|---|---|---|---|
| 131 | 9 | 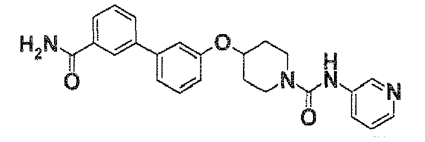 | FP: 417 |
| 132 | 11 | 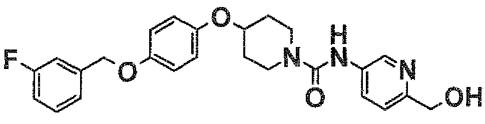 | FP: 452 |
| 133 | 1 | 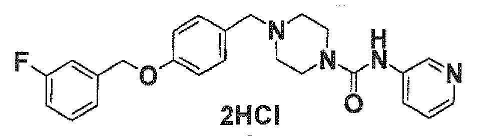 | FP: 421 |
| 134 | 1 | 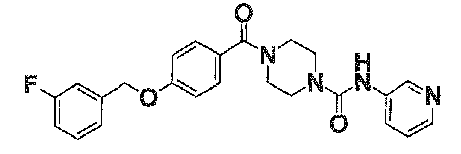 | FP: 435 |
| 135 | 1 | 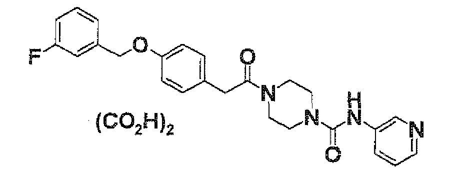 | FP: 449 |
| 136 | 1 | 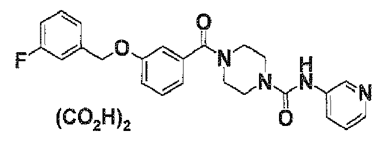 | FP: 435 |
| 137 | 1 | 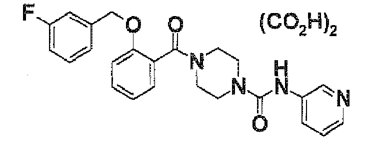 | FP: 435 |
| 138 | 2 | 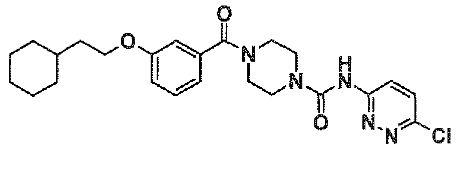 | EP: 472 |

**[Table 34]**

| Ex | Syn | Str | Dat |
|---|---|---|---|
| 139 | 2 | 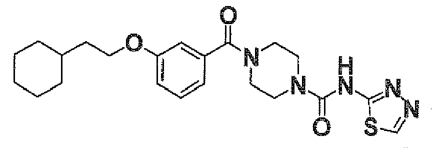 | FP: 444 |
| 140 | 2 | 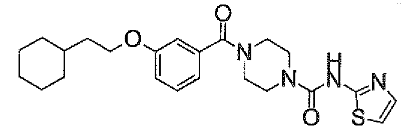 | FP: 443 |
| 141 | 2 | 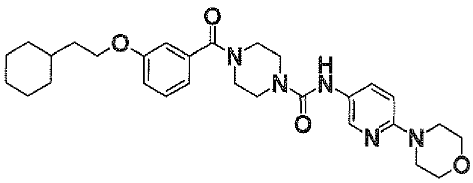 | FP: 522 |
| 142 | 2 | 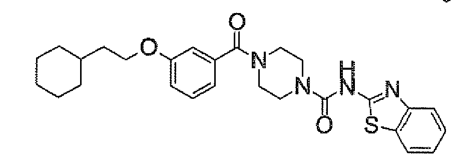 | FP: 493 |
| 143 | 2 | 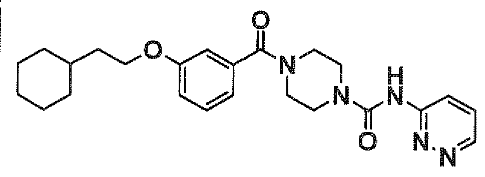 | EP: 438 |
| 144 | 2 | 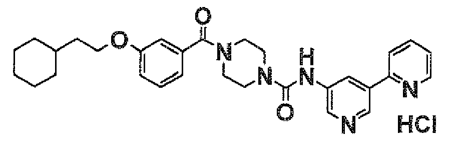 | FP: 514 |
| 145 | 2 | 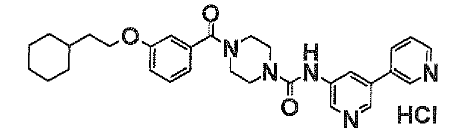 | FN: 512 |
| 146 | 2 | 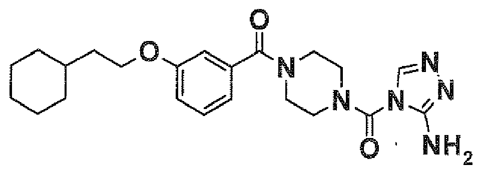 | FP: 427 |

**[Table 35]**

| Ex | Syn | Str | Dat |
|---|---|---|---|
| 147 | 2 | 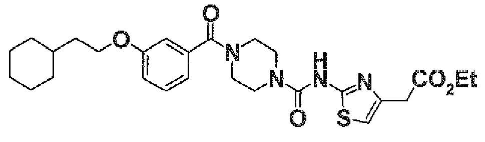 | FP: 529 |
| 148 | 2 | 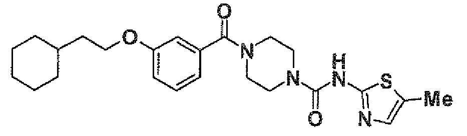 | FP: 457 |
| 149 | 2 | 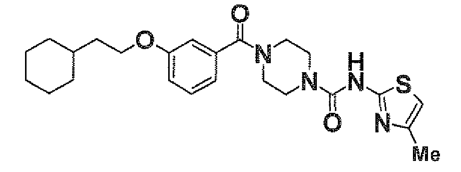 | FP: 457 |
| 150 | 2 | 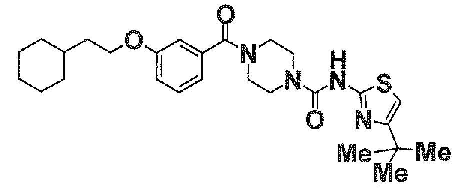 | FP: 499 |
| 151 | 2 | 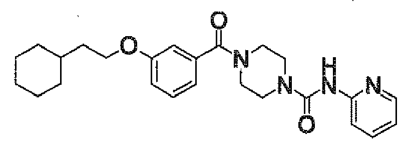 | FP: 437 |
| 152 | 2 | 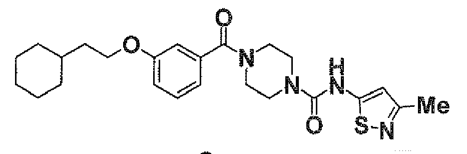 | FP: 457 |
| 153 | 2 | 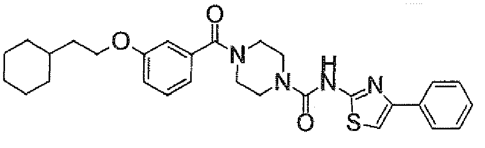 | FP: 519 |
| 154 | 2 | 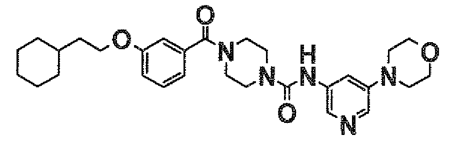 | FP: 522 |

**[Table 36]**

| Ex | Syn | Str | Dat |
|---|---|---|---|
| 155 | 2 | 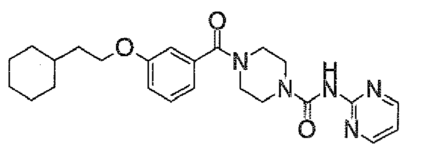 | FP: 438 |
| 156 | 2 | 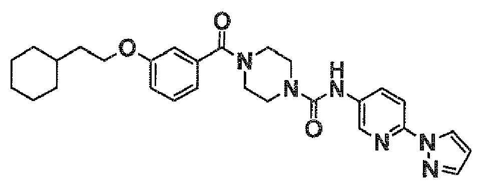 | FP: 503 |
| 157 | 2 | 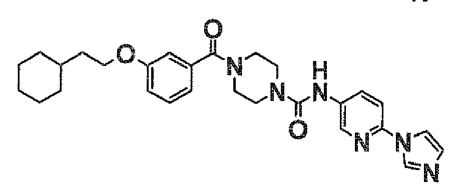 | FN: 501 |
| 158 | 2 | 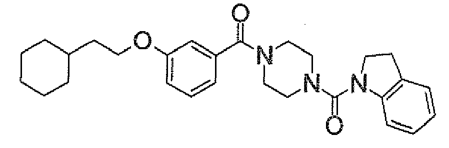 | FP: 462 |
| 159 | 2 | 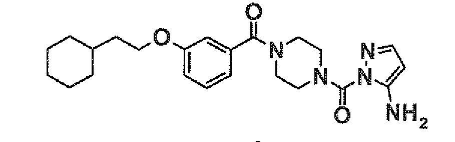 | FP: 426 |
| 160 | 2 | 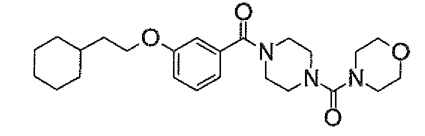 | FP: 430 |
| 161 | 2 | 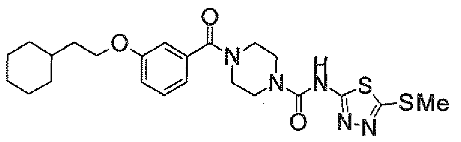 | FP: 490 |
| 162 | 2 | 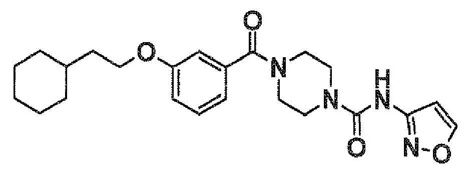 | FP: 427 |

**[Table 37]**

| Ex | Syn | Str | Dat |
|---|---|---|---|
| 163 | 2 | 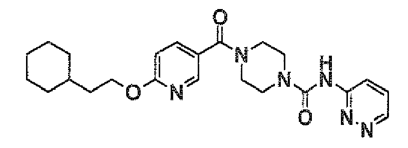 | FP: 439 |
| 164 | 2 | 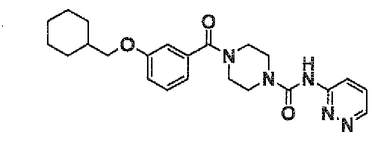 | FP: 424 |
| 165 | 2 | 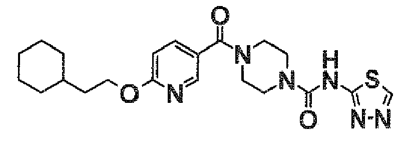 | FP: 445 |
| 166 | 2 | 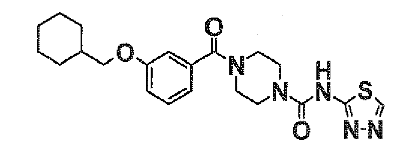 | FP: 430 |
| 167 | 2 | 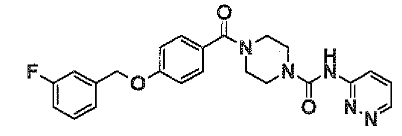 | FP: 436 |
| 168 | 2 | 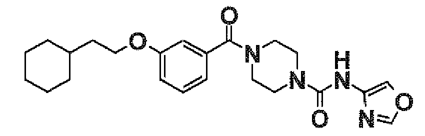 | FP: 427 |
| 169 | 2 | 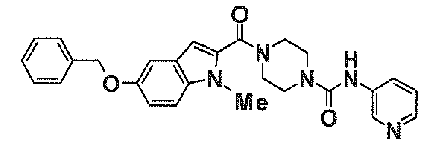 | EP: 470 |
| 170 | 2 | 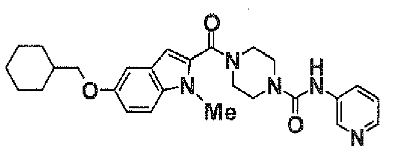 | FP: 476 |
| 171 | 2 | 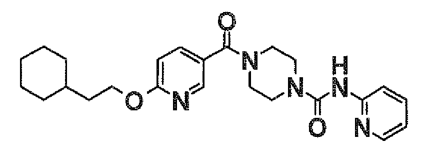 | FP: 438 |

**[Table 38]**

| Ex | Syn | Str | Dat |
|---|---|---|---|
| 172 | 2 | 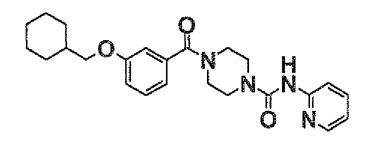 | FP: 423 |
| 173 | 2 | 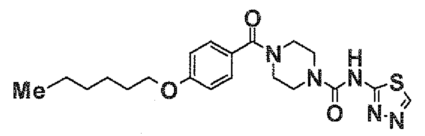 | FP: 418 |
| 174 | 2 | 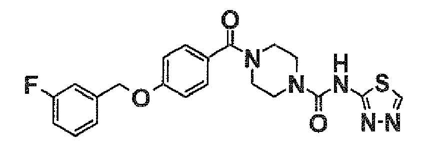 | EP: 442 |
| 175 | 2 | 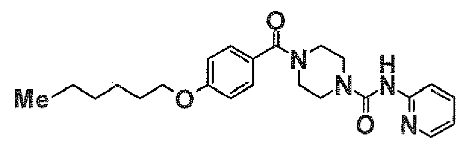 | FP: 411 |
| 176 | 2 | 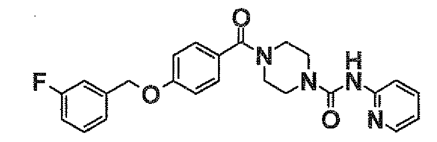 | FP: 435 |
| 177 | 2 | 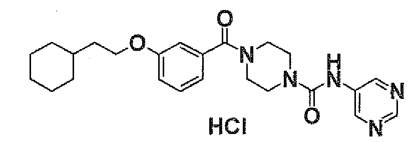 | EP: 438 |
| 178 | 2 | 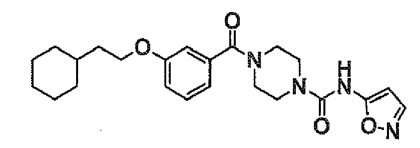 | FP: 427 |
| 179 | 2 | 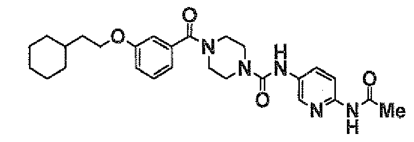 | FP: 494 |
| 180 | 2 | 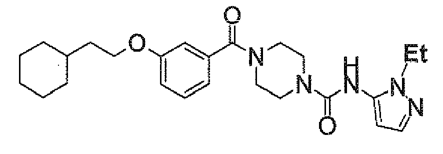 | FP: 454 |

**[Table 39]**

| Ex | Syn | Str | Dat |
|---|---|---|---|
| 181 | 2 | 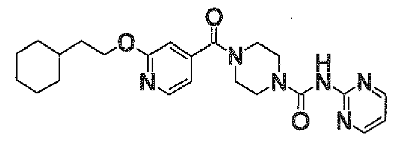 | FP: 439 |
| 182 | 2 | 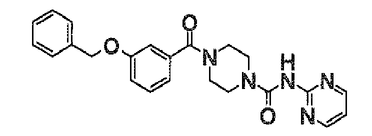 | FP: 418 |
| 183 | 2 | 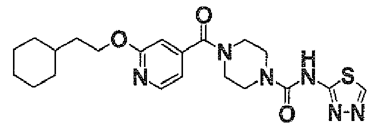 | FP: 445 |
| 184 | 2 | 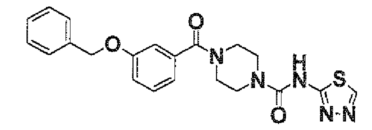 | FP: 424 |
| 185 | 2 | 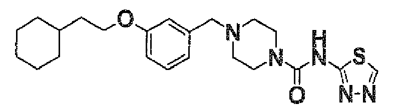 | FP: 430 |
| 186 | 2 | 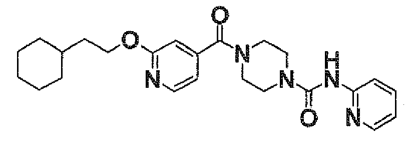 | FP: 438 |
| 187 | 2 | 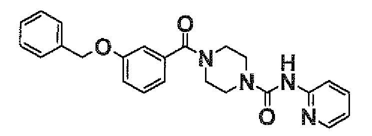 | FP: 417 |
| 188 | 2 | 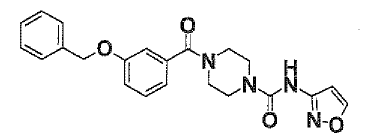 | FN: 405 |
| 189 | 2 | 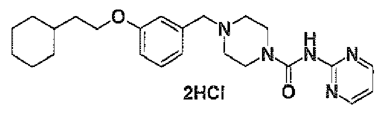 | FP: 424 |

**[Table 40]**

| Ex | Syn | Str | Dat |
|---|---|---|---|
| 190 | 2 | 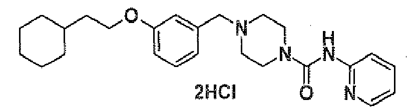 | FP: 423 |
| 191 | 2 | 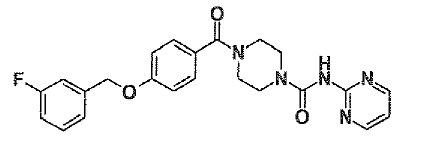 | FP: 436 |
| 192 | 2 | 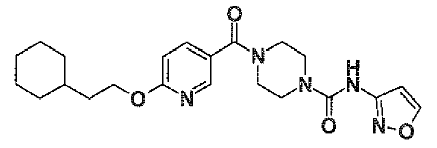 | FP: 428 |
| 193 | 2 | 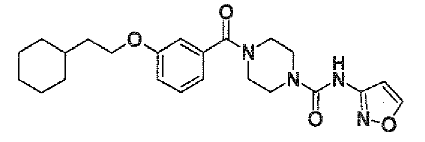 | FP: 413 |
| 194 | 2 | 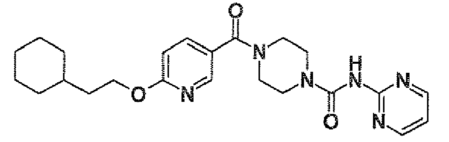 | FP: 439 |
| 195 | 2 | 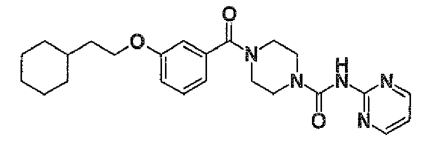 | FP: 424 |
| 196 | 2 | 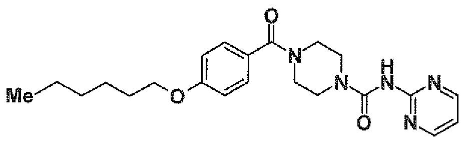 | FP: 412 |
| 197 | 2 | 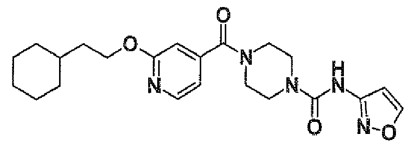 | FP: 428 |
| 198 | 2 | 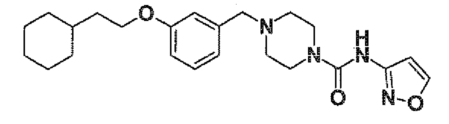 | FP: 413 |

**[Table 41]**

| Ex | Syn | Str | Dat |
|---|---|---|---|
| 199 | 2 | 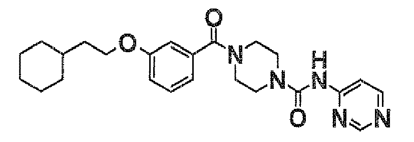 | FP: 438 |
| 200 | 2 | 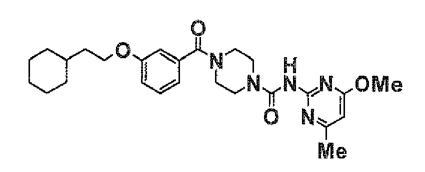 | FP: 482 |
| 201 | 2 | 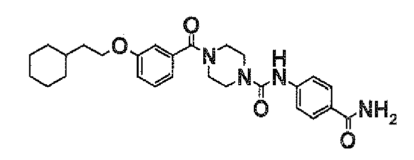 | FP: 479 |
| 202 | 2 | 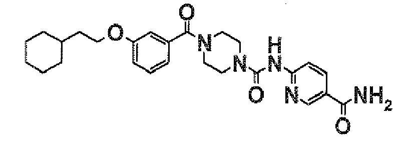 | FP: 480 |
| 203 | 2 | 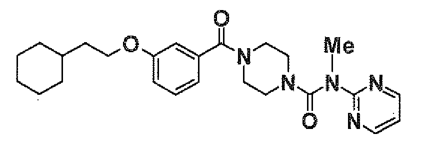 | FP: 452 |
| 204 | 2 | 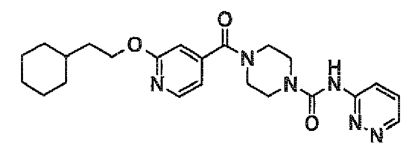 | FP: 439 |
| 205 | 2 | 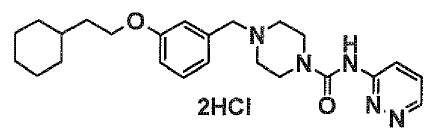 | FP: 424 |
| 206 | 2 | 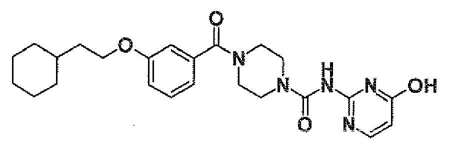 | FP: 454 |
| 207 | 2 | 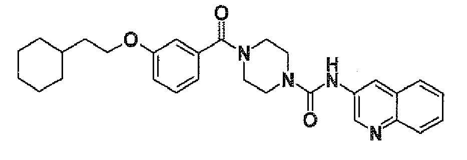 | FP: 487 |

**[Table 42]**

| Ex | Syn | Str | Dat |
|---|---|---|---|
| 208 | 2 | 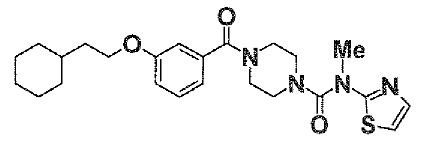 | FP: 457 |
| 209 | 2 | 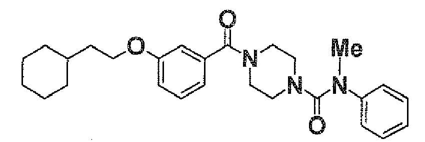 | FP: 450 |
| 210 | 2 | 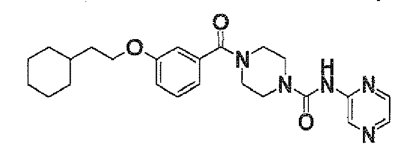 | FP: 488 |
| 211 | 2 | 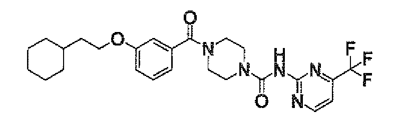 | FP: 506 |
| 212 | 2 | 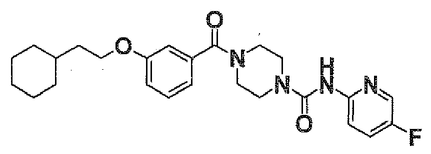 | FP: 455 |
| 213 | 2 | 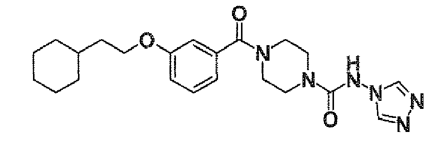 | FP: 427 |
| 214 | 2 | 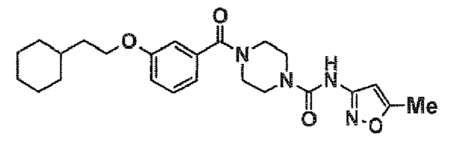 | FP: 441 |
| 215 | 2 | 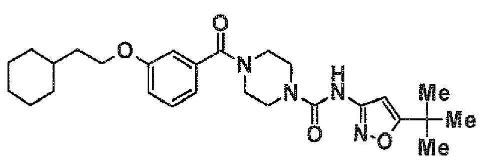 | EP: 483 |
| 216 | 2 | 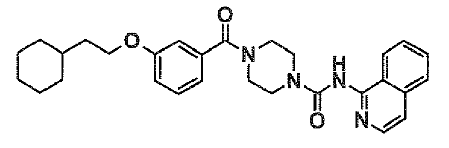 | FP: 487 |

**[Table 43]**

| Ex | Syn | Str | Dat |
|---|---|---|---|
| 217 | 2 | 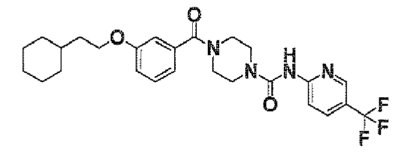 | FP: 505 |
| 218 | 2 | 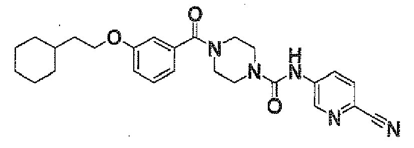 | FP: 462 |
| 219 | 2 | 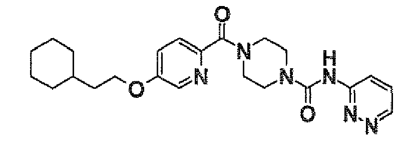 | FP: 439 |
| 220 | 2 | 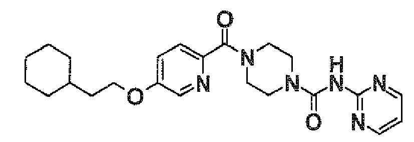 | FP: 439 |
| 221 | 2 | 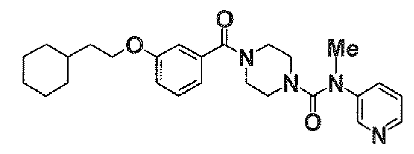 | FP: 451 |
| 222 | 2 | 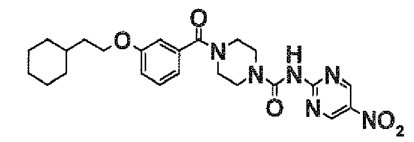 | EP: 483 |
| 223 | 2 | 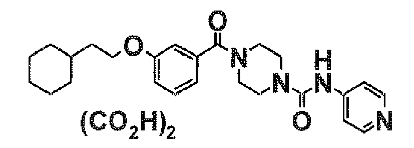 | FP: 437 |
| 224 | 2 | 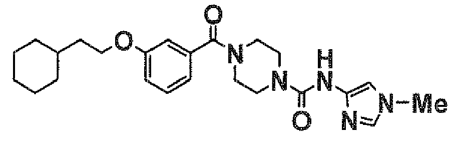 | FP: 440 |
| 225 | 2 | 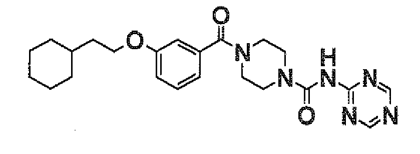 | FP: 439 |

**[Table 44]**

| Ex | Syn | Str | Dat |
|---|---|---|---|
| 226 | 2 | 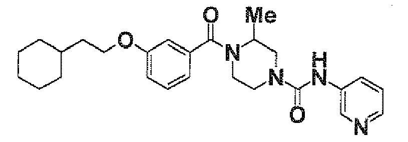 | FP: 451 |
| 227 | 2 | 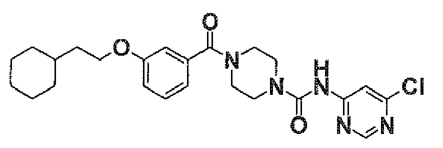 | FN: 470 |
| 228 | 2 | 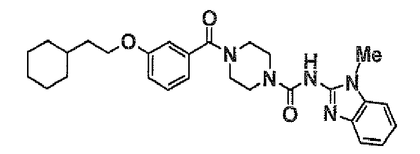 | FP: 490 |
| 229 | 2 | 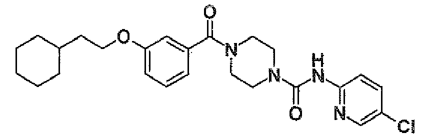 | EP: 471 |
| 230 | 2 | 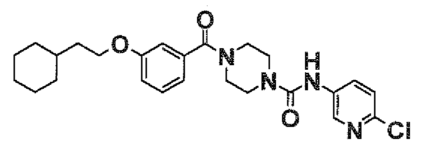 | FP: 471 |
| 231 | 2 | 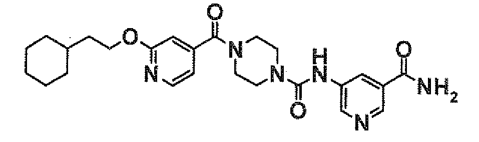 | EP: 481 |
| 232 | 2 | 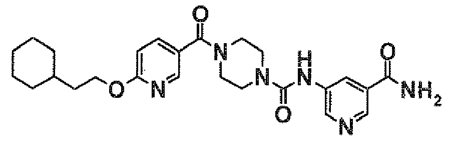 | EP: 481 |
| 233 | 2 | 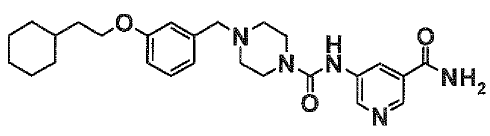 | FP: 466 |
| 234 | 2 | 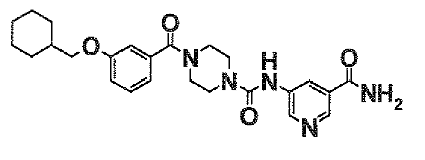 | EP: 466 |

**[Table 45]**

| Ex | Syn | Str | Dat |
|---|---|---|---|
| 235 | 2 | 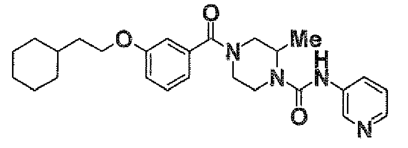 | FP: 451 |
| 236 | 2 | 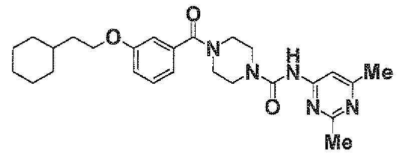 | FP: 466 |
| 237 | 2 | 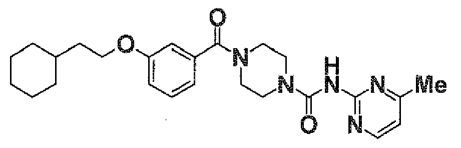 | FP: 452 |
| 238 | 2 | 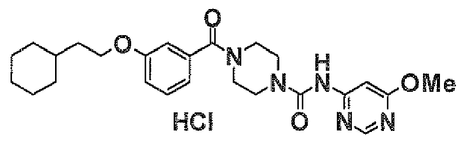 | FP: 468 |
| 239 | 2 | 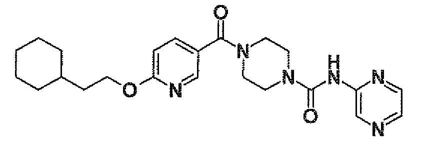 | FP: 439 |
| 240 | 2 | 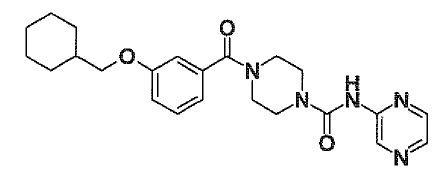 | FP: 424 |
| 241 | 2 | 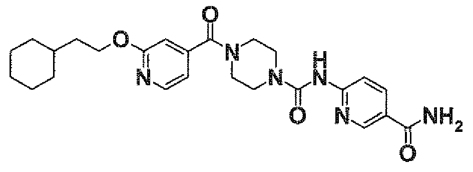 | FP: 481 |
| 242 | 2 | 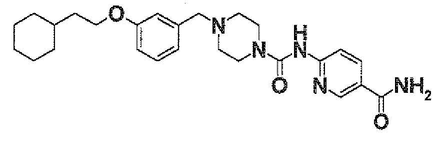 | FP: 466 |

**[Table 46]**

| Ex | Syn | Str | Dat |
|---|---|---|---|
| 243 | 2 | 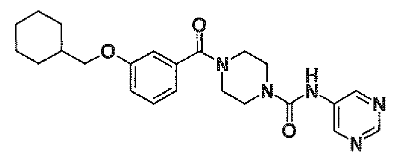 | FP: 424 |
| 244 | 2 | 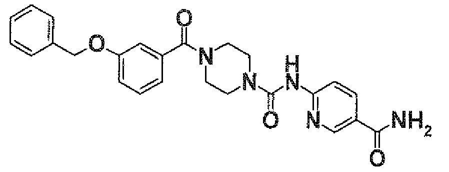 | EP: 460 |
| 245 | 2 | 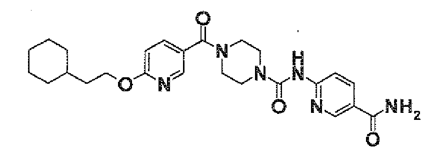 | FP: 481 |
| 246 | 2 | 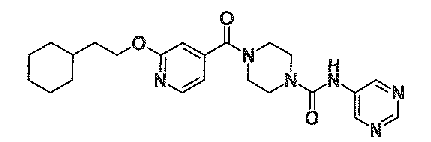 | FP: 439 |
| 247 | 2 | 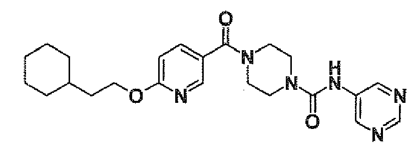 | FP: 439 |
| 248 | 2 | 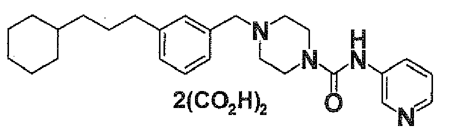 | FP: 421 |
| 249 | 2 | 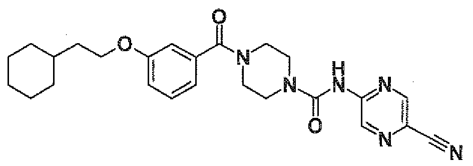 | EP: 463 |
| 250 | 2 | 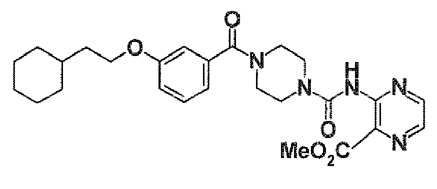 | EP: 496 |

**[Table 47]**

| Ex | Syn | Str | Dat |
|---|---|---|---|
| 251 | 2 | 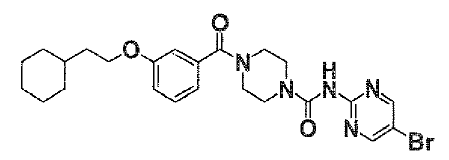 | EP: 516 |
| 252 | 2 | 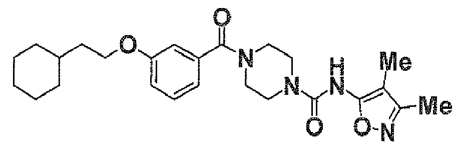 | FP: 455 |
| 253 | 2 | 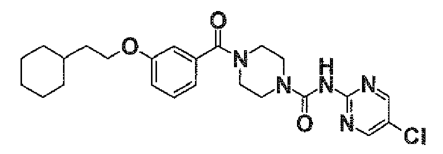 | FP: 472 |
| 254 | 2 | 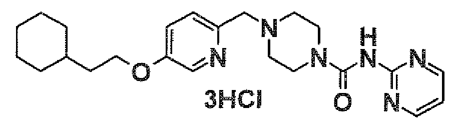 | FP: 425 |
| 255 | 2 | 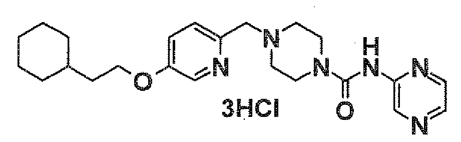 | FP: 425 |
| 256 | 2 | 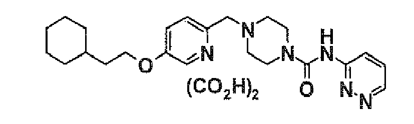 | FP: 425 |
| 257 | 2 | 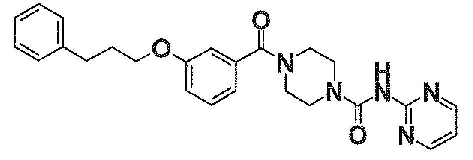 | FP: 446 |
| 258 | 2 | 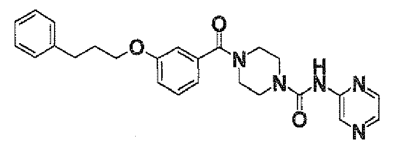 | FP: 446 |
| 259 | 2 | 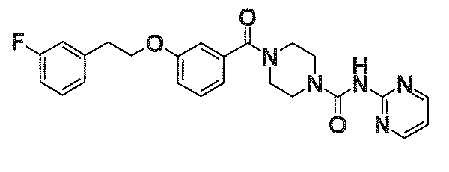 | FP: 450 |

**[Table 48]**

| Ex | Syn | Str | Dat |
|---|---|---|---|
| 260 | 2 | 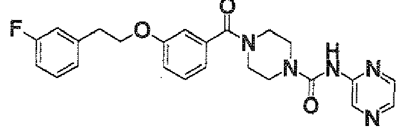 | FP: 450 |
| 261 | 2 | 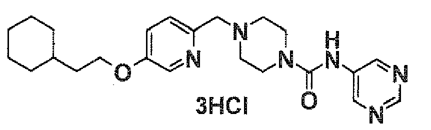 | FP: 425 |
| 262 | 2 | 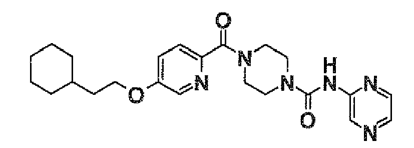 | FP: 439 |
| 263 | 2 | 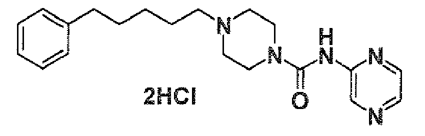 | FP: 354 |
| 264 | 2 | 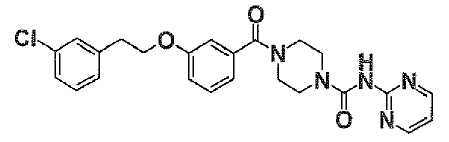 | EP: 466 |
| 265 | 2 | 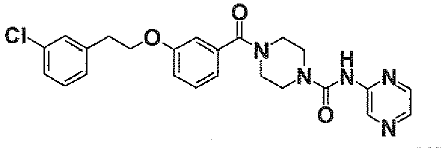 | EP: 466 |
| 266 | 2 | 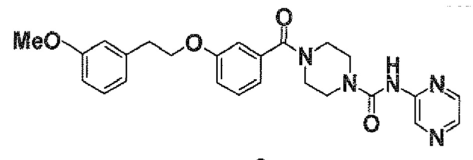 | FP: 462 |
| 267 | 2 | 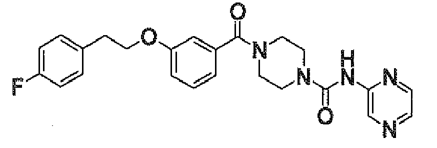 | FP: 450 |
| 268 | 2 | 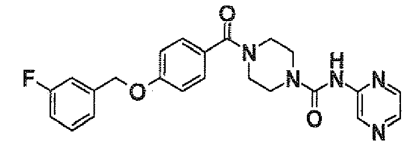 | FP: 436 |

**[Table 49]**

| Ex | Syn | Str | Dat |
|---|---|---|---|
| 269 | 2 | 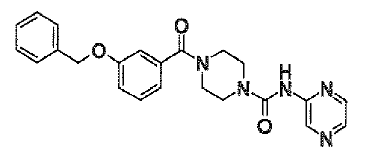 | FP: 418 |
| 270 | 2 | 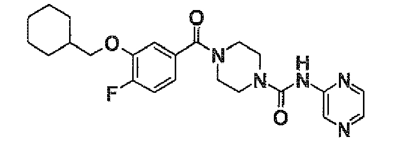 | EP: 442 |
| 271 | 2 | 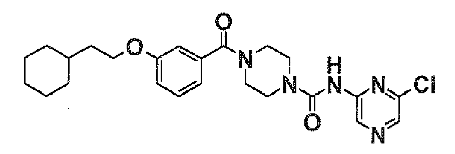 | FP: 471 [M⁺] |
| 272 | 2 | 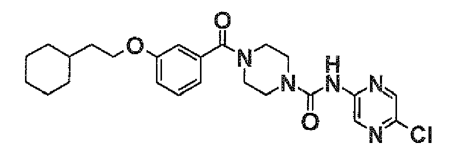 | EP: 472 |
| 273 | 2 | 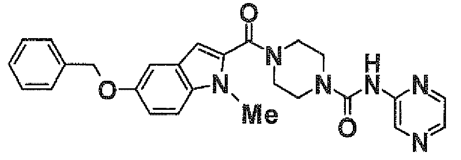 | FP: 471 |
| 274 | 2 | 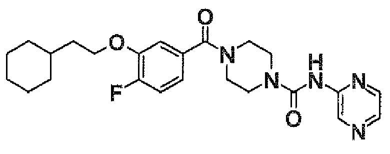 | EP: 456 |
| 275 | 2 | 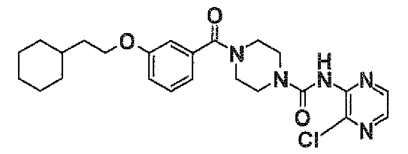 | FP: 472 |
| 276 | 2 | 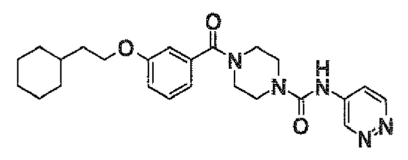 | FP: 438 |

**[Table 50]**

| Ex | Syn | Str | Dat |
|---|---|---|---|
| 277 | 3 | 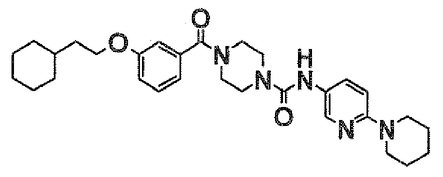 | FP: 520 |
| 278 | 3 | 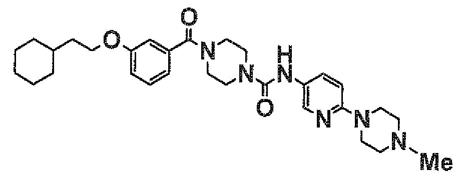 | EP: 535 |
| 279 | 3 | 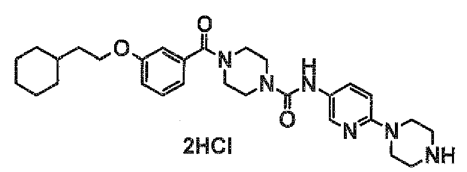 | FP: 521 |
| 280 | 5 | 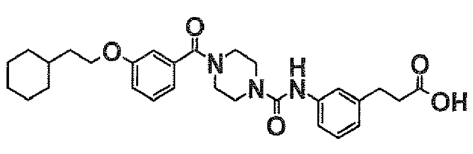 | FP: 508 |
| 281 | 5 | 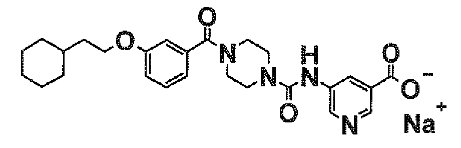 | FP: 503 |
| 282 | 5 | 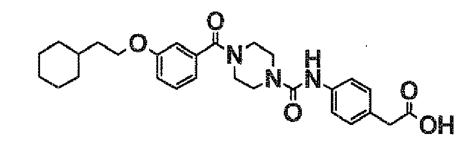 | FP: 494 |
| 283 | 5 | 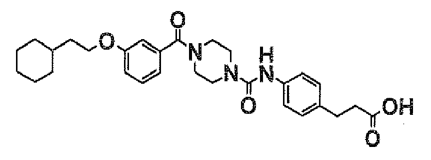 | FN: 506 |
| 284 | 5 | 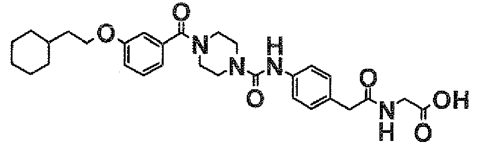 | FN: 549 |

**[Table 51]**

| Ex | Syn | Str | Dat |
|---|---|---|---|
| 285 | 5 | 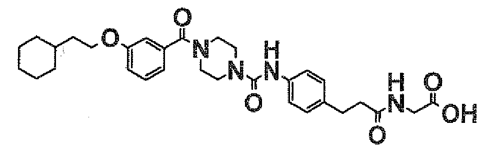 | FP: 565 |
| 286 | 5 | 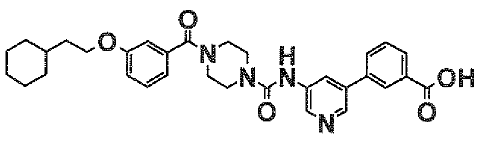 | FN: 555 |
| 287 | 5 | 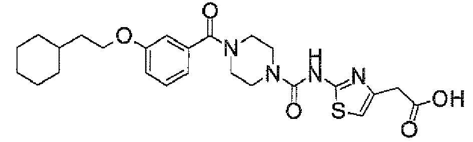 | FP: 501 |
| 288 | 5 | 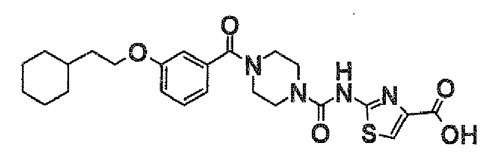 | EP: 487 |
| 289 | 5 | 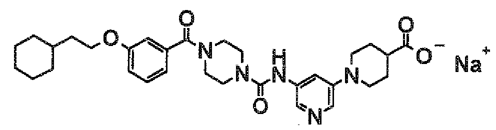 | FP: 564 |
| 290 | 5 | 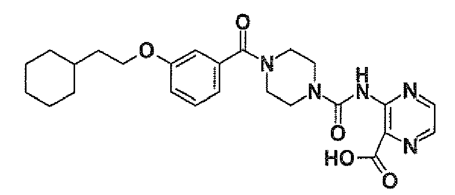 | FP: 482 |
| 291 | 6 | 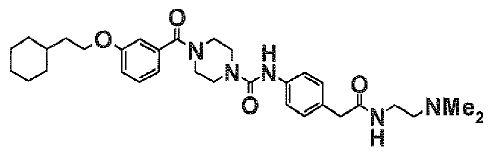 | FP: 564 |
| 292 | 6 | 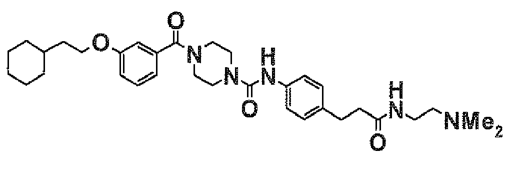 | FP: 578 |

**[Table 52]**

| Ex | Syn | Str | Dat |
|---|---|---|---|
| 293 | 7 | 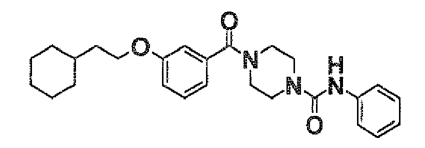 | FP: 436 |
| 294 | 9 | 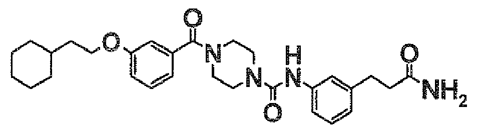 | FP: 507 |
| 295 | 9 | 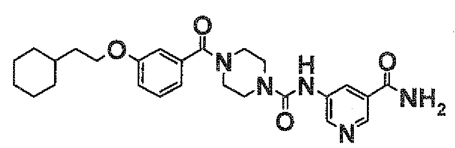 | FP: 480 |
| 296 | 9 | 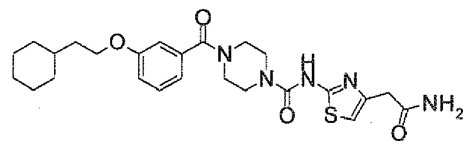 | FP: 500 |
| 297 | 9 | 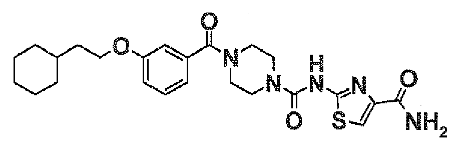 | FP: 486 |
| 298 | 9 | 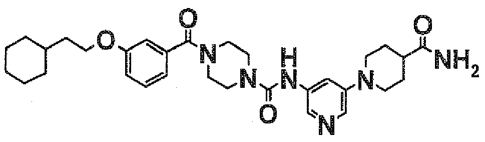 | FP: 563 |
| 299 | 9 | 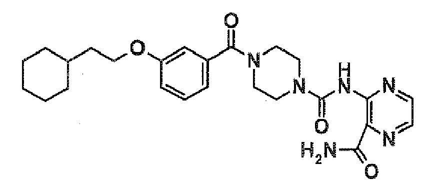 | EP: 481 |
| 300 | 12 | 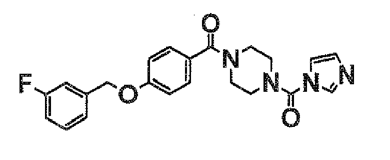 | FP: 409 |

**[Table 53]**

| Ex | Syn | Str | Dat |
|---|---|---|---|
| 301 | 16 | 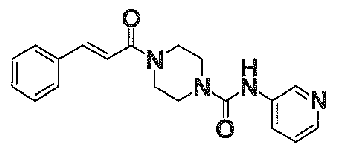 | FP: 337 |
| 302 | 16 | 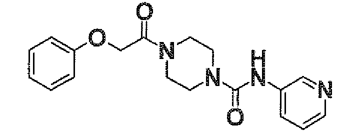 | FP: 341 |
| 303 | 16 | 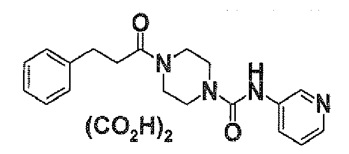 | FP: 339 |
| 304 | 16 | 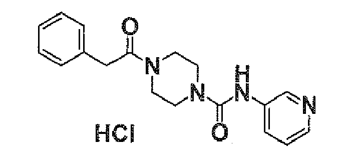 | FP: 325 |
| 305 | 16 | 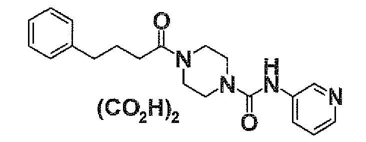 | FP: 353 |
| 306 | 16 | 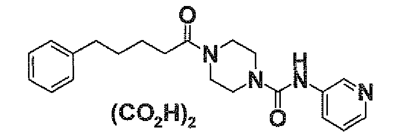 | EP: 367 |
| 307 | 16 | 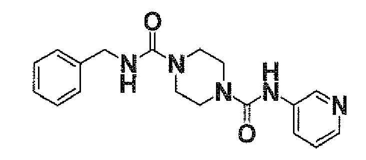 | FP: 340 |
| 308 | 16 | 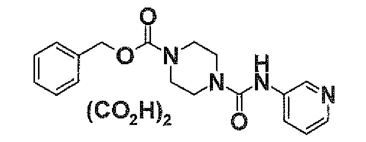 | FP: 341 |
| 309 | 16 | 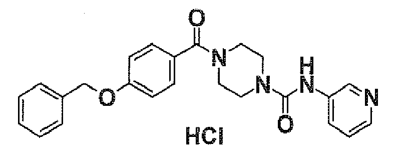 | FP: 417 |

**[Table 54]**

| Ex | Syn | Str | Dat |
|---|---|---|---|
| 310 | 16 | 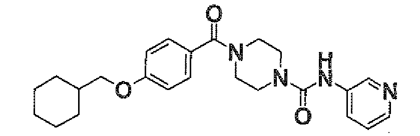 | FP: 423 |
| 311 | 16 | 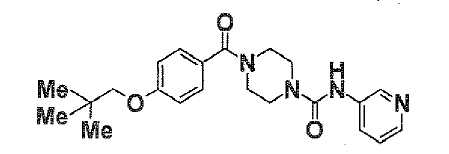 | EP: 397 |
| 312 | 16 | 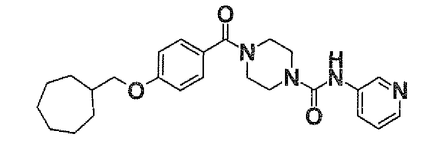 | EP: 437 |
| 313 | 16 | 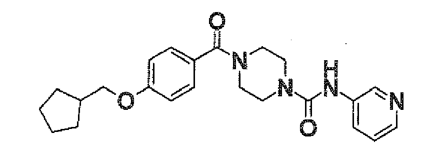 | FP: 409 |
| 314 | 16 | 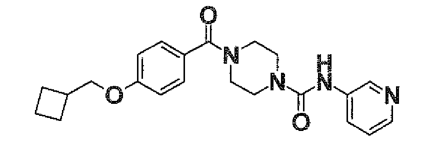 | FP: 395 |
| 315 | 16 | 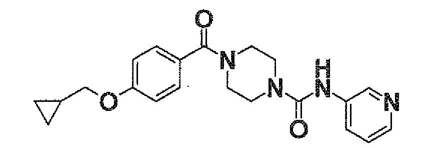 | FP: 381 |
| 316 | 16 | 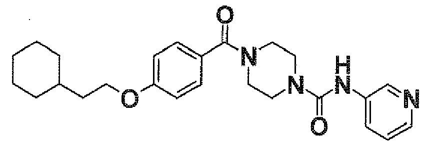 | FP: 437 |
| 317 | 16 | 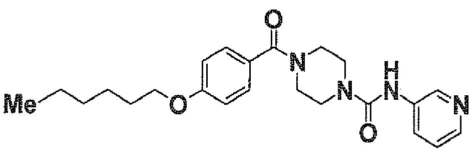 | FP: 411 |
| 318 | 16 | 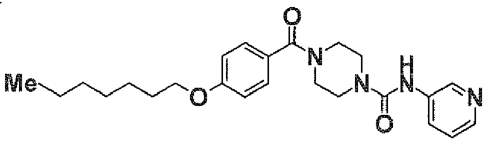 | FP: 425 |

**[Table 55]**

| Ex | Syn | Str | Dat |
|---|---|---|---|
| 319 | 16 | 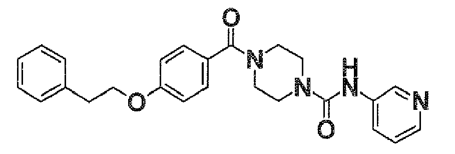 | FP: 431 |
| 320 | 16 | 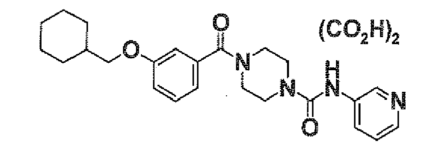 | FP: 423 |
| 321 | 16 | 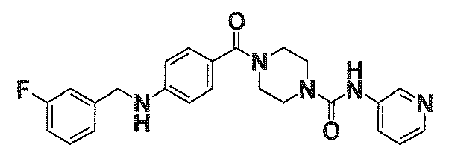 | FP: 434 |
| 322 | 16 | 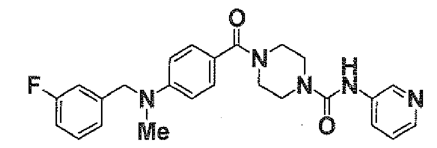 | FP: 448 |
| 323 | 16 | 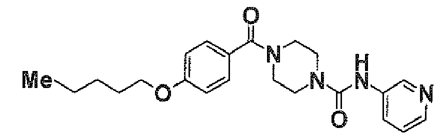 | FP: 397 |
| 324 | 16 | 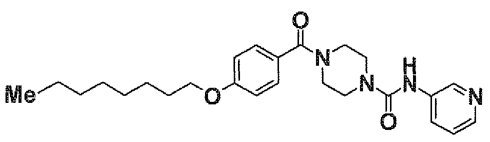 | FP: 439 |
| 325 | 16 | 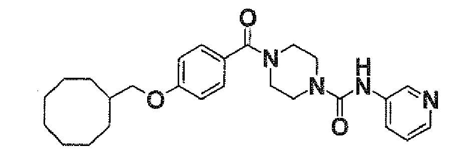 | FP: 451 |
| 326 | 16 | 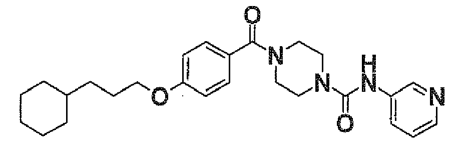 | FP: 451 |
| 327 | 16 | 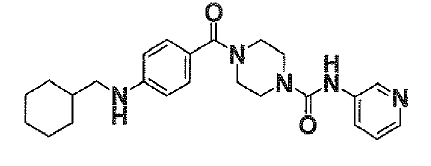 | FP: 422 |

**[Table 56]**

| Ex | Syn | Str | Dat |
|---|---|---|---|
| 328 | 16 | 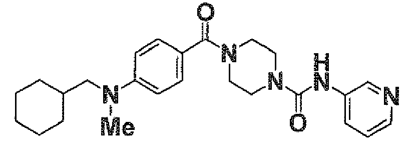 | FP: 436 |
| 329 | 16 | 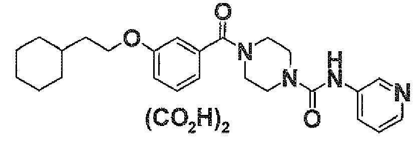 | FP: 437 |
| 330 | 16 | 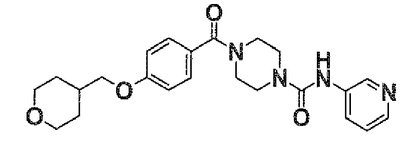 | FP: 425 |
| 331 | 16 | 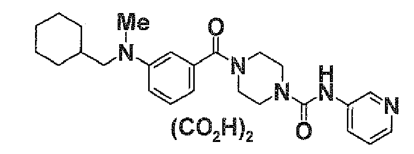 | FP: 436 |
| 332 | 16 | 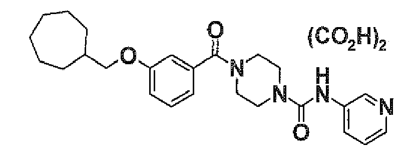 | FP: 437 |
| 333 | 16 | 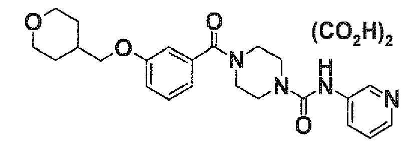 | FP: 425 |
| 334 | 16 | 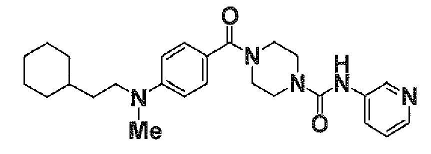 | FP: 450 |
| 335 | 16 | 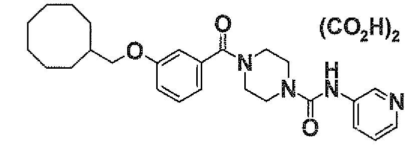 | FP: 451 |

**[Table 57]**

| Ex | Syn | Str | Dat |
|---|---|---|---|
| 336 | 16 | 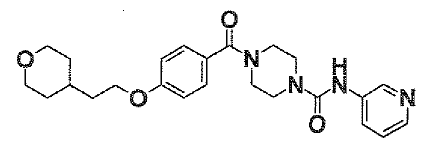 | FP: 439 |
| 337 | 16 | 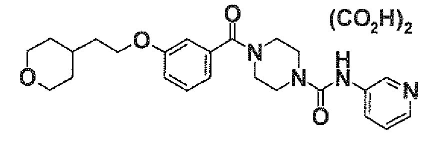 | FP: 439 |
| 338 | 16 | 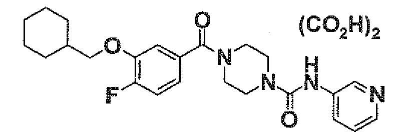 | FP: 441 |
| 339 | 16 | 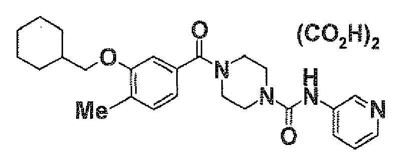 | FP: 437 |
| 340 | 16 | 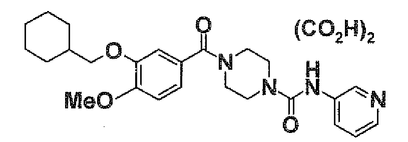 | FP: 453 |
| 341 | 16 | 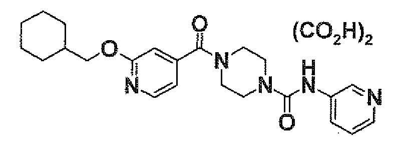 | FP: 424 |
| 342 | 16 | 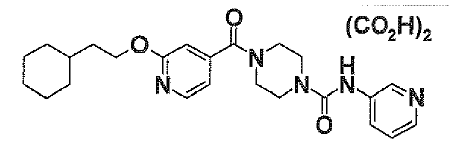 | FP: 438 |
| 343 | 16 | 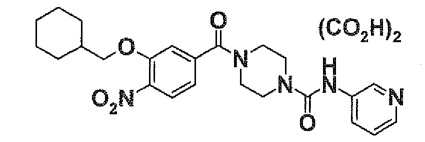 | FP: 468 |
| 344 | 16 | 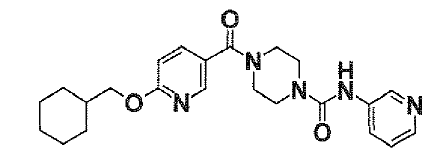 | FP: 424 |

**[Table 58]**

| Ex | Syn | Str | Dat |
|---|---|---|---|
| 345 | 16 | 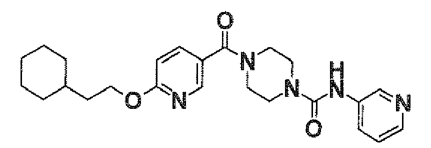 | FP: 438 |
| 346 | 16 | 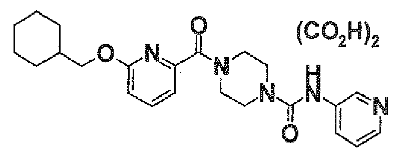 | FP: 424 |
| 347 | 16 | 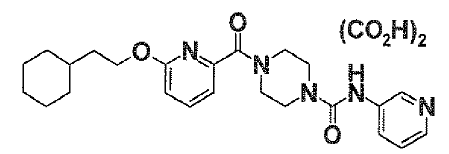 | FP: 438 |
| 348 | 16 | 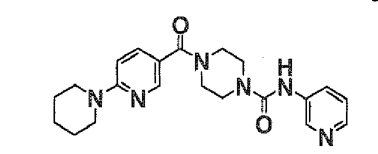 | EP: 395 |
| 349 | 16 | 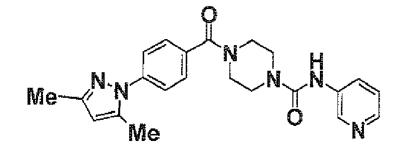 | FP: 405 |
| 350 | 16 | 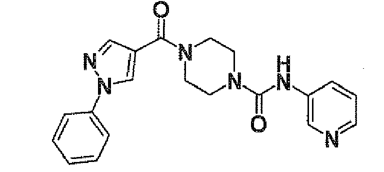 | EP: 377 |
| 351 | 16 | 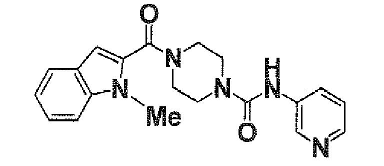 | EP: 364 |
| 352 | 16 | 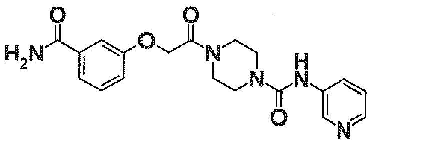 | EP: 384 |

**[Table 59]**

| Ex | Syn | Str | Dat |
|---|---|---|---|
| 353 | 16 | 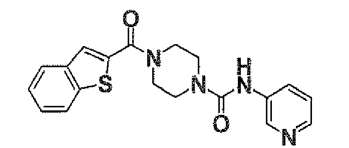 | EP: 367 |
| 354 | 16 | 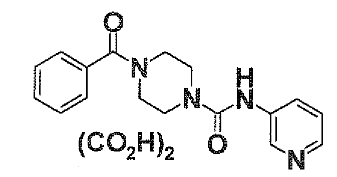 | EP: 311 |
| 355 | 16 | 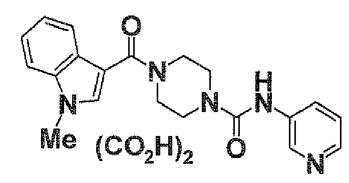 | EP: 364 |
| 356 | 16 | 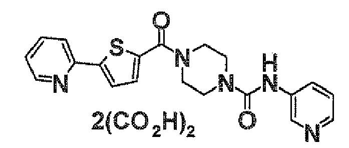 | EP: 394 |
| 357 | 16 | 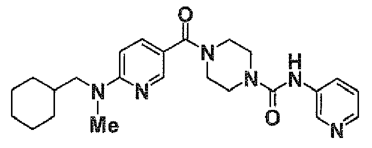 | FP: 437 |
| 358 | 16 | 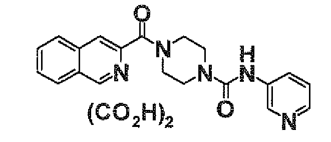 | FN: 360 |
| 359 | 16 | 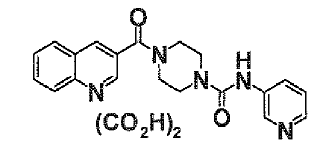 | EP: 362 |
| 360 | 16 | 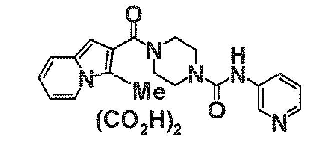 | EP: 364 |

**[Table 60]**

| Ex | Syn | Str | Dat |
|---|---|---|---|
| 361 | 16 | 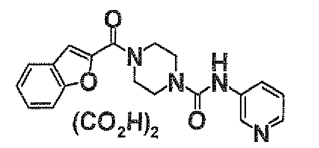 | EP: 351 |
| 362 | 16 | 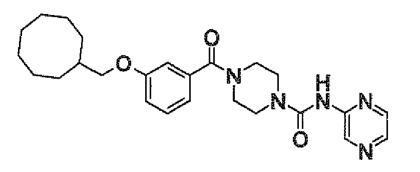 | EP: 452 |
| 363 | 16 | 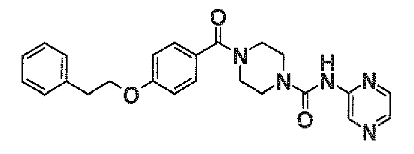 | FP: 432 |
| 364 | 16 | 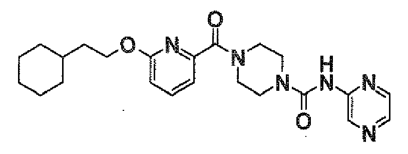 | FP: 439 |
| 365 | 16 | 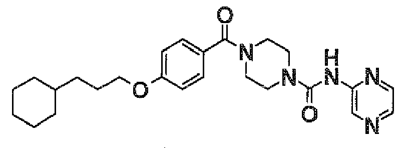 | FP: 452 |
| 366 | 16 | 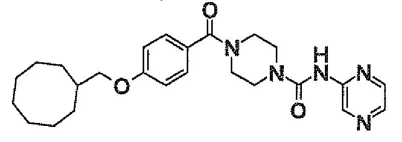 | FP: 452 |
| 367 | 16 | 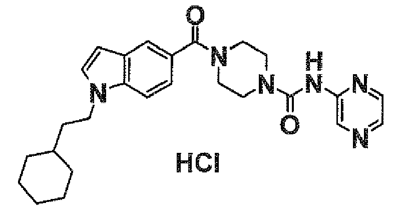 | FP: 461 |
| 368 | 16 | 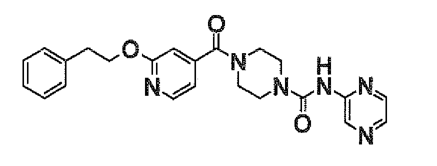 | FP: 433 |

**[Table 61]**

| Ex | Syn | Str | Dat |
|---|---|---|---|
| 369 | 16 | 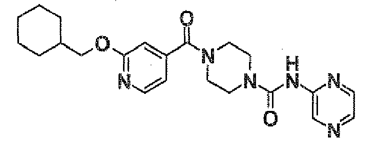 | FP: 425 |
| 370 | 16 | 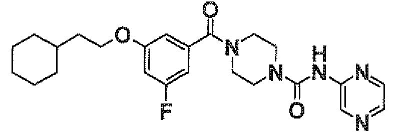 | EP: 456 |
| 371 | 16 | 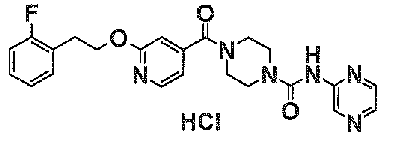 | EP: 451 |
| 372 | 16 | 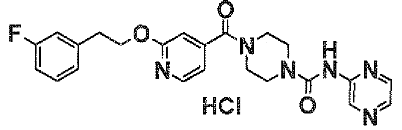 | EP: 451 |
| 373 | 16 | 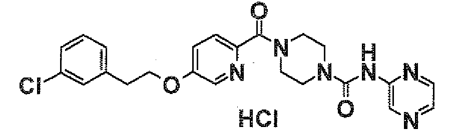 | FP: 467 |
| 374 | 16 | 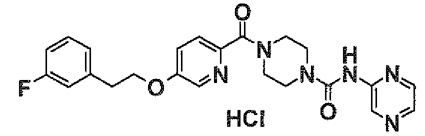 | FP: 451 |
| 375 | 16 | 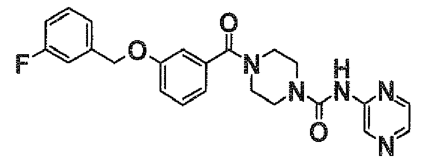 | FP: 436 |
| 376 | 16 | 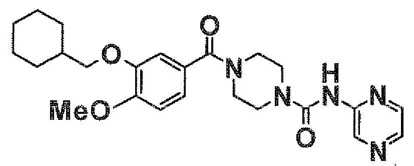 | FP: 454 |

**[Table 62]**

| Ex | Syn | Str | Dat |
|---|---|---|---|
| 377 | 16 | 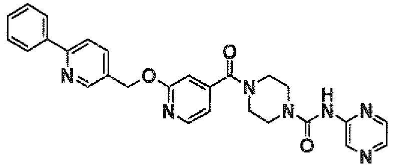 | EP: 496 |
| 378 | 17 | 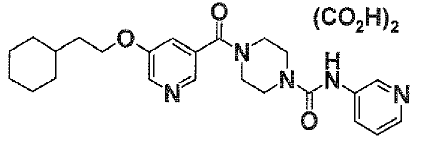 | FP: 438 |
| 379 | 18 | 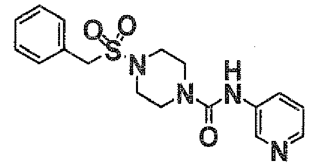 | EP: 361 |
| 380 | 18 | 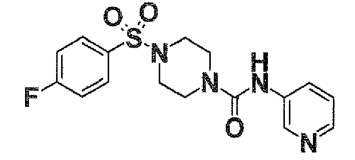 | FP: 365 |
| 381 | 19 | 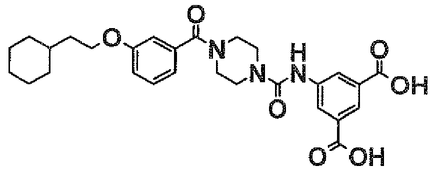 | FP: 524 |
| 382 | 21 | 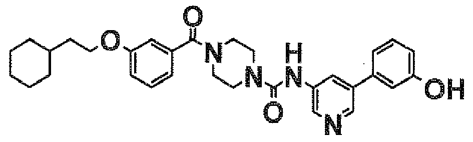 | FP: 529 |
| 383 | 24 | 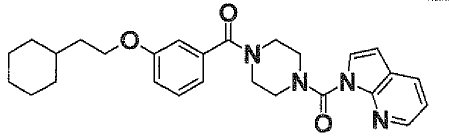 | EP: 461 |
| 384 | 24 | 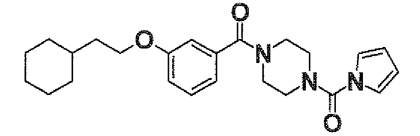 | EP: 410 |

**[Table 63]**

| Ex | Syn | Str | Dat |
|---|---|---|---|
| 385 | 24 | 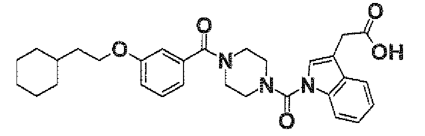 | FN: 516 |
| 386 | 25 | 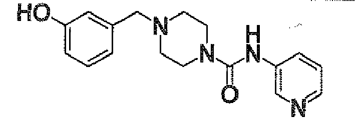 | FP: 313 |
| 387 | 26 | 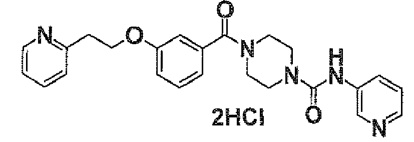 | FP: 432 |
| 388 | 26 | 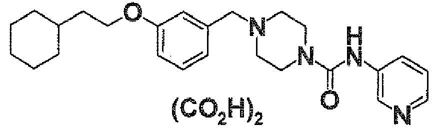 | FP: 423 |
| 389 | 26 | 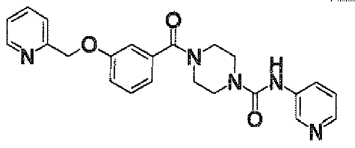 | EP: 418 |
| 390 | 26 | 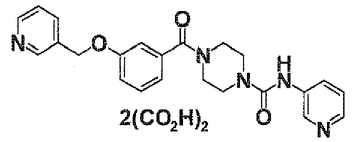 | EP: 418 |
| 391 | 26 | 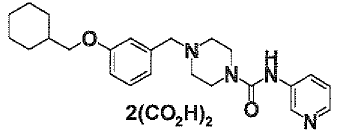 | FP: 409 |
| 392 | 26 | 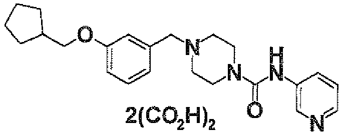 | FP: 395 |
| 393 | 26 | 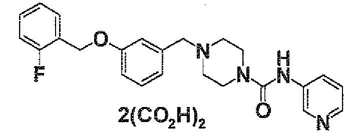 | FP: 421 |

**[Table 64]**

| Ex | Syn | Str | Dat |
|---|---|---|---|
| 394 | 26 | 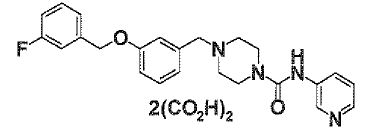 | EP: 421 |
| 395 | 26 | 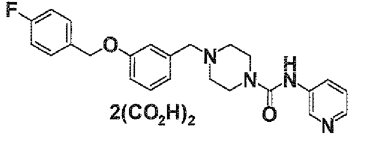 | EP: 421 |
| 396 | 26 | 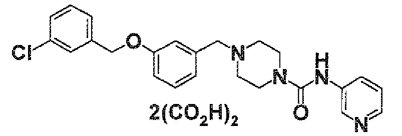 | FP: 437 |
| 397 | 26 | 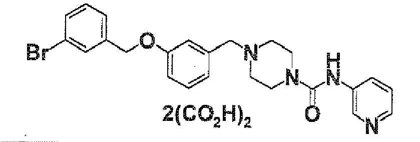 | EP: 481 |
| 398 | 26 | 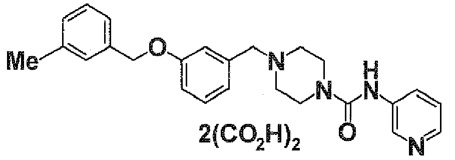 | FP: 417 |
| 399 | 26 | 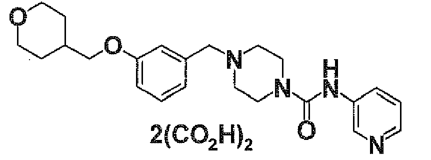 | EP: 411 |
| 400 | 26 | 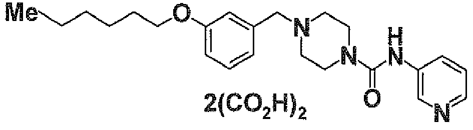 | EP: 397 |
| 401 | 26 | 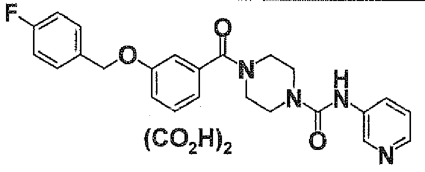 | EP: 435 |

**[Table 65]**

| Ex | Syn | Str | Dat |
|---|---|---|---|
| 402 | 26 | 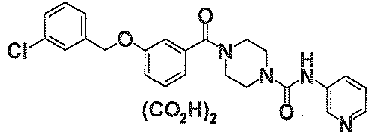 | EP: 451 |
| 403 | 26 | 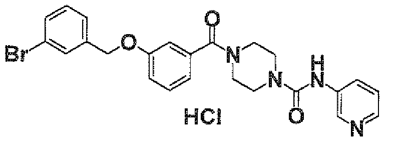 | EP: 497 |
| 404 | 26 | 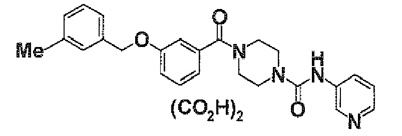 | EP: 431 |
| 405 | 26 | 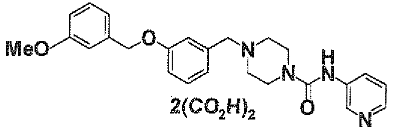 | FP: 433 |
| 406 | 26 | 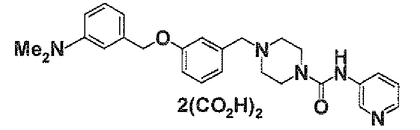 | FP: 446 |
| 407 | 26 | 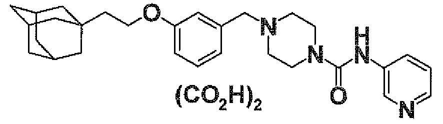 | FP: 475 |
| 408 | 26 | 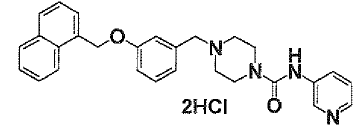 | EP: 453 |
| 409 | 26 | 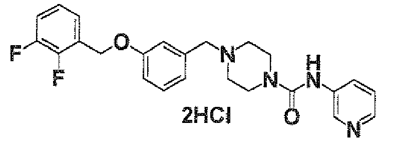 | EP: 439 |
| 410 | 26 | 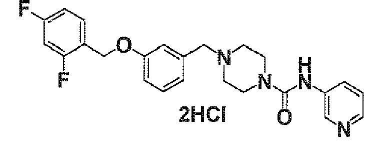 | FP: 439 |

**[Table 66]**

| Ex | Syn | Str | Dat |
|---|---|---|---|
| 411 | 26 | 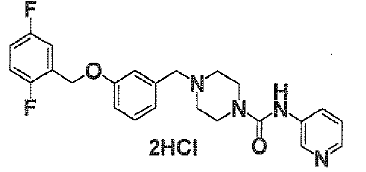 | FP: 439 |
| 412 | 26 | 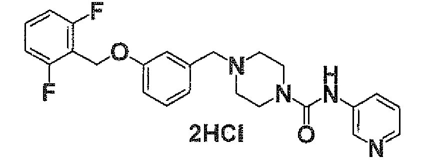 | FP: 439 |
| 413 | 26 | 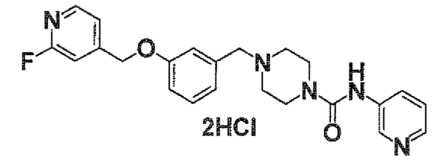 | FP: 422 |
| 414 | 26 | 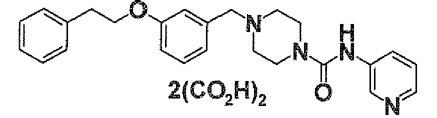 | FP: 417 |
| 415 | 26 | 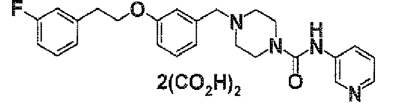 | FP: 435 |
| 416 | 26 | 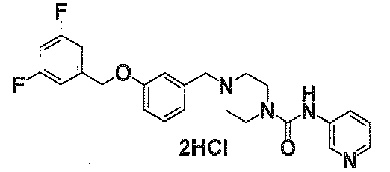 | EP: 439 |
| 417 | 26 | 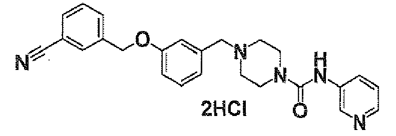 | EN: 426 |
| 418 | 26 | 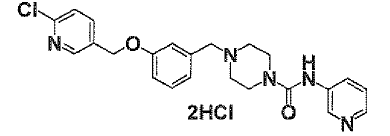 | EN: 436 |

**[Table 67]**

| Ex | Syn | Str | Dat |
|---|---|---|---|
| 419 | 26 | 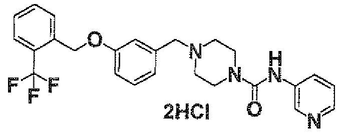 | EP: 471 |
| 420 | 26 | 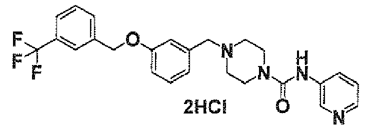 | EP: 471 |
| 421 | 26 | 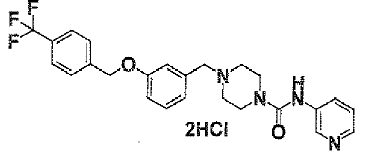 | EP: 471 |
| 422 | 26 | 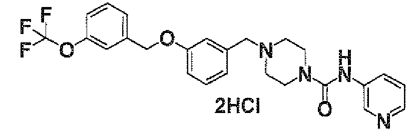 | EP: 487 |
| 423 | 26 | 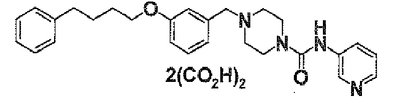 | FP: 445 |
| 424 | 26 | 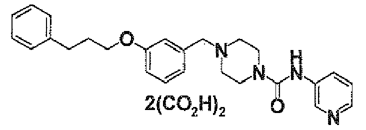 | FP: 431 |
| 425 | 26 | 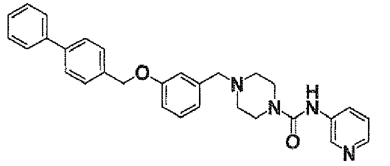 | FP: 479 |
| 426 | 26 | 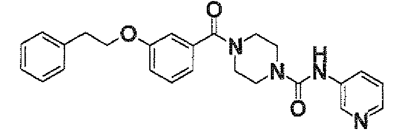 | FP: 431 |

**[Table 68]**

| Ex | Syn | Str | Dat |
|---|---|---|---|
| 427 | 26 | 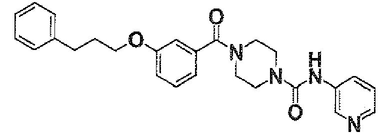 | FP: 445 |
| 428 | 30 | 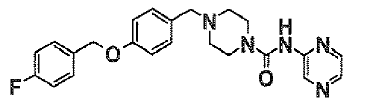 | EP: 422 |
| 429 | 30 | 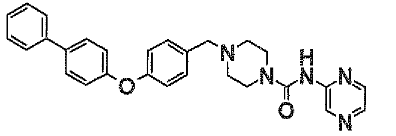 | EP: 466 |
| 430 | 30 | 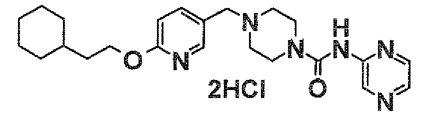 | FP: 425 |
| 431 | 30 | 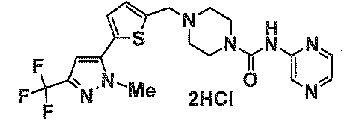 | FP: 452 |
| 432 | 32 | 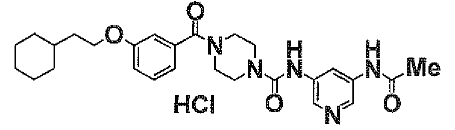 | FP: 494 |
| 433 | 35 | 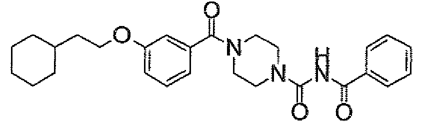 | FP: 464 |
| 434 | 35 | 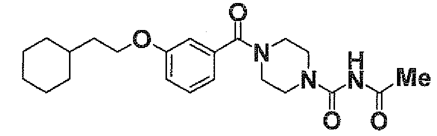 | FP: 402 |
| 435 | 35 | 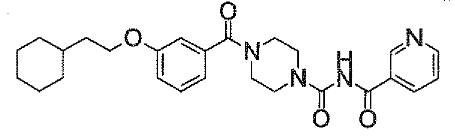 | FP: 465 |

**[Table 69]**

| Ex | Syn | Str | Dat |
|---|---|---|---|
| 436 | 35 | 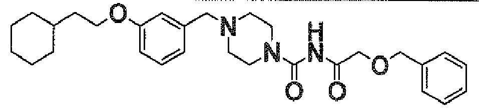 | FP: 494 |
| 437 | 35 | 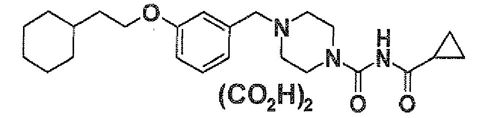 | FP: 414 |
| 438 | 35 | 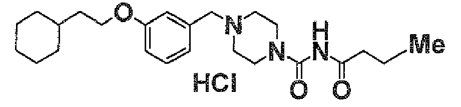 | FP: 416 |
| 439 | 35 | 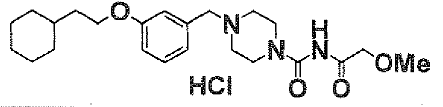 | FP: 418 |
| 440 | 39 | 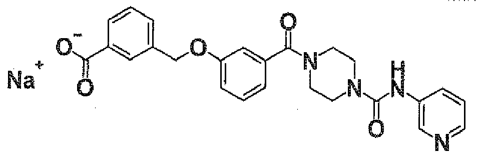 | EP: 482 |
| 441 | 441 | 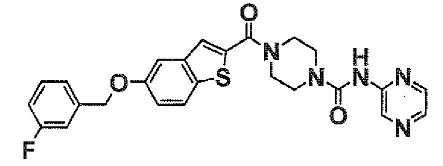 | EP: 492 |
| 442 | 442 | 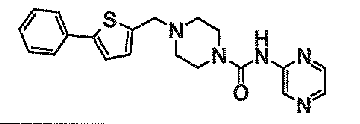 | FP: 380 |
| 443 | 443 | 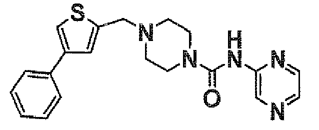 | EP: 380 |

**[Table 70]**

| Ex | Syn | Str | Dat |
|---|---|---|---|
| 444 | 444 | 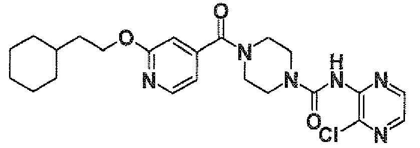 | FP: 473 |
| 445 | 445 | 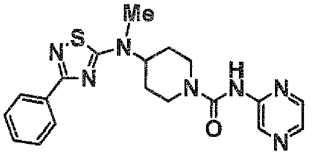 | FP: 396 |
| 446 | 446 | 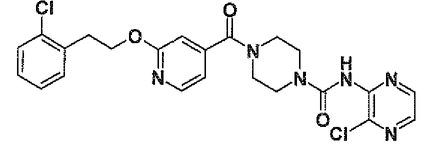 | EP: 501 |
| 447 | 447 | 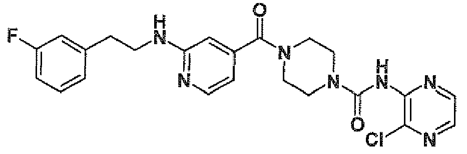 | EP: 484 |
| 448 | 448 | 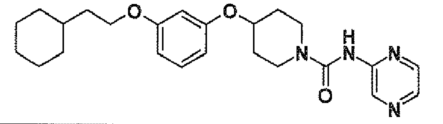 | EP: 425 |
| 449 | 449 | 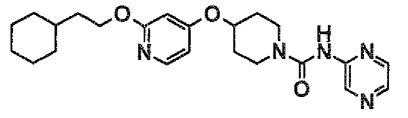 | FP: 426 |
| 450 | 450 | 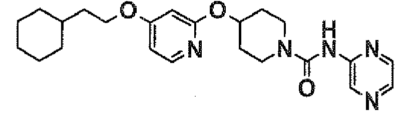 | FP: 426 |

**[Table 71]**

| Ex | Syn | Str | Dat |
|---|---|---|---|
| 451 | 451 | 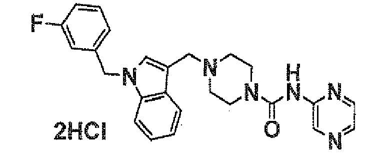 | FP: 445 |
| 452 | 452 | 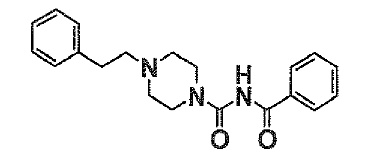 | FP: 338 |
| 453 | 30 | 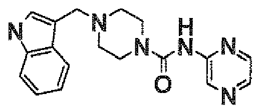 | FP: 337 |
| 454 | 16 | 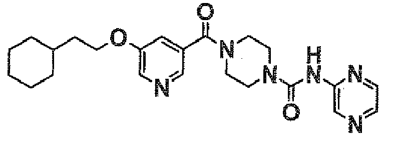 | FP: 439 |
| 455 | 16 | 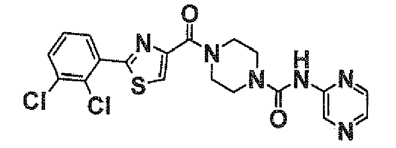 | FP: 463 |
| 456 | 16 | 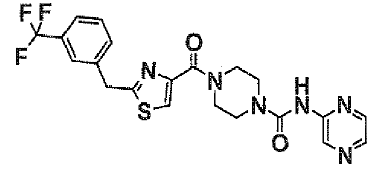 | FP: 477 |
| 457 | 16 | 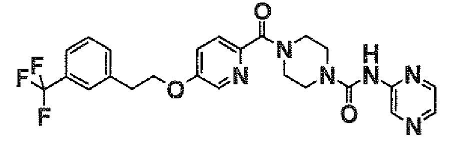 | FP: 501 |

**[Table 72]**

| Ex | Syn | Str | Dat |
|---|---|---|---|
| 458 | 16 | 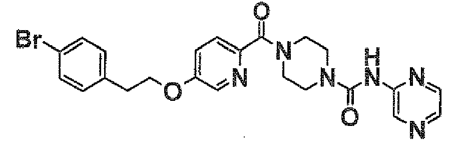 | FP: 511 |
| 459 | 441 | 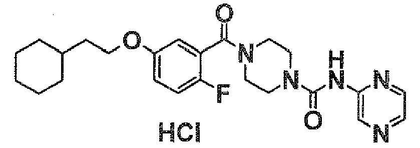 | EP: 456 |
| 460 | 442 | 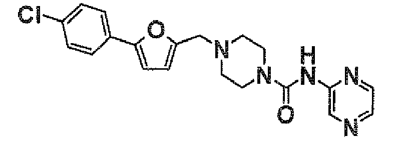 | FP: 398 |
| 461 | 442 | 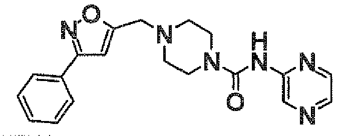 | FP: 365 |
| 462 | 442 | 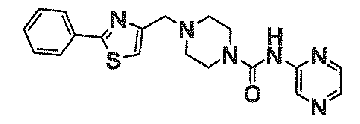 | FP: 381 |
| 463 | 442 | 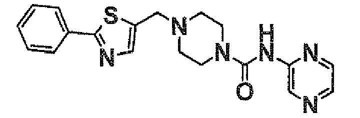 | FP: 381 |
| 464 | 442 | 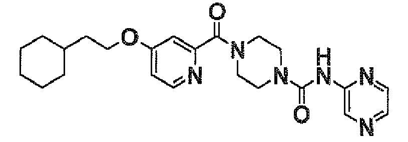 | EP: 439 |

**[Table 73]**

| Ex | Syn | Str | Dat |
|---|---|---|---|
| 465 | 30 | 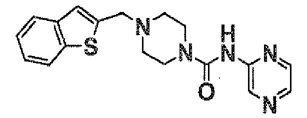 | FP: 354 |
| 466 | 443 | 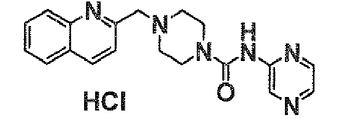 | FP: 349 |
| 467 | 441 | 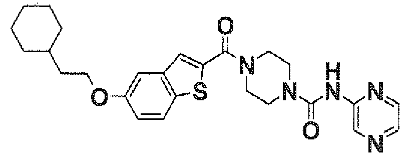 | FP: 494 |
| 468 | 442 | 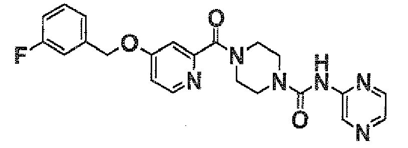 | EP: 437 |
| 469 | 443 | 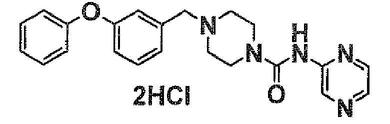 | FP: 390 |
| 470 | 444 | 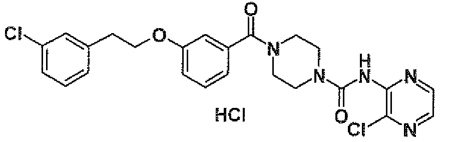 | EP: 500 |
| 471 | 28 | 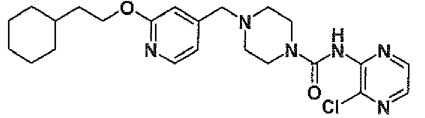 | FP: 459 |

**[Table 74]**

| Ex | Syn | Str | Dat |
|---|---|---|---|
| 472 | 28 | 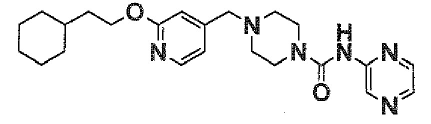 | FP: 425 |
| 473 | 444 | 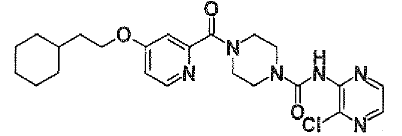 | FP: 473 |
| 474 | 444 | 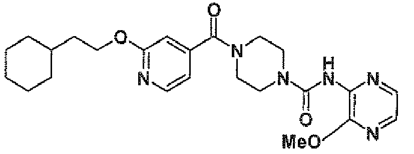 | FP: 469 |
| 475 | 445 | 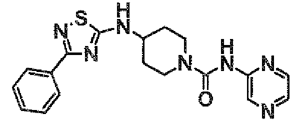 | EP: 382 |
| 476 | 446 | 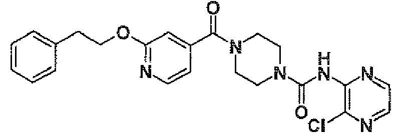 | FP: 467 |
| 477 | 446 | 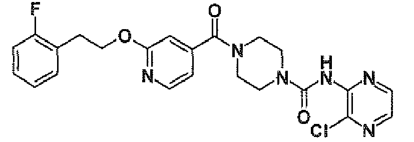 | EP: 485 |
| 478 | 446 | 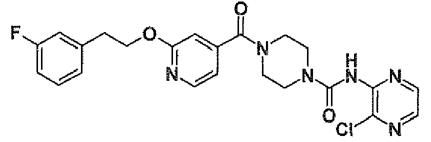 | EP: 485 |
| 479 | 446 | 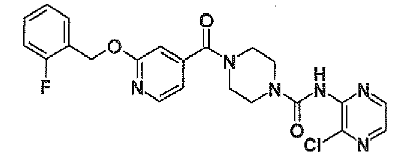 | EP: 471 |

**[Table 75]**

| Ex | Syn | Str | Dat |
|---|---|---|---|
| 480 | 446 | 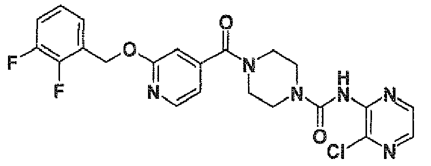 | EP: 489 |
| 481 | 446 | 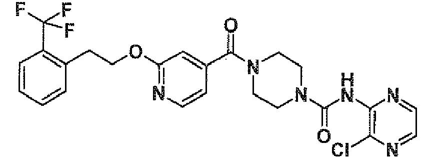 | EP: 535 |
| 482 | 446 | 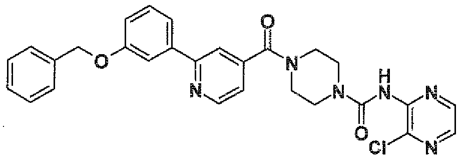 | EP: 529 |
| 483 | 446 | 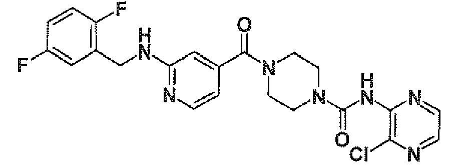 | EP: 488 |
| 484 | 446 | 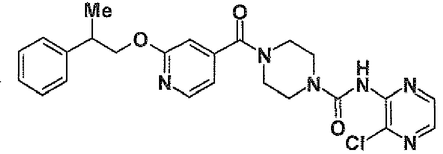 | FP: 481 |
| 485 | 444 | 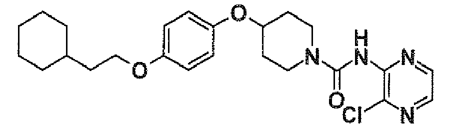 | FP: 459 |
| 486 | 444 | 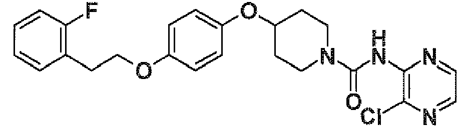 | EP: 471 |

**[Table 76]**

| Ex | Syn | Str | Dat |
|---|---|---|---|
| 487 | 444 | 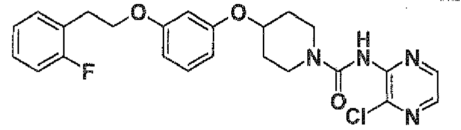 | EP: 471 |
| 488 | 444 | 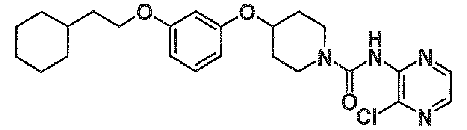 | EP: 459 |
| 489 | 442 | 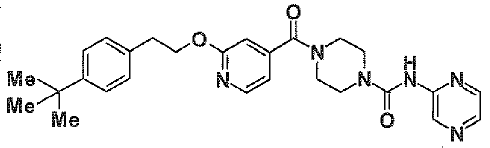 | EP: 489 |
| 490 | 448 | 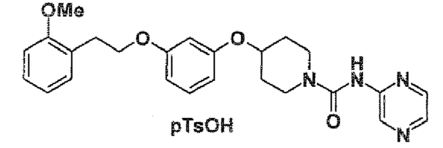 | EP: 449 |
| 491 | 448 | 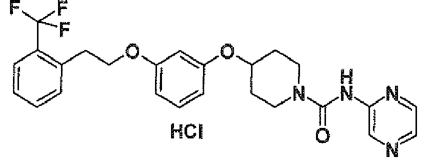 | FP: 487 |
| 492 | 448 | 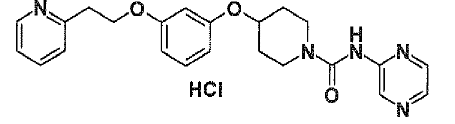 | EP: 420 |

**[Table 77]**

| Ex | **NMR** |
|---|---|
| 1 | NMR1:1.55-1.64(2H, m), 1.93- 1.98(2H, m), 3.35- 3.74(2H, m), 3.85-3.90 (2H, m), 4.47- 4.89 (1H, m), 5.07 (2H, s), 6. 95 (4H, s), 7.13- 7.18 (1H, m), 7.25-7.29 (2H, m), 7.41- 7.46 (1H, m), 7.89- 7.93 (1H, m), 8.47 (1H, d, J=5.2Hz), 8.58-8.60 (1H, m), 9.13 (1H, s), 9.81- 9.83(1H, m) |
| 12 | NMR1 : 1.65- 1.75 (2H, m), 1.94- 2.03 (2H, m), 3.36- 3.46 (2H, m), 3.65-3.75 (2H, m), 4.50- 4.57(1H, m), 5.07 (2H, s), 6.94 (4H, s), 7.03(1H, s), 7.12-7.18(1H, m), 7.23- 7.29 (2H, m), 7.40- 7.48 (2H, m), 8.04 (1H, s) |
| 16 | NMR1:3.22- 3.78 (8H, m), 5.16 (2H, s), 6.98- 7.12 (3H, m), 7.29- 7.48 (7H, m), 7.90 (1H, m), 8.18 (1H, m), 8.67(1H, m), 8.84(1H, s) |
| 26 | NMR1:3.38- 3.68 (8H, m), 5.18 (2H, s), 7.00- 7.13 (3H, m), 7.23- 7.59 (6H, m), 7.90 (1H, m), 8.17 (1H, m), 8.67 (1H, m), 8.85 (1H, s) |
| 28 | NMR1 : 2.89- 2.89 (4H, m), 3.59- 3.67 (4H, m), 3.99 (2H, s), 5.12 (2H, s), 7.01- 7.12 (3H, m), 7.28- 7.48 (7H, m), 7.88 (1H, m), 8.17 (1H, m), 8.65 (1H, m), 8.90 (1H, s) |
| 30 | NMR1 : 0.89- 1.01 (2H, m), 1.10- 1.27 (3H, m), 1.38- 1.49 (1H, m), 1.58- 1.76 (7H, m), 3.01- 3.16 (2H, br), 3.28- 3.54 (4H, br), 4.30 (2H, t, J=6. 6Hz), 4.27- 4.46 (4H, br), 7.13 (1H, s), 7.27 (1H, dd, J=5.2, 1.1Hz), 7.80 (1H, dd, J=8.6, 5.5Hz), 8.24 (1H, d, J =5.2Hz), 8.52 (1H, d, J=5.4Hz), 8.68-8.73 (1H, m), 9.16 (1H, d, J=2.2Hz), 10.50 (1H, s), 11.90- 12.00 (1H, br) |
| 35 | NMR1 : 0.89- 1.02 (2H, m), 1.12- 1.27 (3H, m), 1.40- 1.52(1H, m), 1.58- 1.78 (7H, m), 2.08 (3H, s), 2.57- 2.65 (4H, m), 3.42- 3.50 (4H, m), 3.74 (2H, s), 3.98 (2H, t, J= 6.6Hz), 6.86- 6.94 (3H, m), 7.23- 7.29 (1H, m), 9.83 (1H, s) |
| 36 | NMR1 : 0.90- 1.02 (2H, m), 1.12- 1.27 (3H, m), 1.40- 1.50 (1H, m), 1.58- 1.78 (7H, m), 3.30- 3.68 (8H, m), 4.02 (2H, t, J=6.7Hz), 5.51- 5.53 (2H, br), 6.37 (1H, d, J=8.8 Hz), 6.92- 7.04 (3H, m), 7.32- 7.40 (2H, m), 7.86- 7.88 (1H, m), 8.25 (1H, s) |
| 38 | NMR1 : 0.89- 1.02 (2H, m), 1.10- 1.27 (3H, m), 1.38- 1.50 (1H, m), 1.57- 1.77 (7H, m), 3.26-3.77 (8H, m), 4.31 (2H, t, J=6.8Hz), 6.80 (1H, s), 6.97 (1H, d, J=5.3Hz), 8.20-8.25 (2H, m), 8.29- 8.31 (1H, m), 9.02 (1H, s), 9.64 (1H, s) |

**[Table 78]**

| Ex | **NMR** |
|---|---|
| 133 | NMR1 : 2.97- 3.07 (2H, m), 3.26- 3.50 (4H, m), 4.28 (2H, s), 4.33- 4.45 (2H, m), 5.17 (2H, s), 7.10 (2H, d, J=8.8Hz), 7.17- 7.21 (1H, m), 7.24- 7.33 (2H, m), 7.42- 7.49 (1H, m), 7.56 (2H, d, J=8.8Hz), 7.87- 7.92 (1H, m), 8.46- 8.51 (1H, m), 8.57- 8.64 (1H, m), 9.11 (1H, s), 10.25- 10.33(1H,m) |
| 143 | NMR1 : 0.88- 1.02 (2H, m), 1.08- 1.28 (3H, m), 1.40- 1.52 (1H, m), 1.58- 1.78 (7H, m), 3.30- 3.74 (8H, m), 4.03 (2H, t, J=8. 7Hz), 6.92- 7.04 (3H, m), 7.32- 7.38 (1H, m), 7.54- 7.62 (1H, m), 7.96- 8.02 (1H, m), 8.82- 8.87 (1H, m), 9.94 (1H, s) |
| 155 | NMR2 : 0.91-1.15 (5H, m), 1.43- 1.82 (8H, m), 3.39-3.94 (8H, br), 4.00 (2H, t, J= 5.1Hz), 6.89-7.00 (3H, m), 7.26-7.35 (1H, m), 8.19 (1H, s), 8.55 (2H, d, J=6.0Hz), 9.60 (1H, s) |
| 159 | NMR2 : 0.89- 1.07 (2H, m), 1.12- 1.36 (4H, m), 1.42- 1.59 (1H, m), 1.61- 1.84 (6H, m), 2.80- 3.25 (2H, br), 3.46- 3.95 (6H, br), 4.01 (2H, t, J=7.5Hz), 5.83 (1H, d, J=3. 0Hz), 6.92- 7.00 (4H, m), 7.26- 7.36 (2H, m), 7.94 (1H, d, J=3.0Hz) |
| 163 | NMR1 : 0.89- 1.02 (2H, m), 1.08- 1.28 (3H, m), 1.38- 1.50 (1H, m), 1.57- 1.77 (7H, m), 3.32- 3.41 (2H, br), 3.49- 3.66 (6H, br), 4.34 (2H, t, J=7.1Hz), 6.87 (1H, d, J=8. 6Hz), 7.58 (1H, dd, J=9.1, 4.7Hz), 7.78 (1H, dd, J=8.7, 2.4Hz), 7.97- 8.01 (1H, m), 8.27 (1H, d, J=2.3Hz), 8.83- 8.86 (1H, m), 9.95 (1H, s) |
| 177 | NMR1 : 0.88- 1.28 (5H, m), 1.40- 1.52 (1H, m), 1.58- 1.78 (7H, m), 3.32- 3.74 (8H, m), 4.03 (2H, t, J=6.7Hz), 6.94- 7.04 (3H, m), 7.32- 7.38 (1H, m), 8.79 (1H, s), 8.90-8.92 (2H, br), 9.11 (1H, s) |
| 178 | NMR1 : 0.89- 1.02 (2H, m), 1.12- 1.28 (3H, m), 1.42- 1.52 (1H, m), 1.58- 1.78 (7H, m), 3.32- 3.66 (8H, m), 4.02 (2H, t, J=6.6Hz), 6.02 (1H, d, J=1.8Hz), 6.92- 7.04 (3H, m), 7.32- 7.38 (1H, m), 8.35 (1H, d, J=1.8Hz), 10.43 (1H, s) |
| 179 | NMR1 : 0.89- 1.02 (2H, m), 1.12- 1.27 (3H, m), 1.42- 1.52 (1H, m), 1.58- 1.78 (7H, m), 2.05 (3H, s), 3.30- 3.72 (8H, m), 4.02 (2H, t, J=6.7Hz), 6.94- 7.04 (3H, m), 7.32-7.38 (1H, m), 7.76- 7.80 (1H, m), 7.92- 7.98 (1H, m), 8.36- 8.40 (1H, m), 8.69 (1H, s), 10.32 (1H, s) |
| 189 | NMR1 : 0.89- 1.02 (2H, m), 1.08- 1.28 (3H, m), 1.40- 1.51 (1H, m), 1.58- 1.78 (7H, m), 3.00 (2H, q, J=11.5Hz), 3.30 (2H, d, J=11.8Hz), 3.41 (2H, t, J=12.5Hz), 4.03 (2H, t, J=6.7Hz), 4.17 (2H, d, J=14.2Hz), 4.31 (2H, d, J=4.5Hz), 7.00 (1H, dd, J=8.1, 2.1Hz), 7.09- 7.15 (2H, m), 7.29- 7.38 (1H, m), 8.61 (2H, d, J=4.9Hz), 9.83-10.23 (1H, br), 11.50 (1H, s) |

**[Table 79]**

| Ex | **NMR** |
|---|---|
| 197 | NMR1 : 0.88- 1.01 (2H, m), 1.07- 1.28 (3H, m), 1.38- 1.50 (1H, m), 1.57- 1.77 (7H, m), 3.22- 3.34 (2H, br), 3.41- 3.69 (6H, m), 4.31 (2H, t, J=6.8Hz), 6.77 (1H, d, J=1.5 Hz), 6.80 (1H, s), 6.96 (1H, dd, J=5.1, 1.3Hz), 8.23 (1H, d, J=5.4Hz), 8.67 (1H, d, J=1.8H z), 9.86 (1H, s) |
| 199 | NMR1 : 0.90- 1.02 (2H, m), 1.08- 1.28 (3H, m), 1.40- 1.52 (1H, m), 1.59- 1.78 (7H, m), 3.32- 3.41 (2H,m),3.45- 3.68 (6H, m), 4.02 (2H, t, J=8.6Hz), 6.92- 6.96 (2H, m), 6.99- 7.03 (1H, m), 7.32- 7.36 (1H, m), 7.74- 7.78 (1H, m), 8.50 (1H, d, J=6.0Hz), 8.76 (1H, s), 9.87 (1H, s) |
| 202 | NMR1 : 0.89- 1.02 (2H, m), 1.12- 1.27 (3H, m), 1.40- 1.52 (1H, m), 1.58- 1.78 (7H, m), 3.28- 3.70 (8H, m), 4.02 (2H, t, J=6. 8Hz), 6.93- 7.03 (3H, m), 7.32- 7.40 (2H, m), 7.83 (1H, d, J=8.7Hz), 7.93- 7.98 (1H, br), 8.12- 8.15 (1H, m), 8.72- 8.75 (1H, m), 9.62 (1H, s) |
| 204 | NMR1 : 0.89- 1.01 (2H, m), 1.08- 1.27 (3H, m), 1.38- 1.50 (1H, m), 1.57- 1.77 (7H, m), 3.27-3.71 (8H, m), 4.31 (2H, t, J=6.8Hz), 6.81 (1H, s), 6.97 (1H, dd, J=5 2, 1.2Hz), 7.58 (1H, d d, J=9.0, 4.7Hz), 7.98 (1H, dd, J=9.2, 1.4Hz), 8.24 (1H, d, J=4.8Hz), 8.84 (1H, dd, J=4.4 - 1.4Hz), 9.97 (1H, s) |
| 210 | NMR1 : 0.89- 1.02 (2H, m), 1.08- 1.28 (3H, m), 1.40- 1.52 (1H, m), 1.58- 1.78 (7H, m), 3.34- 3.43 (2H, m), 3.47- 3.70 (6H, m), 4.02 (2H, t, J=6.6Hz), 6.92 - 6.96 (2H, m), 6.99- 7.03 (1H, m), 7.32- 7.38 (1H, m), 8.21 (1H, d, J=2.6Hz), 8.28- 8.31 (1H, m), 9.03 (1H, s), 9.62 (1H, s) |
| 219 | NMR1 : 0.90- 1.80 (13H, m), 3.50- 3.70 (8H, m), 4.13 (2H, t, J=6.6Hz), 7.50 (1H, dd, J=2.8, 8.7Hz), 7.58 (1H, dd, J=4.6, 9.0Hz), 7.62 (1H, d, J=8.6Hz), 7.99 (1H, d, J= 8.9Hz), 8.84 (1H, d, J=4.6Hz), 9.94 (1H, s) |
| 225 | NMR1 : 0.89- 1.02 (2H, m), 1.12- 1.27 (3H, m), 1.40- 1.52 (1H, m), 1.58- 1.78 (7H, m), 3.28- 3.70 (8H, m), 4.02 (2H, t, J=6.7Hz), 6.93- 7.03 (3H, m), 7.31- 7.38 (1H, m), 8.83 (2H, s), 10.32 (1H, s) |
| 239 | NMR1:0.87- 1.27 (5H, m), 1.38- 1.50 (1H, m), 1.57- 1.77 (7H, m), 3.44- 3.67(8H,br ), 4.33 (2H, t, J=9.0Hz), 6.87 (1H, d, J=11.2Hz), 7.78 (1H, dd, J=11.4, 3.2Hz), 8.22 (1H, d, J=3.4Hz), 8.27 (1H, d, J=3.4Hz), 8.298.32 (1H, m), 9.03 (1H, s), 9.65 (1H, s) |
| 243 | NMR1 : 0.99- 1.10 (2H, m), 1.12-1.32 (3H, m), 1.61- 1.84 (6H, m), 3.28- 3.72 (8H, m), 3.80 (2H, d, J=6.4Hz), 6.94-6.97 (2H, m), 6.99-7.03 (1H, m), 7.35 (1H, t, J=8.0Hz), 8.78 (1H, s), 8.88 (1H, s), 9.00 (1H, s) |

**[Table 80]**

| Ex | **NMR** |
|---|---|
| 246 | NMR1 : 0.89- 1.27 (5H, m), 1.38- 1.50 (1H, m), 1.57- 1.77 (7H, m), 3.29- 3.72 (8H, m), 4.31 (2H, t, J=6. 6Hz), 6.81 (1H, s), 6.98 (1H, d, J=5.0Hz), 8.24 (1H, d, J=5. 1Hz), 8.77 ( 1H, s), 8.88 (1H, s), 9.01 (1H, s) |
| 247 | NMR1 : 0.89- 1.27 (5H, m), 1.39- 1.50 (1H, m), 1.58- 1.76 (7H, m), 3.47- 3.68 (8H, br ), 4.33 (2H, t, J=6. 7Hz), 6. 86 (1H, d, J=8.6Hz), 7.78 (1H, dd, J=8.8, 2.4Hz), 8. 28 (1H, d, J=2. 3Hz), 8.78 (1H, s), 8.88 (1H, s), 9.01 (1H, s) |
| 262 | NMR1 : 0.90- 1.80 (13H, m), 3.47- 3. 70 (8H, m), 4.13 (2H, t, J=6. 6Hz), 7.50 (1H, dd, J=2.9, 8.8Hz), 7.62 (1H, d, J=8.7Hz), 8.21 (1H, d, J=2.8Hz), 8.27- 8.33 (2H, m), 9.03 (1H, d, J=1. 4Hz), 9.61 (1H, s) |
| 300 | NMR1 : 3.50- 3.70 (8H, m), 5.19 (2H, s), 7.03- 7.11 (3H, m), 7.14- 7.21 (1H, m), 7.27-7. 33 (2H, m), 7.39- 7.50 (4H, m), 8.05 (1H, s) |
| 320 | NMR1 : 0.98-1.10 (2H, m),1.13-1.32 (3H, m), 1.61-1. 84 (6H, m), 3.30- 3.72(8H,m), 3.81 (2H, d, J=6. 4Hz), 6. 94- 7.03 (3H, m), 7.29- 7.38(2H,m),7.88- 7.92 (1H, m), 8.15- 8.20 (1H, m), 8.66 (1H, s), 8. 84 (1H, s) |
| 328 | NMR1 : 0.90-1.02 (2H, m), 1.07- 1.23 (3H, m), 1.58- 175 (6H, m), 2.96 (3H, s), 3.20 (2H, d, J=7. 2Hz), 3.48- 3.80 (8H, m), 6.56 (2H, d, J=8. 8Hz), 7.25- 7.32 (3H, m), 7.85- 7. 90 (1H, m), 8.14- 8.18 (1H, m), 8.64 (1H, s), 8. 78 (1H, s) |
| 329 | NMR1 : 0.90- 1.02 (2H, m), 1.08- 1.28 (3H, m), 1.42- 1.78 (8H, m), 3.22- 3. 78 (8H, m), 4.00- 4.05 (2H, m), 6.94- 7.04 (3H, m), 7.29- 7.37 (2H, m), 7.88- 7.92 (1H, m), 8.16- 8. 20 (1H, m), 8.64- 8.87 (1H, m), 8.83 (1H, s) |
| 334 | NMR1 : 0.88- 1. 00 (2H, m), 1.18- 1. 32 (4H, m), 1.35- 1.43 (2H, m), 1.57- 1.76(5H,m), 2. 92 (3H, s), 3.34- 3.41 (2H, m), 3. 48- 3. 58 (8H, m), 6. 86 (2H, d, J=8. 8Hz), 7.25-7.32 (3H, m), 7.85- 7.89 (1H, m), 8.14- 8.17 (1H, m), 8.64 (1H, d, J=2. 4Hz), 8.78 (1H, s) |
| 388 | NMR1 : 0.98- 0.99 (2H, m), 1.09- 1.27 (4H, m), 1.46 (1H, m), 1.57- 1.75 (6H, m), 2. 89-2. 94 (4H, m), 3. 62- 3. 67 (4H, m), 4. 00- 4. 05 (4H, m), 6.94- 7.04 (3H, m), 7.28-7.34 (2H, m), 7.88 (1H, m), 8.17 (1H, m), 8.64 (1H, m), 8.97 (1H, s) |

**[Table 81]**

| Ex | **NMR** |
|---|---|
| 400 | NMR1 : 0.86- 0.90 (3H, m), 1.29- 1.34 (4H, m), 1.38- 1.44 (2H, m), 1.68- 1.74 (2H, m), 2.87- 2. 92 (4N, m), 3.63- 3.66 (4H, m), 3.95- 4.00 (2H, m), 6.93- 7.03 (3H, m), 7.28- 7.33 (2H, m), 7.88 (1H, m), 8.17 (1H, m), 8.64 (1H, m), 8.91 (1N, s) |
| 409 | NMR1 : 3.09- 3.48 (8H, m), 5.25 (2H, s), 7.13- 7.29 (3H, m), 7.38- 7.50 (4H, m), 7.92 (1H, m), 8.50 (1H, m), 8. 63 (1H, m), 9.13 (1H, m), 10.35 (1H, s) |
| 412 | NMR1 : 3.08- 3.47 (8H, m), 4.34 (2H, s), 5.15 (2H, s), 7.13- 7.23 (3H, m), 7.39- 7.45 (2H, m), 7.51-7.59 (2H, m), 7.91 (1H, m), 8.49 (1H, m), 8.62 (1H, m), 9.11 (1H, m), 10 32 (1H, s) |
| 415 | NMR1 : 2.76- 2.83 (4H, m), 3.05- 3.09 (2H, m), 3.57- 3.63 (4H, m), 3.90 (2H, s), 4.19 - 4.23 (2H, m), 6.93- 7.08 (4H, m), 7.17- 7.21 (2H, m), 7.27- 7.39 (3H, m), 7. 87 (1H, m), 8. 17 (1H, m), 8.63 (1H, m), 8.85 (1H, s) |
| 416 | NMR1 : 3.08- 3.47 (8H, m), 4.33 (2H, s), 5.21 (2H, s), 7.09- 7.23 (5H, m), 7.37- 7.46 (2H, m), 7.92 (1H, m), 8.49 (1H, m), 8.65 (1N, m), 9.13 (1H, m), 10. 38 (1H, s) |
| 424 | NMR1 : 1.99- 2. 05 (2H, m), 2.73- 2.77 (2H, m), 2.86- 2.90 (4H, m), 3.61- 3.66 (4H, m), 3.96- 4.00 (2H, m), 6.94- 7.04 (3H, m), 7.17- 7.34 (7H, m), 7.88 (1H, m), 8.17 (1H, m), 8.65 (1H, m), 8.90 (1H, s) |
| 426 | NMR1 : 3.04 (2H, t, J=6. 8Hz), 3.33- 3.72 (8H, m), 4.24 (2H, t, J=6. 8Hz), 6.94- 6.98 (2H, m), 7.00- 7. 05 (1H, m), 7.19- 7.38 (7H, m), 7.83- 7. 89 (1H, m), 8.13- 8.17 (1H, m), 8.63 (1H, d, J=2. 4Hz), 8.78 (1H, s) |
| 435 | NMR2 : 0.91- 1.04 (2H, m), 1.10- 1.33 (3H, m), 1.44- 1.56 (1H, m), 1.61 - 1.81 (7H, m), 3.32- 3. 96 (8H, br), 4.00 (2H, t, J=5. 0Hz), 6.89- 7.01 (3H, m), 7.27- 7.35 (1H, m), 7.38- 7.46 (1H, m), 8.13- 8.27 (1H, m), 8.73- 8.85 (1H, br), 8.99- 9.23 (2H, br) |
| 439 | NMR1 : 0.90- 1.01 (2H, m), 1.07- 1.27 (3H, m), 1.40- 1.52 (1H, m), 1.58- 1.78(7H, m), 2.91- 3.02 (2H, m), 3.25-3.35 (4H, m), 3.39 (3H, s), 4.00- 4.10 (4H, m), 4.14 (2H, s), 4.28 (2H, d, J=2. 8Hz), 6.98- 7.03 (1H, m), 7.11 (1H, d, J=5. 6Nz), 7.28 (1H, s), 7.35 (1H, t, J=5. 6Hz), 10. 01 (1H, s) |

### Industrial Availability

Since the compound of the present invention has an excellent FAAH inhibitory activity, it is useful for treatment of FAAH-related diseases, in particular, of a urinary frequency, urinary incontinence and/or overactive bladder.

### Sequence Listing Free Text

Under Numeric Identifier <223> of SEQ ID NO: 1 in the following sequence listing, the present inventors are provided.

## Claims

1. A urea compound represented by the formula (I) or a pharmaceutically acceptable salt thereof. 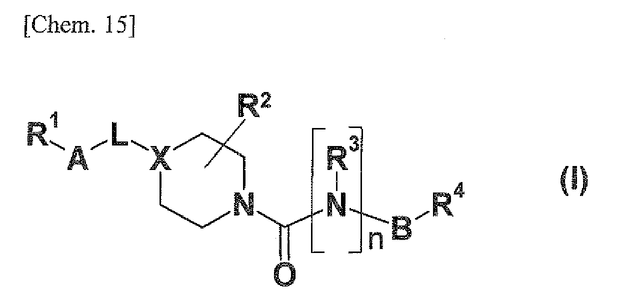 [in the formula, the symbols have the following meanings:
R¹: H, aryl, aryl-O-, aryl-lower alkylene-, aryl-lower alkenylene-, aryl-lower alkylene-O-, aryl-lower alkylene-NR⁰-, aryl-NR⁰-lower alkylene-, aryl-C(O)-NR⁰-, aryl-SO₂-NR⁰-, heteroaryl, heteroaryl-lower alkylene-O-, cycloalkyl-lower alkylene-, cycloalkyl-lower alkylene-O-, cycloalkyl-lower alkylene-NR⁰-, a nitrogen-containing heterocyclic group having a bonding arm on N as a ring-constituting element, or oxygen-containing saturated heterocyclic group-lower alkylene-O-,
wherein each aryl and each heteroaryl in R¹ may be substituted with group(s) selected from the following Group G¹,
Group G¹: halogen, lower alkyl, -O-lower alkyl, -O-beuzyl, halogeno-lower alkyl, -O-halogeno-lower alkyl, -CN, -N(R⁰)₂, -C(O)-OR⁰, -C(O)-N(R⁰)₂, and phenyl,
R⁰: the same or different, H or lower alkyl,
A: a benzene ring or hetero ring, each of which may be substituted with group(s) selected from the following Group G²,
Group G²: halogen, lower alkyl, -O-C₁₋₈alkyl, -OH, -NO₂, -C(O)-OR⁰, and -C(O)-N(R⁰)₂,
X: N or CH,
L: lower alkylene, lower alkenylene, -O-, -O-lower alkylene-, -S(O)ₘ-, -lower alkylene-S(O)ₘ-, -C(O)-, -lower alkylene-C(O)-, -lower alkenylene-C(O)-, -NR⁰-, -C(O)-NR⁰-, -NR⁰-C(O)-, -O-lower alkylene-C(O)-, -lower alkylene-O-C(O)-, or -lower alkylene-NR⁰-C(O)-,
(provided that when X is N, L is not -O-, -S-, -NR⁰-, and -C(O)-NR⁰),
m: the same or different, 0, 1, or 2,
R² and R³: the same or different, H or lower alkyl,
n: 0 or 1,
B: (i) in a case of n=1, a single bond, or a benzene ring or aromatic hetero ring, each of which may be substituted with group(s) selected from the following Group G³, and (ii) in a case of n=0, a single bond,
Group G³: halogen, lower alkyl, -O-lower alkyl, halogeno-lower alkyl, -O-halogeno-lower alkyl, -OH, -O-benzyl, -O-C(O)-lower alkyl, -lower alkylene-OR⁰, -lower alkylene-O-C(O)-lower alkyl, -CN, -NO₂, -C(O)-OR⁰, -C(O)-N(R⁰)₂, -N(R⁰)₂, -NR⁰-C(O)-lower alkyl, -NR⁰-SO₂-lower alkyl, and -S(O)ₘ-lower alkyl,
R⁴: (i) in a case of n=1 and B=a single bond,
-C(O)-Z or -S(O)ₘ-Z,
[wherein
Z: lower alkyl, cycloalkyl, aryl, heteroaryl, -lower alkylene-O-lower alkyl, or -lower alkylene-O-benzyl],
(ii) in a case of n=1 and B= other than a single bond,
H, or phenyl, a nitrogen-containing heterocyclic group, a -C(O)-nitrogen-containing heterocyclic group, each of which may be substituted with group(s) selected from the following Group G⁴, or -W-lower alkylene-Y,
[wherein
W: -lower alkylene-C(O)-NR⁰-, -C(O)-NR⁰-, -O-, or a single bond, and
Y: -OH, -N(R⁰)₂, -C(O)-OR⁰, or -C(O)-N(R⁰)₂], and
(iii) in a case of n=0,
a nitrogen-containing heterocyclic group having a bonding arm on N as a ring-constituting element, which may be substituted with group(s) selected from the following Group G⁴, and
Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂,
provided that when L is -C(O)- or-lower alkylene-C(O)-, X is N, n=1, and R¹, R², R³, and R⁴ are all H, for B, unsubstituted benzoisoxazole is excluded. The same shall apply hereinafter.]

2. The urea compound according to claim 1, which is represented by the formula (I-A) or a pharmaceutically acceptable salt thereof. 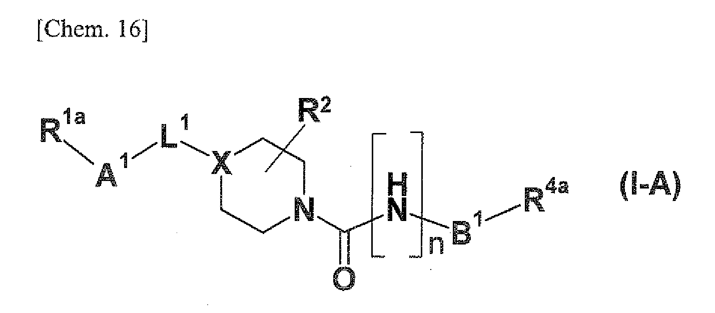 [in the formula, the symbols have the following meanings:
R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
wherein each aryl and each heteroaryl in R^{1a} may be substituted with group(s) selected from the following Group G¹,
Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
R⁰: the same or different, H or lower alkyl,
A¹: a benzene ring or aromatic hetero ring, each of which may be substituted with group(s) selected from the following Group G²,
Group G²: halogen, lower alkyl, -O-C₁₋₈ alkyl, -OH, -NO₂, -C(O)-OR⁰, and -C(O)-N(R⁰)₂,
X: N or CH,
L¹: methylene, -O-, -S(O)ₘ-, -C(O)-, or -NR⁰-,
(provided that when X is N, L¹ is methylene, -S(O)₂-, or -C(O)-),
m: the same or different, 0, 1, or 2,
R²: H or lower alkyl,
n: 0 or 1,
B¹: (i) in a case of n=1, a single bond, or a 5- or 6-membered aromatic nitrogen-containing hetero ring which may be substituted with group(s) selected from the following Group G³, (ii) in a case of n=0, a single bond,
Group G³: halogen, lower alkyl, -O-lower alkyl, halogeno-lower alkyl, -O-halogeno-lower alkyl, -OH, -O-benzyl, -O-C(O)-lower alkyl, -lower alkylene-OR⁰, -lower alkylene-O-C(O)-lower alkyl, -CN, -NO₂, -C(O)-OR⁰, -C(O)-N(R⁰)₂, -N(R⁰)₂, -NR⁰-C(O)-lower alkyl, -NR⁰-SO₂-lower alkyl, and -S(O)ₘ-lower alkyl,
R^{4a}: (i) in a case of n=1 and B¹=a single bond,
-C(O)-Z or -S(O)ₘ-Z,
[wherein
Z: lower alkyl, cycloalkyl, aryl, heteroaryl, -lower alkylene-O-lower alkyl, or -lower alkylene-O-benzyl],
(ii) in a case of n=1 and B¹= a 5- or 6-membered aromatic nitrogen-containing hetero ring which may be substituted with group(s) selected from the above-mentioned Group G³,
H, or phenyl, a nitrogen-containing heterocyclic group, -C(O)-nitrogen-containing heterocyclic group, each of which may be substituted with group(s) selected from the following Group G⁴, or -W-lower alkylene-Y,
[wherein
W: -lower alkylene-C(O)-NR⁰-, -C(O)-NR⁰-, -O-, or a single bond, and
Y: -OH, -N(R⁰)₂, -C(O)-OR⁰, or -C(O)-N(R⁰)₂], and
(iii) in a case of n=0,
a 5-membered aromatic nitrogen-containing heterocyclic group having a bonding arm on N as a ring-constituting element, which may be substituted with group(s) selected from the following Group G⁴, and
Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂.]

3. The urea compound according to claim 2, which is represented by the formula (I-B) or a pharmaceutically acceptable salt thereof. 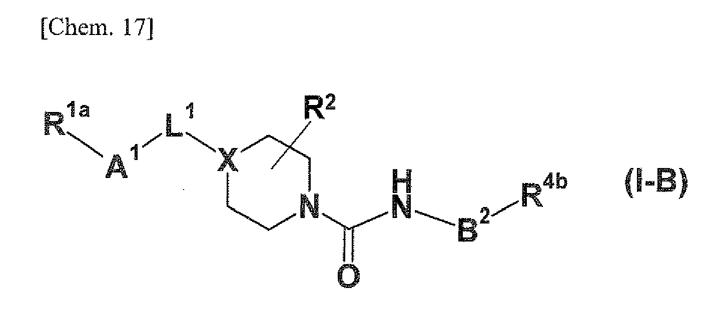 [in the formula, the symbols have the following meanings:
R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
wherein each aryl and each heteroaryl in R^{1a} may be substituted with group(s) selected from the following Group G¹,
Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
R⁰: the same or different, H or lower alkyl,
A¹: a benzene ring or aromatic hetero ring, each of which may be substituted with group(s) selected from the following Group G²,
Group G²: halogen, lower alkyl, -O-C₁₋₈ alkyl, -OH, -NO₂, -C(O)-OR⁰, and -C(O)-N(R⁰)₂,
X: N or CH,
L¹: methylene, -O-, -S(O)ₘ-, -C(O)-, or -NR⁰-,
(provided that when X is N, L¹ is methylene, -S(O)₂-, or -C(O)-),
m: the same or different, 0, 1, or 2,
R²: H or lower alkyl,
B²: a 5- or 6-membered aromatic nitrogen-containing hetero ring, which may be substituted with group(s) selected from the following Group G³,
Group G³: halogen, lower alkyl, -O-lower alkyl, halogeno-lower alkyl, -O-halogeno-lower alkyl, -OH, -O-benzyl, -O-C(O)-lower alkyl, -lower alkylene-OR⁰, -lower alkylene-O-C(O)-lower alkyl, -CN, -NO₂, -C(O)-OR⁰, -C(O)-N(R⁰)₂, -N(R⁰)₂, -NR⁰-C(O)-lower alkyl, -NR⁰-SO₂-lower alkyl, and -S(O)ₘ-lower alkyl,
R^{4b}: H, or phenyl, a nitrogen-containing heterocyclic group, -C(O)-nitrogen-containing heterocyclic group, each of which may be substituted with group(s) selected from the following Group G⁴, or -W-lower alkylene-Y,
[wherein
W: -lower alkylene-C(O)-NR⁰, -C(O)-NR⁰-, -O-, or a single bond, and
Y: -OH, -N(R⁰)₂, -C(O)-OR⁰, or -C(O)-N(R⁰)₂], and
Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂.]

4. The urea compound according to claim 3, wherein A¹ is a benzene ring or a 5- or 6-membered aromatic hetero ring or a pharmaceutically acceptable salt thereof.

5. The urea compound according to claim 4, wherein R^{1a} is aryl-lower alkylene-O-, aryl-lower alkylene-NR⁰-, heteroaryl-lower alkylene-O-, cycloalkyl-lower alkylene-O-, cycloalkyl-lower alkylene-NR⁰-, or oxygen-containing saturated heterocyclic group-lower alkylene-O- or a pharmaceutically acceptable salt thereof.

6. The urea compound according to claim 5, wherein X=N, and L¹ is -C(O)-or methylene or a pharmaceutically acceptable salt thereof.

7. The urea compound according to claim 5, wherein X=CH, and L¹ is -O- or a pharmaceutically acceptable salt thereof.

8. The urea compound according to claim 2, which is represented by the formula (I-C) or a pharmaceutically acceptable salt thereof. 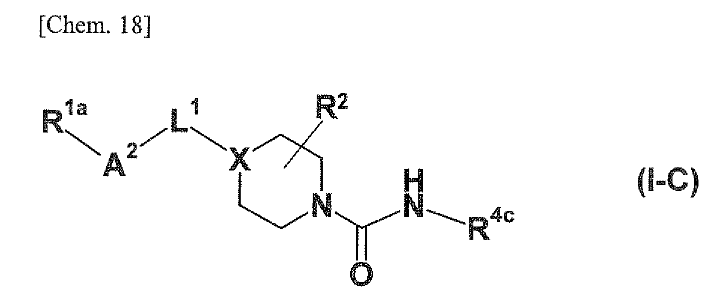 [in the formula, the symbols have the following meanings:
R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
wherein each aryl and each heteroaryl in R^{1a} may be substituted with group(s) selected from the following Group G¹,
Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
R⁰: the same or different, H or lower alkyl,
A²: a benzene ring or 5- or 6-membered aromatic hetero ring, each of which may be substituted with group(s) selected from the following Group G²,
Group G²: halogen, lower alkyl, -O-C₁₋₈ alkyl, -OH, -NO₂, -C(O)-OR⁰, and -C(O)-N(R⁰)₂,
X: N or CH,
L¹: methylene, -O-, -S(O)ₘ-, -C(O)-, or -NR⁰-,
(provided that when X is N, L¹ is methylene, -S(O)₂-, or -C(O)-),
m: the same or different from, 0, 1, or 2,
R²: H or lower alkyl, and
R^{4c}: -C(O)-Z or -S(O)ₘ-Z,
[wherein
Z: lower alkyl, cycloalkyl, aryl, heteroaryl, -lower alkylene-O-lower alkyl, or -lower alkylene-O-benzyl.]

9. The urea compound according to claim 2, which is represented by the formula (I-D) or a pharmaceutically acceptable salt thereof. 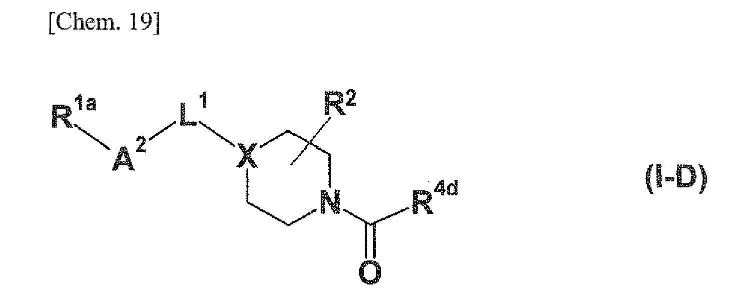 [in the formula, the symbols have the following meanings:
R^{1a}: aryl-lower alkylene-, aryl-lower alkenylene-, aryl-lower alkylene-O-, aryl-lower alkylene-NR⁰-, heteroaryl-lower alkylene-O-, cycloalkyl-lower alkylene-, cycloalkyl-lower alkylene-O-, cycloalkyl-lower alkylene-NR⁰-, or oxygen-containing saturated heterocyclic group-lower alkylene-O-,
wherein each aryl and each heteroaryl in R^{1a} may be substituted with group(s) selected from the following Group G¹,
Group G¹: halogen, lower alkyl, -O-lower alkyl, -O-benzyl, halogeno-lower alkyl, -O-halogeno-lower alkyl, -CN, -N(R⁰)₂, -C(O)-OR⁰, -C(O)-N(R⁰)₂, and phenyl,
R⁰: the same or different, H or lower alkyl,
A²: a benzene ring or 5- or 6-membered aromatic hetero ring, each of which may be substituted with group(s) selected from the following Group G²,
Group G²: halogen, lower alkyl, -O-C₁₋₈ alkyl, -OH, -NO₂, -C(O)-OR⁰, and -C(O)-N(R⁰)₂,
X: N or CH,
L¹: methylene, -O-, -S(O)ₘ-, -C(O)-, or -NR⁰-,
(provided that when X is N, L¹ is methylene, -S(O)₂-, or -C(O)-),
m: 0,1, or 2,
R²_{:} H or lower alkyl,
R^{4d}: a 5-membered aromatic nitrogen-containing heterocyclic group having a bonding arm on N as a ring-constituting element, which may be substituted with group(s) selected from the following Group G⁴, and
Group G⁴: lower alkyl, -OH, -N(R⁰)₂, -C(O)-OR⁰, -C(O)-N(R⁰)₂, -lower alkylene-C(O)-OR⁰, and -lower alkylene-C(O)-N(R⁰)₂.]

10. The compound according to claim 1, which is selected from the group consisting of
4-{4-[(3-fluorobenzyl)oxy]phenoxy}-N-pyridin-3-ylpiperidine-1-carboxamide,
4-{4-[(3-fluorobenzyl)oxy]benzyl}-N-pyridin-3-ylpiperazine-1-carboxamide,
4-{4-(2-cyclohexylethyl)(methyl)amino]benzoyl}-N-pyridin-3-ylpiperazine-1-carboxamide,
4-[3-(2-cyclohexylethoxy)benzoyl]-N-pyrimidin-2-ylpiperazine-1-carboxamide,
4-[3-(2-cyclohexylethoxy)benzoyl]-N-pyridazin-3-ylpiperazine-1-carboxamide,
4-[3-(2-cyclohexylethoxy)benzoyl]-N-pyrazin-2-ylpiperazine-1-carboxamide,
4-[2-(2-cyclohexylethoxy)isonicotinoyl]-N-pyrazin-2-ylpiperazine-1-carboxamide,
4-[3-(2-cyclohexylethoxy)benzyl]-N-pyridin-3-ylpiperazine-1-carboxamide,
4-[3-(2-cyclohexylethoxy)benzoyl]-N-pyrimidin-5-ylpiperazine-1-carboxamide,
N-(6-aminopyridin-3-yl)-4-[3-(2-cyclohexylethoxy)benzoyl]piperazine-1-carboxamide,
4-[3-(2-cyclohexylethoxy)benzyl]-N-pyridazin-3-ylpiperazine-1-carboxamide,
4-{3-[(2,3-difluorobenzyl)oxy]benzyl}-N-pyridin-3-ylpiperazine-1-carboxamide,
4-{[2-(2-cyclohexylethoxy)pyridin-4-yl]methyl}-N-pyridin-3-ylpiperazine-1-carboxamide,
4-{3-[2-(3-fluorophenyl)ethoxy]benzoyl}-N-pyrazin-2-ylpiperazine-1-carboxamide,
4-{[5-(2-cyclohexylethoxy)pyridin-2-yl]carbonyl}-N-pyrazin-2-ylpiperazine-1-carboxamide,
4-[3-(2-cyclohexylethoxy)-5-fluorobenzoyl]-N-pyrazin-2-ylpiperazine-1-carboxamide,
4-[3-(2-cyclohexylethoxy)-4-fluorobenzoyl]-N-pyrazin-2-ylpiperazine-1-carboxamide,
4-{2-[2-(2-fluorophenyl)ethoxy]isonicotinoyl}-N-pyrazin-2-ylpiperazine-1-carboxamide,
N-(3-chloropyrazin-2-yl)-4-[3-(2-cyclohexylethoxy)benzoyl]piperazine-1-carboxamide,
N-(3-chloropyrazin-2-yl)-4-[3-(2-cyclohexylethoxy)phenoxy]piperidine-1-carboxamide,
4-[5-(2-cyclohexylethoxy)-2-fluorobenzoyl]-N-pyrazin-2-ylpiperazine-1-carboxamide,
N-(3-chloropyrazin-2-yl)-4-[2-(2-cyclohexylethoxy)isonicotinoyl]piperazine-1-carboxamide,
N-(3-chloropyrazin-2-yl)-4-{[2-(2-cyclohexylethoxy)pyridin-4-yl]methyl}piperazine-1-carboxamide,
4-{[2-(2-cyclohexylethoxy)pyridin-4-yl]methyl}-N-pyrazin-2-ylpiperazine-1-carboxamide,
N-(3-chloropyrazin-2-yl)-4-{2-[2-(3-fluorophenyl)ethoxy]isonicotinoyl}piperazine-1-carboxamide,
4-{2-[2-(2-chlorophenyl)ethoxy]isonicotinoyl}-N-(3-chloropyrazin-2-yl)piperazine-1-carboxamide,
N-(3-chloropyrazin-2-yl)-4-(2-{2-[2-(trifluoromethyl)phenyl]ethoxy}isonicotinoyl)piperazine-1-carboxamide,
N-(3-chloropyrazin-2-yl)-4-{2-[2-(2-fluorophenyl)ethoxy]isonicotinoyl}piperazine-1-carboxamide,
4-{4-[(3-fluorobenzyl)oxy]phenoxy}-1-(1H-imidazol-1-ylcarbonyl)piperidine,
1-{4-[(3-fluorobenzyl)oxy]benzoyl}-4-(1H-imidazol-1-ylcarbonyl)piperazine,
4-({4-[3-(2-cyclohexylethoxy)benzoyl]piperazin-1-yl}carbonyl)-4H-1,2,4-triazol-3-amine,
1-({4-[3-(2-cyclohexylethoxy)benzoyl]piperazin-1-yl}carbonyl)-1H-pyrazol-5-amine, and
4-[3-(2-cyclohexylethoxy)benzyl]-N-(methoxyacetyl)piperazine-1-carboxamide, or a pharmaceutically acceptable salt thereof.

11. A pharmaceutical composition comprising the compound according to claim 1 or a pharmaceutically acceptable salt thereof as an active ingredient.

12. The pharmaceutical composition according to claim 11, which is an FAAH inhibitor.

13. The pharmaceutical composition according to claim 11, which is an agent for treating urinary frequency, urinary incontinence, and/or overactive bladder.

14. A use of the compound according to claim 1 or a pharmaceutically acceptable salt thereof for the preparation of an agent for treating urinary frequency, urinary incontinence, and/or overactive bladder.

15. A method for treating urinary frequency, urinary incontinence, and/or overactive bladder, comprising administering an effective amount of the compound according to claim 1 or a pharmaceutically acceptable salt thereof to a patient.


SEQUENCE LISTING

<110>  Astellas Pharma Inc.

<120>  Urea compound or a salt thereof

<130>  A07016

<150>  JP2006-226072
<151>  2006-08-23

<160>  8

<170>  PatentIn version 3.1

<210>  1
<211>  2063
<212>  DNA
<213>  Homo sapiens

<220>
<221>  CDS
<222>  (36)..(1772)
<223>  Inventor;Ishii, Takahiro; Sugane, Takashi; Kakefuda, Akio; Takahashi,
Tatsuhisa; Kanayama, Takatoshi; Sato, Kentaro; Kuriwaki, Ikumi; Kitada, Chika;
Suzuki, Jotaro

<400>  1
tgccgggcgg taggcagcag caggctgaag ggatc atg gtg cag tac gag ctg        53
                                       Met Val Gln Tyr Glu Leu
                                       1               5

tgg gcc gcg ctg cct ggc gcc tcc ggg gtc gcc ctg gcc tgc tgc ttc       101
Trp Ala Ala Leu Pro Gly Ala Ser Gly Val Ala Leu Ala Cys Cys Phe
            10                  15                  20

gtg gcg gcg gcc gtg gcc ctg cgc tgg tcc ggg cgc cgg acg gcg cgg       149
Val Ala Ala Ala Val Ala Leu Arg Trp Ser Gly Arg Arg Thr Ala Arg
        25                  30                  35

ggc gcg gtg gtc cgg gcg cga cag aag cag cga gcg ggc ctg gag aac       197
Gly Ala Val Val Arg Ala Arg Gln Lys Gln Arg Ala Gly Leu Glu Asn
    40                  45                  50

atg gac agg gcg gcg cag cgc ttc cgg ctc cag aac cca gac ctg gac       245
Met Asp Arg Ala Ala Gln Arg Phe Arg Leu Gln Asn Pro Asp Leu Asp
55                  60                  65                  70

tca gag gcg ctg cta gcc ctg ccc ctg cct cag ctg gtg cag aag tta       293
Ser Glu Ala Leu Leu Ala Leu Pro Leu Pro Gln Leu Val Gln Lys Leu
                75                  80                  85

cac agt aga gag ctg gcc cct gag gcc gtg ctc ttc acc tat gtg gga       341
His Ser Arg Glu Leu Ala Pro Glu Ala Val Leu Phe Thr Tyr Val Gly
            90                  95                  100

aag gcc tgg gaa gtg aac aaa ggg acc aac tgt gtg acc tcc tat ctg       389
Lys Ala Trp Glu Val Asn Lys Gly Thr Asn Cys Val Thr Ser Tyr Leu
        105                 110                 115

gct gac tgt gag act cag ctg tct cag gcc cca agg cag ggc ctg ctc       437
Ala Asp Cys Glu Thr Gln Leu Ser Gln Ala Pro Arg Gln Gly Leu Leu
    120                 125                 130

tat ggc gtc cct gtg agc ctc aag gag tgc ttc acc tac aag ggc cag       485
Tyr Gly Val Pro Val Ser Leu Lys Glu Cys Phe Thr Tyr Lys Gly Gln
135                 140                 145                 150

gac tcc acg ctg ggc ttg agc ctg aat gaa ggg gtg ccg gcg gag tgc       533

Asp Ser Thr Leu Gly Leu Ser Leu Asn Glu Gly Val Pro Ala Glu Cys
                155                 160                 165

gac agc gta gtg gtg cat gtg ctg aag ctg cag ggt gcc gtg ccc ttc 581
Asp Ser Val Val Val His Val Leu Lys Leu Gln Gly Ala Val Pro Phe
            170                 175                 180

gtg cac acc aat gtt cca cag tcc atg ttc agc tat gac tgc agt aac 629
Val His Thr Asn Val Pro Gln Ser Met Phe Ser Tyr Asp Cys Ser Asn
        185                 190                 195

ccc ctc ttt ggc cag acc gtg aac cca tgg aag tcc tcc aaa agc cca 677
Pro Leu Phe Gly Gln Thr Val Asn Pro Trp Lys Ser Ser Lys Ser Pro
    200                 205                 210

ggg ggc tcc tca ggg ggt gaa ggg gcc ctc atc ggg tct gga ggc tcc 725
Gly Gly Ser Ser Gly Gly Glu Gly Ala Leu Ile Gly Ser Gly Gly Ser
215                 220                 225                 230

ccc ctg ggc tta ggc act gat atc gga ggc agc atc cgc ttc ccc tcc 773
Pro Leu Gly Leu Gly Thr Asp Ile Gly Gly Ser Ile Arg Phe Pro Ser
                235                 240                 245

tcc ttc tgc ggc atc tgc ggc ctc aag ccc aca ggg aac cgc ctc agc 821
Ser Phe Cys Gly Ile Cys Gly Leu Lys Pro Thr Gly Asn Arg Leu Ser
            250                 255                 260

aag agt ggc ctg aag ggc tgt gtc tat gga cag gag gca gtg cgt ctc 869
Lys Ser Gly Leu Lys Gly Cys Val Tyr Gly Gln Glu Ala Val Arg Leu
        265                 270                 275

tcc gtg ggc ccc atg gcc cgg gac gtg gag agc ctg gca ctg tgc ctg 917
Ser Val Gly Pro Met Ala Arg Asp Val Glu Ser Leu Ala Leu Cys Leu
    280                 285                 290

cga gcc ctg ctg tgc gag gac atg ttc cgc ttg gac ccc act gtg cct 965
Arg Ala Leu Leu Cys Glu Asp Met Phe Arg Leu Asp Pro Thr Val Pro
295                 300                 305                 310

ccc ttg ccc ttc aga gaa gag gtc tac acc agc tct cag ccc ctg cgt 1013
Pro Leu Pro Phe Arg Glu Glu Val Tyr Thr Ser Ser Gln Pro Leu Arg
                315                 320                 325

gtg ggg tac tat gag act gac aac tat acc atg ccc tcc ccg gcc atg 1061
Val Gly Tyr Tyr Glu Thr Asp Asn Tyr Thr Met Pro Ser Pro Ala Met
            330                 335                 340

agg cgg gcc gtg ctg gag acc aaa cag agc ctt gag gct gcg ggg cac 1109
Arg Arg Ala Val Leu Glu Thr Lys Gln Ser Leu Glu Ala Ala Gly His
        345                 350                 355

acg ctg gtt ccc ttc ttg cca agc aac ata ccc cat gct ctg gag acc 1157
Thr Leu Val Pro Phe Leu Pro Ser Asn Ile Pro His Ala Leu Glu Thr
    360                 365                 370

ctg tca aca ggt ggg ctc ttc agt gat ggt ggc cac acc ttc cta cag 1205
Leu Ser Thr Gly Gly Leu Phe Ser Asp Gly Gly His Thr Phe Leu Gln
375                 380                 385                 390

aac ttc aaa ggt gat ttc gtg gac ccc tgc ctg ggg gac ctg gtc tca 1253
Asn Phe Lys Gly Asp Phe Val Asp Pro Cys Leu Gly Asp Leu Val Ser
                395                 400                 405

att ctg aag ctt ccc caa tgg ctt aaa gga ctg ctg gcc ttc ctg gtg 1301
Ile Leu Lys Leu Pro Gln Trp Leu Lys Gly Leu Leu Ala Phe Leu Val
            410                 415                 420

aag cct ctg ctg cca agg ctg tca gct ttc ctc agc aac atg aag tct 1349
Lys Pro Leu Leu Pro Arg Leu Ser Ala Phe Leu Ser Asn Met Lys Ser

425                 430                 435

cgt tcg gct gga aaa ctc tgg gaa ctg cag cac gag atc gag gtg tac      1397
Arg Ser Ala Gly Lys Leu Trp Glu Leu Gln His Glu Ile Glu Val Tyr
    440                 445                 450

cgc aaa acc gtg att gcc cag tgg agg gcg ctg gac ctg gat gtg gtg      1445
Arg Lys Thr Val Ile Ala Gln Trp Arg Ala Leu Asp Leu Asp Val Val
455                 460                 465                 470

ctg acc ccc atg ctg gcc cct gct ctg gac ttg aat gcc cca ggc agg      1493
Leu Thr Pro Met Leu Ala Pro Ala Leu Asp Leu Asn Ala Pro Gly Arg
                475                 480                 485

gcc aca ggg gcc gtc agc tac act atg ctg tac aac tgc ctg gac ttc      1541
Ala Thr Gly Ala Val Ser Tyr Thr Met Leu Tyr Asn Cys Leu Asp Phe
            490                 495                 500

cct gca ggg gtg gtg cct gtc acc acg gtg act gct gag gac gag gcc      1589
Pro Ala Gly Val Val Pro Val Thr Thr Val Thr Ala Glu Asp Glu Ala
        505                 510                 515

cag atg gaa cat tac agg ggc tac ttt ggg gat atc tgg gac aag atg      1637
Gln Met Glu His Tyr Arg Gly Tyr Phe Gly Asp Ile Trp Asp Lys Met
    520                 525                 530

ctg cag aag ggc atg aag aag agt gtg ggg ctg ccg gtg gcc gtg cag      1685
Leu Gln Lys Gly Met Lys Lys Ser Val Gly Leu Pro Val Ala Val Gln
535                 540                 545                 550

tgt gtg gct ctg ccc tgg caa gaa gag ttg tgt ctg cgg ttc atg cgg      1733
Cys Val Ala Leu Pro Trp Gln Glu Glu Leu Cys Leu Arg Phe Met Arg
                555                 560                 565

gag gtg gag cga ctg atg acc cct gaa aag cag tca tcc tgatggctct      1782
Glu Val Glu Arg Leu Met Thr Pro Glu Lys Gln Ser Ser
            570                 575

ggctccagag gacctgagac tcacactctc tgcagcccag cctagtcagg gcacagctgc      1842

cctgctgcca cagcaaggaa atgtcctgca tggggcagag gcttccgtgt cctctccccc      1902

aaccccctgc aagaagcgcc gactccctga gtctggacct ccatccctgc tctggtcccc      1962

tctcttcgtc ctgatccctc cacccccatg tggcagccca tgggtatgac ataggccaag      2022

gcccaactaa cagtcaagaa acaaaaaaaa aaaaaaaaaa a                          2063

<210>  2
<211>  579
<212>  PRT
<213>  Homo sapiens

<400>  2

Met Val Gln Tyr Glu Leu Trp Ala Ala Leu Pro Gly Ala Ser Gly Val
1               5                   10                  15

Ala Leu Ala Cys Cys Phe Val Ala Ala Ala Val Ala Leu Arg Trp Ser
            20                  25                  30

Gly Arg Arg Thr Ala Arg Gly Ala Val Val Arg Ala Arg Gln Lys Gln
        35                  40                  45

Arg Ala Gly Leu Glu Asn Met Asp Arg Ala Ala Gln Arg Phe Arg Leu

50                  55                  60

Gln Asn Pro Asp Leu Asp Ser Glu Ala Leu Leu Ala Leu Pro Leu Pro
65                  70                  75                  80

Gln Leu Val Gln Lys Leu His Ser Arg Glu Leu Ala Pro Glu Ala Val
                85                  90                  95

Leu Phe Thr Tyr Val Gly Lys Ala Trp Glu Val Asn Lys Gly Thr Asn
            100                 105                 110

Cys Val Thr Ser Tyr Leu Ala Asp Cys Glu Thr Gln Leu Ser Gln Ala
        115                 120                 125

Pro Arg Gln Gly Leu Leu Tyr Gly Val Pro Val Ser Leu Lys Glu Cys
    130                 135                 140

Phe Thr Tyr Lys Gly Gln Asp Ser Thr Leu Gly Leu Ser Leu Asn Glu
145                 150                 155                 160

Gly Val Pro Ala Glu Cys Asp Ser Val Val Val His Val Leu Lys Leu
                165                 170                 175

Gln Gly Ala Val Pro Phe Val His Thr Asn Val Pro Gln Ser Met Phe
            180                 185                 190

Ser Tyr Asp Cys Ser Asn Pro Leu Phe Gly Gln Thr Val Asn Pro Trp
        195                 200                 205

Lys Ser Ser Lys Ser Pro Gly Gly Ser Ser Gly Gly Glu Gly Ala Leu
    210                 215                 220

Ile Gly Ser Gly Gly Ser Pro Leu Gly Leu Gly Thr Asp Ile Gly Gly
225                 230                 235                 240

Ser Ile Arg Phe Pro Ser Ser Phe Cys Gly Ile Cys Gly Leu Lys Pro
                245                 250                 255

Thr Gly Asn Arg Leu Ser Lys Ser Gly Leu Lys Gly Cys Val Tyr Gly
            260                 265                 270

Gln Glu Ala Val Arg Leu Ser Val Gly Pro Met Ala Arg Asp Val Glu
        275                 280                 285

Ser Leu Ala Leu Cys Leu Arg Ala Leu Leu Cys Glu Asp Met Phe Arg
    290                 295                 300

Leu Asp Pro Thr Val Pro Pro Leu Pro Phe Arg Glu Glu Val Tyr Thr
305                 310                 315                 320

Ser Ser Gln Pro Leu Arg Val Gly Tyr Tyr Glu Thr Asp Asn Tyr Thr
                325                 330                 335

Met Pro Ser Pro Ala Met Arg Arg Ala Val Leu Glu Thr Lys Gln Ser
            340                 345                 350

Leu Glu Ala Ala Gly His Thr Leu Val Pro Phe Leu Pro Ser Asn Ile
        355                 360                 365

Pro His Ala Leu Glu Thr Leu Ser Thr Gly Gly Leu Phe Ser Asp Gly
    370                 375                 380

Gly His Thr Phe Leu Gln Asn Phe Lys Gly Asp Phe Val Asp Pro Cys
385                 390                 395                 400

Leu Gly Asp Leu Val Ser Ile Leu Lys Leu Pro Gln Trp Leu Lys Gly
                405                 410                 415

Leu Leu Ala Phe Leu Val Lys Pro Leu Leu Pro Arg Leu Ser Ala Phe
            420                 425                 430

Leu Ser Asn Met Lys Ser Arg Ser Ala Gly Lys Leu Trp Glu Leu Gln
        435                 440                 445

His Glu Ile Glu Val Tyr Arg Lys Thr Val Ile Ala Gln Trp Arg Ala
    450                 455                 460

Leu Asp Leu Asp Val Val Leu Thr Pro Met Leu Ala Pro Ala Leu Asp
465                 470                 475                 480

Leu Asn Ala Pro Gly Arg Ala Thr Gly Ala Val Ser Tyr Thr Met Leu
                485                 490                 495

Tyr Asn Cys Leu Asp Phe Pro Ala Gly Val Val Pro Val Thr Thr Val
            500                 505                 510

Thr Ala Glu Asp Glu Ala Gln Met Glu His Tyr Arg Gly Tyr Phe Gly
        515                 520                 525

Asp Ile Trp Asp Lys Met Leu Gln Lys Gly Met Lys Lys Ser Val Gly
    530                 535                 540

Leu Pro Val Ala Val Gln Cys Val Ala Leu Pro Trp Gln Glu Glu Leu
545                 550                 555                 560

Cys Leu Arg Phe Met Arg Glu Val Glu Arg Leu Met Thr Pro Glu Lys
                565                 570                 575

Gln Ser Ser

<210>  3
<211>  3805
<212>  DNA

<213>  Mus musculus

<220>
<221>  CDS
<222>  (247)..(1983)
<223>

<400>  3
ggttccgcct gccttaaaac gctggcacgc caggaaccgg gccagaaggg gtctaggctt      60

gaccttgacc gttggagctg ctagcttggc tctcgctgcc gggcaacggc gcgctccccg     120

cgggcccgcg gctgcgcctg cccctccctc aagcggaatc gcgggcgatc caggccgggt     180

tttgcagcgg agctgttggt gtgcgcgtgc cgagtcctct cgggtggcgg tcggctgcag     240

gagatc atg gtg ctg agc gaa gtg tgg acc gcg ctg tct gga ctc tcc        288
       Met Val Leu Ser Glu Val Trp Thr Ala Leu Ser Gly Leu Ser
       1               5                   10

ggg gtt tgc cta gcc tgc agc ttg ctg tcg gcg gcg gtg gtc ctg cga       336
Gly Val Cys Leu Ala Cys Ser Leu Leu Ser Ala Ala Val Val Leu Arg
15                  20                  25                  30

tgg acc agg agc cag acc gcc cgg ggc gcg gtg acc agg gcg cgg cag       384
Trp Thr Arg Ser Gln Thr Ala Arg Gly Ala Val Thr Arg Ala Arg Gln
                35                  40                  45

aag cag cga gcc ggc ctg gag acc atg gac aag gcg gtg cag cgc ttc       432
Lys Gln Arg Ala Gly Leu Glu Thr Met Asp Lys Ala Val Gln Arg Phe
            50                  55                  60

cgg ctg cag aat cct gac ctg gat tca gag gcc ttg ctg gct ctg ccc       480
Arg Leu Gln Asn Pro Asp Leu Asp Ser Glu Ala Leu Leu Ala Leu Pro
        65                  70                  75

ctg ctc caa ctg gta cag aag tta cag agt ggg gaa ctg tcc cca gaa       528
Leu Leu Gln Leu Val Gln Lys Leu Gln Ser Gly Glu Leu Ser Pro Glu
    80                  85                  90

gct gtg ctc ttt acc tac ctg gga aag gcc tgg gaa gtg aac aaa ggg       576
Ala Val Leu Phe Thr Tyr Leu Gly Lys Ala Trp Glu Val Asn Lys Gly
95                  100                 105                 110

acc aac tgt gtg acc tcc tat ctg act gac tgt gag act cag ctg tcc       624
Thr Asn Cys Val Thr Ser Tyr Leu Thr Asp Cys Glu Thr Gln Leu Ser
                115                 120                 125

cag gcc cca cgg cag ggc ctg ctc tat ggc gtc ccc gtg agc ctc aag       672
Gln Ala Pro Arg Gln Gly Leu Leu Tyr Gly Val Pro Val Ser Leu Lys
            130                 135                 140

gaa tgc ttc agc tac aag ggc cat gct tcc aca ctg ggc tta agt ttg       720
Glu Cys Phe Ser Tyr Lys Gly His Ala Ser Thr Leu Gly Leu Ser Leu
        145                 150                 155

aac gag ggt gtg aca tcg gag agt gac tgt gtg gtg gtg cag gta ctg       768
Asn Glu Gly Val Thr Ser Glu Ser Asp Cys Val Val Val Gln Val Leu
    160                 165                 170

aag ctg cag gga gct gtg ccc ttt gtg cac acc aac gtc ccc cag tcc       816
Lys Leu Gln Gly Ala Val Pro Phe Val His Thr Asn Val Pro Gln Ser
175                 180                 185                 190

atg cta agc tat gac tgc agt aac ccc ctc ttt ggc cag acc atg aac       864
Met Leu Ser Tyr Asp Cys Ser Asn Pro Leu Phe Gly Gln Thr Met Asn
                195                 200                 205

ccg tgg aag ccc tcc aag agt cca gga ggt tcc tca ggg ggt gag ggg       912

Pro Trp Lys Pro Ser Lys Ser Pro Gly Gly Ser Ser Gly Gly Glu Gly
            210                 215                 220

gct ctc att gga tct gga ggc tcc cct ctg ggt tta ggc act gac atc 960
Ala Leu Ile Gly Ser Gly Gly Ser Pro Leu Gly Leu Gly Thr Asp Ile
        225                 230                 235

ggc ggc agc atc cgg ttc cct tct gcc ttc tgt ggc atc tgt ggc ctc 1008
Gly Gly Ser Ile Arg Phe Pro Ser Ala Phe Cys Gly Ile Cys Gly Leu
    240                 245                 250

aag cct act ggg aac cgc ctc agc aag agt ggc ctg aag agc tgt gtt 1056
Lys Pro Thr Gly Asn Arg Leu Ser Lys Ser Gly Leu Lys Ser Cys Val
255                 260                 265                 270

tat gga cag aca gca gtg cag ctt tct gtt ggc ccc atg gca cgg gat 1104
Tyr Gly Gln Thr Ala Val Gln Leu Ser Val Gly Pro Met Ala Arg Asp
                275                 280                 285

gtg gat agc ctg gca ttg tgc atg aaa gcc cta ctt tgt gag gat ttg 1152
Val Asp Ser Leu Ala Leu Cys Met Lys Ala Leu Leu Cys Glu Asp Leu
            290                 295                 300

ttc cgc ttg gac tcc acc atc ccc ccc ttg ccc ttc agg gag gag atc 1200
Phe Arg Leu Asp Ser Thr Ile Pro Pro Leu Pro Phe Arg Glu Glu Ile
        305                 310                 315

tac aga agt tct cga ccc ctt cgt gtg gga tac tat gaa act gac aac 1248
Tyr Arg Ser Ser Arg Pro Leu Arg Val Gly Tyr Tyr Glu Thr Asp Asn
    320                 325                 330

tac acc atg ccc act cca gcc atg agg agg gct gtg atg gag acc aag 1296
Tyr Thr Met Pro Thr Pro Ala Met Arg Arg Ala Val Met Glu Thr Lys
335                 340                 345                 350

cag agt ctc gag gct gct ggc cac acg ctg gtc ccc ttc tta cca aac 1344
Gln Ser Leu Glu Ala Ala Gly His Thr Leu Val Pro Phe Leu Pro Asn
                355                 360                 365

aac ata cct tat gcc ctg gag gtc ctg tcg gca ggt ggg ctg ttc agt 1392
Asn Ile Pro Tyr Ala Leu Glu Val Leu Ser Ala Gly Gly Leu Phe Ser
            370                 375                 380

gat ggt ggc tgc tct ttt ctc caa aac ttc aaa ggc gac ttt gtg gat 1440
Asp Gly Gly Cys Ser Phe Leu Gln Asn Phe Lys Gly Asp Phe Val Asp
        385                 390                 395

ccc tgc ttg ggg gac ctg gtc tta gtg ctg aag ctg ccc agg tgg ttt 1488
Pro Cys Leu Gly Asp Leu Val Leu Val Leu Lys Leu Pro Arg Trp Phe
    400                 405                 410

aaa aaa ctg ctg agc ttc ctg ctg aag cct ctg ttt cct cgg ctg gca 1536
Lys Lys Leu Leu Ser Phe Leu Leu Lys Pro Leu Phe Pro Arg Leu Ala
415                 420                 425                 430

gcc ttt ctc aac agt atg tgt cct cgg tca gcc gaa aag ctg tgg gaa 1584
Ala Phe Leu Asn Ser Met Cys Pro Arg Ser Ala Glu Lys Leu Trp Glu
                435                 440                 445

ctg cag cat gag att gag atg tat cgc cag tcc gtc att gcc cag tgg 1632
Leu Gln His Glu Ile Glu Met Tyr Arg Gln Ser Val Ile Ala Gln Trp
            450                 455                 460

aag gca atg aac ttg gac gtg gtg cta acc ccc atg ctg ggt cct gct 1680
Lys Ala Met Asn Leu Asp Val Val Leu Thr Pro Met Leu Gly Pro Ala
        465                 470                 475

ctg gat ttg aac gca ccg ggc aga gcc aca ggg gct atc agc tac act 1728
Leu Asp Leu Asn Ala Pro Gly Arg Ala Thr Gly Ala Ile Ser Tyr Thr

480                 485                 490

gtt ctc tat aac tgc ctg gac ttc cct gcg ggg gtg gtg cct gtc acc      1776
Val Leu Tyr Asn Cys Leu Asp Phe Pro Ala Gly Val Val Pro Val Thr
495                 500                 505                 510

act gtg acc gct gag gac gat gcc cag atg gaa cac tac aaa ggc tac      1824
Thr Val Thr Ala Glu Asp Asp Ala Gln Met Glu His Tyr Lys Gly Tyr
                515                 520                 525

ttt ggg gat atg tgg gac aac att ctg aag aag ggc atg aaa aag ggt      1872
Phe Gly Asp Met Trp Asp Asn Ile Leu Lys Lys Gly Met Lys Lys Gly
            530                 535                 540

ata ggc ctg cct gtg gct gtg cag tgc gtg gct ctg ccc tgg cag gaa      1920
Ile Gly Leu Pro Val Ala Val Gln Cys Val Ala Leu Pro Trp Gln Glu
        545                 550                 555

gag ctg tgt ctg cgg ttc atg cgg gag gtg gaa cgg ctg atg acc cct      1968
Glu Leu Cys Leu Arg Phe Met Arg Glu Val Glu Arg Leu Met Thr Pro
    560                 565                 570

gaa aag cgg cca tct tgagggtcat tcatctgccc agctctggag gacctaaggc      2023
Glu Lys Arg Pro Ser
575

ccatgcgctc tgcactgcag ccccatctat tcaggatcct gccacccatg aggagatgcc      2083

cagcacggga agaggcaacc acctgccctc ccctggactc ctacagaaac ccaggacatg      2143

ccctccataa ccaagtctgg accttgctcc cctttctggt ctactttcca tcctgacccc      2203

ctactctatg tgacagccca gcaggaacga cacgggccaa ggaccaccaa cagtcaaaaa      2263

aagcaatgtg tttctgtatt tttctgggta tttttctatt aggaccttgg aaccagagcc      2323

tgctgagagg gctgtgctgt ccctccagag ctggctgtaa tcatgtcact ctcctgctcc      2383

aaagcctccc taggccatca cccacaaggt agacacaggg acatgtcctt ggcacttggc      2443

tcctgccctt ccttccttgt tcagattggc cccagctttg atggacaccg cccgggtctt      2503

cctccctcat tccacctctc tctcttctga cttggccttt ttacttctct agctgttgta      2563

gagaacaagg tttctctgtg tagccctggc tgtccaggat ctcactctgt agatcaggct      2623

ggctttcagc tcacaaggct gcctgcctgg gtgctgggat taaaggcgtg tgttaccaga      2683

gtctggctgg ctgggatatc ttttagggtc atctctgtaa cccattttct acttacatat      2743

ccacaagtca gaacactgga ctgaggaagg actatgtcac cctggatacc tgtcagggaa      2803

cacttacaga gataccagtg tcatcaattt gagcttccca gccaaataag tctcctccag      2863

tctgtgtttg gttagcttgc cagtattgac ctggctcctg acggctcctc catgtaggtt      2923

gtactggcga cctaggatac cccttgtggt gagtgagtga gaccacttgt ggtctgggct      2983

ctgtaaggtt tatcatcaca tgcctagcac ccagcatgtt acctggcact tagtagatag      3043

ccagtcaggc ttgttggagg gatggcagat ttatttctag cgaatccatt ttggcacctc      3103

ccgattactt cctatgccat ccataatagg gaattttcag tttgcctctg acctgctacc      3163

tttatctaat ctacttaatt cactttgatt catgaccaca gggacaaata tctctttgtc      3223

tgtgatcaac gtagagcttc ctcctggtca agaaggctgc atgcatcaac ttctaccaac      3283

cctcagctcc tccatacttc ttccacccag cccacaagca cattgaattc ctcccactat      3343

gctcaggata agcaaacaga aacagcccct gcttgaatgg tttctcattc taccagcaac 3403

tgtgtattta cactgtaggt cacacaaatg ctataaagca gaagtccctg ggtatgatga 3463

catcaagcag gctacctggc ccgcaggtca tgtgtgcaag agtaggagta tcagggagtg 3523

ctctctgtgt gaagtgacat gtaagctgga aagctcacag ttaatgggag ccaaggaaca 3583

agtatttcaa gaagagtgga gtcgacttaa acgctgctga tgtaactgga taaggacaag 3643

tgaagggagg gatggagaac tccacctgcc atcattgaat cgagtgccta ctgtttgcca 3703

gacactttac caaaggctgc caaggtcctt gctcttaggg aactcgtttt ctagagagag 3763

ggcatgcaaa taagatcata aataaaataa tttatgctga tg 3805

<210> 4
<211> 579
<212> PRT
<213> Mus musculus

<400> 4

Met Val Leu Ser Glu Val Trp Thr Ala Leu Ser Gly Leu Ser Gly Val
1               5                   10                  15

Cys Leu Ala Cys Ser Leu Leu Ser Ala Ala Val Val Leu Arg Trp Thr
            20                  25                  30

Arg Ser Gln Thr Ala Arg Gly Ala Val Thr Arg Ala Arg Gln Lys Gln
        35                  40                  45

Arg Ala Gly Leu Glu Thr Met Asp Lys Ala Val Gln Arg Phe Arg Leu
    50                  55                  60

Gln Asn Pro Asp Leu Asp Ser Glu Ala Leu Leu Ala Leu Pro Leu Leu
65                  70                  75                  80

Gln Leu Val Gln Lys Leu Gln Ser Gly Glu Leu Ser Pro Glu Ala Val
                85                  90                  95

Leu Phe Thr Tyr Leu Gly Lys Ala Trp Glu Val Asn Lys Gly Thr Asn
            100                 105                 110

Cys Val Thr Ser Tyr Leu Thr Asp Cys Glu Thr Gln Leu Ser Gln Ala
        115                 120                 125

Pro Arg Gln Gly Leu Leu Tyr Gly Val Pro Val Ser Leu Lys Glu Cys
    130                 135                 140

Phe Ser Tyr Lys Gly His Ala Ser Thr Leu Gly Leu Ser Leu Asn Glu
145                 150                 155                 160

Gly Val Thr Ser Glu Ser Asp Cys Val Val Val Gln Val Leu Lys Leu
                165                 170                 175

Gln Gly Ala Val Pro Phe Val His Thr Asn Val Pro Gln Ser Met Leu
            180                 185                 190

Ser Tyr Asp Cys Ser Asn Pro Leu Phe Gly Gln Thr Met Asn Pro Trp
        195                 200                 205

Lys Pro Ser Lys Ser Pro Gly Gly Ser Ser Gly Gly Glu Gly Ala Leu
    210                 215                 220

Ile Gly Ser Gly Gly Ser Pro Leu Gly Leu Gly Thr Asp Ile Gly Gly
225                 230                 235                 240

Ser Ile Arg Phe Pro Ser Ala Phe Cys Gly Ile Cys Gly Leu Lys Pro
                245                 250                 255

Thr Gly Asn Arg Leu Ser Lys Ser Gly Leu Lys Ser Cys Val Tyr Gly
            260                 265                 270

Gln Thr Ala Val Gln Leu Ser Val Gly Pro Met Ala Arg Asp Val Asp
        275                 280                 285

Ser Leu Ala Leu Cys Met Lys Ala Leu Leu Cys Glu Asp Leu Phe Arg
    290                 295                 300

Leu Asp Ser Thr Ile Pro Pro Leu Pro Phe Arg Glu Glu Ile Tyr Arg
305                 310                 315                 320

Ser Ser Arg Pro Leu Arg Val Gly Tyr Tyr Glu Thr Asp Asn Tyr Thr
                325                 330                 335

Met Pro Thr Pro Ala Met Arg Arg Ala Val Met Glu Thr Lys Gln Ser
            340                 345                 350

Leu Glu Ala Ala Gly His Thr Leu Val Pro Phe Leu Pro Asn Asn Ile
        355                 360                 365

Pro Tyr Ala Leu Glu Val Leu Ser Ala Gly Gly Leu Phe Ser Asp Gly
    370                 375                 380

Gly Cys Ser Phe Leu Gln Asn Phe Lys Gly Asp Phe Val Asp Pro Cys
385                 390                 395                 400

Leu Gly Asp Leu Val Leu Val Leu Lys Leu Pro Arg Trp Phe Lys Lys
                405                 410                 415

Leu Leu Ser Phe Leu Leu Lys Pro Leu Phe Pro Arg Leu Ala Ala Phe
            420                 425                 430

Leu Asn Ser Met Cys Pro Arg Ser Ala Glu Lys Leu Trp Glu Leu Gln
        435                 440                 445

His Glu Ile Glu Met Tyr Arg Gln Ser Val Ile Ala Gln Trp Lys Ala

450                 455                 460

Met Asn Leu Asp Val Val Leu Thr Pro Met Leu Gly Pro Ala Leu Asp
465                 470                 475                 480

Leu Asn Ala Pro Gly Arg Ala Thr Gly Ala Ile Ser Tyr Thr Val Leu
                485                 490                 495

Tyr Asn Cys Leu Asp Phe Pro Ala Gly Val Val Pro Val Thr Thr Val
            500                 505                 510

Thr Ala Glu Asp Asp Ala Gln Met Glu His Tyr Lys Gly Tyr Phe Gly
        515                 520                 525

Asp Met Trp Asp Asn Ile Leu Lys Lys Gly Met Lys Lys Gly Ile Gly
    530                 535                 540

Leu Pro Val Ala Val Gln Cys Val Ala Leu Pro Trp Gln Glu Glu Leu
545                 550                 555                 560

Cys Leu Arg Phe Met Arg Glu Val Glu Arg Leu Met Thr Pro Glu Lys
                565                 570                 575

Arg Pro Ser

<210>  5
<211>  2472
<212>  DNA
<213>  Rattus norvegicus

<220>
<221>  CDS
<222>  (50)..(1786)
<223>

<400>  5
ggtttgtgcg agccgagttc tctcgggtgg cggtcggctg caggagatc atg gtg ctg      58
                                                      Met Val Leu
                                                      1

agc gaa gtg tgg acc acg ctg tct ggg gtc tcc ggg gtt tgc cta gcc      106
Ser Glu Val Trp Thr Thr Leu Ser Gly Val Ser Gly Val Cys Leu Ala
    5                   10                  15

tgc agc ttg ttg tcg gcg gcg gtg gtc ctg cga tgg acc ggg cgc cag      154
Cys Ser Leu Leu Ser Ala Ala Val Val Leu Arg Trp Thr Gly Arg Gln
20                  25                  30                  35

aag gcc cgg ggc gcg gcg acc agg gcg cgg cag aag cag cga gcc agc      202
Lys Ala Arg Gly Ala Ala Thr Arg Ala Arg Gln Lys Gln Arg Ala Ser
                40                  45                  50

ctg gag acc atg gac aag gcg gtg cag cgc ttc cgg ctg cag aat cct      250
Leu Glu Thr Met Asp Lys Ala Val Gln Arg Phe Arg Leu Gln Asn Pro
            55                  60                  65

gac ctg gac tcg gag gcc ttg ctg acc ctg ccc cta ctc caa ctg gta      298
Asp Leu Asp Ser Glu Ala Leu Leu Thr Leu Pro Leu Leu Gln Leu Val
        70                  75                  80

cag aag tta cag agt gga gag ctg tcc cca gag gct gtg ttc ttt act 346
Gln Lys Leu Gln Ser Gly Glu Leu Ser Pro Glu Ala Val Phe Phe Thr
    85                  90                  95

tac ctg gga aag gcc tgg gaa gtg aac aaa ggg acc aac tgc gtg acc 394
Tyr Leu Gly Lys Ala Trp Glu Val Asn Lys Gly Thr Asn Cys Val Thr
100                 105                 110                 115

tcc tat ctg acc gac tgt gag act cag ctg tcc cag gcc cca cgg cag 442
Ser Tyr Leu Thr Asp Cys Glu Thr Gln Leu Ser Gln Ala Pro Arg Gln
                120                 125                 130

ggc ctg ctc tat ggt gtc cct gtg agc ctc aag gaa tgc ttc agc tac 490
Gly Leu Leu Tyr Gly Val Pro Val Ser Leu Lys Glu Cys Phe Ser Tyr
            135                 140                 145

aag ggc cac gac tcc aca ctg ggc ttg agc ctg aat gag ggc atg cca 538
Lys Gly His Asp Ser Thr Leu Gly Leu Ser Leu Asn Glu Gly Met Pro
        150                 155                 160

tcg gaa tct gac tgt gtg gtg gtg caa gtg ttg aag ctg cag gga gct 586
Ser Glu Ser Asp Cys Val Val Val Gln Val Leu Lys Leu Gln Gly Ala
    165                 170                 175

gtg ccc ttt gtg cat acc aat gtc ccc cag tcc atg tta agc ttt gac 634
Val Pro Phe Val His Thr Asn Val Pro Gln Ser Met Leu Ser Phe Asp
180                 185                 190                 195

tgc agt aac cct ctc ttt ggc cag acc atg aac cca tgg aag tcc tcc 682
Cys Ser Asn Pro Leu Phe Gly Gln Thr Met Asn Pro Trp Lys Ser Ser
                200                 205                 210

aag agc cca gga ggt tcc tca ggg ggt gag ggg gct ctc att gga tct 730
Lys Ser Pro Gly Gly Ser Ser Gly Gly Glu Gly Ala Leu Ile Gly Ser
            215                 220                 225

gga ggt tcc cct ctg ggt tta ggc act gac att ggc ggc agc atc cgg 778
Gly Gly Ser Pro Leu Gly Leu Gly Thr Asp Ile Gly Gly Ser Ile Arg
        230                 235                 240

ttc cct tct gcc ttc tgc ggc atc tgt ggc ctc aag cct act ggc aac 826
Phe Pro Ser Ala Phe Cys Gly Ile Cys Gly Leu Lys Pro Thr Gly Asn
    245                 250                 255

cgc ctc agc aag agt ggc ctg aag ggc tgt gtc tat gga cag acg gca 874
Arg Leu Ser Lys Ser Gly Leu Lys Gly Cys Val Tyr Gly Gln Thr Ala
260                 265                 270                 275

gtg cag ctt tct ctt ggc ccc atg gcc cgg gat gtg gag agc ctg gcg 922
Val Gln Leu Ser Leu Gly Pro Met Ala Arg Asp Val Glu Ser Leu Ala
                280                 285                 290

cta tgc ctg aaa gct cta ctg tgt gag cac ttg ttc acc ttg gac cct 970
Leu Cys Leu Lys Ala Leu Leu Cys Glu His Leu Phe Thr Leu Asp Pro
            295                 300                 305

acc gtg cct ccc ttg ccc ttc aga gag gag gtc tat aga agt tct aga 1018
Thr Val Pro Pro Leu Pro Phe Arg Glu Glu Val Tyr Arg Ser Ser Arg
        310                 315                 320

ccc ctg cgt gtg ggg tac tat gag act gac aac tat acc atg ccc agc 1066
Pro Leu Arg Val Gly Tyr Tyr Glu Thr Asp Asn Tyr Thr Met Pro Ser
    325                 330                 335

cca gct atg agg agg gct ctg ata gag acc aag cag aga ctt gag gct 1114
Pro Ala Met Arg Arg Ala Leu Ile Glu Thr Lys Gln Arg Leu Glu Ala
340                 345                 350                 355

gct ggc cac acg ctg att ccc ttc tta ccc aac aac ata ccc tac gcc     1162
Ala Gly His Thr Leu Ile Pro Phe Leu Pro Asn Asn Ile Pro Tyr Ala
                360                 365                 370

ctg gag gtc ctg tct gcg ggc ggc ctg ttc agt gac ggt ggc cgc agt     1210
Leu Glu Val Leu Ser Ala Gly Gly Leu Phe Ser Asp Gly Gly Arg Ser
            375                 380                 385

ttt ctc caa aac ttc aaa ggt gac ttt gtg gat ccc tgc ttg gga gac     1258
Phe Leu Gln Asn Phe Lys Gly Asp Phe Val Asp Pro Cys Leu Gly Asp
        390                 395                 400

ctg atc tta att ctg agg ctg ccc agc tgg ttt aaa aga ctg ctg agc     1306
Leu Ile Leu Ile Leu Arg Leu Pro Ser Trp Phe Lys Arg Leu Leu Ser
    405                 410                 415

ctc ctg ctg aag cct ctg ttt cct cgg ctg gca gcc ttt ctc aac agt     1354
Leu Leu Leu Lys Pro Leu Phe Pro Arg Leu Ala Ala Phe Leu Asn Ser
420                 425                 430                 435

atg cgt cct cgg tca gct gaa aag ctg tgg aaa ctg cag cat gag att     1402
Met Arg Pro Arg Ser Ala Glu Lys Leu Trp Lys Leu Gln His Glu Ile
                440                 445                 450

gag atg tat cgc cag tct gtg att gcc cag tgg aaa gcg atg aac ttg     1450
Glu Met Tyr Arg Gln Ser Val Ile Ala Gln Trp Lys Ala Met Asn Leu
            455                 460                 465

gat gtg ctg ctg acc ccc atg ttg ggc cct gct ctg gat ttg aac aca     1498
Asp Val Leu Leu Thr Pro Met Leu Gly Pro Ala Leu Asp Leu Asn Thr
        470                 475                 480

ccg ggc aga gcc aca ggg gct atc agc tac acc gtt ctc tac aac tgc     1546
Pro Gly Arg Ala Thr Gly Ala Ile Ser Tyr Thr Val Leu Tyr Asn Cys
    485                 490                 495

ctg gac ttc cct gcg ggg gtg gtg cct gtc acc act gtg acc gcc gag     1594
Leu Asp Phe Pro Ala Gly Val Val Pro Val Thr Thr Val Thr Ala Glu
500                 505                 510                 515

gac gat gcc cag atg gaa ctc tac aaa ggc tac ttt ggg gat atc tgg     1642
Asp Asp Ala Gln Met Glu Leu Tyr Lys Gly Tyr Phe Gly Asp Ile Trp
                520                 525                 530

gac atc atc ctg aag aag gcc atg aaa aat agt gtc ggt ctg cct gtg     1690
Asp Ile Ile Leu Lys Lys Ala Met Lys Asn Ser Val Gly Leu Pro Val
            535                 540                 545

gct gtg cag tgc gtg gct ctg ccc tgg cag gaa gag ctg tgt ctg agg     1738
Ala Val Gln Cys Val Ala Leu Pro Trp Gln Glu Glu Leu Cys Leu Arg
        550                 555                 560

ttc atg cgg gag gtg gaa cag ctg atg acc cct caa aag cag cca tcg     1786
Phe Met Arg Glu Val Glu Gln Leu Met Thr Pro Gln Lys Gln Pro Ser
    565                 570                 575

tgagggtcgt tcatccgcca gctctggagg acctaaggcc catgcgctgt gcactgtagc     1846

cccatgtatt caggagccac cacccacgag ggaacgccca gcacagggaa gaggtgtcta     1906

cctgccctcc cctggactcc tgcagccaca accaagtctg gaccttcctc cccgttatgg     1966

tctactttcc atcctgattc cctgcttttt atggcagcca gcaggaatga cgtgggccaa     2026

ggatcaccaa cattcaaaaa caatgcgttt atctattttc tgggtatctc cattagggcc     2086

ctgggaacca gagtgctggg aaggctgtcc agaccctcca gagctggctg taaccacatc     2146

actctcctgc tccaaagcct ccctagttct gtcacccaca agatagacac agggacatgt     2206

ccttggcact tgactcctgt ccttcctttc ttattcagat tgaccccagc cttgatggac 2266

cctgcccctg cacttccttc ctcagtccac ctctctgccg acacgccctt tttatggctc 2326

ctctatttgt tgtggagaca aggtttctct cagtagccct ggctgtccag gacctcactc 2386

tgtagatgag gctggctttc aactcacaag gctgcctgcc tgggtgctgg gattaaaggc 2446

gtatgccacc acaaagaaaa aaaaaa 2472

<210> 6
<211> 579
<212> PRT
<213> Rattus norvegicus

<400> 6

Met Val Leu Ser Glu Val Trp Thr Thr Leu Ser Gly Val Ser Gly Val
1               5                   10                  15

Cys Leu Ala Cys Ser Leu Leu Ser Ala Ala Val Val Leu Arg Trp Thr
            20                  25                  30

Gly Arg Gln Lys Ala Arg Gly Ala Ala Thr Arg Ala Arg Gln Lys Gln
        35                  40                  45

Arg Ala Ser Leu Glu Thr Met Asp Lys Ala Val Gln Arg Phe Arg Leu
    50                  55                  60

Gln Asn Pro Asp Leu Asp Ser Glu Ala Leu Leu Thr Leu Pro Leu Leu
65                  70                  75                  80

Gln Leu Val Gln Lys Leu Gln Ser Gly Glu Leu Ser Pro Glu Ala Val
                85                  90                  95

Phe Phe Thr Tyr Leu Gly Lys Ala Trp Glu Val Asn Lys Gly Thr Asn
            100                 105                 110

Cys Val Thr Ser Tyr Leu Thr Asp Cys Glu Thr Gln Leu Ser Gln Ala
        115                 120                 125

Pro Arg Gln Gly Leu Leu Tyr Gly Val Pro Val Ser Leu Lys Glu Cys
    130                 135                 140

Phe Ser Tyr Lys Gly His Asp Ser Thr Leu Gly Leu Ser Leu Asn Glu
145                 150                 155                 160

Gly Met Pro Ser Glu Ser Asp Cys Val Val Val Gln Val Leu Lys Leu
                165                 170                 175

Gln Gly Ala Val Pro Phe Val His Thr Asn Val Pro Gln Ser Met Leu
            180                 185                 190

Ser Phe Asp Cys Ser Asn Pro Leu Phe Gly Gln Thr Met Asn Pro Trp
        195                 200                 205

Lys Ser Ser Lys Ser Pro Gly Gly Ser Ser Gly Gly Glu Gly Ala Leu
    210                 215                 220

Ile Gly Ser Gly Gly Ser Pro Leu Gly Leu Gly Thr Asp Ile Gly Gly
225                 230                 235                 240

Ser Ile Arg Phe Pro Ser Ala Phe Cys Gly Ile Cys Gly Leu Lys Pro
                245                 250                 255

Thr Gly Asn Arg Leu Ser Lys Ser Gly Leu Lys Gly Cys Val Tyr Gly
            260                 265                 270

Gln Thr Ala Val Gln Leu Ser Leu Gly Pro Met Ala Arg Asp Val Glu
        275                 280                 285

Ser Leu Ala Leu Cys Leu Lys Ala Leu Leu Cys Glu His Leu Phe Thr
    290                 295                 300

Leu Asp Pro Thr Val Pro Pro Leu Pro Phe Arg Glu Glu Val Tyr Arg
305                 310                 315                 320

Ser Ser Arg Pro Leu Arg Val Gly Tyr Tyr Glu Thr Asp Asn Tyr Thr
                325                 330                 335

Met Pro Ser Pro Ala Met Arg Arg Ala Leu Ile Glu Thr Lys Gln Arg
            340                 345                 350

Leu Glu Ala Ala Gly His Thr Leu Ile Pro Phe Leu Pro Asn Asn Ile
        355                 360                 365

Pro Tyr Ala Leu Glu Val Leu Ser Ala Gly Gly Leu Phe Ser Asp Gly
    370                 375                 380

Gly Arg Ser Phe Leu Gln Asn Phe Lys Gly Asp Phe Val Asp Pro Cys
385                 390                 395                 400

Leu Gly Asp Leu Ile Leu Ile Leu Arg Leu Pro Ser Trp Phe Lys Arg
                405                 410                 415

Leu Leu Ser Leu Leu Leu Lys Pro Leu Phe Pro Arg Leu Ala Ala Phe
            420                 425                 430

Leu Asn Ser Met Arg Pro Arg Ser Ala Glu Lys Leu Trp Lys Leu Gln
        435                 440                 445

His Glu Ile Glu Met Tyr Arg Gln Ser Val Ile Ala Gln Trp Lys Ala
    450                 455                 460

Met Asn Leu Asp Val Leu Leu Thr Pro Met Leu Gly Pro Ala Leu Asp
465                 470                 475                 480

Leu Asn Thr Pro Gly Arg Ala Thr Gly Ala Ile Ser Tyr Thr Val Leu
                485                 490                 495

Tyr Asn Cys Leu Asp Phe Pro Ala Gly Val Val Pro Val Thr Thr Val
            500                 505                 510

Thr Ala Glu Asp Asp Ala Gln Met Glu Leu Tyr Lys Gly Tyr Phe Gly
        515                 520                 525

Asp Ile Trp Asp Ile Ile Leu Lys Lys Ala Met Lys Asn Ser Val Gly
    530                 535                 540

Leu Pro Val Ala Val Gln Cys Val Ala Leu Pro Trp Gln Glu Glu Leu
545                 550                 555                 560

Cys Leu Arg Phe Met Arg Glu Val Glu Gln Leu Met Thr Pro Gln Lys
                565                 570                 575

Gln Pro Ser

<210>  7
<211>  2300
<212>  DNA
<213>  Sus scrofa

<220>
<221>  CDS
<222>  (20)..(1756)
<223>

<400>  7
cggtcctcgg tgggagatc atg gtg cag gaa gaa ctg tgg gct gcg ttc tcc      52
                     Met Val Gln Glu Glu Leu Trp Ala Ala Phe Ser
                     1               5                   10

ggc ccc tcc ggg gtt gcc ctg gcc tgc tgc ttg gtg gca gcg gcc ttg      100
Gly Pro Ser Gly Val Ala Leu Ala Cys Cys Leu Val Ala Ala Ala Leu
            15                  20                  25

gcc ctg cgt tgg tcc agt cgc cgg atg gcg cgg ggc gcg gcg gcc cgg      148
Ala Leu Arg Trp Ser Ser Arg Arg Met Ala Arg Gly Ala Ala Ala Arg
        30                  35                  40

gcg cga cag agg cag caa gcg gcc ctg gag acc atg gac aag gcg gcg      196
Ala Arg Gln Arg Gln Gln Ala Ala Leu Glu Thr Met Asp Lys Ala Ala
    45                  50                  55

cag cgc ttc cgg ctc cag aac ccc gat ctg gac tcg gag atg ctg ctg      244
Gln Arg Phe Arg Leu Gln Asn Pro Asp Leu Asp Ser Glu Met Leu Leu
60                  65                  70                  75

gcc ctg cca ctg cct cag ctg gta cag aag gta cga agt ggg gag ctg      292
Ala Leu Pro Leu Pro Gln Leu Val Gln Lys Val Arg Ser Gly Glu Leu
                80                  85                  90

tct cca gag gct gtg ctc ttt tcc tac ctg caa aag gcc tgg gaa gtg      340
Ser Pro Glu Ala Val Leu Phe Ser Tyr Leu Gln Lys Ala Trp Glu Val
            95                  100                 105

aac aga ggg acc aac tgc gtg acc acc tac ctg gca gac tgt gag gct      388

Asn Arg Gly Thr Asn Cys Val Thr Thr Tyr Leu Ala Asp Cys Glu Ala
        110                 115                 120

cag ctg tgc cag gcg ccc ggg cag ggc ctg ctc tac ggt gtc ccc gtc     436
Gln Leu Cys Gln Ala Pro Gly Gln Gly Leu Leu Tyr Gly Val Pro Val
    125                 130                 135

agc ctc aag gag tgc ttc agc tgc aag ggc cat gac tcc acg ctg ggc     484
Ser Leu Lys Glu Cys Phe Ser Cys Lys Gly His Asp Ser Thr Leu Gly
140                 145                 150                 155

ttg agc cgg aac cag ggg aca cca gca gaa tgt gac tgc gtg gtg gtg     532
Leu Ser Arg Asn Gln Gly Thr Pro Ala Glu Cys Asp Cys Val Val Val
                160                 165                 170

cag gtg ctg aaa ctg cag ggt gct gtg cct ttc gtg cac acc aac gtc     580
Gln Val Leu Lys Leu Gln Gly Ala Val Pro Phe Val His Thr Asn Val
            175                 180                 185

ccc cag tcc atg ttc agc tat gac tgc agt aac ccc ctc ttt ggc cag     628
Pro Gln Ser Met Phe Ser Tyr Asp Cys Ser Asn Pro Leu Phe Gly Gln
        190                 195                 200

acc acg aac cca tgg atg tcg tcc aag agc ccg ggc ggc tcc tcg gga     676
Thr Thr Asn Pro Trp Met Ser Ser Lys Ser Pro Gly Gly Ser Ser Gly
    205                 210                 215

ggt gag ggg gcc ctc att gct gct gga ggc tcc cca ctg ggc tta ggc     724
Gly Glu Gly Ala Leu Ile Ala Ala Gly Gly Ser Pro Leu Gly Leu Gly
220                 225                 230                 235

acc gac atc ggg ggc agc atc cgc ttt ccc tcc gcc ttc tgt ggc atc     772
Thr Asp Ile Gly Gly Ser Ile Arg Phe Pro Ser Ala Phe Cys Gly Ile
                240                 245                 250

tgc ggc atc aaa ccc acg ggg aac cgc atc agc aag agt ggt ctg aag     820
Cys Gly Ile Lys Pro Thr Gly Asn Arg Ile Ser Lys Ser Gly Leu Lys
            255                 260                 265

ggc tct gtc tat gga cag gta gca gtg cag ctc tca gtg ggc ccc atg     868
Gly Ser Val Tyr Gly Gln Val Ala Val Gln Leu Ser Val Gly Pro Met
        270                 275                 280

gcg cgg gac gtg gag agc ctg gcc ctg tgc ctg cgt gcg ctg ctg tgc     916
Ala Arg Asp Val Glu Ser Leu Ala Leu Cys Leu Arg Ala Leu Leu Cys
    285                 290                 295

gaa gac atg ttc cgc ctg gac ccc acg gtg cct ccc ctg ccc ttc aac     964
Glu Asp Met Phe Arg Leu Asp Pro Thr Val Pro Pro Leu Pro Phe Asn
300                 305                 310                 315

gag gag gtc tac gca agc tct cgg ccc ctg cgt gtc ggg tat tat gag    1012
Glu Glu Val Tyr Ala Ser Ser Arg Pro Leu Arg Val Gly Tyr Tyr Glu
                320                 325                 330

acc gac aac tac acc atg ccc acg ccg gcc atg agg cgg gcc ctg ctg    1060
Thr Asp Asn Tyr Thr Met Pro Thr Pro Ala Met Arg Arg Ala Leu Leu
            335                 340                 345

gag acc aag cgg agc ctt gag gct gcg ggc cac acg ctg att ccc ttc    1108
Glu Thr Lys Arg Ser Leu Glu Ala Ala Gly His Thr Leu Ile Pro Phe
        350                 355                 360

ctg ccg gcc aac ata ccc cac gct ctg gag gcc ctg tca acg ggc ggg    1156
Leu Pro Ala Asn Ile Pro His Ala Leu Glu Ala Leu Ser Thr Gly Gly
    365                 370                 375

ctc ttc agt gat ggt ggg aag agg ttg cta cag aac ttc gaa ggc gat    1204
Leu Phe Ser Asp Gly Gly Lys Arg Leu Leu Gln Asn Phe Glu Gly Asp

380                 385                 390                 395

tac gtg gac tcc tgc tta ggg gac ctg atc tca att ctg agg ctg ccc     1252
Tyr Val Asp Ser Cys Leu Gly Asp Leu Ile Ser Ile Leu Arg Leu Pro
                400                 405                 410

aaa tgg ctt aaa gga ctg ctg gct ttc atg ctg agg cct ctg ctc cca     1300
Lys Trp Leu Lys Gly Leu Leu Ala Phe Met Leu Arg Pro Leu Leu Pro
            415                 420                 425

agg ttg gca ggc ttt ctc agc agc ctg agg cct cgg tcg gct gga aag     1348
Arg Leu Ala Gly Phe Leu Ser Ser Leu Arg Pro Arg Ser Ala Gly Lys
        430                 435                 440

ctc tgg gaa ctg cag cac gag att gag atg tac cgt cac tcc gtg att     1396
Leu Trp Glu Leu Gln His Glu Ile Glu Met Tyr Arg His Ser Val Ile
    445                 450                 455

gcc cag tgg cga gcg ctg gac ctg gat gtg gtg cta acc ccc atg ctg     1444
Ala Gln Trp Arg Ala Leu Asp Leu Asp Val Val Leu Thr Pro Met Leu
460                 465                 470                 475

agc cct gcc cta gac ttg aat gcc cca ggc aag gcc aca ggg gcc gtc     1492
Ser Pro Ala Leu Asp Leu Asn Ala Pro Gly Lys Ala Thr Gly Ala Val
                480                 485                 490

agc tac acg ctg ctc tac aac tgc ctg gac ttc ccc gcg ggg gtg gtg     1540
Ser Tyr Thr Leu Leu Tyr Asn Cys Leu Asp Phe Pro Ala Gly Val Val
            495                 500                 505

cct gtc acc acg gtg act gcc gag gac gag gcc cag atg gag cat tac     1588
Pro Val Thr Thr Val Thr Ala Glu Asp Glu Ala Gln Met Glu His Tyr
        510                 515                 520

aag ggc tac ttt ggg gac att tgg gac aag gtg gtg cag aag gcc atg     1636
Lys Gly Tyr Phe Gly Asp Ile Trp Asp Lys Val Val Gln Lys Ala Met
    525                 530                 535

aag agg agc gtg ggg ctg cct gtg gcc gtg cag tgt gtg gct ctg ccc     1684
Lys Arg Ser Val Gly Leu Pro Val Ala Val Gln Cys Val Ala Leu Pro
540                 545                 550                 555

tgg cag gag gag ctg tgt ttg cgg ttc atg cgg gag gtg gag cga ctg     1732
Trp Gln Glu Glu Leu Cys Leu Arg Phe Met Arg Glu Val Glu Arg Leu
                560                 565                 570

atg gct cct ggg cgg cag ccc tcc tgaccgctgc ccgcccggcc ccccaggacc     1786
Met Ala Pro Gly Arg Gln Pro Ser
            575

tgagacccac tggatccgcg cccagcggag tcaggacaca actgccaccg tgcaagaaaa     1846

tgttcaacct caggcagagg cttcccggtc tctccccctc gcccctgcca gaagcccaga     1906

accactgagt ctggaccttg ctcttcccgt ggtccctgct ctgccctgac cccgccaatg     1966

tggcagctag tgggtatgac atggcaaagg ccccccaacc gtcaaaaacc ggttcctggt     2026

ctccatactt tctggcagtc gttgttaggg cagtgggggt tggagacctg accttctgga     2086

acccgactcc agccatgtcc gtctcgtgct gcagaagctt ctctggtcct cgtcactcac     2146

gggcagacac cggcttctcc gagtgggcct tgcagcccag gacttcaccc cgccgccccc     2206

agcctaagcc ctactttgcg aggcattgtc ttctctcctg ccctctgctg agggtgccct     2266

ttctgctcct ctaccattaa atcctttgag gccc                                 2300

<210>  8
<211>  579
<212>  PRT
<213>  Sus scrofa

<400>  8

Met Val Gln Glu Glu Leu Trp Ala Ala Phe Ser Gly Pro Ser Gly Val
1               5                   10                  15

Ala Leu Ala Cys Cys Leu Val Ala Ala Ala Leu Ala Leu Arg Trp Ser
            20                  25                  30

Ser Arg Arg Met Ala Arg Gly Ala Ala Ala Arg Ala Arg Gln Arg Gln
        35                  40                  45

Gln Ala Ala Leu Glu Thr Met Asp Lys Ala Ala Gln Arg Phe Arg Leu
    50                  55                  60

Gln Asn Pro Asp Leu Asp Ser Glu Met Leu Leu Ala Leu Pro Leu Pro
65                  70                  75                  80

Gln Leu Val Gln Lys Val Arg Ser Gly Glu Leu Ser Pro Glu Ala Val
                85                  90                  95

Leu Phe Ser Tyr Leu Gln Lys Ala Trp Glu Val Asn Arg Gly Thr Asn
            100                 105                 110

Cys Val Thr Thr Tyr Leu Ala Asp Cys Glu Ala Gln Leu Cys Gln Ala
        115                 120                 125

Pro Gly Gln Gly Leu Leu Tyr Gly Val Pro Val Ser Leu Lys Glu Cys
    130                 135                 140

Phe Ser Cys Lys Gly His Asp Ser Thr Leu Gly Leu Ser Arg Asn Gln
145                 150                 155                 160

Gly Thr Pro Ala Glu Cys Asp Cys Val Val Val Gln Val Leu Lys Leu
                165                 170                 175

Gln Gly Ala Val Pro Phe Val His Thr Asn Val Pro Gln Ser Met Phe
            180                 185                 190

Ser Tyr Asp Cys Ser Asn Pro Leu Phe Gly Gln Thr Thr Asn Pro Trp
        195                 200                 205

Met Ser Ser Lys Ser Pro Gly Gly Ser Ser Gly Gly Glu Gly Ala Leu
    210                 215                 220

Ile Ala Ala Gly Gly Ser Pro Leu Gly Leu Gly Thr Asp Ile Gly Gly
225                 230                 235                 240

Ser Ile Arg Phe Pro Ser Ala Phe Cys Gly Ile Cys Gly Ile Lys Pro
                245                 250                 255

Thr Gly Asn Arg Ile Ser Lys Ser Gly Leu Lys Gly Ser Val Tyr Gly
            260                 265                 270

Gln Val Ala Val Gln Leu Ser Val Gly Pro Met Ala Arg Asp Val Glu
        275                 280                 285

Ser Leu Ala Leu Cys Leu Arg Ala Leu Leu Cys Glu Asp Met Phe Arg
    290                 295                 300

Leu Asp Pro Thr Val Pro Pro Leu Pro Phe Asn Glu Glu Val Tyr Ala
305                 310                 315                 320

Ser Ser Arg Pro Leu Arg Val Gly Tyr Tyr Glu Thr Asp Asn Tyr Thr
                325                 330                 335

Met Pro Thr Pro Ala Met Arg Arg Ala Leu Leu Glu Thr Lys Arg Ser
            340                 345                 350

Leu Glu Ala Ala Gly His Thr Leu Ile Pro Phe Leu Pro Ala Asn Ile
        355                 360                 365

Pro His Ala Leu Glu Ala Leu Ser Thr Gly Gly Leu Phe Ser Asp Gly
    370                 375                 380

Gly Lys Arg Leu Leu Gln Asn Phe Glu Gly Asp Tyr Val Asp Ser Cys
385                 390                 395                 400

Leu Gly Asp Leu Ile Ser Ile Leu Arg Leu Pro Lys Trp Leu Lys Gly
                405                 410                 415

Leu Leu Ala Phe Met Leu Arg Pro Leu Leu Pro Arg Leu Ala Gly Phe
            420                 425                 430

Leu Ser Ser Leu Arg Pro Arg Ser Ala Gly Lys Leu Trp Glu Leu Gln
        435                 440                 445

His Glu Ile Glu Met Tyr Arg His Ser Val Ile Ala Gln Trp Arg Ala
    450                 455                 460

Leu Asp Leu Asp Val Val Leu Thr Pro Met Leu Ser Pro Ala Leu Asp
465                 470                 475                 480

Leu Asn Ala Pro Gly Lys Ala Thr Gly Ala Val Ser Tyr Thr Leu Leu
                485                 490                 495

Tyr Asn Cys Leu Asp Phe Pro Ala Gly Val Val Pro Val Thr Thr Val
            500                 505                 510

Thr Ala Glu Asp Glu Ala Gln Met Glu His Tyr Lys Gly Tyr Phe Gly
        515                 520                 525

Asp Ile Trp Asp Lys Val Val Gln Lys Ala Met Lys Arg Ser Val Gly
    530                 535                 540

Leu Pro Val Ala Val Gln Cys Val Ala Leu Pro Trp Gln Glu Glu Leu
545                 550                 555                 560

Cys Leu Arg Phe Met Arg Glu Val Glu Arg Leu Met Ala Pro Gly Arg
                565                 570                 575

Gln Pro Ser